(19) Europäisches Patentamt
European Patent Office
Office européen des brevets

(11) EP 4 028 399 B1

(12) # EUROPEAN PATENT SPECIFICATION

(45) Date of publication and mention of the grant of the patent:
**14.02.2024 Bulletin 2024/07**

(21) Application number: **20776062.0**

(22) Date of filing: **11.09.2020**

(51) International Patent Classification (IPC):
***C07D 471/04*** ^(2006.01) ***C07D 487/04*** ^(2006.01)
***C07D 495/04*** ^(2006.01) ***A61P 31/04*** ^(2006.01)
***A61P 31/06*** ^(2006.01) ***A61K 31/53*** ^(2006.01)
***A61K 31/5383*** ^(2006.01) ***C07D 487/14*** ^(2006.01)
***C07D 491/147*** ^(2006.01) ***C07D 498/14*** ^(2006.01)
***C07D 513/04*** ^(2006.01)

(52) Cooperative Patent Classification (CPC):
**C07D 471/04; A61P 31/04; A61P 31/06; C07D 487/04; C07D 487/14; C07D 491/147; C07D 495/04; C07D 498/14; C07D 513/04**

(86) International application number:
**PCT/EP2020/075458**

(87) International publication number:
**WO 2021/048342 (18.03.2021 Gazette 2021/11)**

(54) **ANTIBACTERIAL COMPOUNDS**
ANTIBAKTERIELLE VERBINDUNGEN
COMPOSÉS ANTIBACTÉRIENS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.09.2019 EP 19197183**

(43) Date of publication of application:
**20.07.2022 Bulletin 2022/29**

(73) Proprietor: **Janssen Sciences Ireland Unlimited Company**
**Ringaskiddy, Co Cork (IE)**

(72) Inventors:
- **GUILLEMONT, Jérôme, Émile, Georges**
**92787 Issy-les-Moulineaux Cedex 9 (FR)**
- **MOTTE, Magali, Madeleine, Simone**
**92787 Issy-les-Moulineaux Cedex 9 (FR)**
- **VILLELLAS ARILLA, Maria, Cristina**
**2340 Beerse (BE)**
- **LAMMENS, Godelieve, Maria, J**
**2340 Beerse (BE)**
- **RENÉ, Adeline, Julie, Dominique, Marie**
**67405 Ilkirch Cedex (FR)**
- **JEANTY, Matthieu, Ludovic**
**67405 Illkirch Cedex (FR)**
- **LAMPRECHT, Dirk, Antonie**
**2340 Beerse (BE)**

(74) Representative: **Purewal, Savroop**
**Janssen Pharmaceutica NV**
**J&J Patent Law Department**
**Turnhoutseweg 30**
**2340 Beerse (BE)**

(56) References cited:
**WO-A1-2017/049321** **WO-A1-2017/216283**
**WO-A1-2018/084809** **WO-A2-2014/015167**
**WO-A2-2015/014993** **US-A1- 2017 313 697**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- SUNHEE KANG ET AL: "Synthesis and structure-activity studies of side chain analogues of the anti-tubercular agent, Q203", EUROPEAN JOURNAL OF MEDICINAL CHEMISTRY, vol. 125, 28 September 2016 (2016-09-28), pages 807-815, XP055329689, AMSTERDAM, NL ISSN: 0223-5234, DOI: 10.1016/j.ejmech.2016.09.082
- HONGJIAN WANG ET AL: "Synthesis and antitubercular evaluation of reduced lipophilic imidazo[1,2-a]pyridine-3-carboxamide derivatives", EUROPEAN JOURNAL OF MEDICINAL CHEMISTRY, vol. 165, 1 March 2019 (2019-03-01), pages 11-17, XP055611092, AMSTERDAM, NL ISSN: 0223-5234, DOI: 10.1016/j.ejmech.2018.12.071

## Description

The present invention relates to novel compounds. The invention also relates to such compounds for use as a pharmaceutical and further for the use in the treatment of bacterial diseases, including diseases caused by pathogenic mycobacteria such as *Mycobacterium tuberculosis.* Such compounds may work by interfering with ATP synthase in *M. tuberculosis,* with the inhibition of cytochrome *b*$c_1$ activity as the primary mode of action. Hence, primarily, such compounds are antitubercular agents.

BACKGROUND OF THE INVENTION

*Mycobacterium tuberculosis* is the causative agent of tuberculosis (TB), a serious and potentially fatal infection with a world-wide distribution. Estimates from the World Health Organization indicate that more than 8 million people contract TB each year, and 2 million people die from tuberculosis yearly. In the last decade, TB cases have grown 20% worldwide with the highest burden in the most impoverished communities. If these trends continue, TB incidence will increase by 41% in the next twenty years. Fifty years since the introduction of an effective chemotherapy, TB remains after AIDS, the leading infectious cause of adult mortality in the world. Complicating the TB epidemic is the rising tide of multi-drug-resistant strains, and the deadly symbiosis with HIV. People who are HIV-positive and infected with TB are 30 times more likely to develop active TB than people who are HIV-negative and TB is responsible for the death of one out of every three people with HIV/AIDS worldwide.

Existing approaches to treatment of tuberculosis all involve the combination of multiple agents. For example, the regimen recommended by the U.S. Public Health Service is a combination of isoniazid, rifampicin and pyrazinamide for two months, followed by isoniazid and rifampicin alone for a further four months. These drugs are continued for a further seven months in patients infected with HIV. For patients infected with multi-drug resistant strains of *M. tuberculosis,* agents such as ethambutol, streptomycin, kanamycin, amikacin, capreomycin, ethionamide, cycloserine, ciprofoxacin and ofloxacin are added to the combination therapies. There exists no single agent that is effective in the clinical treatment of tuberculosis, nor any combination of agents that offers the possibility of therapy of less than six months' duration.

There is a high medical need for new drugs that improve current treatment by enabling regimens that facilitate patient and provider compliance. Shorter regimens and those that require less supervision are the best way to achieve this. Most of the benefit from treatment comes in the first 2 months, during the intensive, or bactericidal, phase when four drugs are given together; the bacterial burden is greatly reduced, and patients become noninfectious. The 4- to 6-month continuation, or sterilizing, phase is required to eliminate persisting bacilli and to minimize the risk of relapse. A potent sterilizing drug that shortens treatment to 2 months or less would be extremely beneficial. Drugs that facilitate compliance by requiring less intensive supervision also are needed. Obviously, a compound that reduces both the total length of treatment and the frequency of drug administration would provide the greatest benefit.

Complicating the TB epidemic is the increasing incidence of multi-drug-resistant strains or MDR-TB. Up to four percent of all cases worldwide are considered MDR-TB - those resistant to the most effective drugs of the four-drug standard, isoniazid and rifampin. MDR-TB is lethal when untreated and cannot be adequately treated through the standard therapy, so treatment requires up to 2 years of "second-line" drugs. These drugs are often toxic, expensive and marginally effective. In the absence of an effective therapy, infectious MDR-TB patients continue to spread the disease, producing new infections with MDR-TB strains. There is a high medical need for a new drug with a new mechanism of action, which is likely to demonstrate activity against drug resistant, in particular MDR strains.

The term "drug resistant" as used hereinbefore or hereinafter is a term well understood by the person skilled in microbiology. A drug resistant Mycobacterium is a Mycobacterium which is no longer susceptible to at least one previously effective drug; which has developed the ability to withstand antibiotic attack by at least one previously effective drug. A drug resistant strain may relay that ability to withstand to its progeny. Said resistance may be due to random genetic mutations in the bacterial cell that alters its sensitivity to a single drug or to different drugs.

NMR tuberculosis is a specific form of drug resistant tuberculosis due to a bacterium resistant to at least isoniazid and rifampicin (with or without resistance to other drugs), which are at present the two most powerful anti-TB drugs. Thus, whenever used hereinbefore or hereinafter "drug resistant" includes multi drug resistant.

Another factor in the control of the TB epidemic is the problem of latent TB. In spite of decades of tuberculosis (TB) control programs, about 2 billion people are infected by M. tuberculosis, though asymptomatically. About 10% of these individuals are at risk of developing active TB during their lifespan. The global epidemic of TB is fuelled by infection of HIV patients with TB and rise of multi-drug resistant TB strains (MDR-TB). The reactivation of latent TB is a high risk factor for disease development and accounts for 32% deaths in HIV infected individuals. To control TB epidemic, the need is to discover new drugs that can kill dormant or latent bacilli. The dormant TB can get reactivated to cause disease by several factors like suppression of host immunity by use of immunosuppressive agents like antibodies against tumor necrosis factor $\alpha$ or interferon-$\gamma$. In case of HIV positive patients the only prophylactic treatment available for latent TB is two- three months regimens of rifampicin, pyrazinamide. The efficacy of the treatment regime is still not clear and

furthermore the length of the treatments is an important constrain in resource-limited environments. Hence there is a drastic need to identify new drugs, which can act as chemoprophylatic agents for individuals harboring latent TB bacilli.

The tubercle bacilli enter healthy individuals by inhalation; they are phagocytosed by the alveolar macrophages of the lungs. This leads to potent immune response and formation of granulomas, which consist of macrophages infected with M. tuberculosis surrounded by T cells. After a period of 6-8 weeks the host immune response cause death of infected cells by necrosis and accumulation of caseous material with certain extracellular bacilli, surrounded by macrophages, epitheloid cells and layers of lymphoid tissue at the periphery. In case of healthy individuals, most of the mycobacteria are killed in these environments but a small proportion of bacilli still survive and are thought to exist in a non-replicating, hypometabolic state and are tolerant to killing by anti-TB drugs like isoniazid. These bacilli can remain in the altered physiological environments even for individual's lifetime without showing any clinical symptoms of disease. However, in 10% of the cases these latent bacilli may reactivate to cause disease. One of the hypothesis about development of these persistent bacteria is patho-physiological environment in human lesions namely, reduced oxygen tension, nutrient limitation, and acidic pH. These factors have been postulated to render these bacteria phenotypically tolerant to major anti-mycobacterial drugs.

In addition to the management of the TB epidemic, there is the emerging problem of resistance to first-line antibiotic agents. Some important examples include penicillinresistant Streptococcus pneumoniae, vancomycin-resistant enterococci, methicillinresistant Staphylococcus aureus, multi-resistant salmonellae.

The consequences of resistance to antibiotic agents are severe. Infections caused by resistant microbes fail to respond to treatment, resulting in prolonged illness and greater risk of death. Treatment failures also lead to longer periods of infectivity, which increase the numbers of infected people moving in the community and thus exposing the general population to the risk of contracting a resistant strain infection.

Hospitals are a critical component of the antimicrobial resistance problem worldwide. The combination of highly susceptible patients, intensive and prolonged antimicrobial use, and cross-infection has resulted in infections with highly resistant bacterial pathogens.

Self-medication with antimicrobials is another major factor contributing to resistance. Self-medicated antimicrobials may be unnecessary, are often inadequately dosed, or may not contain adequate amounts of active drug.

Patient compliance with recommended treatment is another major problem. Patients forget to take medication, interrupt their treatment when they begin to feel better, or may be unable to afford a full course, thereby creating an ideal environment for microbes to adapt rather than be killed.

Because of the emerging resistance to multiple antibiotics, physicians are confronted with infections for which there is no effective therapy. The morbidity, mortality, and financial costs of such infections impose an increasing burden for health care systems worldwide.

Therefore, there is a high need for new compounds to treat bacterial infections, especially mycobacterial infections including drug resistant and latent mycobacterial infections, and also other bacterial infections especially those caused by resistant bacterial strains.

Anti-infective compounds for treating tuberculosis have been disclosed in e.g. international patent application WO 2011/113606. Such a document is concerned with compounds that would prevent *M. tuberculosis* multiplication inside the host macrophage and relates to compounds with a bicyclic core, imidazopyridines, which are linked (e.g. via an amido moiety) to e.g. an optionally substituted benzyl group.

International patent application WO 2014/015167 also discloses compounds that are disclosed as being of potential use in the treatment of tuberculosis. Such compounds disclosed herein have a bicycle (a 5,5-fused bicycle) as an essential element, which is substituted by a linker group (e.g. an amido group), which itself may be attached to another bicycle or aromatic group. Such compounds in this document do not contain a series of more than three rings.

Journal article Nature Medicine, 19, 1157-1160 (2013) by Pethe et al "Discovery of Q203, a potent clinical candidate for the treatment of tuberculosis" identifies a specific compound that was tested against *M. tuberculosis.* This compound Q203 is depicted below.

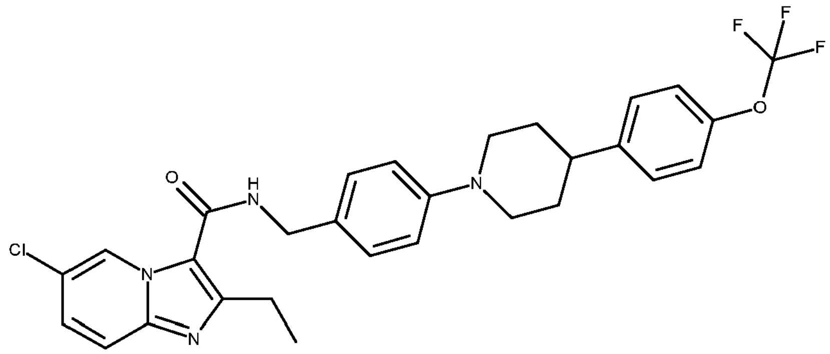

This clinical candidates is also discussed in journal article, J. Medicinal Chemistry, 2014, 57 (12), pp 5293-5305. It is stated to have activity against NMR tuberculosis, and have activity against the strain *M. tuberculosis* H37Rv at a $MIC_{50}$ of 0.28 nM inside macrophages. Positive control data (using known anti-TB compounds bedaquiline, isoniazid and moxifloxacin) are also reported. This document also suggests a mode of action, based on studies with mutants. It postulates that it acts by interfering with ATP synthase in *M. tuberculosis,* and that the inhibition of cytochrome *b*$c_1$ activity is the primary mode of action. Cytochrome *b*$c_1$ is an essential component of the electron transport chain required for ATP synthesis. It appeared that Q203 was highly active against both replicating and non-replicating bacteria.

International patent application WO 2015/014993 also discloses compounds as having activity against *M. tuberculosis,* as do international patent applications WO 2014/4015167, WO 2017/001660, WO 2017/001661, WO 2017/216281 and WO 2017/216283. International patent applications WO 2013/033070 and WO 2013/033167 disclose various compounds as kinase modulators.

The purpose of the present invention is to provide compounds for use in the treatment of bacterial diseases, particularly those diseases caused by pathogenic bacteria such as *Mycobacterium tuberculosis* (including the latent disease and including drug resistant *M. tuberculosis* strains). Such compounds may also be novel and may act by interfering with ATP synthase in *M. tuberculosis,* with the inhibition of cytochrome *b*$c_1$ activity being considered the primary mode of action.

SUMMARY OF THE INVENTION

There is now provided a compound of formula (I)

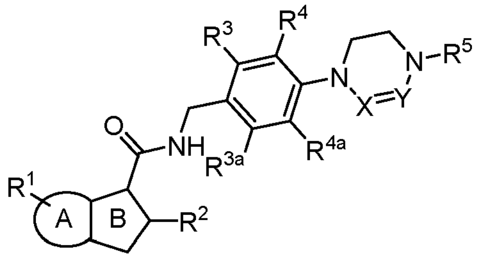

(I)

wherein

A is a 5- or 6-membered ring, which may be aromatic or non-aromatic, and optionally containing 1 or 2 heteroatoms selected from nitrogen and sulfur;

B is a 5-membered aromatic ring containing 1 or 2 nitrogen heteroatoms;

$R^1$ represents one or more (e.g. one, two or three) optional substituents independently selected from halo (e.g. Cl, F), $-R^{6a}$, $-O-R^{6b}$, $-C(=O)-R^{6c}$, $-C(=O)-N(R^7)(R^8)$, -CN and $-N(R^{7a})R^{7b}$;

$R^2$ is $-C_{1-4}$ alkyl optionally substituted by one or more substituents selected from halo and $-OC_{1-3}$ alkyl;

any two of $R^3$, $R^{3a}$, $R^4$ and $R^{4a}$ represent H, and the other two independently represent a substituent selected from H, F, $-C_{1-3}$ alkyl and $-O-C_{1-3}$ alkyl;

$R^5$ is H, $-R^{9a}$, $-C(=O)-R^{9b}$, $-SO_2-R^{10}$ or $Het^1$;

either one of X and Y represents $-CR^{11a}$ and the other represents N or $-CR^{11b}$;

$R^{6a}$ and $R^{6b}$ independently represent $-C_{1-4}$ alkyl optionally substituted by one or more substituents selected from halo (e.g. F) and $-O-CH_3$;

$R^{6c}$ is $-C_{1-3}$ alkyl;
$R^7$ and $R^8$ are independently selected from H and $-C_{1-3}$ alkyl;
$R^{7a}$ and $R^{7b}$ independently represent H, $C_{1-6}$ alkyl or $R^{7a}$ and $R^{7b}$ are linked together to form a 3- to 6-membered ring;
$R^{9a}$ represents $-C_{1-4}$ alkyl, optionally substituted by one or more substituents selected from halo, $-OC_{1-3}$ alkyl and $Het^2$;
$R^{9b}$ is hydrogen or $-C_{1-3}$ alkyl (optionally substituted by one or more fluoro atoms);
$R^{10}$ is $-C_{1-4}$ alkyl optionally substituted by one or more substituents selected from halo (e.g. F) and $-O-CH_3$;
$R^{11a}$ and $R^{11b}$ independently represent H, $C_{1-4}$ alkyl (itself optionally substituted by one or more, e.g. one, substituent(s) selected from fluoro, -CN, $-R^{12a}$, $-OR^{12b}$, $-N(R^{12c})R^{12d}$ and/or $-C(O)N(R^{12e})R^{12f}$) or $-O-C_{1-4}$ alkyl (itself optionally substituted by one or more, e.g. one, substituent(s) selected from fluoro, $-R^{12g}$, $-OR^{12h}$ and/or $-N(R^{12i})R^{12j}$);
$R^{12a}$, $R^{12b}$, $R^{12c}$, $R^{12d}$, $R^{12e}$, $R^{12f}$, $R^{12g}$, $R^{12h}$, $R^{12i}$ and $R^{12j}$ independently represent hydrogen or $C_{1-3}$ alkyl (optionally substituted by one or more fluoro atoms);
$Het^1$ and $Het^2$ independently represent a 5- or 6-membered aromatic ring containing one or two heteroatoms, preferably selected from nitrogen and sulfur, optionally substituted by one or more substitutents selected from halo and $C_{1-3}$ alkyl (itself optionally substituted by one or more fluoro atoms),

or a pharmaceutically-acceptable salt thereof,

which compounds may be referred to herein as "compounds of the invention".

In an embodiment, there is now also provided a compound of formula (Ia)

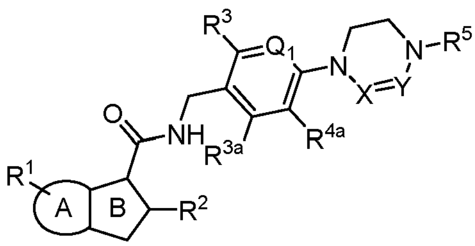

(Ia)

wherein

$Q_1$ represents N or $C(R^4)$;
A is a 5- or 6-membered ring, which may be aromatic or non-aromatic, and optionally containing 1 or 2 heteroatoms selected from nitrogen and sulfur;
B is a 5-membered aromatic ring containing 1 or 2 nitrogen heteroatoms;
$R^1$ represents one or more (e.g. one, two or three) optional substituents independently selected from halo (e.g. Cl, F), $-R^{6a}$, $-O-R^{6b}$, $-C(=O)-R^{6c}$, $-C(=O)-N(R^7)(R^8)$, -CN and $-N(R^{7a})R^{7b}$; or any two $R^1$ groups may be taken together (when attached to adjacent atoms of the A ring) to form a 5- or 6-membered ring optionally containing one or two heteroatoms, and which ring is optionally substituted by one or two $C_{1-3}$ alkyl substituents;
$R^2$ is $-C_{1-4}$ alkyl optionally substituted by one or more substituents selected from halo and $-OC_{1-3}$ alkyl;
any two of $R^3$, $R^{3a}$, $R^4$ and $R^{4a}$ represent H, and the other two independently represent a substituent selected from H, F, $-C_{1-3}$ alkyl and $-O-C_{1-3}$ alkyl;
$R^5$ is H, $-R^{9a}$, $-C(=O)-R^{9b}$, $-SO_2-R^{10}$ or $Het^1$;
either one of X and Y represents $-CR^{11a}$ and the other represents N or $-CR^{11b}$;
$R^{6a}$ and $R^{6b}$ independently represent hydrogen or $-C_{1-4}$ alkyl optionally substituted by one or more substituents selected from halo (e.g. F), $-O-CH_3$ and phenyl;
$R^{6c}$ is $-C_{1-3}$ alkyl;
$R^7$ and $R^8$ are independently selected from Hand $-C_{1-3}$ alkyl;
$R^{7a}$ and $R^{7b}$ independently represent H, $C_{1-6}$ alkyl or $R^{7a}$ and $R^{7b}$ are linked together to form a 3- to 6-membered ring;
$R^{9a}$ represents $-C_{1-4}$ alkyl, optionally substituted by one or more substituents selected from halo, $-OC_{1-3}$ alkyl and $Het^2$;
$R^{9b}$ is hydrogen or $-C_{1-3}$ alkyl (optionally substituted by one or more fluoro atoms);
$R^{10}$ is $-C_{1-4}$ alkyl optionally substituted by one or more substituents selected from halo (e.g. F) and $-O-CH_3$;
$R^{11a}$ and $R^{11b}$ independently represent H, $C_{1-4}$ alkyl (itself optionally substituted by one or more, e.g. one, substituent(s) selected from fluoro, -CN, $-R^{12a}$, $-OR^{12b}$, $-N(R^{12c})R^{12d}$ and/or $-C(O)N(R^{12e})R^{12f}$) or $-O-C_{1-4}$ alkyl (itself optionally substituted by one or more, e.g. one, substituent(s) selected from fluoro, $-R^{12g}$, $-OR^{12h}$ and/or $-N(R^{12i})R^{12j}$);

$R^{12a}$, $R^{12b}$, $R^{12c}$, $R^{12d}$, $R^{12e}$, $R^{12f}$, $R^{12g}$, $R^{12h}$, $R^{12i}$ and $R^{12j}$ independently represent hydrogen or $C_{1-3}$ alkyl (optionally substituted by one or more fluoro atoms);

$Het^1$ and $Het^2$ independently represent a 5- or 6-membered aromatic ring containing one or two heteroatoms, preferably selected from nitrogen and sulfur, optionally substituted by one or more substitutents selected from halo and $C_{1-3}$ alkyl (itself optionally substituted by one or more fluoro atoms),

or a pharmaceutically-acceptable salt thereof,

which compounds may also be referred to herein as "compounds of the invention".

Pharmaceutically-acceptable salts include acid addition salts and base addition salts. Such salts may be formed by conventional means, for example by reaction of a free acid or a free base form of a compound of formula I with one or more equivalents of an appropriate acid or base, optionally in a solvent, or in a medium in which the salt is insoluble, followed by removal of said solvent, or said medium, using standard techniques (e.g. *in vacuo,* by freeze-drying or by filtration). Salts may also be prepared by exchanging a counter-ion of a compound of the invention in the form of a salt with another counter-ion, for example using a suitable ion exchange resin.

The pharmaceutically acceptable acid addition salts as mentioned hereinabove are meant to comprise the therapeutically active non-toxic acid addition salt forms that the compounds of formula (I) are able to form. These pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (*i.e.* ethanedioic), malonic, succinic (*i.e.* butanedioic acid), maleic, fumaric, malic, tartaric, citric, methanesulfonic, ethanesulfonic, benzenesulfonic, *p*-toluenesulfonic, cyclamic, salicylic, *p*-aminosalicylic, pamoic and the like acids.

For the purposes of this invention solvates, N-oxides and stereoisomers of compounds of the invention are also included within the scope of the invention.

The term "prodrug" of a relevant compound of the invention includes any compound that, following oral or parenteral administration, is metabolised *in vivo* to form that compound in an experimentally-detectable amount, and within a predetermined time (e.g. within a dosing interval of between 6 and 24 hours (i.e. once to four times daily)). For the avoidance of doubt, the term "parenteral" administration includes all forms of administration other than oral administration.

Prodrugs of compounds of the invention may be prepared by modifying functional groups present on the compound in such a way that the modifications are cleaved, *in vivo* when such prodrug is administered to a mammalian subject. The modifications typically are achieved by synthesising the parent compound with a prodrug substituent. Prodrugs include compounds of the invention wherein a hydroxyl, amino, sulfhydryl, carboxy or carbonyl group in a compound of the invention is bonded to any group that may be cleaved *in vivo* to regenerate the free hydroxyl, amino, sulfhydryl, carboxy or carbonyl group, respectively.

Examples of prodrugs include, but are not limited to, esters and carbamates of hydroxy functional groups, esters groups of carboxyl functional groups, N-acyl derivatives and N-Mannich bases. General information on prodrugs may be found e.g. in Bundegaard, H. "Design of Prodrugs" p. I-92, Elesevier, New York-Oxford (1985).

Compounds of the invention may contain double bonds and may thus exist as *E* (*entgegen*) and *Z* (*zusammen*) geometric isomers about each individual double bond. All such isomers (e.g. if a compound of the invention incorporates a double bond or a fused ring, the cis- and trans- forms, are embraced) and mixtures thereof are included within the scope of the invention.

Compounds of the invention may also exhibit tautomerism. All tautomeric forms (or tautomers) and mixtures thereof are included within the scope of the invention. The term "tautomer" or "tautomeric form" refers to structural isomers of different energies which are interconvertible *via* a low energy barrier. For example, proton tautomers (also known as prototropic tautomers) include interconversions *via* migration of a proton, such as keto-enol and imine-enamine isomerisations. Valence tautomers include interconversions by reorganisation of some of the bonding electrons.

Compounds of the invention may also contain one or more asymmetric carbon atoms and may therefore exhibit optical and/or diastereoisomerism. Diastereoisomers may be separated using conventional techniques, e.g. chromatography or fractional crystallisation. The various stereoisomers may be isolated by separation of a racemic or other mixture of the compounds using conventional, e.g. fractional crystallisation or HPLC, techniques. Alternatively the desired optical isomers may be made by reaction of the appropriate optically active starting materials under conditions which will not cause racemisation or epimerisation (i.e. a ‘chiral pool’ method), by reaction of the appropriate starting material with a ‘chiral auxiliary’ which can subsequently be removed at a suitable stage, by derivatisation (i.e. a resolution, including a dynamic resolution), for example with a homochiral acid followed by separation of the diastereomeric derivatives by conventional means such as chromatography, or by reaction with an appropriate chiral reagent or chiral catalyst

all under conditions known to the skilled person.

All stereoisomers (including but not limited to diastereoisomers, enantiomers and atropisomers) and mixtures thereof (e.g. racemic mixtures) are included within the scope of the invention.

In the structures shown herein, where the stereochemistry of any particular chiral atom is not specified, then all stereoisomers are contemplated and included as the compounds of the invention. Where stereochemistry is specified by a solid wedge or dashed line representing a particular configuration, then that stereoisomer is so specified and defined.

The compounds of the present invention may exist in unsolvated as well as solvated forms with pharmaceutically acceptable solvents such as water, ethanol, and the like, and it is intended that the invention embrace both solvated and unsolvated forms.

The present invention also embraces isotopically-labeled compounds of the present invention which are identical to those recited herein, but for the fact that one or more atoms are replaced by an atom having an atomic mass or mass number different from the atomic mass or mass number usually found in nature (or the most abundant one found in nature). All isotopes of any particular atom or element as specified herein are contemplated within the scope of the compounds of the invention. Exemplary isotopes that can be incorporated into compounds of the invention include isotopes of hydrogen, carbon, nitrogen, oxygen, phosphorus, sulfur, fluorine, chlorine and iodine, such as $^{2}H$, $^{3}H$ , $^{11}C$, $^{13}C$, $^{14}C$ , $^{13}N$, $^{15}O$, $^{17}O$, $^{18}O$, $^{32}P$, $^{33}P$, $^{35}S$, $^{18}F$, $^{36}Cl$, $^{123}I$, and $^{125}I$. Certain isotopically-labeled compounds of the present invention (e.g., those labeled with $^{3}H$ and $^{14}C$) are useful in compound and for substrate tissue distribution assays. Tritiated ($^{3}H$) and carbon-l4 ($^{14}C$) isotopes are useful for their ease of preparation and detectability. Further, substitution with heavier isotopes such as deuterium (i.e., $^{2}H$ may afford certain therapeutic advantages resulting from greater metabolic stability (e.g., increased *in vivo* half-life or reduced dosage requirements) and hence may be preferred in some circumstances. Positron emitting isotopes such as $^{15}O$, $^{13}N$, $^{11}C$ and $^{18}F$ are useful for positron emission tomography (PET) studies to examine substrate receptor occupancy. Isotopically labeled compounds of the present invention can generally be prepared by following procedures analogous to those disclosed in the description/Examples hereinbelow, by substituting an isotopically labeled reagent for a non-isotopically labeled reagent.

Unless otherwise specified, $C_{1-q}$ alkyl groups (where q is the upper limit of the range) defined herein may be straight-chain or, when there is a sufficient number (i.e. a minimum of two or three, as appropriate) of carbon atoms, be branched-chain, and/or cyclic (so forming a $C_{3-q}$-cycloalkyl group). Such cycloalkyl groups may be monocyclic or bicyclic and may further be bridged. Further, when there is a sufficient number (i.e. a minimum of four) of carbon atoms, such groups may also be part cyclic. Such alkyl groups may also be saturated or, when there is a sufficient number (i.e. a minimum of two) of carbon atoms, be unsaturated (forming, for example, a $C_{2-q}$ alkenyl or a $C_{2-q}$ alkynyl group).

$C_{3-q}$ cycloalkyl groups (where q is the upper limit of the range) that may be specifically mentioned may be monocyclic or bicyclic alkyl groups, which cycloalkyl groups may further be bridged (so forming, for example, fused ring systems such as three fused cycloalkyl groups). Such cycloalkyl groups may be saturated or unsaturated containing one or more double bonds (forming for example a cycloalkenyl group). Substituents may be attached at any point on the cycloalkyl group. Further, where there is a sufficient number (i.e. a minimum of four) such cycloalkyl groups may also be part cyclic.

The term "halo", when used herein, preferably includes fluoro, chloro, bromo and iodo.

Heterocyclic groups when referred to herein may include aromatic or non-aromatic heterocyclic groups, and hence encompass heterocycloalkyl and hetereoaryl. Equally, "aromatic or non-aromatic 5- or 6-membered rings" may be heterocyclic groups (as well as carbocyclic groups) that have 5- or 6-members in the ring.

Heterocycloalkyl groups that may be mentioned include non-aromatic monocyclic and bicyclic heterocycloalkyl groups in which at least one (e.g. one to four) of the atoms in the ring system is other than carbon (i.e. a heteroatom), and in which the total number of atoms in the ring system is between 3 and 20 (e.g. between three and ten, e.g between 3 and 8, such as 5- to 8-). Such heterocycloalkyl groups may also be bridged. Further, such heterocycloalkyl groups may be saturated or unsaturated containing one or more double and/or triple bonds, forming for example a $C_{2-q}$ heterocycloalkenyl (where q is the upper limit of the range) group. $C_{2-q}$ heterocycloalkyl groups that may be mentioned include 7-azabicyclo[2.2.1]heptanyl, 6-azabicyclo[3.1.1]heptanyl, 6-azabicyclo[3.2.1]-octanyl, 8-azabicyclo-[3.2.1]octanyl, aziridinyl, azetidinyl, dihydropyranyl, dihydropyridyl, dihydropyrrolyl (including 2,5-dihydropyrrolyl), dioxolanyl (including 1,3-dioxolanyl), dioxanyl (including 1,3-dioxanyl and 1,4-dioxanyl), dithianyl (including 1,4-dithianyl), dithiolanyl (including 1,3-dithiolanyl), imidazolidinyl, imidazolinyl, morpholinyl, 7-oxabicyclo[2.2.1]heptanyl, 6-oxabicyclo-[3.2.1]octanyl, oxetanyl, oxiranyl, piperazinyl, piperidinyl, non-aromatic pyranyl, pyrazolidinyl, pyrrolidinonyl, pyrrolidinyl, pyrrolinyl, quinuclidinyl, sulfolanyl, 3-sulfolenyl, tetrahydropyranyl, tetrahydrofuranyl, tetrahydropyridyl (such as 1,2,3,4-tetrahydropyridyl and 1,2,3,6-tetrahydropyridyl), thietanyl, thiiranyl, thiolanyl, thiomorpholinyl, trithianyl (including 1,3,5-trithianyl), tropanyl and the like.

Substituents on heterocycloalkyl groups may, where appropriate, be located on any atom in the ring system including a heteroatom. The point of attachment of heterocycloalkyl groups may be *via* any atom in the ring system including (where appropriate) a heteroatom (such as a nitrogen atom), or an atom on any fused carbocyclic ring that may be present as part of the ring system. Heterocycloalkyl groups may also be in the *N*- or *S*- oxidised form. Heterocycloalkyl mentioned herein may be stated to be specifically monocyclic or bicyclic.

Aromatic groups may be aryl or heteroaryl. Aryl groups that may be mentioned include $C_{6-20}$, such as $C_{6-12}$ (e.g. $C_{6-10}$) aryl groups. Such groups may be monocyclic, bicyclic or tricyclic and have between 6 and 12 (e.g. 6 and 10) ring carbon atoms, in which at least one ring is aromatic. $C_{6-10}$ aryl groups include phenyl, naphthyl and the like, such as 1,2,3,4-tetrahydronaphthyl. The point of attachment of aryl groups may be *via* any atom of the ring system. For example, when the aryl group is polycyclic the point of attachment may be *via* atom including an atom of a non-aromatic ring. However, when aryl groups are polycyclic (e.g. bicyclic or tricyclic), they are preferably linked to the rest of the molecule *via* an aromatic ring. Most preferred aryl groups that may be mentioned herein are "phenyl".

Unless otherwise specified, the term "heteroaryl" when used herein refers to an aromatic group containing one or more heteroatom(s) (e.g. one to four heteroatoms) preferably selected from N, O and S. Heteroaryl groups include those which have between 5 and 20 members (e.g. between 5 and 10) and may be monocyclic, bicyclic or tricyclic, provided that at least one of the rings is aromatic (so forming, for example, a mono-, bi-, or tricyclic heteroaromatic group). When the heteroaryl group is polycyclic the point of attachment may be *via* any atom including an atom of a non-aromatic ring. However, when heteroaryl groups are polycyclic (e.g. bicyclic or tricyclic), they are preferably linked to the rest of the molecule *via* an aromatic ring. Heteroaryl groups that may be mentioned include 3,4-dihydro-1*H*-isoquinolinyl, 1,3-dihydroisoindolyl, 1,3-dihydroisoindolyl (e.g. 3,4-dihydro-1*H*-isoquinolin-2-yl, 1,3-dihydroisoindol-2-yl, 1,3-dihydroisoindol-2-yl; i.e. heteroaryl groups that are linked *via* a non-aromatic ring), or, preferably, acridinyl, benzimidazolyl, benzodioxanyl, benzodioxepinyl, benzodioxolyl (including 1,3-benzodioxolyl), benzofuranyl, benzofurazanyl, benzothiadiazolyl (including 2,1,3-benzothiadiazolyl), benzothiazolyl, benzoxadiazolyl (including 2,1,3-benzoxadiazolyl), benzoxazinyl (including 3,4-dihydro-2*H*-1,4-benzoxazinyl), benzoxazolyl, benzomorpholinyl, benzoselenadiazolyl (including 2,1,3-benzoselenadiazolyl), benzothienyl, carbazolyl, chromanyl, cinnolinyl, furanyl, imidazolyl, imidazo[1,2-*a*]pyridyl, indazolyl, indolinyl, indolyl, isobenzofuranyl, isochromanyl, isoindolinyl, isoindolyl, isoquinolinyl, isothiaziolyl, isothiochromanyl, isoxazolyl, naphthyridinyl (including 1,6-naphthyridinyl or, preferably, 1,5-naphthyridinyl and 1,8-naphthyridinyl), oxadiazolyl (including 1,2,3-oxadiazolyl, 1,2,4-oxadiazolyl and 1,3,4-oxadiazolyl), oxazolyl, phenazinyl, phenothiazinyl, phthalazinyl, pteridinyl, purinyl, pyranyl, pyrazinyl, pyrazolyl, pyridazinyl, pyridyl, pyrimidinyl, pyrrolyl, quinazolinyl, quinolinyl, quinolizinyl, quinoxalinyl, tetrahydroisoquinolinyl (including 1,2,3,4-tetrahydroisoquinolinyl and 5,6,7,8-tetrahydroisoquinolinyl), tetrahydroquinolinyl (including 1,2,3,4-tetrahydroquinolinyl and 5,6,7,8-tetrahydroquinolinyl), tetrazolyl, thiadiazolyl (including 1,2,3-thiadiazolyl, 1,2,4-thiadiazolyl and 1,3,4-thiadiazolyl), thiazolyl, thiochromanyl, thiophenetyl, thienyl, triazolyl (including 1,2,3-triazolyl, 1,2,4-triazolyl and 1,3,4-triazolyl) and the like. Substituents on heteroaryl groups may, where appropriate, be located on any atom in the ring system including a heteroatom. The point of attachment of heteroaryl groups may be *via* any atom in the ring system including (where appropriate) a heteroatom (such as a nitrogen atom), or an atom on any fused carbocyclic ring that may be present as part of the ring system. Heteroaryl groups may also be in the *N*- or *S*- oxidised form. Heteroaryl groups mentioned herein may be stated to be specifically monocyclic or bicyclic. When heteroaryl groups are polycyclic in which there is a non-aromatic ring present, then that non-aromatic ring may be substituted by one or more =O group. Most preferred heteroaryl groups that may be mentioned herein are 5- or 6-membered aromatic groups containing 1, 2 or 3 heteroatoms (e.g. preferably selected from nitrogen, oxygen and sulfur).

It may be specifically stated that the heteroaryl group is monocyclic or bicyclic. In the case where it is specified that the heteroaryl is bicyclic, then it may consist of a five-, six- or seven-membered monocyclic ring (e.g. a monocyclic heteroaryl ring) fused with another five-, six- or seven-membered ring (e.g. a monocyclic aryl or heteroaryl ring).

Heteroatoms that may be mentioned include phosphorus, silicon, boron and, preferably, oxygen, nitrogen and sulfur.

When "aromatic" groups are referred to herein, they may be aryl or heteroaryl. When "aromatic linker groups" are referred to herein, they may be aryl or heteroaryl, as defined herein, are preferably monocyclic (but may be polycyclic) and attached to the remainder of the molecule *via* any possible atoms of that linker group. However, when, specifically carbocylic aromatic linker groups are referred to, then such aromatic groups may not contain a heteroatom, i.e. they may be aryl (but not heteroaryl).

For the avoidance of doubt, where it is stated herein that a group may be substituted by one or more substituents (e.g. selected from $C_{1-6}$ alkyl), then those substituents (e.g. alkyl groups) are independent of one another. That is, such groups may be substituted with the same substituent (e.g. same alkyl substituent) or different (e.g. alkyl) substituents.

All individual features (e.g. preferred features) mentioned herein may be taken in isolation or in combination with any other feature (including preferred feature) mentioned herein (hence, preferred features may be taken in conjunction with other preferred features, or independently of them).

The skilled person will appreciate that compounds of the invention that are the subject of this invention include those that are stable. That is, compounds of the invention include those that are sufficiently robust to survive isolation from e.g. a reaction mixture to a useful degree of purity.

Compounds of the invention may refer to compounds of formula (I) or compounds of formula (Ia). Embodiments of the invention may therefore refer to either (or both) of compounds of formula (I) or of formula (Ia). Compounds of

formula (I) are an embodiment of compounds of formula (Ia). In this respect, compounds of formula (Ia) that may be mentioned include those in which:

$Q_1$ represents $CR^4$;
two $R^1$ substituents on the A ring cannot be linked together to form a 5- or 6-membered ring as hereinbefore defined (i.e. $R^1$ represents one or more (e.g. one, two or three) optional substituents independently selected from selected from halo (e.g. Cl, F), $-R^{6a}$, - O-$R^{6b}$, -C(=O)-$R^{6c}$, -C(=O)-N($R^7$)($R^8$), -CN and -N($R^{7a}$)$R^{7b}$); and/or
$R^{6a}$ and $R^{6b}$ independently represent $-C_{1-4}$ alkyl optionally substituted by one or more substituents selected from halo (e.g. F) and $-O-CH_3$.

In an embodiment of the invention, preferred compounds include those in which:

there may be none, one or two $R^1$ substituents present on ring A;
$R^1$ (when present) represents one or two substituents independently selected from F, Cl, $-R^{6a}$, -O-$R^{6b}$, -C(=O)-$R^{6c}$, -C(=O)-N($R^7$)($R^8$), -CN and -N($R^{7a}$)$R^{7b}$;
$R^{6a}$ represents $C_{1-3}$ alkyl (e.g. methyl, ethyl, *n*-propyl) optionally substituted (e.g. by one substituent) selected from $-O-C_{1-2}$ alkyl (e.g. $-OCH_3$);
$R^{6b}$ and $R^{6c}$ represent $C_{1-3}$ alkyl (e.g. methyl), which is preferably unsubstituted;
$R^7$ and $R^8$ independently represent hydrogen or $C_{1-3}$ alkyl (e.g. methyl), which is preferably unsubstituted;
$R^{7a}$ and $R^{7b}$ are linked together to form a 4-6- (e.g. 5-) membered ring.

Hence, in an embodiment, specific $R^1$ groups may be: F, Cl, $-CH_3$, $-CH_2-OCH_3$, $-(CH_2)_3-OH$, $-OCH_3$, $-C(O)CH_3$, $-C(O)N(CH_3)_2$, $-C(O)N(H)CH_3$, -CN and/or pyrrolidine-1-yl.

In an embodiment of the invention, preferred compounds include those in which:

$R^2$ is linear $-C_{1-4}$ alkyl optionally substituted by one or more substituents (e.g one substituent), for example selected from $-O-C_{1-2}$ alkyl (e.g. $-OCH_3$);
any two of $R^3$, $R^{3a}$, $R^4$ and $R^{4a}$ represent H, and the other two independently represent a substituent selected from H, F, $-CH_3$ and $-OCH_3$.

In an embodiment of the invention, preferred compounds include those in which:

$R^5$ is H, $-R^{9a}$, -C(=O)-$R^{9b}$, $-SO_2-R^{10}$ or Het[1];
$R^{9a}$ represents $C_{1-3}$ alkyl (e.g. methyl) unsubstituted or substituted with one substituent (e.g. selected from Het[2]);
$R^{9b}$ represents H or $C_{1-3}$ alkyl (e.g. methyl) optionally substituted by one or more fluoro atoms (so forming a $-CF_3$ group);
$R^{10}$ represents $C_{1-4}$ alkyl optionally substituted by one or more substituents selected from fluoro and $-OC_{1-2}$ alkyl (e.g. $-OCH_3$), and hence $R^{10}$ may represent $-CF_3$, $-CH_3$, *i*-propyl, $-CH_2C(H)(CH_3)_2$ (*i*-butyl), $-CH_2CH_2-OCH_3$;
Het[1] and Het[2] independently represent a 5- or 6-membered heteroaryl ring containing one or two heteroatoms selected from nitrogen and sulfur (so forming, e.g. a thiazolyl ring, e.g. a 2-thiazolyl ring), which ring is unsubstitued or substituted by one or two (e.g. one) substituent selected from $C_{1-3}$ alkyl (itself optionally substituted by one or more fluoro atoms, so forming a $-CF_3$ group), and, hence, Het[1] and Het[2] may independently represent a thiazolyl group optionally substituted by a $-CF_3$ substituent.

In a further embodiment:

either one of X and Y represents $-CR^{11a}$ and the other represents N or $-CR^{11b}$ (and in an embodiment X represents N and Y represents $-CR^{11a}$);
when $R^{11a}$ or $R^{11b}$ represents $C_{1-4}$ alkyl, then it may be unsubstituted or substituted (e.g. by one substituent) with e.g. -CN, $-OR^{12b}$ and/or -N($R^{12c}$)$R^{12d}$;
$R^{12b}$ represents H or $C_{1-2}$ alkyl (e.g. methyl);
$R^{12c}$ and $R^{12d}$ may independently represent $C_{1-2}$ alkyl (e.g. methyl);
hence, when $R^{11a}$ or $R^{11b}$ represents such a $C_{1-4}$ alkyl group, then it may be $-CH_3$, $-CH_2CH_3$, $-CH_2CH_2-OH$, $-CH_2CH_2-OCH_3$, $-C(H)(CH_3)_2$, $-CH_2-N(CH_3)_2$ or $-CH_2-CN$);
when $R^{11a}$ or $R^{11b}$ represents $-O-C_{1-4}$ alkyl, then it is preferably unsubstituted and may represent $-OC_{1-2}$ alkyl (e.g. $-OCH_3$).

In an embodiment of the invention, preferred compounds include those in which:

$R^2$ is linear -$C_{1-4}$ alkyl (e.g. unsubtituted $C_{1-2}$ alkyl, such as methyl or ethyl), cyclopropyl or -$CH_2$-O-$CH_3$;
$R^5$ is H, -$C_{1-4}$ alkyl, -C(=O)-$R^{9b}$ or -$SO_2$-$R^{10}$; for the avoidance of doubt where "Tf" is mentioned as a substituent, it refers to -$S(O)_2CF_3$;
$R^7$ and $R^8$ are independently selected from H and -$CH_3$;
$R^{9b}$ is H, or in another embodiment, -$CH_3$; and/or
$R^{10}$ is -$CF_3$, linear unsubstituted -$C_{1-4}$ alkyl or -$C_{1-4}$ alkyl substituted with -O-$CH_3$.

In an embodiment, compounds of the invention in which $R^5$ is H are useful intermediates, for example in order to prepare compounds of the invention in which $R^5$ is other than H.

In another embodiment of the invention, compounds of the invention include those in which:

$R^3$ is H, F or -O-$CH_3$;
$R^4$ is H, F, -$CH_3$ or -O-$CH_3$;
$R^{3a}$ is H;
$R^{4a}$ is H or F; and/or
all of $R^3$, $R^4$, $R^{3a}$ and $R^{4a}$ represent hydrogen, or, any one or two of $R^3$, $R^4$, $R^{3a}$ and $R^{4a}$ represents a substituent other than hydrogen (and the others represents hydrogen), for example: (i) $R^3$ represents a substituent other than H (e.g. F or -$OCH_3$) and the others, i.e. $R^4$, $R^{3a}$ and $R^{4a}$, represent hydrogen; (ii) $R^4$ represents a substituent other than H (e.g. F, -$CH_3$ or -$OCH_3$) and the others, i.e. $R^3$, $R^{3a}$ and $R^{4a}$, represent hydrogen; (iii) $R^4$ and $R^{4a}$ represent a substituent other than H (e.g. F) and the others, i.e. $R^3$ and $R^{3a}$, represent hydrogen.

In an additional or alternative embodiment:

$Q_1$ represents =N- or =C($R^4$)- (in an embodiment $Q^1$ represents =C($R^4$)-; and/or
all of $R^3$, $R^4$, $R^{3a}$ and $R^{4a}$ represent hydrogen, or one or $R^4$ or $R^{4a}$ represent a substituent as defined herein (e.g. fluoro, methyl or methoxy; in an embodiment, it represents fluoro).

In an additional or alternative embodiment:

X represents N and Y represents $CR^{11a}$; and/or
$R^{11a}$ represents H, $C_{1-3}$ alkyl (e.g. methyl or isopropyl) or -$OC_{1-2}$ alkyl (e.g. -$OCH_3$).

In an embodiment:

there is one or two (e.g. one) $R^1$ substituent(s) present on ring A (where $R^1$ is, in an embodiment, not hydrogen but a substituent as defined herein);
there is one $R^2$ group presents on ring B.

In a further embodiment of the invention, preferred compounds include those in which:
Ring A is represented as follow:

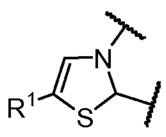
(II)

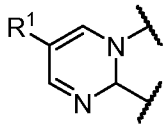
(III)

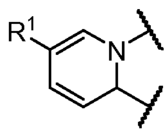
(IV)

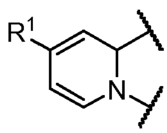
(V)

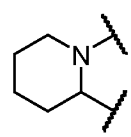
(VI)

In another embodiment of the invention, preferred compounds include those in which: Ring B is represented as follow:

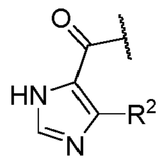
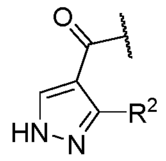

(VII) (VIII)

In an embodiment of the invention, preferred compounds of the invention include those in which: the combined ring system, i.e. ring A and ring B may be represented as follow:

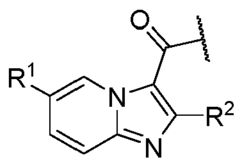
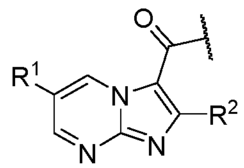
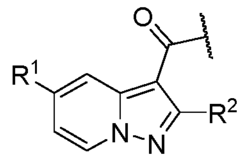

(IX) (X) (XI)

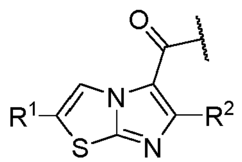
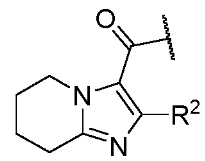

(XII) (XIII)

In another embodiment of the invention, the combined ring system, i.e. ring A and ring B may be represented by any of the following sub-groups:

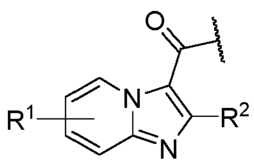
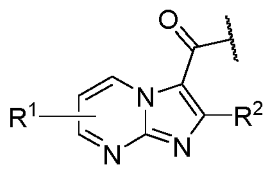
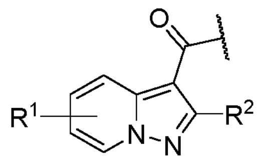

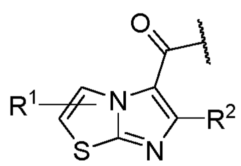
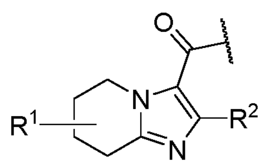
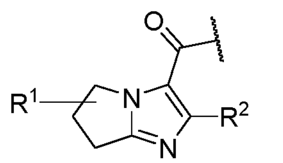

where $R^2$ is as defined herein, and $R^1$ represents one or more (e.g. one, two or three) optional substituents as defined herein (e.g. in respect of compounds of formula (I), compounds of formula (Ia), or further embodiments of either).

In an additional or alternative embodiment, $R^1$ is not present or may represent a substituent selected from halo (e.g. chloro, fluoro, bromo), $C_{1-3}$ alkyl (e.g. methyl) and - $N(R^{7a})R^{7b}$ (where $R^{7a}$ and $R^{7b}$ independently represent hydrogen or $C_{1-3}$ alkyl, such as methyl, or are linked together to form a 4- to 6-membered ring, and hence may form - $NH_2$, $-N(H)CH_2$, $-N(CH_3)_2$ and/or pyrrolidinyl). Optionally, two $R^1$ groups may be taken together to form a 5- or 6-membered ring.

In an additional or alternative embodiment of the invention when two $R^1$ groups are taken together to form a 5- or 6-membered ring, then:

- it may contain only carbon atoms or may contain one or two heteroatoms selected from nitrogen and oxygen;
- it may contain no further double bonds (it may be saturated) or it may contain one or two double bonds and may therefore form a further aromatic ring;

- it may form one of the following moieties:

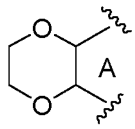
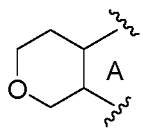
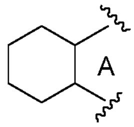

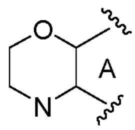
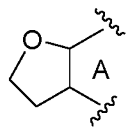
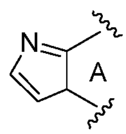
;

and/or

- it may be optionally substituted by one or two (e.g. one) $C_{1-3}$ alkyl (e.g. methyl) groups,

In an embodiment, two $R^1$ groups may not be taken together to form a further 5- or 6-membered ring as defined herein.

In an embodiment of the invention:

$R^1$ represents one or more (e.g. one, two or three) optional (hence, $R^1$ may also represent hydrogen) substituents independently selected from halo (e.g Cl, F), $-R^{6a}$, $-O-R^{6b}$, $-C(=O)-R^{6c}$, $-C(=O)-N(R^7)(R^8)$, -CN and $-N(R^{7a})R^{7b}$;
$R^{6a}$, $R^{6b}$ and $R^{6c}$ independently represent $C_{1-3}$ alkyl (e.g. methyl, cyclopropyl);
$R^7$ and $R^8$ are independently selected from H and $C_{1-3}$ alkyl;
$R^{7a}$ and $R^{7b}$ independently represent H, $C_{1-3}$ alkyl or are linked together to form a 4-6 membered ring (e.g. a 5-membered); and/or
$R^2$ represents $C_{1-4}$ alkyl optionally substituted by one substituent (e.g. selected from $-O-C_{1-3}$ alkyl).

In an additional or alternative embodiment of the invention, $R^2$ may represent $C_{1-4}$ alkyl optionally substituted by one or more substituents selected from halo (e.g. fluoro) and - $OC_{1-3}$ alkyl, for instance $R^2$ may represent $-CF_3$, $-CHF_2$, $-CH_2CH_3$, $-CH_3$, cyclopropyl, $-OCH_3$.

Further embodiments of the invention include those in which:

$R^1$ represents one or two (e.g. one) substituent selected from H, Cl, F, $-R^{6a}$, $-O-R^{6b}$, $-C(=O)-R^{6c}$ and $-C(=O)-N(R^7)(R^8)$;
$R^{6a}$, $R^{6b}$ and $R^{6c}$ independently represent $-CH_3$;
$R^7$ and $R^8$ are independently selected from H and $-CH_3$; and/or
$R^2$ is linear $C_{1-4}$ alkyl, cyclopropyl or $CH_2-O-CH_3$.

In an additional or alternative embodiment, $R^1$ is not present or may represent a substituent selected from halo (e.g. chloro, fluoro, bromo), $C_{1-3}$ alkyl (e.g. methyl) and $-N(R^{7a})R^{7b}$ (where $R^{7a}$ and $R^{7b}$ independently represent hydrogen or $C_{1-3}$ alkyl, such as methyl, or are linked together to form a 4- to 6-membered ring, and hence may form $-NH_2$, $-N(H)CH_2$, $-N(CH_3)_2$ and/or pyrrolidinyl).

Yet further embodiments of the invention include those in which:

$R^5$ is $-C_{1-4}$ alkyl (e.g. methyl), $-C(=O)-R^{9b}$ (e.g. -C(O)H, or, in another embodiment, $-C(O)CH_3$) or $-SO_2-R^{10}$;
the combined ring system, i.e. ring A and ring B, is a ring of formula (IX) or formula (X) and $R^5$ is $-SO_2-R^{10}$;
$R^1$ is H, Cl, F, $-C_{1-4}$ alkyl (e.g. methyl, ethyl or $-CH_2-OCH_3$) or $-O-C_{1-4}$ alkyl (e.g. $OCH_3$), and, in a further embodiment, $R^1$ more preferably represents Cl;
$R^2$ is $-C_{1-4}$ alkyl (e.g. methyl, ethyl, cyclopropyl or $-CH_2-OCH_3$); and/or
$R^{10}$ is isopropyl ($-CH_2CH(CH_2)_2$), $-CH_3$, $-CH_2-CH_2-OCH_3$ or, in a ceratin embodiment, is $-CF_3$.

In an alternative embodiment:

- $R^5$ represents hydrogen, $-S(O)_2R^{10}$ or $Het^1$ (and in a particular embodiment $R^5$ represents $-S(O)_2R^{10}$);
- $R^{10}$ represents $C_{1-3}$ alkyl (e.g. methyl) optionally substituted by one or more fluoro atoms (so forming, in a particular embodiment, $CF_3$); and/or

- $Het^1$ represents a 5-membered heteroaryl group containing one or two (e.g. one) heteroatom (e.g. selected from oxygen, nitrogen and sulfur; in particular sulfur), so forming for example a thienyl group.

In a particular embodiment, $R^5$ represents $-S(O)_2R^{10}$; and in a further particular embodiment, $R^{10}$ represents $C_{1-3}$ alkyl (e.g. methyl) optionally substituted by one or more fluoro atoms (so forming, in a particular embodiment, $CF_3$).
Further embodiments of the invention include those in which:

$R^{11a}$ and $R^{11b}$ independently represent H, $-CH_3$, $-CH_2CH_3$ or $-OCH_3$;
X represents N and Y represents $-CR^{11a}$, in which $R^{11a}$ represents H, $-CH_3$, $-CH_2CH_3$ or $-OCH_3$.
It is stated that either one of X and Y represents $-CR^{11a}$ and the other represents Nor $-CR^{11b}$ and, in an embodiment, X represents N and Y represents $-CR^{11a}$ (as defined herein).

PHARMACOLOGY

The compounds according to the invention have surprisingly been shown to be suitable for the treatment of a bacterial infection including a mycobacterial infection, particularly those diseases caused by pathogenic mycobacteria such as *Mycobacterium tuberculosis* (including the latent and drug resistant form thereof). The present invention thus also relates to compounds of the invention as defined hereinabove, for use as a medicine, in particular for use as a medicine for the treatment of a bacterial infection including a mycobacterial infection.
Such compounds of the invention may act by interfering with ATP synthase in *M. tuberculosis,* with the inhibition of cytochrome *b*$c_1$ activity being the primary mode of action. Cytochrome *b*$c_1$ is an essential component of the electron transport chain required for ATP synthesis.
Further, the present invention also relates to the use of a compound of the invention, as well as any of the pharmaceutical compositions thereof as described hereinafter for the manufacture of a medicament for the treatment of a bacterial infection including a mycobacterial infection.
Disclosed but not claimed is a method of treating a patient suffering from, or at risk of, a bacterial infection, including a mycobacterial infection, which comprises administering to the patient a therapeutically effective amount of a compound or pharmaceutical composition according to the invention.
The compounds of the present invention also show activity against resistant bacterial strains.
Whenever used hereinbefore or hereinafter, that the compounds can treat a bacterial infection it is meant that the compounds can treat an infection with one or more bacterial strains.
The invention also relates to a composition comprising a pharmaceutically acceptable carrier and, as active ingredient, a therapeutically effective amount of a compound according to the invention. The compounds according to the invention may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in addition salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, in particular, for administration orally or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, diluents, lubricants, binders, disintegrating agents and the like in the case of powders, pills, capsules and tablets. Because of their ease in administration, tablets and capsules represent the most advantageous oral dosage unit forms in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations which are intended to be converted, shortly before use, to liquid form preparations.
Depending on the mode of administration, the pharmaceutical composition will preferably comprise from 0.05 to 99 % by weight, more preferably from 0.1 to 70 % by weight, even more preferably from 0.1 to 50 % by weight of the active ingredient(s), and, from 1 to 99.95 % by weight, more preferably from 30 to 99.9 % by weight, even more preferably from 50 to 99.9 % by weight of a pharmaceutically acceptable carrier, all percentages being based on the total weight of the composition.
The pharmaceutical composition may additionally contain various other ingredients known in the art, for example, a lubricant, stabilising agent, buffering agent, emulsifying agent, viscosity-regulating agent, surfactant, preservative, flavouring or colorant.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and the like, and segregated multiples thereof.

The daily dosage of the compound according to the invention will, of course, vary with the compound employed, the mode of administration, the treatment desired and the mycobacterial disease indicated. However, in general, satisfactory results will be obtained when the compound according to the invention is administered at a daily dosage not exceeding 1 gram, e.g. in the range from 10 to 50 mg/kg body weight.

Given the fact that the compounds of formula (Ia) or Formula (Ib) are active against bacterial infections, the present compounds may be combined with other antibacterial agents in order to effectively combat bacterial infections.

Therefore, the present invention also relates to a combination of (a) a compound according to the invention, and (b) one or more other antibacterial agents.

The present invention also relates to a combination of (a) a compound according to the invention, and (b) one or more other antibacterial agents, for use as a medicine.

The present invention also relates to the use of a combination or pharmaceutical composition as defined directly above for the treatment of a bacterial infection.

A pharmaceutical composition comprising a pharmaceutically acceptable carrier and, as active ingredient, a therapeutically effective amount of (a) a compound according to the invention, and (b) one or more other antibacterial agents, is also comprised by the present invention.

The weight ratio of (a) the compound according to the invention and (b) the other antibacterial agent(s) when given as a combination may be determined by the person skilled in the art. Said ratio and the exact dosage and frequency of administration depends on the particular compound according to the invention and the other antibacterial agent(s) used, the particular condition being treated, the severity of the condition being treated, the age, weight, gender, diet, time of administration and general physical condition of the particular patient, the mode of administration as well as other medication the individual may be taking, as is well known to those skilled in the art. Furthermore, it is evident that the effective daily amount may be lowered or increased depending on the response of the treated subject and/or depending on the evaluation of the physician prescribing the compounds of the instant invention. A particular weight ratio for the present compound of the invention and another antibacterial agent may range from 1/10 to 10/1, more in particular from 1/5 to 5/1, even more in particular from 1/3 to 3/1.

The compounds according to the invention and the one or more other antibacterial agents may be combined in a single preparation or they may be formulated in separate preparations so that they can be administered simultaneously, separately or sequentially. Thus, the present invention also relates to a product containing (a) a compound according to the invention, and (b) one or more other antibacterial agents, as a combined preparation for simultaneous, separate or sequential use in the treatment of a bacterial infection.

The other antibacterial agents which may be combined with the compounds of the invention are for example antibacterial agents known in the art. For example, the compounds of the invention may be combined with antibacterial agents known to interfere with the respiratory chain of Mycobacterium tuberculosis, including for example direct inhibitors of the ATP synthase (e.g. bedaquiline, bedaquiline fumarate or any other compounds that may have be disclosed in the prior art, e.g. compounds disclosed in WO2004/011436), inhibitors of ndh2 (e.g. clofazimine) and inhibitors of cytochrome bd. Additional mycobacterial agents which may be combined with the compounds of the invention are for example rifampicin (=rifampin); isoniazid; pyrazinamide; amikacin; ethionamide; ethambutol; streptomycin; para-aminosalicylic acid; cycloserine; capreomycin; kanamycin; thioacetazone; PA-824; delamanid; quinolones/fluoroquinolones such as for example moxifloxacin, gatifloxacin, ofloxacin, ciprofloxacin, sparfloxacin; macrolides such as for example clarithromycin, amoxycillin with clavulanic acid; rifamycins; rifabutin; rifapentin; as well as others, which are currently being developed (but may not yet be on the market; see e.g. http://www.newtbdrugs.org/pipeline.php).

Compounds of the invention (including forms and compositions/combinations comprising compounds of the invention) may have the advantage that they may be more efficacious than, be less toxic than, be longer acting than, be more potent than, produce fewer side effects than, be more easily absorbed than, and/or have a better pharmacokinetic profile (e.g. higher oral bioavailability and/or lower clearance) than, and/or have other useful pharmacological, physical, or chemical properties over, compounds known in the prior art, whether for use in the above-stated indications or otherwise. For instance compounds of the invention may advantages associated with: lower cardiotoxicity; no reactive metabolite formation (e.g. that may cause toxicity issues, e.g. genotoxicity); no formation of degradants (e.g. that are undesired or may elicit unwanted side-effects); and/or faster oral absorption and improved bioavailability.

GENERAL PREPARATION

The compounds according to the invention can generally be prepared by a succession of steps, each of which may be known to the skilled person or described herein.

EXPERIMENTAL PART

Compounds of formula I may be prepared in accordance with the techniques employed in the examples hereinafter (and those methods know by those skilled in the art), for example by using the following techniques.

Compounds of formula (I) or (Ia) may be prepared by:

(i) reaction of a compound of formula (XIV),

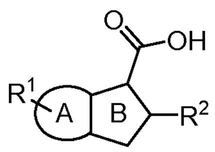

(XIV)

in which the integers are hereinbefore defined, with a compound of formula (XV) or (XVA), respectively,

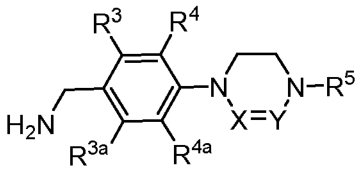

(XV)

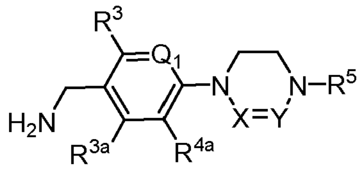

(XVA)

wherein the integers are as hereinbefore defined, and in an embodiment $R^5$ is as hereinbefore defined but preferably represents -$C_{1-4}$ alkyl, -C(=O)-$R^{9b}$ or -$S(O)_2$-$R^{10}$, which reaction may be performed in the presence of a suitable coupling reagent, for instance selected from diisopropylethylamine (DIPEA), 1-[bis(dimethylamino)methylene]-1H-1,2,3-triazolo[4,5-b]pyridinium-3-oxid hexafluorophosphate (HATU), 1-(3-dimethylaminopropyl)-3-ethylcarbodiimide (EDCI), 1-hydroxybenzotriazole (HOBt), O-(benzotriazole-1-yl)-N,N,N',N'-tetramethyluronium tetrafluoroborate (TBTU), or a combination thereof, unders suitable conditions such as those described in the examples hereinafter; for example, in the presence of a suitable coupling reagent (e.g. 1,1'-carbonyldiimidazole, *N,N'*-dicyclohexylcarbodiimide, 1-(3-dimethylaminopropyl)-3-ethylcarbodiimide (or hydrochloride thereof) or *N,N'*-disuccinimidyl carbonate), optionally in the presence of a suitable base (e.g. sodium hydride, sodium bicarbonate, potassium carbonate, pyridine, triethylamine, dimethylaminopyridine, diisopropylamine, sodium hydroxide, potassium *tert*-butoxide and/or lithium diisopropylamide (or variants thereof) and an appropriate solvent (e.g. tetrahydrofuran, pyridine, toluene, dichloromethane, chloroform, acetonitrile, dimethylformamide, trifluoromethylbenzene, dioxane or triethylamine). Alternatively, the carboxylic acid group of the compound of formula (XIV) may first be converted under standard conditions to the corresponding acyl chloride (e.g. in the presence of $POCl_3$, $PCl_5$, $SOCl_2$ or oxalyl chloride), which acyl chloride is then reacted with a compound of formula (XV), for example under similar conditions to those mentioned above;

(ii) coupling of a compound of formula (XVII) or (XVIIA), respectively,

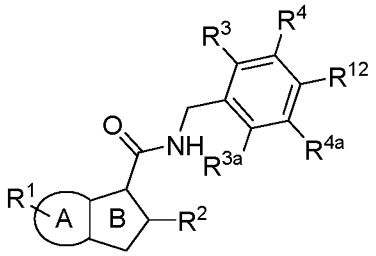

(XVII)

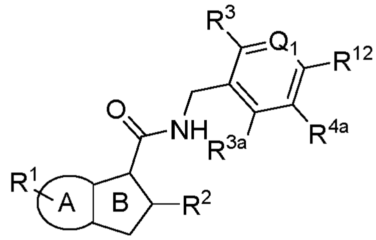

(XVIIA)

wherein the integers are as hereinbefore defined, and $R^{12}$ represents a suitable group, e.g. a suitable leaving group such as chloro, bromo, iodo or a sulfonate group (for example a type of group that may be deployed for a coupling), with a compound of formula (XVI),

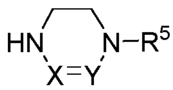

(XVI)

wherein $R^5$ is as hereinbefore defined (but preferably does not represent H), under standard conditions, for example optionally in the presence of an appropriate metal catalyst (or a salt or complex thereof) such as $Pd(dba)_2$, $Pd(OAc)_2$, Cu, $Cu(OAc)_2$, CuI, NiClz or the like, with an optional additive such as $Ph_3P$, X-phos or the like, in the presence of an appropriate base (e.g. t-BuONa, or the like) in a suitable solvent (e.g. dioxane or the like) under reaction conditions known to those skilled in the art;

(iii) for compounds of formula (I) or (Ia) in which X represents N (and $R^5$ preferably represents H), reaction of a compound of formula (XVIII) or (XVIIIA), respectively,

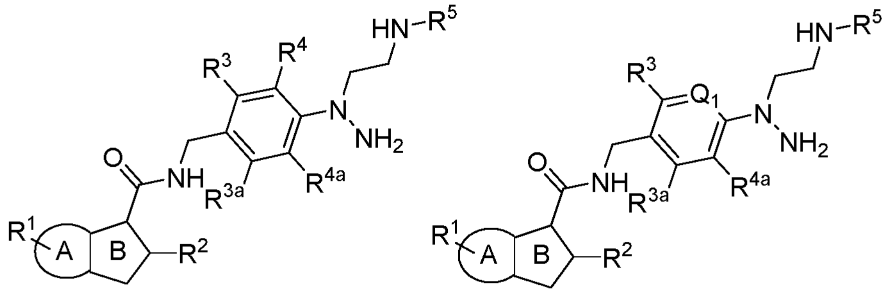

(XVIII) (XVIIIA)

wherein the integers are as hereinbefore defined (and $R^5$ preferably represents H), reaction with a compound of formula (XIX)

$R^{11x}C(OCH_3)_3$ (XIX)

or the like, wherein $R^{11x}$ represents $R^{11a}$ or $R^{11b}$ (as appropriate), under reaction conditions such as those herein described, for instance in the examples;

(iv) for compounds of formula (I) or (Ia) in which X represents N (and preferably $R^5$ represents H), reaction of a comound of formula (XX) or (XXA), respectively

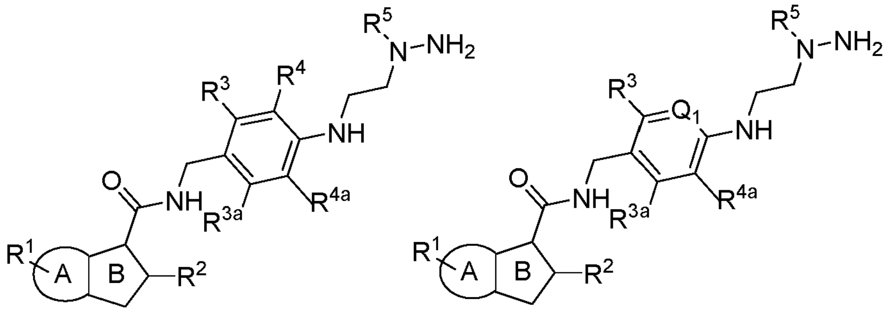

(XX) (XXA)

wherein the integers are as hereinbefore defined (and $R^5$ preferably represents H), reaction with a compound of formula (XIX) as defined above, under reaction conditions such as those herein described, for instance in the examples; and/or

(v) for the preparation of a compound of formula (I) or (Ia) in which $R^5$ represents $-C(=O)-R^{9b}$, $-S(O)_2-R^{10}$ or $Het^1$, reaction of a corresponding compound of formula (I) in which $R^5$ represents H, with a compound of formula (XXI),

$LG^1$-Z (XXI)

wherein Z represents $-C(=O)-R^{9b}$, $-S(O)_2-R^{10}$ or $Het^1$, and $LG^1$ represents a suitable leaving group e.g. chloro, bromo, iodo or a sulfonate group, and wherein the integers are as defined herein and in the case of $Het^1$, the $LG^1$ is attached to an appropriate C atom of that heteroaromatic ring such that the N atom attached to $R^5$ can react with $Het^1$ (e.g. via its lone pair of electrons) and substituted the $LG^1$.

It is evident that in the foregoing and in the following reactions, the reaction products may be isolated from the reaction medium and, if necessary, further purified according to methodologies generally known in the art, such as extraction, crystallization and chromatography. It is further evident that reaction products that exist in more than one enantiomeric form, may be isolated from their mixture by known techniques, in particular preparative chromatography, such as preparative HPLC, chiral chromatography. Individual diastereoisomers or individual enantiomers can also be obtained by Supercritical Fluid Chromatography (SCF).

The starting materials and the intermediates are compounds that are either commercially available or may be prepared according to conventional reaction procedures generally known in the art.

## Examples

1. General Information

### Melting points

Melting points were recorded using a differential scanning calorimeter DSC 1 Mettler Toledo. Melting points were measured with a temperature gradient of 10°C per min from 25 to 350 °C. Values are peak values. Unless indicated, this method is used.

An alternative method is with open capilliary tubes on a Mettler Toledo MP50, which may be indicated at "MT". With this method, melting points are measured with a temperature gradient of 10 °C/minute. Maximum temperature is 300 °C. The melting point data is read from a digital display and checked from a video recording system.

### $^1$H NMR

$^1$H NMR spectra were recorded on a Bruker Avance DRX 400 spectrometer or Bruker Advance III 400 spectrometer using internal deuterium lock and equipped with reverse double-resonance ($^1$H, 13C, SEI) probe head with z gradients and operating at 400 MHz for proton and 100 MHz for carbon and a Bruker Avance 500 MHz spectrometer equipped with a Bruker 5mm BBFO probe head with z gradients and operating at 500 MHz for proton and 125 MHz for carbon.

NMR spectra were recorded at ambient temperature unless otherwise stated.

Data are reported as follow: chemical shift in parts per million (ppm) relative to TMS ($\delta$ = 0 ppm) on the scale, integration, multiplicity (s = singulet, d = doublet, t = triplet, q = quartet, quin = quintuplet, sex = sextuplet, m = multiplet, b = broad, or a combination of these), coupling constant(s) J in Hertz (Hz).

**HPLC- LCMS**

Analytical methods

*LCMS*

The mass of some compounds was recorded with LCMS (liquid chromatography mass spectrometry). The methods used are described below.

*General procedure LCMS Methods A and B*

The High Performance Liquid Chromatography (HPLC) measurement was performed using a LC pump, a diode-array (DAD) or a UV detector and a column as specified in the respective methods. If necessary, additional detectors were included (see table of methods below). Flow from the column was brought to the Mass Spectrometer (MS) which was configured with an atmospheric pressure ion source. It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software.

Compounds are described by their experimental retention times ($R_t$) and ions. If not specified differently in the table of data, the reported molecular ion corresponds to the $[M+H]^+$ (protonated molecule) and/or $[M-H]^-$ (deprotonated molecule). In case the compound was not directly ionizable the type of adduct is specified (i.e. $[M+NH_4]^+$, $[M+HCOO]^-$, etc...). For molecules with multiple isotopic patterns (Br, Cl..), the reported value is the one obtained for the lowest isotope mass. All results were obtained with experimental uncertainties that are commonly associated with the method used. Hereinafter, "SQD" means Single Quadrupole Detector, "RT" room temperature, "BEH" bridged ethylsiloxane/silica hybrid, "HSS" High Strength Silica, "DAD" Diode Array Detector, "MSD" Mass Selective Detector.

*Table:* LCMS Method codes (Flow expressed in mL/min; column temperature (T) in °C; Run time in minutes).

| Method code | Instrument | Column | Mobile phase | gradient | Flow / Column T | Run time |
|---|---|---|---|---|---|---|
| A | Waters: Acquity UPLC® - DAD and Quattro Micro™ | Waters: BEH C18 (1.7μm, 2.1x100 mm) | A: 95% $CH_3COONH_4$ 7mM / 5% $CH_3CN$<br>B: $CH_3CN$ | 84.2% A for 0.49 min, to 10.5% A in 2.18 min, held for 1.94 min, back to 84.2% A in 0.73 min, held for 0.73 min. | 0.343<br>40 | 6.2 |
| B | Waters: Acquity® H-Class - DAD and SQD2™ | Waters: BEH C18 (1.7μm, 2.1x100m m) | A: 95% $CH_3COONH_4$ 7mM / 5% $CH_3CN$<br>B: $CH_3CN$ | 84.2% A to 10.5% A in 2.18 min, held for 1.96 min, back to 84.2% A in 0.73 min, held for 0.73 min. | 0.343<br>40 | 6.1 |
| C | Waters: Acquity UPLC® H-Class - DAD and QDa | Waters: BEH C18 (1.7μm, 2.1x100m m) | A: 95% $CH_3COONH_4$ 7mM / 5% $CH_3CN$, B: $CH_3CN$ | From 85% A to 10% A in 2.1min, held for 2min, back to 85% A in 0.8min, held for 0.7min. | 0.35<br>40 | 6.1 |
| D | Agilent 1100 HPLC DAD LC/MS G1956A | YMC-pack ODS-AQ C18 (50 x 4.6 mm, 3 μm) | A: 0.1% HCOOH in H2O<br>B: CH3CN | From 95% A to 5% A in 4.8 min, held for 1.0 min, to 95% A in 0.2 min. | 2.6<br>35 | 6.2 |

When a compound is a mixture of isomers which give different peaks in the LCMS method, only the retention time of the main component is given in the LCMS table.

2. Abbreviations (and formulae)

| | |
|---|---|
| AcOH | Acetic acid |
| AcCl | Acetyl chloride |
| BINAP | 2,2'-bis(diphenylphosphino)-1,1'-binaphthyl |
| BrettPhos | 2-(Dicyclohexylphosphino)3,6-dimethoxy-2',4',6'-triisopropyl-1,1'-biphenyl |
| BrettPhos Pd G3 | [(2-Di-cyclohexylphosphino-3,6-dimethoxy-2',4',6'-triisopropyl-1,1'-biphenyl)-2-(2'-amino-1,1'-biphenyl)]palladium(II) methanesulfonate methanesulfonate |
| $CBr_4$ | Tetrabromomethane |
| CbzCl | Benzyl chloroformate |
| $CH_3CN$ / ACN | Acetonitrile |
| $Cs_2CO_3$ | Cesium carbonate |
| CSA | Camphor-10-sulfonic acid |
| DCE | Dichloroethane |
| DCM or $CH_2Cl_2$ | Dichloromethane |
| DIPEA | *N,N*-Diisopropylethylamine |
| DMAP | 4-(Dimethylamino)pyridine |
| DME | 1,2-Dimethoxyethane |
| DMF | Dimethylformamide |
| DMF-DMA | *N,N*-dimethylformamide dimethyl acetal |
| DMSO | Methyl sulfoxide |
| EDCI•HCl | N-(3-Dimethylaminopropyl)-N'-ethylcarbodiimide hydrochloride |
| $Et_2O$ | Diethylether |
| $Et_3N$ or TEA | Triethylamine |
| EtOAc | Ethyl acetate |
| EtOH | Ethanol |
| h | hour |
| $H_2$ | Dihydrogen gas |
| HATU | Hexafluorophosphate Azabenzotriazole Tetramethyl Uronium |
| HCl | Hydrochloric acid |
| HFIP | Hexafluoroisopropanol |
| HOBT•$H_2O$ | 1-Hydroxybenzotriazole hydrate |
| i-PrOH | Isopropyl alcohol |
| $K_2CO_3$ | Potassium carbonate |
| $KHSO_4$ | Potassium bisulfate |
| LiOH | Lithium hydroxide |
| LiHMDS | Lithium bis(trimethylsilyl)amide |
| MeOH | Methanol |
| MeTHF / 2-MeTHF | Methyltetrahydrofurane |
| $MgSO_4$ | Magnesium sulfate |
| min | Minute |
| $N_2$ | Nitrogen |
| NaCl | Sodium Chloride |
| $NaHCO_3$ | Sodium Bicarbonate |
| NaOH | Sodium hydroxide |
| NBS | 1-bromopyrrolidine-2,5-dione |
| $NH_3$ | Ammonia |
| $NH_4Cl$ | Ammonium, chloride |
| $NH_4HCO_3$ | Ammonium bicarbonate |
| NMR | Nuclear Magnetic Resonance |
| Pd/C | Palladium on carbon |
| $PdCl_2(PPh_3)_2$ | Dichlorobis(triphenylphosphine)palladium(II) |
| $Pd(OAc)_2$ | Palladium(II) acetate |

| | |
|---|---|
| $Pd_2dba_3$ | Tris(dibenzylideneacetone)dipalladium(0) |
| $Pd(PPh_3)_4$ | Palladium-tetrakis(triphenylphosphine) |
| PIDA | (Diacetoxyiodo)benzene |
| $POCl_3$ | Phosphorous Oxychloride |
| Ra-Ni / Ni Raney | Raney®-Nickel |
| rt / RT | Room temperature |
| RuPhos | 2-Dicyclohexylphosphino-2',6'-diisopropoxybiphenyl |
| RuPhos Pd G3 | (2-Dicyclohexylphosphino-2',6'-diisopropoxy-1,1'-biphenyl)[2-(2'-amino-1,1'-biphenyl)] palladium(II) methanesulfonate |
| t-AmylOH | *tert*-Amyl alcohol |
| SiOH | Silica Gel |
| TBTU | O-(benzotriazole-1-yl)-N,N,N',N'-tetramethyluronium tetrafluoroborate |
| $Tf_2O$ | Trifluoromethanesulfonic Anhydride |
| TFA | Trifluoroactetic acid |
| THF | Tetrahydrofuran |
| TMSCI | Trimethylsilyl chloride |
| TsOH or PTSA | p-Toluensulfonic acid |
| XantPhos | 4,5-Bis(diphenylphosphino)-9,9-dimethylxanthene |

3. Procedures

**Synthesis of Compound 1**

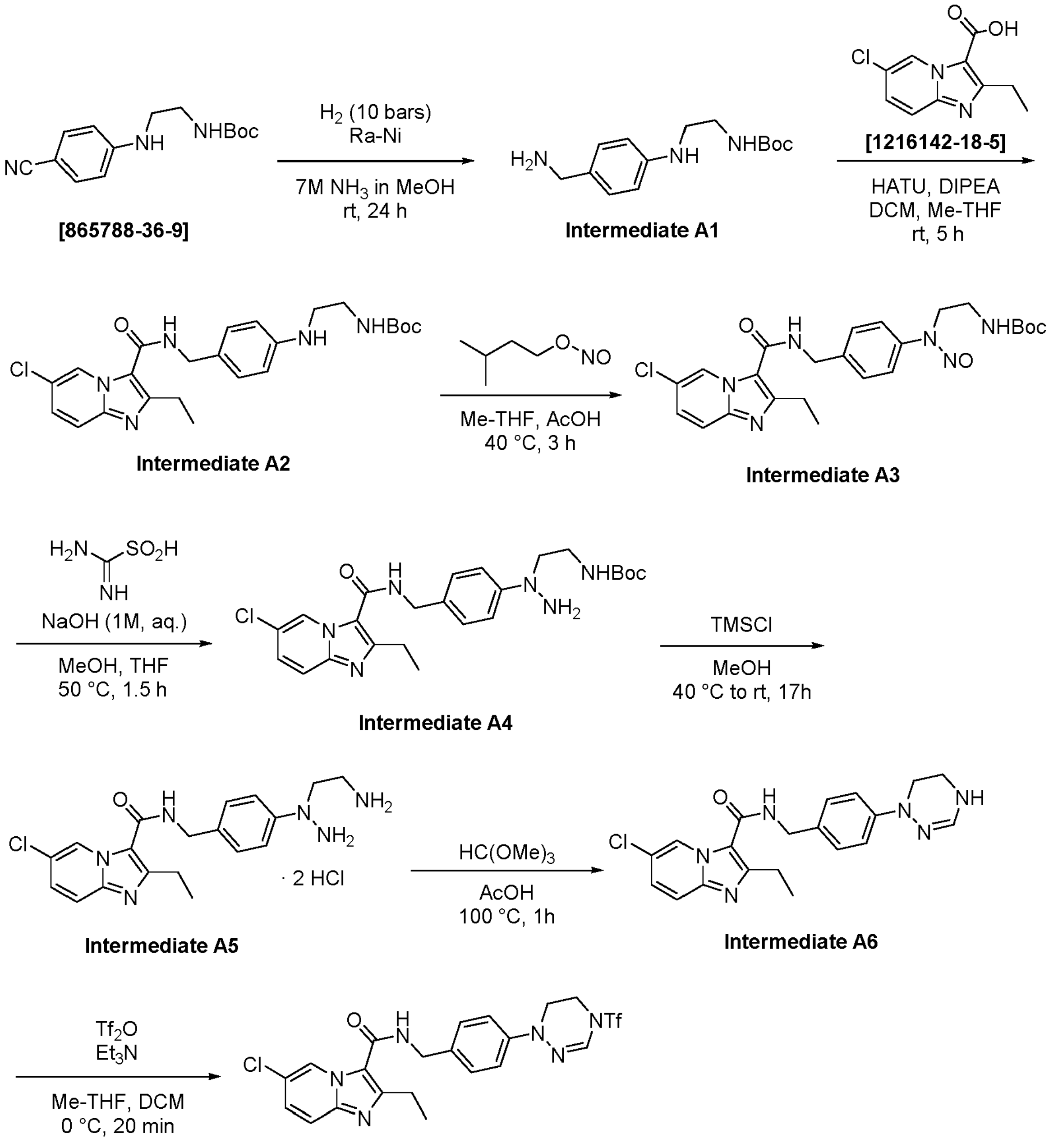


[865788-36-9]

Intermediate A1

[1216142-18-5]

Intermediate A2

Intermediate A3

Intermediate A4

Intermediate A5

Intermediate A6

**Compound 1**

Preparation of intermediate **A1**

In an 1 L autoclave, a mixture of N Boc-[2-[(4-cyanophenyl)amino]ethyl] [865788-36-9] (50.0 g, 191 mmol) and Raney Nickel (2.25 g, 38.2 mmol) in a 7M solution of $NH_3$ in MeOH (600 mL) was hydrogenated at room temperature under 10 bars of $H_2$ for 24 h. The reaction mixture was filtered through a pad of Celite® and washed with a mixture of DCM and MeOH (9/1). The filtrate was evaporated *in vacuo* to afford 50.2 g of intermediate **A1** as a greenish oil (99%).

Preparation of intermediate **A2**

A 2 L flask was charged with 6-chloro-2-ethylimidazo[1,2-a]pyridine-3-carboxylic acid [1216142-18-5] (15.0 g, 66.8 mmol), intermediate **A1** (18.6 g, 70.1 mmol) and DIPEA (17.3 mL, 100 mmol) in DCM (600 mL) and Me-THF (100 mL). The reaction mixture was stirred for 10 min at room temperature, then HATU (27.9 g, 73.4 mmol) was added portionwise over 5 minutes and the reaction mixture was stirred at room temperature for 5 h. The mixture was diluted with DCM (1 L) and water (800 mL). The organic layer was separated and washed with water (400 mL), dried over $MgSO_4$, filtered and evaporated *in* vacuo. The residue was solubilized in a minimum amount of warm EtOAc. The solution

was cooled to room temperature, and then to 0 °C. The suspension was collected by filtration and the solid was washed with cold EtOAc, then with $Et_2O$ before being dried under vacuum to afford 21.7 g of intermediate **A2** as an off-white solid (69%).

Preparation of intermediate **A3**

Intermediate **A2** (5.00 g, 10.6 mmol) was solubilized at 40 °C in Me-THF (80 mL) and acetic acid (6.1 mL, 106 mmol). Isopentyl nitrite (7.12 mL, 53.0 mmol) was added dropwise and the reaction mixture was stirred at 40 °C for 3 h. The solution was diluted in EtOAc and water, washed with $NaHCO_3$ (sat., aq.) (twice) and brine, dried over $MgSO_4$ and evaporated *in vacuo.* The residue was triturated in $Et_2O$. the product was collected by filtration, washed with $Et_2O$ and dried under vacuum to give 4.26 g of intermediate **A3** as a beige solid (80%).

Preparation of intermediate **A4**

A solution of intermediate **A3** (5.00 g, 9.98 mmol) in THF (100 mL) and MeOH (65 mL) was treated with a NaOH (1M, aq., 100 mL). Formamidinesulfinic acid (5.40 g, 49.9 mmol) was added and the reaction mixture was stirred at 50 °C for 1.5 h. The reaction mixture was diluted in DCM and $K_2CO_3$ (10%, aq.) was added. The layers were separated. The aqueous phase was extracted with DCM and MeOH (95/5). The combined organic extracts were dried over $MgSO_4$, filtered and evaporated *in vacuo* to give 4.67 g of intermediate **A4** as a white solid (Quant.).

Preparation of intermediate **A5**

To a solution of intermediate **A4** (4.67 g, 9.59 mmol) in MeOH (96 mL) was added dropwise TMSCl (9.73 mL, 76.7 mmol). The reaction mixture was stirred at 40 °C for 1.5 h and at room temperature for another 17 h. The mixture was concentrated *in vacuo.* The residue was triturated in $Et_2O$. the solid was collected by filtration, washed with $Et_2O$, and dried under vacuum to afford 4.76 g of intermediate **A5** as a pale yellow solid (Quant.).

Preparation of intermediate **A6**

A mixture of intermediate **A5** (4.76 g, 10.4 mmol) and trimethyl orthoformate (3.40 mL, 31.1 mmol) in acetic acid (52 mL) was stirred for 1 h at 100 °C. The reaction mixture was concentrated *in vacuo.* The residue was diluted in DCM and $K_2CO_3$ (10%, aq.) was added. The aqueous layer was extracted with DCM and MeOH (95/5) twice. The combined organic extracts were dried over $MgSO_4$, filtered and evaporated *in vacuo* to give 3.44 g of intermediate **A6** as a beige solid (83%).

Preparation of Compound **1**

A solution of intermediate **A6** (80 mg, 0.202 mmol) in DCM (6 mL) and Me-THF (3 mL) was treated with $Et_3N$ (70 μL, 0.50 mmol). The mixture was cooled to 0 °C and a solution of $Tf_2O$ (1M in DCM, 302 μL, 0.302 mmol) was added dropwise. The reaction mixture was stirred at 0 °C for 20 min. MeOH (0.3 mL) was added, followed by $K_2CO_3$ (10%, aq., 5 mL) and DCM. The layers were separated. The organic phase was dried over $MgSO_4$, filtered and evaporated *in vacuo.* The crude mixture was purified by preparative LC (irregular SiOH 15-40 μm, 12 g, dry loading (Celite®), mobile phase: heptane/EtOAc, gradient from 70:30 to 0:100). The residue (62 mg) was dissolved in warm EtOAc (3 mL) and allowed to cool down to room temperature. The supernatent was removed. The solid was triturated in $Et_2O$. The product was collected by filtration and dried under vacuum to afford 42 mg of compound **1** as a white solid (36%).

**$^1$H NMR** (400 MHz, DMSO-*d*$_6$) δ ppm 9.07 (s, 1 H), 8.47 (br s, 1 H), 7.67 (d, J = 8.1 Hz, 1 H), 7.46 (br d, J = 9.1 Hz, 1 H), 7.30 (br d, J = 8.1 Hz, 2 H), 7.20 (br d, J = 7.6 Hz, 2 H), 4.49 (br d, J = 5.1 Hz, 2 H), 4.41 (s, 2 H), 4.18 (s, 2 H), 3.39-3.31 (m, 1 H), 2.98 (q, J = 7.4 Hz, 2 H), 2.63 - 2.58 (m, 2 H), 2.34-2.29 (m, 2 H), 1.26 (br t, J = 7.3 Hz, 3 H)
**$^1$H NMR** (400 MHz, DMSO-*d*$_6$) δ ppm 9.12 (s, 1 H) 8.71 (m, 1 H) 7.79 (d, *J*=9.4 Hz, 1 H) 7.68 (d, *J*=8.8 Hz, 1 H) 7.26 - 7.37 (m, 3 H) 7.19 (d, *J*=8.7 Hz, 2 H) 4.48 (d, *J*=5.9 Hz, 2 H) 4.08 (t, *J*=4.5 Hz, 2 H) 3.83 (t, *J*=4.8 Hz, 2 H) 3.01 (q, *J*=7.6 Hz, 2 H) 1.27 (t, *J*=7.5 Hz, 3 H)

**Synthesis of Compound 2**

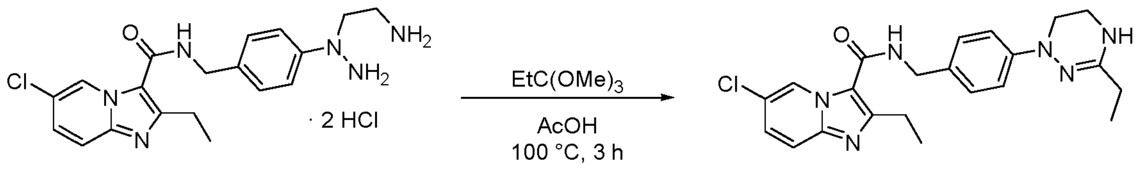


**Intermediate A5** **Intermediate A7**

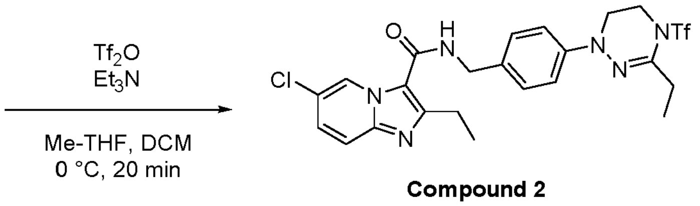


**Compound 2**

Preparation of intermediate **A7**

A mixture of intermediate **A5** (300 mg, 0.652 mmol) and trimethyl orthopropionate (0.102 mL, 0.718 mmol) in acetic acid (6 mL) was stirred for 1 h at 100 °C. Aditionnal amount of trimethylorthopropionate (0.102 mL, 0.718 mmol) was added and the reaction mixture was stirred for at 100 °C for another 2 h. The reaction mixture was diluted in DCM and NaOH (3M, aq.). The layers were separated and the organic phase was dried over $MgSO_4$, filtered and evaporated *in vacuo* to give 138 mg of intermediate **A7** as a foam (50%).

Preparation of Compound **2**

A solution of intermediate **A7** (138 mg, 0.325 mmol) in DCM (4 mL) was treated with $Et_3N$ (113 μL, 0.812 mmol). The mixture was cooled to 0 °C and a solution of $Tf_2O$ in DCM (1M in DCM, 357 μL, 0.357 mmol) was added dropwise. The reaction mixture was stirred at 0 °C for 20 min. The reaction was quenched with MeOH (0.2 mL) and pyridine (0.1 mL). Celite® was added and the mixture was evaporated *in vacuo.* The residue was purified by preparative LC (irregular SiOH 15-40 μm, 24 g, dry loading (Celite®), mobile phase: heptane/EtOAc, gradient from 70:30 to 0:100). A second purification was performed by reverse phase (stationary phase: YMC-actus Triaroom temperature C18 10μm 30*150mm, mobile phase: $NH_4HCO_3$ (0.2% in water)/MeCN, gradient from 40:60 to 10:90) to give 60 mg of compound 2 as a white solid (33%). **$^1$H NMR** (400 MHz, DMSO-*d*$_6$) δ ppm 9.07 (d, *J*=1.6 Hz, 1 H) 8.43 (t, *J*=5.9 Hz, 1 H) 7.66 (d, *J*=9.5 Hz, 1 H) 7.45 (dd, *J*=9.5, 2.1 Hz, 1 H) 7.32 (d, *J*=8.7 Hz, 2 H) 7.18 (d, *J*=8.8 Hz, 2 H) 4.46 (d, *J*=5.9 Hz, 2 H) 3.91 - 4.02 (m, 2 H) 3.79 - 3.90 (m, 2 H) 2.98 (q, *J*=7.5 Hz, 2 H) 2.61 (q, *J*=7.3 Hz, 2 H) 1.26 (t, *J*=7.5 Hz, 3 H) 1.18 (t, *J*=7.3 Hz, 3 H).

**Synthesis of Compound 3**

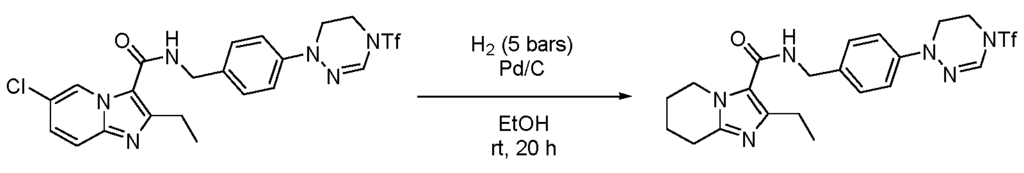


**Compound 1** **Compound 3**

In a pressure vessel reactor, a mixture of compound 1 (250 mg, 0.473 mmol) and Pd/C (54 mg, 50.5 μmol) in EtOH (15 mL) was stirred at room temperature under 5 bar of $H_2$ for 20 h. The mixture was filtered over a pad of Celite®. The filtered cake was washed with EtOH and DCM, and the filtrate was evaporated *in vacuo.* The residue was combined with another batch to give 250 mg of a crude mixture. The residue was purified by reverse phase (Stationary phase: YMC-actus Triaroom temperature C18 10μm 30*150mm, mobile phase: $NH_4HCO_3$ (0.2% in water)/MeCN, gradient from 55:45 to 30:70). The residue was triturated in $Et_2O$, and the solvent was removed under reduced pressure to give 165 mg of compound **3** as a white solid (58%).

**$^1$H NMR** (400 MHz, DMSO-*d*$_6$) δ ppm 8.16 (t, *J*=6.1 Hz, 1 H) 7.28 (s, 1H) 7.26 (d, *J*=8.6 Hz, 2 H) 7.16 (d, *J*=8.6 Hz, 2 H) 4.35 (d, *J*=6.1 Hz, 2 H) 4.07 (t, *J*=4.6 Hz, 2 H) 3.97 (t, *J*=5.7 Hz, 2 H) 3.77 - 3.87 (m, 2 H) 2.68 - 2.75 (t, *J*=6.4

Hz, 2 H) 2.60 (q, *J*=7.5 Hz, 2 H) 1.73 - 1.90 (m, 4 H) 1.09 (t, *J*=7.5 Hz, 3 H).

**Synthesis of Compound 4**

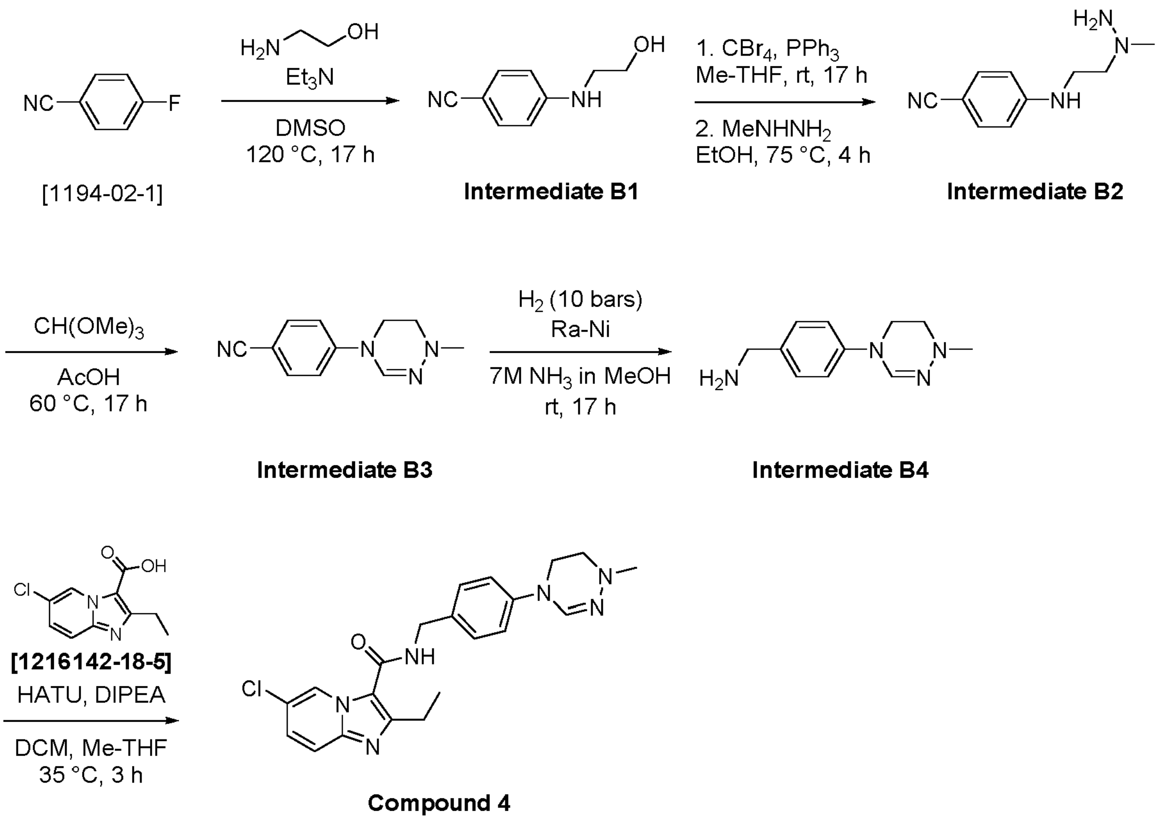


[1194-02-1] **Intermediate B1** **Intermediate B2**

**Intermediate B3** **Intermediate B4**

**[1216142-18-5]**

**Compound 4**

Preparation of intermediate **B1**

A flask (equipped with a findenser) was charged with 4-fluorobenzonitrile [1194-02-1] (1.00 g, 8.26 mmol), DMSO (5.9 mL) and ethanolamine (0.757 g, 12.4 mmol). $Et_3N$ (1.72 mL, 12.4 mmol) was added and the reaction mixture was stirred at 120 °C for 17 h. The mixture was poured into brine. The layers were separated and the aqueous phase was extracted with EtOAc. The combined organic extracts were washed with brine (3 times), dried over $MgSO_4$, filtered and evaporated *in vacuo* to afford intermediate **B1** as pale-yellow oil (Quant.).

Preparation of intermediate **B2**

A solution of the intermediate **B1** (2.00 g, 12.3 mmol) and triphenylphosphine (4.21 g, 16.0 mmol) in Me-THF (100 mL) was treated with $CBr_4$ (5.32 g, 16.0 mmol). The reaction mixture was stirred at room temperature for 17 h. The mixture was evaporated *in vacuo.* The residue was solubilized in EtOH (40 mL) and treated with methylhydrazine (5.19 mL, 98.6 mmol). The reaction mixture was stirred at 75 °C for 4 h and concentrated *in vacuo.* The residue was diluted with DCM and HCl (3M, aq.) was added. The layers were separated and the organic phase was washed with water. The combined aqueous extracts were basified by the addition of $K_2CO_3$. The aqueous phase was extracted with DCM (twice). The combined organic layers were dried over $MgSO_4$, filtered and evaporated *in vacuo* to afford 2.54 g of compound **B2** as an orange oil (Quant.).

Preparation of intermediate **B3**

A solution of intermediate **B2** (2.15 g, 11.3 mmol) and trimethyl orthoformate (3.71 mL, 33.9 mmol) in acetic acid (60 mL) was stirred at 60 °C for 17 h. The yellow solution was cooled to room temperature. Water (150 mL) and EtOAc (150 mL) were added. $K_2CO_3$ was added portionwise until basification of the aqueous layer. The organic layer

was separated, washed with water, and brine, dried over $MgSO_4$, filtered and evaporated *in vacuo* to give 1.50 g of intermediate **B3** as an orange solid (66%).

Preparation of intermediate **B4**

In an autoclave, a mixture of intermediate **B3** (1.5 g, 7.49 mmol) and Raney Nickel (440 mg, 7.49 mmol) in a 7M solution of $NH_3$ in MeOH (64 mL) was hydrogenated at room temperature under 5 bars of $H_2$ for 17 h. The reaction mixture was filtered through a pad of Celite®, and washed with a mixture of DCM and MeOH (9/1). The filtrate was evaporated *in vacuo* to afford 1.53 g of intermediate **B4** as a grey solid (Quant.).

Preparation of Compound **4**

6-Chloro-2-ethylimidazo[1,2-a]pyridine-3-carboxylic acid [1216142-18-5] (600 mg, 2.67 mmol) was solubilized in Me-THF (30 mL), and DCM (15 mL) and DIPEA (0.736 mL, 4.27 mmol) was added. After complete solubilization, intermediate **B4** (627 mg, 3.07 mmol) was added followed by HATU (1.17 g, 3.07 mmol). The reaction mixture was stirred for 3 h at 35 °C. EtOAc and water was added. The organic layer was separated and washed with water, then brine. The combined organic extracts were dried over $MgSO_4$, filtered and evaporated *in vacuo.* The residue was solubilized in a minimum amount of warm EtOAc. The solution was cooled to room temperature and the suspension was filtered. The solid was washed with EtOAc, then with EtOH and $Et_2O$. The solid was collected by filtration and dried under vacuum to afford 210 mg of an off-white solid. The solid was combined with the filtrate and evaporated *in vacuo.* The residue was purified by preparative LC (irregular SiOH 15-40 µm, 80 g, mobile phase: DCM/(DCM/MeOH/$NH_3$ aq., 18/20/2), gradient from 90:10 to 60:40). The residue was crystallized from EtOAc, washed with $Et_2O$ and dried under vacuum to afford 317 mg of compound **4.**

**$^1$H NMR** (400 MHz, DMSO-$d_6$) δ ppm 9.07 (d, *J*=1.47 Hz, 1 H) 8.45 (t, *J*=5.81 Hz, 1 H) 7.67 (d, *J*=9.66 Hz, 1 H) 7.46 (dd, *J*=9.41, 2.08 Hz, 1 H) 7.30 - 7.36 (m, 3 H) 7.11 (d, *J*=8.56 Hz, 2 H) 4.47 (d, *J*=5.87 Hz, 2 H) 3.70 (t, *J*=5.01 Hz, 2 H) 3.17 (d, *J=5.14* Hz, 1 H) 2.88 - 3.01 (m, 4 H) 2.54 - 2.65 (m, 4 H) 1.26 (t, *J*=7.52 Hz, 3 H).

**Synthesis of Compound 5**

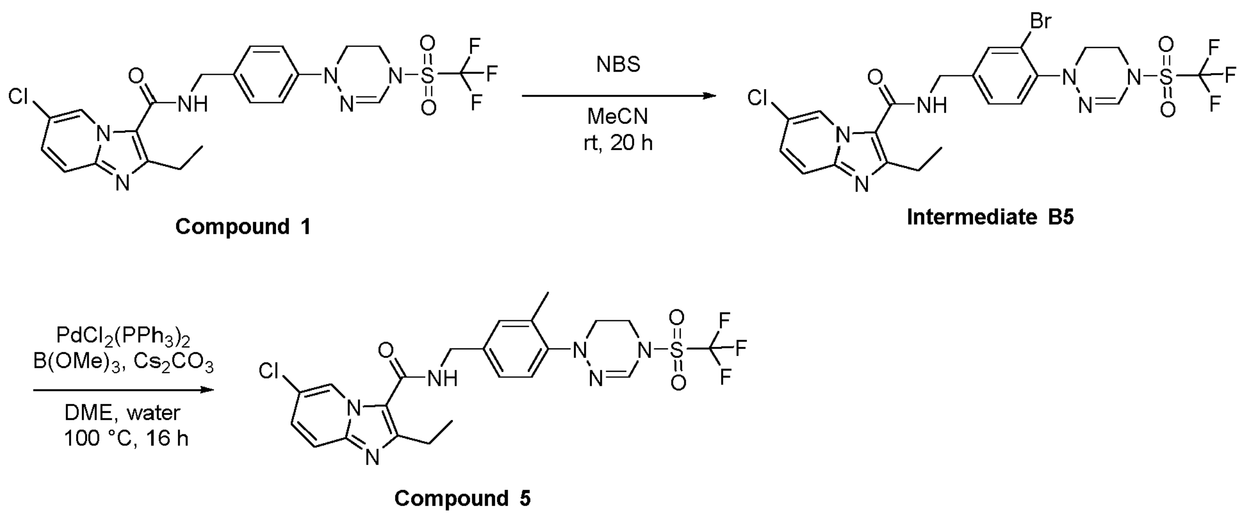


Compound 1

Intermediate B5

Compound 5

Preparation of intermediate **B5**

NBS (204 mg, 1.15 mmol) was added to a solution of Compound **1** (600 mg, 1.13 mmol) in MeCN (9.5 mL) and the reaction mixture was stirred at room temperature for 20 h. The mixture was diluted with EtOAc and water. The layers were separated. The organic phase was washed $NaHCO_3$ (sat., aq.), dried over $MgSO_4$, filtered and the solvent was removed under reduced pressure to give 700 mg of intermediate **B5** as a brown residue.

Preparation of Compound **5**

A mixture of intermediate **B5** (250 mg, 0.234 mmol), trimethylboroxine (131 $\mu$L, 0.938 mmol) and $Cs_2CO_3$ (229 mg, 0.703 mmol) in DME (3.6 mL) and water (3.6 mL) was purged with $N_2$. $PdCl_2(PPh_3)_2$ (32.9 mg, 0.0469 mmol) was added and the mixture was purged again with $N_2$. The reaction mixture was stirred at 100 °C for 16 h. Water and EtOAc were added. The layers were separated and the aqueous phase was extracted with EtOAc. The combined organic extracts were washed with brine, dried over $MgSO_4$, filtered and evaporated to dryness *in vacuo.* The residue was purified by preparative LC (irregular SiOH 15-40 $\mu$m, 24 g, dry loading (Celite®), mobile phase: DCM/MeOH, gradient from 99:1 to 95:5). A second purification was performed via reverse phase (stationary phase: YMC-actus Triaroom temperature C18 10$\mu$m 30*150mm, mobile phase $NH_4HCO_3$ (0.2% in water/MeCN, gradient from 55:45 to 35:65) to give 14 mg of a white residue which was solubilized in MeCN, extended with water and freeze-dried to give 12 mg of compound 5 as a white powder (7%).

**$^1$H NMR** (400 MHz, DMSO-$d_6$) $\delta$ ppm 9.07 (d, *J*=1.34 Hz, 1 H) 8.48 (t, *J*=5.99 Hz, 1 H) 7.67 (d, *J*=9.41 Hz, 1 H) 7.46 (dd, *J*=9.54, 2.08 Hz, 1 H) 7.29 (s, 1 H) 7.22 (s, 1 H) 7.21 (d, *J*=7.74 Hz, 2 H) 7.12 - 7.17 (m, 1 H) 4.49 (d, *J*=6.11 Hz, 2 H) 4.10 (br d, *J*=4.28 Hz, 2 H) 3.38 - 3.54 (m, 4 H) 3.00 (q, *J*=7.42 Hz, 2 H) 2.67 - 2.69 (m, 1 H) 2.52 - 2.56 (m, 5 H) 2.33 - 2.45 (m, 2 H) 2.25 (s, 3 H) 1.19 - 1.33 (m, 3 H).

**Synthesis of Compound 6**

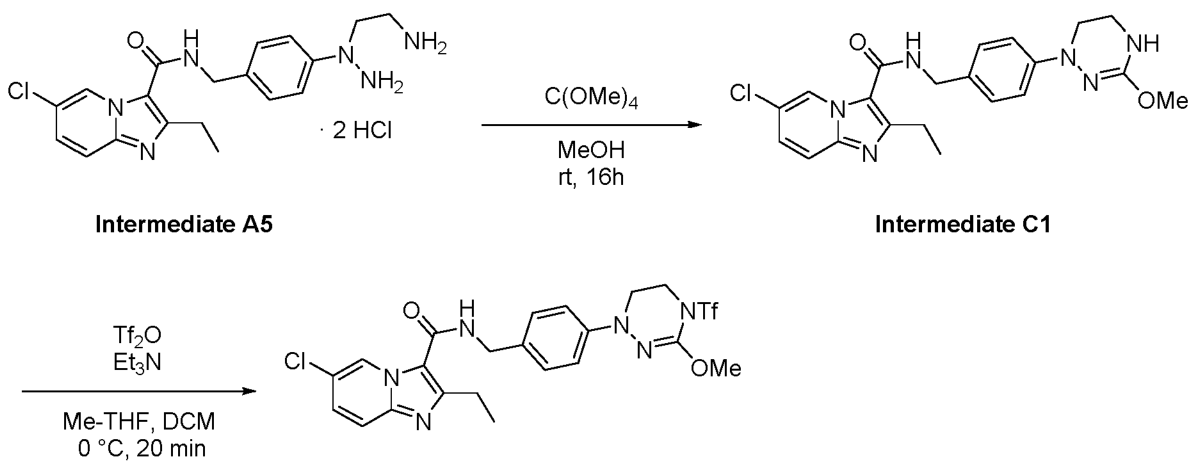


**Intermediate A5**

**Intermediate C1**

**Compound 6**

Preparation of intermediate **C1**

In a sealed tube, a mixture of intermediate **A5** (300 mg, 0.652 mmol) and molecular sieves 3Å in MeOH (4.3 mL) was stirred at room temperature for 10 min. Tetramethyl orthocarbonate (347 $\mu$L, 2.61 mmol) was added and the reaction mixture was stirred at room temperature for 16 h. Water and DCM were added. The layers were separated and the organic phase was dried over $MgSO_4$, filtered and evaporated *in vacuo* to dryness. The residue was purified by preparative LC (irregular SiOH 15-40 $\mu$m, 24 g, dry loading (Celite®), mobile phase: heptane/EtOAc, gradient from 60:40 to 0:100) to give 77 mg of intermediate **C1** as a white solid (24%).

Preparation of Compound **6**

To a solution of intermediate **C1** (48 mg, 0.112 mmol) in anhydrous DCM (1.3 mL) at room temperature was added $Et_3N$ (23.4 $\mu$L, 0.169 mmol) and the mixture was stirred at room temperature for 10 min. The mixture was cooled at 0 °C and a solution of TfzO in DCM (1M in DCM, 112 $\mu$L, 0.112 mmol) was added dropwise. The mixture was stirred warming to room temperature for 1 h. A solution of $Tf_2O$ in DCM (1M in DCM, 112 $\mu$L, 0.112 mmol) was added and the mixture was stirred at room temperature for another 1 h. $NaHCO_3$ (sat., aq.) and DCM were added. The layers were separated, and the organic phase was washed with $NaHCO_3$ (twice) and brine. The combined organic extracts were dried over $MgSO_4$, filtered and concentrated *in vacuo.* The residue was purified by preparative LC (irregular SiOH 15-40 $\mu$m, 24 g, dry loading (Celite®), mobile phase: heptane/EtOAc, gradient from 50:50 to 0:100). A second purification was

performed via reverse phase (stationary phase: YMC-actus Triaroom temperature C18 10μm 30*150mm, mobile phase: $NH_4HCO_3$ (0.2% in water)/MeCN, gradient from 45:55 to 25:75) to give 33 mg of compound **6** as a white solid (37%).

**$^1$H NMR** (500 MHz, DMSO-$d_6$) δ ppm 9.07 (d, *J*=1.58 Hz, 1 H) 8.39 (t, *J*=5.83 Hz, 1 H) 7.66 (d, *J*=9.46 Hz, 1 H) 7.44 (dd, *J*=9.46, 2.21 Hz, 1 H) 7.29 (d, *J*=8.51 Hz, 2 H) 7.15 (d, *J*=8.83 Hz, 2 H) 4.46 (d, *J*=5.99 Hz, 2 H) 4.06 - 4.14 (m, 2 H) 3.85 (s, 3 H) 3.71 - 3.77 (m, 2 H) 3.32 - 3.46 (m, 2 H) 3.17 (d, *J*=5.36 Hz, 1 H) 2.97 (q, *J*=7.36 Hz, 2 H) 2.52 - 2.58 (m, 6 H) 1.26 (t, *J*=7.57 Hz, 3 H).

## Synthesis of Compound 7

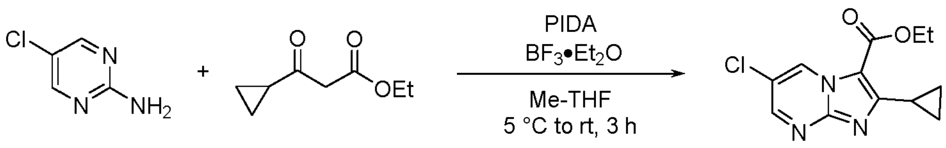


[428-89-7] [24922-02-9] **intermediate C2**

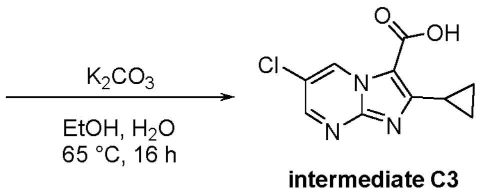


**intermediate C3**

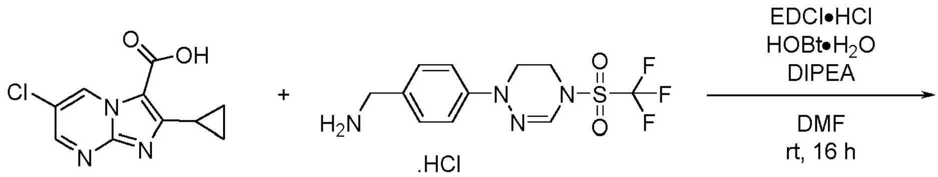


**intermediate C3** **intermediate E9**

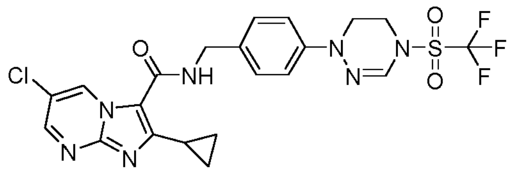

**Compound 7**

### Preparation of Intermediate **C2**

To a solution of 2-amino-5-chloropyrimidine [428-89-7] (500 mg, 3.86 mmol) in Me-THF (40 mL) at 5 °C were added ethyl 3-cyclopropyl-3-oxopropanoate [24922-02-9] (0.603 g, 3.86 mmol) and (diacetoxyiodo)benzene (1.24 g, 3.86 mmol). Boron trifluoride etherate (50 μL, 0.191 mmol) was added dropwise, and the reaction mixture was stirred at 5 °C for 30 min, then at room temperature for 1 h. Extra amounts of ethyl 3-cyclopropyl-3-oxopropanoate (0.301 g, 1.93 mmol) (diacetoxyiodo)benzene (0.622 g, 1.93 mmol) and boron trifluoride etherate (50 μL, 0.191 mmol) were added. The mixture was purged with $N_2$ and stirred at room temperature for 1 h. Extra amounts of ethyl 3-cyclopropyl-3-oxopropanoate (0.301 g, 1.93 mmol), (diacetoxyiodo)benzene (0.622 g, 1.93 mmol) and boron trifluoride etherate (50 μL, 0.191 mmol) were added again. The mixture was purged with $N_2$ and stirred at room temperature for another 1 h. EtOAc and water were added. The layers were separated, and the organic phase was dried over $MgSO_4$, filtered and concentrated *in vacuo.* The crude mixture was purified by preparative LC (irregular SiOH 15-40 μm, 80 g, dry loading (Celite®), mobile phase: heptane/EtOAc, 80:20, 65:35). The residue was triturated in pentane. The solid was collected by filtration and dried under vacuum to give 598 mg of intermediate **C2** as a white solid (58%).

Preparation of Intermediate **C3**

To a solution of the intermediate **C2** (125 mg, 0.47 mmol) in EtOH (2.2 mL) and water (2.2 mL) was added $K_2CO_3$ (196 mg, 1.42 mmol). The reaction mixture was stirred at 65 °C for 16 h. The mixture was cooled to room temperature and the reaction was quenched with HCl (1M in water) until pH~3. The mixture was evaporated *in vacuo* to afford 294 mg of intermediate **C3** as a white solid. The crude product was used as such in the next step.

Preparation of Compound **7**

To a solution of intermediate **C3** (294 mg, 0.472 mmol) in DMF (4.5 mL) were added EDCI•HCl (110 mg, 0.574 mmol), $HOB_t$•$H_2O$ (76 mg, 0.496 mmol), DIPEA (0.245 mL, 1.42 mmol) and intermediate **E9** (185 mg, 0.516 mmol). The reaction mixture was stirred at room temperature for 16 h evaporated *in vacuo.* The residue was taken-up in EtOAc, washed with $NaHCO_3$ (sat., aq.) and brine. The organic layer was dried over $MgSO_4$, filtered and evaporated *in vacuo.* The crude mixture was purified by preparative LC (irregular SiOH 15-40 μm, 24 g Büchi, dry loading (Celite®), mobile phase: heptane/(EtOAc/MeOH, 9:1), gradient from 90:10 to 40:60) to afford a light yellow solid. The solid was crystallized from EtOAc and sonicated in pentane. The solid was collected by filtration and dried under vacuum to obtain 121 mg of compound 7 as a white solid (47%).

**$^1$H NMR** (400 MHz, DMSO-$d_6$) δ ppm 9.40 (d, *J*=1.8 Hz, 1 H) 8.58 - 8.75 (m, 2 H) 7.34 (d, *J*=8.1 Hz, 2 H) 7.29 (s, 1H) 7.19 (d, *J*=8.4 Hz, 2 H) 4.50 (d, *J*=5.6 Hz, 2 H) 4.08 (s, 2 H) 3.83 (s, 2 H) 2.38 - 2.46 (m, 1 H) 1.03 - 1.13 (m, 4 H).

**Synthesis of Compound 8**

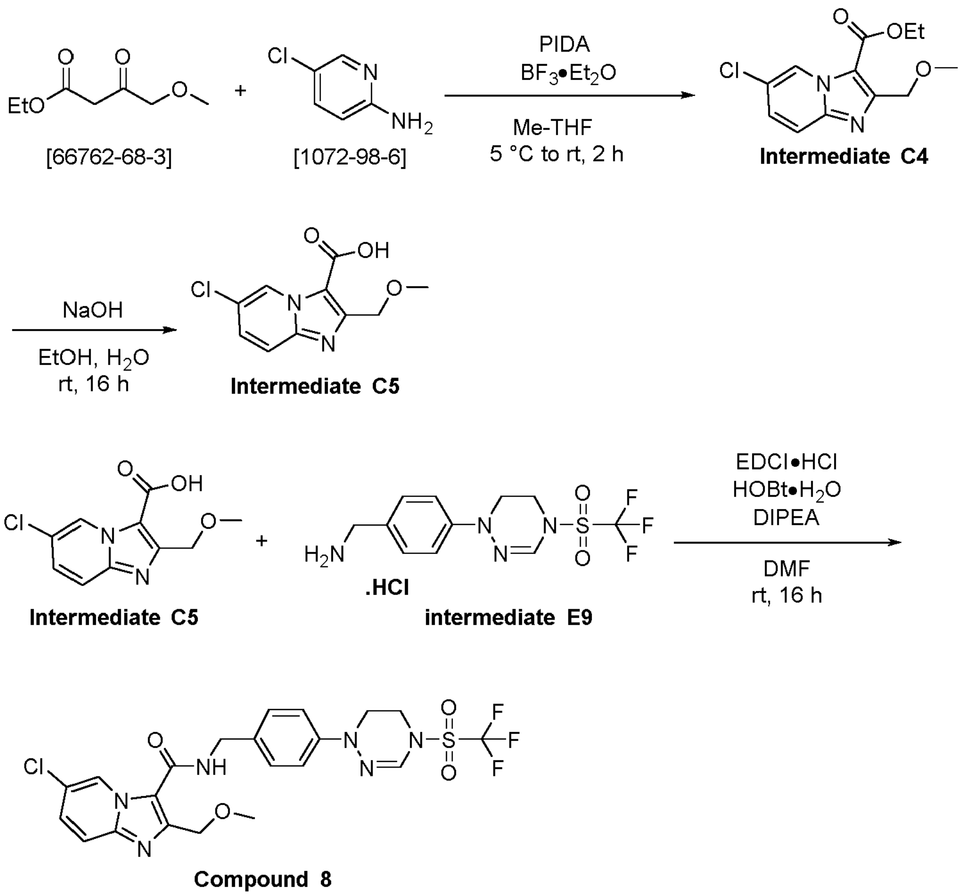


Intermediate C4

Intermediate C5

Intermediate C5

intermediate E9

Compound 8

Preparation of Intermediate **C4**

To a solution of 2-amino-5-chloropyridine [1072-98-6] (3.00 g, 23.3 mmol) in Me-THF (100 mL) were added iodobenzene diacetate (7.50 g, 23.3 mmol) and and ethyl-4-methoxy-3-oxobutanoate [66762-68-3] (6.00 g, 34.8 mmol).

Then boron trifluoride etherate (0.30 mL, 1.15 mmol) was added dropwise. The solution was stirred at 5 °C for 1 h. The mixture was warmed to room temperature and stirred for another 1 h. EtOAc and $NaHCO_3$ (sat., aq.) were added. The layers were separated, and the aqueous layer was extracted with EtOAc. The combined organic extracts were washed with brine (twice), dried over $MgSO_4$, filtered and evaporated to give a brown liquid. The crude mixture was purified by preparative LC (irregular SiOH 15-40 μm, 120 g, dry loading (Celite®), mobile phase: heptane/EtOAc, gradient from 90:10 to 40:60) to afford 2.44 g of the intermediate **C4** as a yellow solid (39%).

Preparation of Intermediate **C5**

To a solution of intermediate **C4** (1.44 g, 5.36 mmol) in EtOH (11.5 mL) and water (11.5 mL) was added NaOH (650 mg, 16.3 mmol) and the reaction mixture was stirred at room temperature overnight. The reaction was quenched with HCl (3N in water) until pH-3. The mixture was filtered to afford 996 mg of the intermediate **C5** as an off-white solid (77%).

Preparation of Compound **8**

To a mixture of intermediate **C5** (125 mg, 0.519 mmol) and DIPEA (270 μL, 1.57 mmol) in DMF (5 mL) at room temperature were added EDCI•HCl (125 mg, 0.652 mmol) and $HOB_t{\bullet}H_2O$ (85 mg, 0.555 mmol). Intermediate **E9** (205 mg, 0.571 mmol) was added and the resulting mixture was stirred for 16 h. $NaHCO_3$ (1%, aq.) and EtOAc were added and the layers were separated. The organic layer was washed with brine (3 times), dried over $MgSO_4$, filtered and concentrated *in vacuo* until dryness to give an orange solid which was purified by preparative LC (irregular SiOH 15-40 μm, 24 g, dry loading (Celite®), mobile phase: heptane/(EtOAc/MeOH, 9:1), gradient from 75:20 to 30:70) to obtain a white solid. The residue was purified by reverse phase (spherical C18, 25 μm, 40 g YMC-ODS-25, dry loading (Celite®), mobile phase: $NH_4HCO_3$ (0.2% in water)/MeCN, gradient from 60:40 to 0:100) to give 233 mg of compound **8** as a white solid (71%).

**$^1$H NMR** (400 MHz, $CDCl_3$-*d*) δ ppm 9.68 (dd, *J*=2.0, 0.8 Hz, 1 H) 8.51 (t, *J*=4.7 Hz, 1 H) 7.56 (d, *J*=9.4 Hz, 1 H) 7.31 - 7.36 (m, 3 H) 7.18 (d, *J*=7.9 Hz, 2 H) 7.11 (s, 1 H) 4.75 (s, 2 H) 4.59 (d, *J*=5.5 Hz, 2 H) 4.06 (t, *J*=4.7 Hz, 2 H) 3.79 (t, *J*=4.7 Hz, 2 H) 3.28 (s, 3 H)

**Synthesis of Compound 9**

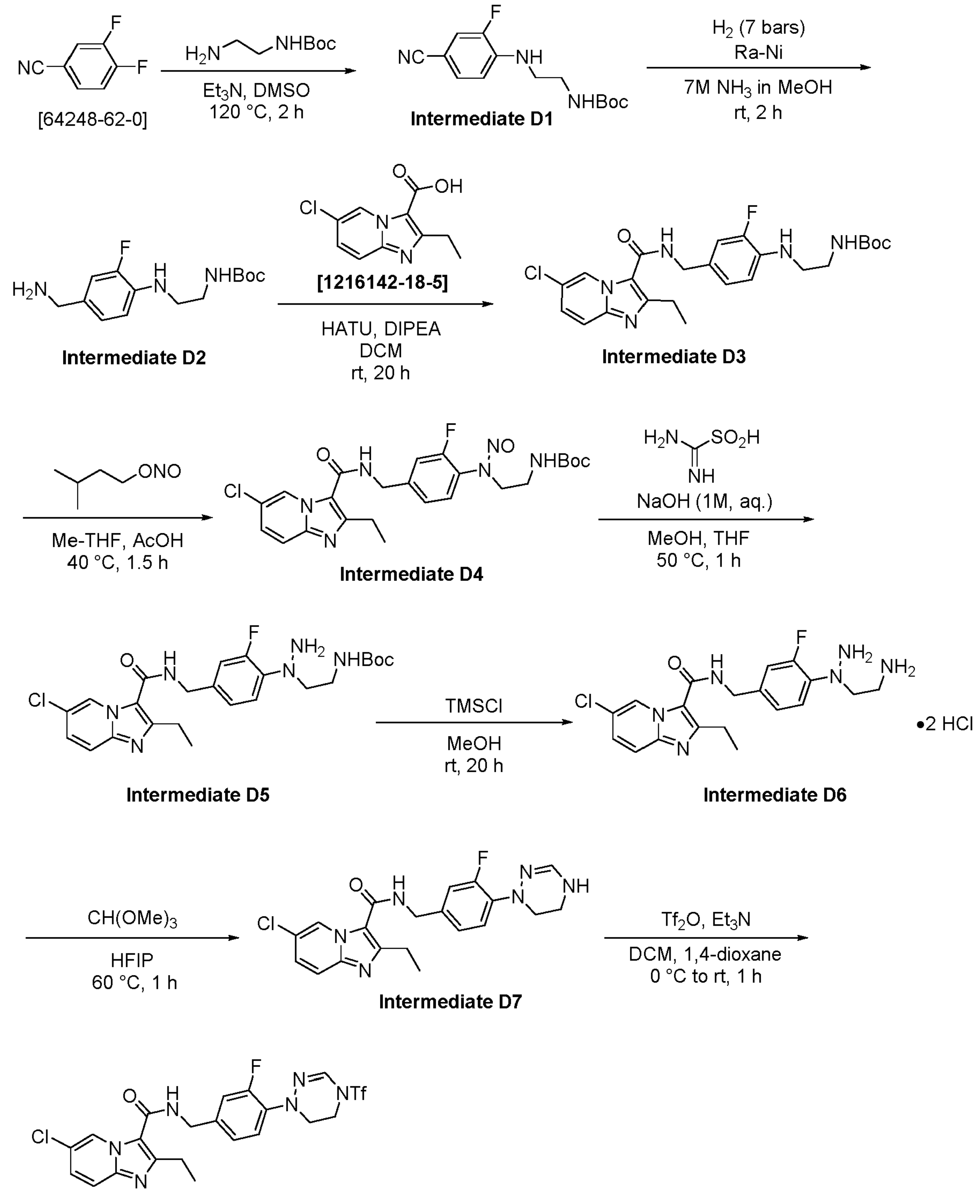


**Compound 9**

Preparation of intermediate **D1**

A mixture of 3,4-difluorobenzonitrile [64248-62-0] (3.67 g, 26.4 mmol), *N*-Boc-1,2-diaminoethane (5.50 g, 34.3 mmol) and $Et_3N$ (14.7 mL, 105 mmol) in DMSO (47 mL) was stirred at 120 °C for 2 h. The reaction mixture was cooled down and diluted with EtOAc and water. The layers were separated and the aqueous phase was extracted with EtOAc (twice). The combined organic layers were washed with brine (3 times), dried over $MgSO_4$, filtered and evaporated *in vacuo.* The residue was purified by preparative LC (irregular SiOH 15-40 μm, 80 g, liquid injection (DCM), mobile phase: heptane/EtOAc, gradient from 100:0 to 50:50) to give 5.02 g of intermediate **D1** as a white solid (68%).

Preparation of intermediate **D2**

In an autoclave, to a solution of intermediate **D1** (2.00 g, 7.16 mmol) in a 7M solution of $NH_3$ in MeOH (70 mL), purged with nitrogen, was added Raney-Nickel (3.39 g, 57.7 mmol). The reaction mixture was hydrogenated under 7 bars at room temperature for 2 h. The mixture was filtered through a pad of Celite® and rinsed with MeOH. The filtrate was concentrated *in vacuo* to give 2.11 g of the intermediate **D2** as a white solid (Quant.).

Preparation of intermediate **D3**

HATU (2.57 g, 6.77 mmol) was added to a mixture of 6-chloro-2-ethylimidazo[1,2-a]pyridine-3-carboxylic acid [1216142-18-5] (1.52 g, 6.77 mmol) and DIPEA (4.7 mL, 27.1 mmol) in DCM (126 mL). The reaction mixture was stirred at room temperature for 10 min and then intermediate **D2** (2.11 g, 7.45 mmol) was added and the reaction mixture was stirred at room temperature for 20 h. The reaction mixture was diluted with DCM and water. The aqueous layer was extracted with DCM (twice). The combined organic layers were washed with brine (twice), dried over $MgSO_4$, filtered and evaporated *in vacuo.* The residue was purified by preparative LC (irregular SiOH 15-40 μm, 120 g, liquid injection (DCM), mobile phase: heptane/EtOAc, gradient from 50:50 to 0:100) to give 2.76 g of intermediate **D3** as a pale brown solid (83%).

Preparation of intermediate **D4**

Intermediate **D3** (1.5 g, 3.06 mmol) was solubilized at 40 °C in Me-THF (23.2 mL) and AcOH (1.75 mL). Isopentyl nitrite (2.06 mL, 15.3 mmol) was added dropwise over 10 min and the reaction mixture was stirred at 40 °C for 1 h. The solution was diluted in EtOAc and $NaHCO_3$ (sat., qa.). The layers were separated and the organic layer was washed with $NaHCO_3$ (sat., aq.) (twice), and brine, dried over $MgSO_4$ and evaporated *in vacuo* to give 1.74 g of intermediate **D4** as a pale-yellow oil.

Preparation of intermediate **D5**

A solution of intermediate **D4** (1.59 g, 3.06 mmol) in THF (47 mL) and MeOH (32 mL) was treated with NaOH (1M, aq., 37 mL). Thisurea dioxide (formamidinesulfonic acid) (1.66 g, 15.3 mmol) was added and the reaction mixture was stirred at 50 °C for 1 h (using findeser equipment). The reaction mixture was diluted with DCM and $K_2CO_3$ (10%, aq.) was added. The layers were separated, and the organic layer was dried over $MgSO_4$, filtered and the solvent was removed under reduced pressure to give 1.44 g of intermediate **D5** as a yellow oil.

Preparation of intermediate **D6**

A solution of intermediate **A5** (1.55 g, 3.06 mmol) in MeOH (34 mL) was treated with TMSCl (3.88 mL, 30.6 mmol) and the reaction mixture was stirred at room temperature for 20 h. The solvent was removed under reduced pressure and the resulting solid was triturated in $Et_2O$. The solvent was evaporated to give 1.51 g of intermediate **D6** as a pale-yellow solid (Quant.).

Preparation of intermediate **D7**

Trimethyl orthoformate (0.618 mL, 5.65 mmol) was added to a suspension of intermediate **D6** (900 mg, 1.88 mmol) in HFIP (18 mL) and the reaction mixture was stirred at 60 °C for 1 h. The reaction mixture was cooled down to room temperature, diluted with EtOAc and then basified with $NaHCO_3$ (sat., aq.). The layers were separated, and the aqueous layer was extracted with EtOAc. The combined organic layers were dried $_{over}$ $MgSO_4$, filtered and the solvent was removed under reduced pressure. The residue was purified by preparative LC (irregular SiOH 15-40 μm, 24 g, liquid injection (DCM), mobile phase: DCM/MeOH, gradient from 100:0 to 90:10) to give 202 mg of the intermediate **D7** as an off-white solid (33%).

Preparation of Compound 9

$Et_3N$ (0.169 mL, 1.22 mmol) was added to a solution of intermediate **D7** (202 mg, 0.487 mmol) in DCM (9 mL) and 1,4-dioxane (6 mL). The solution was cooled to 5 °C and a solution of $Tf_2O$ in DCM (1M in DCM, 0.487 mL, 0.487 mmol) was added dropwise over 5 min. The reaction mixture was diluted with DCM and with $NaHCO_3$ (sat., aq.). The layers were separated. The organic layer was washed with brine, dried over $MgSO_4$, filtered and the solvent was removed under reduced pressure. The residue was purified by preparative LC (irregular SiOH 15-40 μm, 12 g, liquid injection

(DCM), mobile phase: heptane/EtOAc, gradient from 70:30 to 0:100) to give 183 mg of a yellow solid. The solid was triturated and sonicated in EtOAc. The suspension was filtered off. The solid and the filtrate were combined. The residue was triturated in $Et_2O$ and sonicated, filtered off, washed with $Et_2O$ and collected to give 125 mg of compound 9 as a white solid (47%).

**$^1$H NMR** (400 MHz, DMSO-$d_6$) δ ppm 9.09 (d, *J*=1.5 Hz, 1 H) 8.48 (t, *J*=5.9 Hz, 1 H) 7.67 (d, *J*=9.5 Hz, 1 H) 7.47 (dd, *J*=9.5, 2.0 Hz, 1 H) 7.30 - 7.41 (m, 2 H) 7.16 - 7.30 (m, 2 H) 4.50 (d, *J*=5.9 Hz, 2 H) 4.10 (br t, *J*=4.2 Hz, 2 H) 3.65 (t, *J*=4.6 Hz, 2 H) 3.00 (q, *J*=7.5 Hz, 2 H) 1.27 (t, *J*=7.5 Hz, 3 H).

**Synthesis of Compound 10**

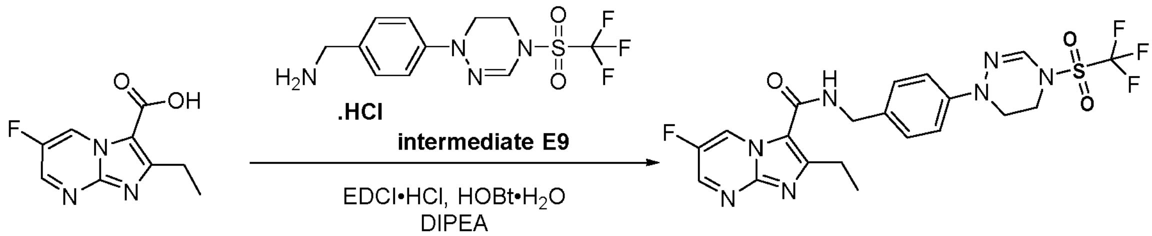


**[1368682-64-7]** **Compound 10**

To a solution of 2-ethyl-6-fluoroimidazo[1,2-a]pyridine-3-carboxylic acid [1368682-64-7] (82 mg 0.393 mmol) in DMF (4.5 mL) were added EDCI•HCl (91 mg, 0.474 mmol), $HOB_t$•$H_2O$ (63 mg, 0.415 mmol) and DIPEA (203 μL, 1.18 mmol). The mixture was stirred at room temperature for 15 min. Intermediate **B9** (155 mg, 0.432 mmol) was added and the reaction mixture was stirred at room temperature for 20 h. The solvent was removed under reduced pressure and the residue was diluted with EtOAc and water. The layers were separated and the aqueous layer was extracted with EtOAc. The combined organic layers were washed with brine (twice), dried over $MgSO_4$, filtered and the solvent was removed under reduced pressure. The residue was purified by preparative LC (irregular SiOH 15-40 μm, 12 g, liquid injection (DCM), mobile phase: DCM/MeOH, gradient from 100:0 to 90:10). A second purification was performed by reverse phase (stationary phase: YMC-actus Triart C18 10μm 30*150mm, mobile phase: $NH_4HCO_3$ (0.2% in water)/MeCN, gradient from 50:50 to 25:75). The residue was solubilized in MeCN and MeOH (50:50), extended with water and freeze-dried to give 44 mg of compound **10** as a white solid (22%).

**$^1$H NMR** (400 MHz, DMSO-$d_6$) δ ppm 9.40 (dd, *J*=4.8, 2.9 Hz, 1 H) 8.82 (d, *J*=3.1 Hz, 1 H) 8.51 (t, *J*=5.7 Hz, 1 H) 7.26 - 7.35 (m, 3 H) 7.18 (d, *J*=8.7 Hz, 2 H) 4.48 (d, *J*=5.7 Hz, 2 H) 4.08 (t, *J*=4.6 Hz, 2 H) 3.82 (t, *J*=4.8 Hz, 2 H) 3.02 (q, *J*=7.5 Hz, 2 H) 1.27 (t, *J*=7.5 Hz, 3 H).

**Synthesis of Compound 11**

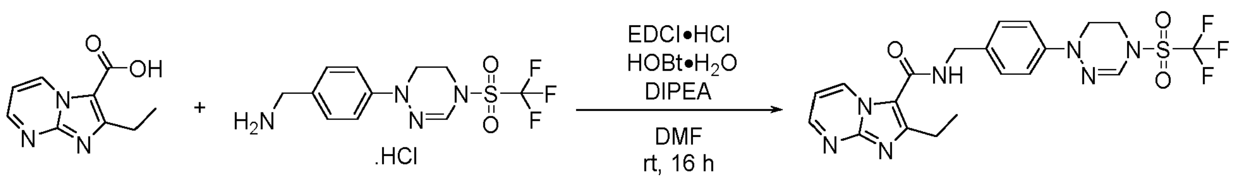


**[1403942-20-0]** **intermediate E9** **Compound 11**

To a mixture of 2-ethyl-imidazo[1,2-a]pyrimidine-3-carboxylic acid [1403942-20-0] (125 mg, 0.654 mmol) and DIPEA (228 μL, 1.32 mmol) in DMF (6.5 mL) at room temperature were added EDCI•HCl (150 mg, 0.782 mmol) and $HOB_t$•$H_2O$ (105 mg, 0.686 mmol). Intermediate **E9** (230 mg, 0.714 mmol) was added and the resulting mixture was stirred for 16 h. NaHCOs (1%, aq.) and EtOAc were added. The layers were separated, and the organic layer was washed with brine (twice), dried over $MgSO_4$, filtered and concentrated *in vacuo* until dryness. The residue was purified by preparative LC (irregular SiOH 15-40 μm, 24 g, dry loading (Celite®), mobile phase: heptane/(EtOAc/MeOH, 9/1), gradient from 60:40 to 10:90). The residue was crystallized from EtOAc and collected by filtration to give 170 mg of compound **11** as a white solid (52%).

**$^1$H NMR** (400 MHz, DMSO-$d_6$) δ ppm 9.30 (dd, *J*=7.0, 2.0 Hz, 1 H) 8.61 (dd, *J*=4.2, 2.0 Hz, 1 H) 8.48 (t, *J*=5.9

Hz, 1 H) 7.27 - 7.35 (m, 3 H) 7.13 - 7.21 (m, 3 H) 4.47 (d, *J*=6.0 Hz, 2 H) 4.05 - 4.11 (m, 2 H) 3.83 (t, *J*=4.8 Hz, 2 H) 3.01 (q, *J*=7.5 Hz, 2 H) 1.27 (t, *J*=7.5 Hz, 3 H).

## Synthesis of Compound 12

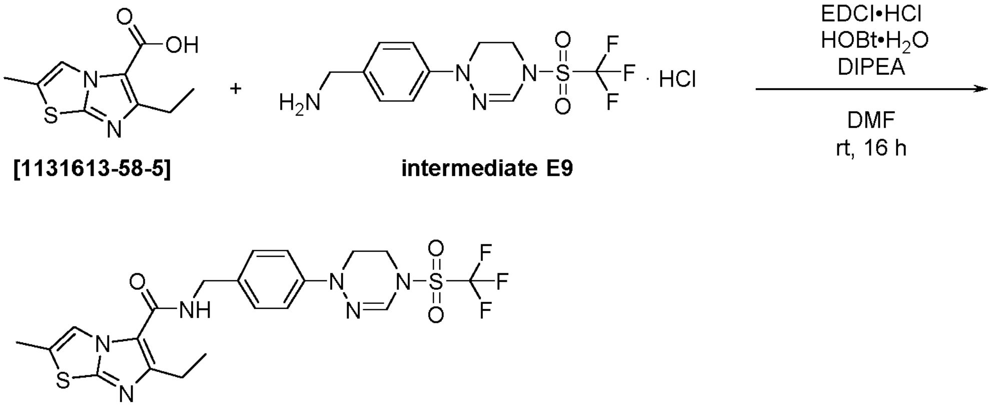


**[1131613-58-5]** **intermediate E9**

**Compound 12**

To a mixture of 6-ethyl-2-methyl-imidazo[2,1-b]thiazole-5-carboxylic acid [1131613-58-5] (150 mg, 0.608 mmol) and DIPEA (345 μL, 2.00 mmol) in DMF (6.5 mL) were added EDCI•HCl (140 mg, 0.730 mmol) and $HOB_t$•$H_2O$ (100 mg, 0.653 mmol). The mixture was stirred at room temperature for 15 min. Then intermediate **E9** (240 mg, 0.669 mmol) was added and the resulting mixture was stirred for 16 h. The mixture was evaporated *in vacuo*. NaHCOs (1%, aq.) and EtOAc were added and the layers were separated. The organic layer was washed with brine, dried over $MgSO_4$ and concentrated to dryness. The residue was purified by preparative LC (irregular SiOH 15-40 μm, 24 g, dry loading (Celite®), mobile phase: heptane/(EtOAc/MeOH, 9/1), gradient from 95:5 to 50:50). A second purification was performed by reverse phase (spherical C18, 25 μm, 40 g YMC-ODS-25, dry loading (Celite®), mobile phase: $NH_4HCO_3$ (0.2% in water)/MeCN, gradient from 60:40 to 5:95) to give 206 mg of compound **12** as a white solid (66%).

**$^1$H NMR** (500 MHz, DMSO-$d_6$) δ ppm 8.05 (t, J=6.0 Hz, 1 H) 7.87 (s, 1 H) 7.24 - 7.30 (m, 3 H) 7.17 (d, J=8.5 Hz, 2 H) 4.41 (d, J=6.0 Hz, 2 H) 4.04 - 4.10 (m, 2 H) 3.81 (br t, J=4.7 Hz, 2 H) 2.86 (q, J=7.6 Hz, 2 H) 2.41 (s, 3 H) 1.20 (t, J=7.6 Hz, 3 H).

## Synthesis of Compound 13 and Compound 14

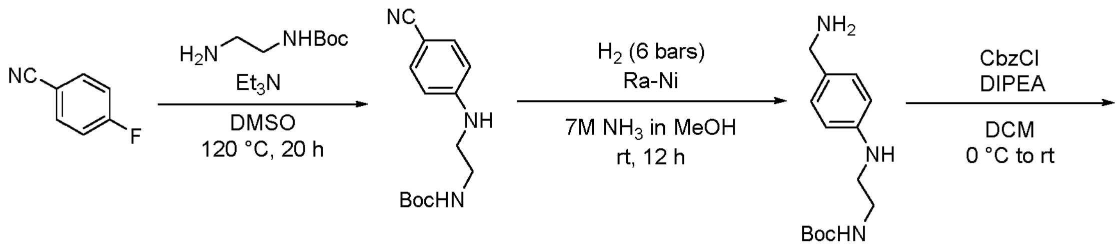


[1194-02-1] Intermediate E1 Intermediate E2

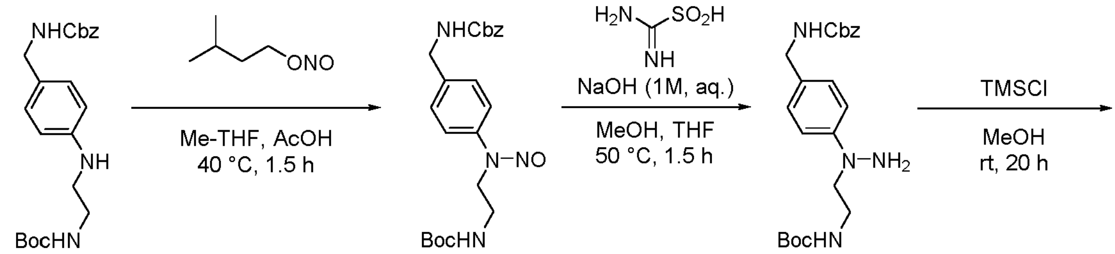


Intermediate E3 Intermediate E4 Intermediate E5

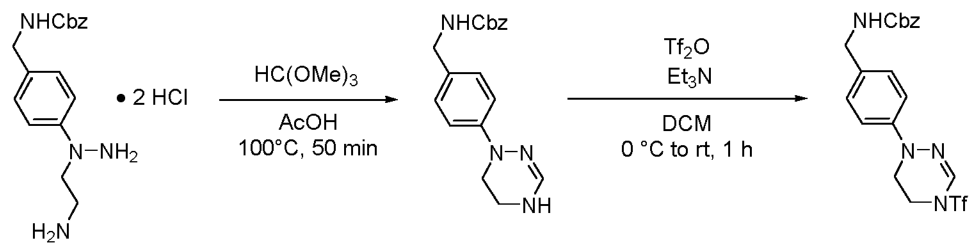


Intermediate E6 Intermediate E7 Intermediate E8

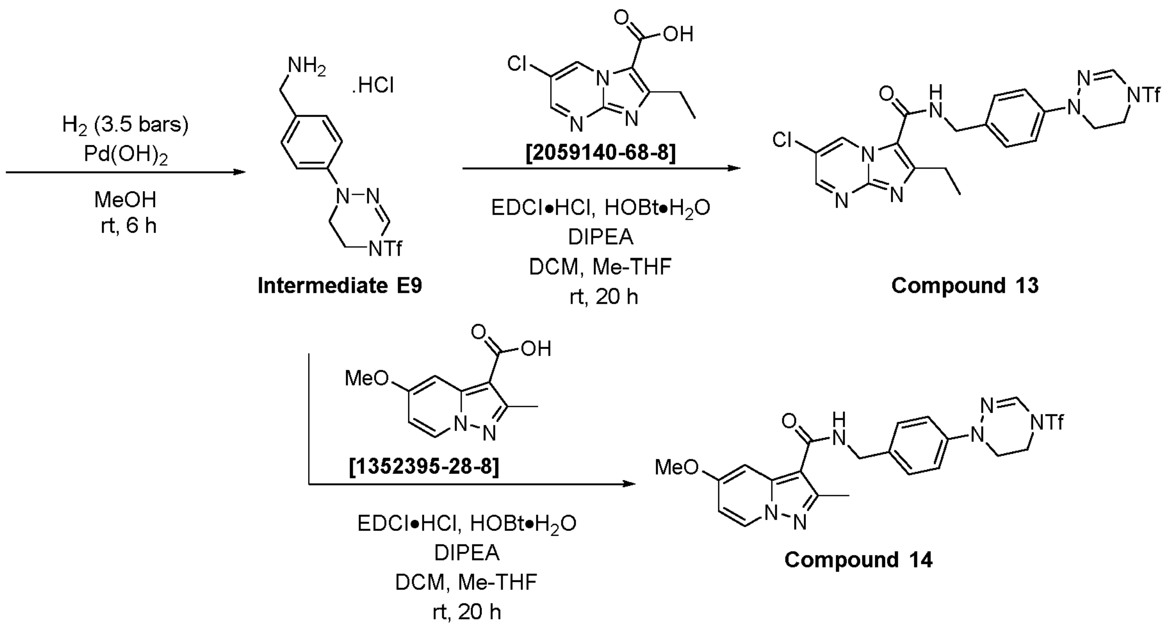


Intermediate E9 Compound 13 Compound 14

Preparation of intermediate **E1**

The reaction was performed on 2 batches. Herein is reported the procedure for one batch. Herein, where "Tf" is used, for avoidance of doubt, it represents $-S(O)_2CH_3$. Further, Intermediate E9 may be prepared and/or employed

as the HCl salt. A 1L flask equipped with a findenser was charged with 4-fluorobenzonitrile [1194-02-1] (20 g, 165 mmol), DMSO (320 mL) and *N*-boc-1,2-diaminoethane (39.7 g, 248 mmol). $Et_3N$ (92 mL, 661 mmol) was added and the reaction mixture was stirred at 120 °C for 20 h. The two batches were combined and poured in a mixture of crushed ice and water (1 L). Brine (1 kg) was added and the mixture was stirred at room temperature for 30 min. EtOAc (1 L) was added. The layers were separated and the aqueous layer was extracted with EtOAc (2 x 500 mL). The combined organic layers were washed with brine (2 × 1 L), dried over $MgSO_4$, filtered and evaporated *in vacuo.* The residue was triturated in pentane (500 mL). The solid was collected by filtration, washed with cold $Et_2O$, and dried under vaccum to give 48.28 g of intermediate **E1** as a white solid (46%, 92% purity).

Preparation of intermediate **E2**

In an 1L autoclave, a mixture of intermediate **E1** (41.5 g, 159 mmol) and Raney-Nickel (4.66 g, 79.4 mmol) in a 7M solution of $NH_3$ in MeOH (500 mL) was hydrogenated at room temperature under 6 bars of $H_2$ for 12 h. The reaction mixture was filtered through a pad of Celite®, washed with a mixture of DCM and MeOH (9/1) and the filtrate was evaporated *in vacuo* to afford 41.8 g of intermediate **E2** as a green oil (99%).

Preparation of intermediate **E3**

Under $N_2$ at 0°C, benzylchloroformate (0.592 mL, 4.15 mmol) was added dropwise to a mixture of intermediate **E2** (1 g, 3.8 mmol) and DIPEA (0.78 mL, 4.52 mmol) in DCM (38 mL). The reaction mixture was stirred at room temperature for 16 h and diluted with DCM. The mixture was washed with $NaHCO_3$ (sat., aq.), dried over $MgSO_4$, filtered and the solvent was removed under reduced pressure to give 1.11 g of intermediate **E3** as a white solid (74%).

Preparation of intermediate **E4**

Intermediate **E3** (1.11 g, 2.78 mmol) was solubilized at 40 °C in Me-THF (21 mL) and AcOH (1.6 mL). Isopentylnitrite (1.87 mL, 13.9 mmol) was added dropwise over 15 min and the reaction mixture was stirred at 40 °C for 1.5 h. The solution was diluted with EtOAc and $NaHCO_3$ (sat., aq.). The layers were separated and the organic phase was washed with $NaHCO_3$ (sat., aq., twice), brine, dried over $MgSO_4$ and evaporated *in vacuo* to give 1.23 g of intermediate **E4** as a pale-yellow solid (Quant.).

Preparation of intermediate **E5**

A solution of intermediate **E4** (1.24 g, 2.89 mmol) in THF (29 mL) and MeOH (19 mL) was treated with NaOH (1M, aq., 29 mL). Thiourea dioxide (formamidinesulfonic acid) (1.56 g, 14.5 mmol) was then added and the reaction mixture was stirred at 50 °C for 1.5 h. The reaction mixture was diluted with DCM and $K_2CO_3$ (10%, aq.) was added. The layers were separated. The aqueous layer was extracted with DCM and MeOH (95/5). The combined organic layers were dried over $MgSO_4$, filtered and evaporated *in vacuo* to give 970 mg of intermediate **E5** as a pale-yellow oil (81%).

Preparation of intermediate **E6**

To a solution of intermediate **E5** (970 mg, 2.34 mmol) in MeOH (23 mL) was added dropwise TMSCl (2.4 mL, 18.7 mmol). The reaction mixture was stirred at room temperature for 20 h and concentrated *in vacuo* to give 710 mg of intermediate **E6** as a brown solid (78%).

Preparation of intermediate **E7**

A mixture of intermediate **E6** (0.71 g, 1.83 mmol) and trimethyl orthoformate (0.602 mL, 5.50 mmol) in AcOH (9.2 mL) was stirred for 50 min at 100 °C. The reaction mixture was concentrated *in vacuo.* The residue was diluted in a solution of DCM and $K_2CO_3$ (10%, aq.). The layers were separated and the aqueous layer was extracted with DCM and MeOH (95/5) (twice). The combined organic layers were dried over $MgSO_4$, filtered and evaporated *in vacuo.* The residue was purified by preparative LC (irregular SiOH 15-40 μm, 40 g, liquid injection (DCM), mobile phase: DCM/MeOH, gradient from 100:0 to 90:10) to give 273 mg of intermediate **E7** as a yellow residue (46%).

Preparation of intermediate **E8**

$Et_3N$ (0.292 mL, 2.10 mmol) was added to a solution of intermediate **E7** (273 mg, 0.842 mmol) in DCM (12 mL). The solution was then cooled to 5 °C and a solution of $Tf_2O$ (1M in DCM, 1.0 mL, 1.0 mmol) was added dropwise

over 5 min. The reaction mixture was stirred for 1 h and diluted with DCM and $NaHCO_3$ (sat., aq.). The layers were separated. The aqueous layer was extracted with DCM (twice). The combined organic layers were dried over $MgSO_4$, filtered and the solvent was removed under reduced pressure. The residue was purified by preparative LC (irregular SiOH 15-40 μm, 12 g, dry loading (Celite®), mobile phase: heptane/EtOAc, gradient from 100:0 to 0:100) to give 105 mg of intermediate **E8** as a white solid (27%).

Preparation of intermediate **E9**

In a steal bomb, a mixture of intermediate **E8** (85 mg, 0.186 mmol) and $Pd(OH)_2$ (21 mg, 0.075 mmol) in MeOH (8.5 mL) was hydrogenated at room temperature under 10 bars of $H_2$ for 6 h. The mixture was filtered on a pad of Celite® and the filtrate was evaporated *in vacuo* to give 65 mg of intermediate **E9** as a white residue (Quant.).

Preparation of Compound **13**

To a mixture of 6-chloro-2-ethyl-imidazo[1,2-a]pyrimidine-3-carboxylic acid [2059140-68-8] (46 mg, 0.202 mmol) and DIPEA (0.070 mL, 0.403 mmol) in DCM (3 mL) and Me-THF (3 mL) were added EDCI•HCl (39 mg, 0.202 mmol), $HOB_t$•$H_2O$ (31 mg, 0.202 mmol) and intermediate **E9** (65 mg, 0.202 mmol). The reaction mixture was stirred at room temperature for 20 h. The reaction mixture was diluted with DCM and washed with $NaHCO_3$ (sat., aq.). The organic layer was dried over $MgSO_4$, filtered and the solvent was removed under reduced pressure. The residue was purified by preparative LC (irregular SiOH 15-40 μm, 12 g, liquid injection (DCM), mobile phase: DCM/MeOH, gradient from 100:0 to 90:10). The solid (70 mg) was triturated and sonicated in $Et_2O$ and the solvent was removed under reduced pressure. The residue (68 mg) was purified by reverse phase (stationary phase: YMC-actus Triart C18 10μm 30* 150mm, mobile phase: $NH_4HCO_3$ (0.2% in water)/MeCN, gradient from 55:45 to 35:65) to give 42 mg of compound **13** as a white solid (39%).

$^1$**H NMR** (400 MHz, DMSO-*$d_6$*) δ ppm 9.40 (d, J=2.69 Hz, 1 H) 8.68 (d, J=2.57 Hz, 1 H) 8.55 (t, J=5.87 Hz, 1 H) 7.32 (m, J=8.68 Hz, 2 H) 7.28 (s, 1 H) 7.19 (m, J=8.68 Hz, 2 H) 4.47 (d, J=5.87 Hz, 2 H) 4.08 (t, J=4.58 Hz, 2 H) 3.83 (t, J=4.77 Hz, 2 H) 3.01 (q, J=7.46 Hz, 2 H) 1.29 (t, J=7.46 Hz, 3 H).

Preparation of compound **14**

Compound **14** was prepared following the procedure reported for the synthesis of compound **13** starting from intermediate **E9** and 5-methoxy-2-methylpyrazolo[1,5-a]pyridine-3-carboxylic acid [1352395-28-8] affording 32 mg as white fluffy solid (40%).

$^1$**H NMR** (400 MHz, DMSO-*$d_6$*) δ ppm 8.50 (d, J=7.46 Hz, 1 H) 7.86 (t, J=5.99 Hz, 1 H) 7.25 - 7.33 (m, 3 H) 7.24 (d, J=2.69 Hz, 1 H) 7.18 (d, J=8.68 Hz, 2 H) 6.63 (dd, J=7.46, 2.81 Hz, 1 H) 4.43 (d, J=5.99 Hz, 2 H) 4.08 (t, J=4.59 Hz, 2 H) 3.85 (s, 3 H) 3.79 - 3.83 (m, 2 H).

**Synthesis of compound 15**

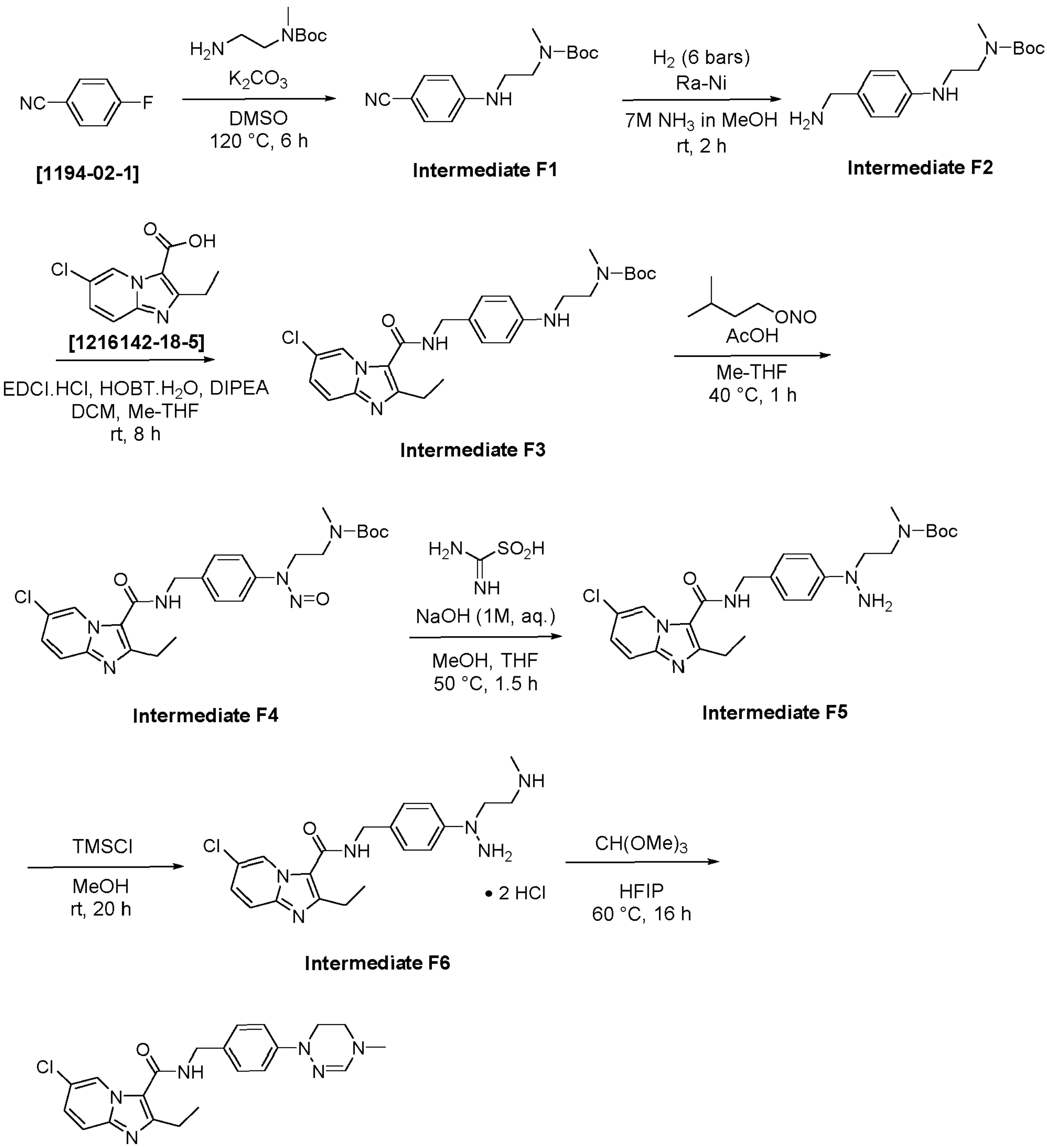


**Compound 15**

Preparation of intermediate **F1**

A mixture of 4-fluorobenzonitrile [1194-02-1] (10.0 g, 82.6 mmol), *N*-boc-*N*-methylethylenediamine (20.2 mL, 116 mmol) and $K_2CO_3$ (13.7 g, 99.1 mmol) in anhydrous DMSO (40 mL) was heated at 120 °C for 6 h. The reaction mixture was poured in brine and EtOAc was added. The layers were separated and the aqueous layer was extracted with EtOAc. The combined organic layers were washed with water and brine, dried over $MgSO_4$, filtered and evaporated *in vacuo.* The crude mixture was purified by preparative LC (irregular SiOH 15-40 μm, 330 g, liquid injection (DCM), mobile phase: heptane/EtOAc, gradient from 90:10 to 30:70) to give 18.04 g of intermediate **F1** as a colorless oil (80 %).

Preparation of intermediate **F2**

In a 1L autoclave, a mixture of intermediate **F1** (17.0 g, 61.7 mmol) and Raney-Nickel (14.5 g, 247 mmol) in

MeOH (330 mL) was stirred at room temperature for 2 h under 6 bars of $H_2$. The mixture was filtered on a pad of Celite®, washed with MeOH and the filtrate was evaporated *in vacuo* to give 17.25 g of intermediate **F2** as a blue/green oil (Quant.).

Preparation of intermediate **F3**

To a mixture of 6-chloro-2-ethylimidazo[1,2-a]pyridine-3-carboxylic acid [1216142-18-5] (2.35 g, 10.0 mmol), intermediate **F2** (3.07 g, 11.0 mmol) and DIPEA (3.45 mL, 20.0 mmol) in DCM (70 mL) and Me-THF (70 mL) were added EDCI•HCl (2.30 g, 12.0 mmol) and $HOB_t$•$H_2O$ (1.62 g, 12.0 mmol). The reaction mixture was stirred at room temperature for 8 h. The mixture was evaporated and the crude mixture was purified by preparative LC (irregular SiOH 15-40 μm, 220 g, dry loading (Celite®), mobile phase: heptane/EtOAc, gradient from 70:30 to EtOAc 0:100) to give 3.703 g of intermediate **F3** as a brown foam (76%).

Preparation of intermediate **F4**

Intermediate **F3** (3.54 g, 7.28 mmol) was solubilized in Me-THF (62 mL) and AcOH (4.17 mL, 72.8 mmol). Isopentyl nitrite (4.89 mL, 36.4 mmol) was added dropwise and the reaction mixture was stirred at 40 °C for 1 h. The resulting solution was diluted in EtOAc. The organic layer was washed with $K_2CO_3$ (10%, aq.) (twice) and brine, dried over $MgSO_4$ and evaporated *in vacuo.* The residue was purified by preparative LC (irregular SiOH 15-40 μm, 80 g, dry loading (Celite®), mobile phase: heptane/EtOAc, gradient from 50:50 to 0:100) to give 3.54 g of intermediate **F4** as an orange paste (94%).

Preparation of intermediate **F5**

A solution of intermediate **F4** (1.13 g, 2.19 mmol) in THF (22 mL) and MeOH (14 mL) was treated *with* NaOH (1M aq., 22 mL, 22 mmol). Formamidinesulfonic acid (1.19 g, 11.0 mmol) was added and the reaction mixture was stirred at 50 °C for 1.5 h. The reaction mixture was diluted in DCM and $K_2CO_3$ (10% aq.) was added. The aqueous layer was extracted with DCM and MeOH (95/5) (twice). The combined organic layers were dried over $MgSO_4$, filtered and evaporated *in vacuo* to give 970 mg of intermediate **F5** as a yellow foam (91% purity, 80%).

Preparation of intermediate **F6**

A solution of intermediate **F5** (932 mg, 1.69 mmol) in MeOH (18 mL) was treated with TMSCl (2.15 mL, 16.9 mmol). The reaction mixture was stirred at room temperature for 20 h and evaporated *in vacuo.* The solid was triturated in $Et_2O$. The supernatant was removed and the yellow powder was dried under vacuum to give 915 mg of intermediate **F6** (Quant.).

Preparation of compound **15**

To a solution of intermediate **F6** (270 mg, 0.570 mmol) in HFIP (4.86 mL) was added trimethyl orthoformate (187 μL, 1.71 mmol) and the reaction mixture was stirred at 60 °C for 16 h. The reaction mixture was diluted with EtOAc and quenched with $K_2CO_3$ (10%, aq.). The organic layer was washed with $H_2O$ (once) and brine (once), dried over $MgSO_4$, filtered and evaporated *in vacuo.* The crude mixture was purified by preparative LC (irregular SiOH 15-40 μm, 12 g, dry loading (Celite®), mobile phase: DCM/(DCM/MeOH, 80:20), gradient from 95:5 to 75:25). The residue was heated under reflux in EtOH for 20 min. The solution was cooled to room temperature and at 0 °C. The mixture was filtered. The solid was rinsed with cold EtOH and dried under vacuum at 60 °C for 7 h to give 51 mg of compound **15** as a beige downy solid (22%). **$^1$H NMR** (400 MHz, DMSO-$d_6$) δ ppm 9.03 (s, 1 H) 8.40 (t, *J*=5.8 Hz, 1 H) 7.66 (d, *J*=9.4 Hz, 1 H) 7.45 (dd, *J*=9.5, 2.08 Hz, 1 H) 7.18 (d, *J*=8.7 Hz, 2 H) 7.10 (d, *J*=8.7 Hz, 2 H) 6.70 (s, 1 H) 4.42 (d, *J*=5.8 Hz, 2 H) 3.51 (t, *J*=5.2 Hz, 2 H) 3.34 (t, *J*=5.2 Hz, 2 H) 2.96 (q, *J*=7.6 Hz, 2 H) 2.83 (s, 3 H) 1.25 (t, *J*=7.5 Hz, 3 H).

**Synthesis of Compound 16**

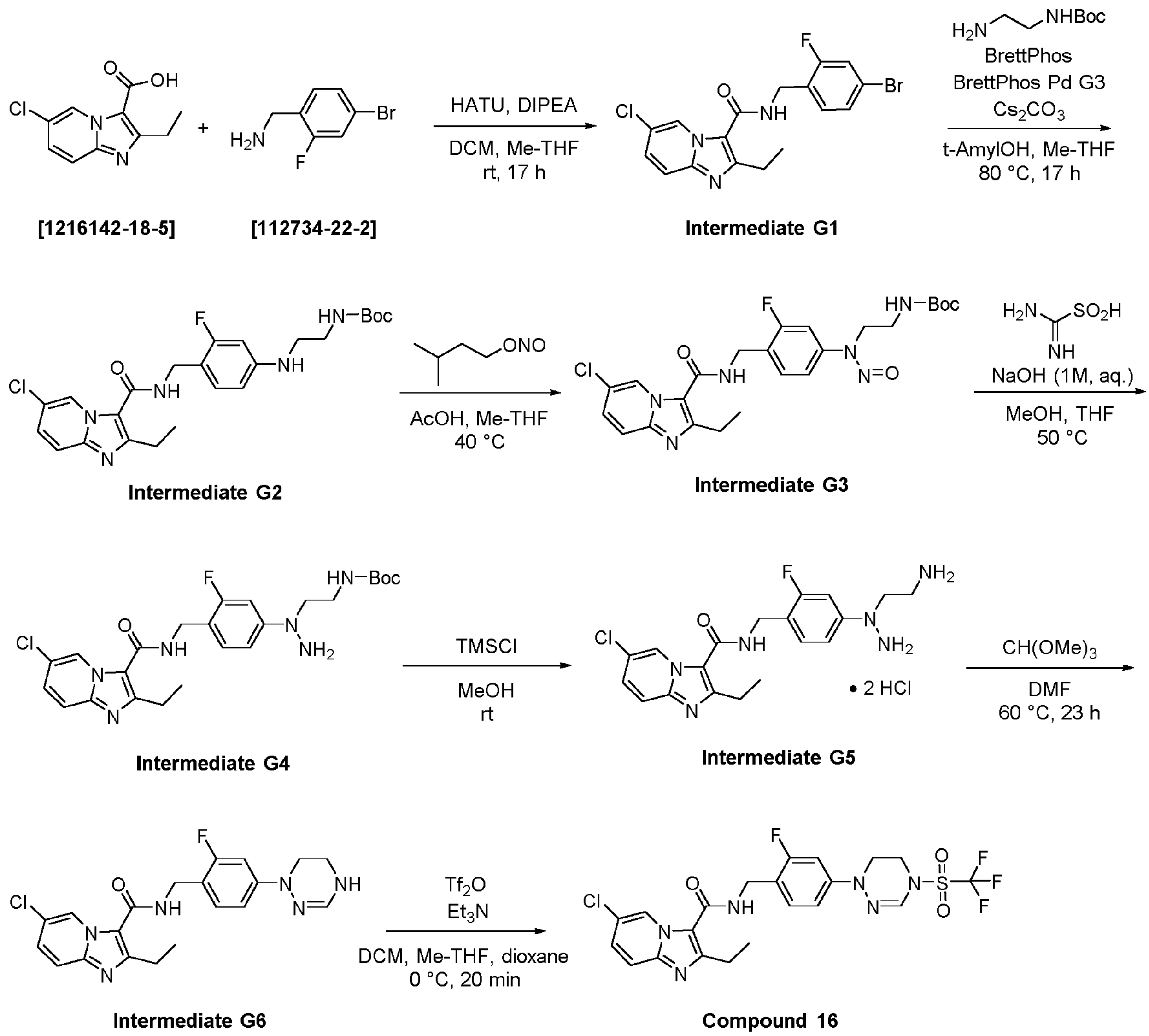


[1216142-18-5] [112734-22-2] Intermediate G1

Intermediate G2 Intermediate G3

Intermediate G4 Intermediate G5

Intermediate G6 Compound 16

Preparation of intermediate **G1**

A flask was charged with 6-chloro-2-ethylimidazo[1,2-a]pyridine-3-carboxylic acid [1216142-18-5] (1.00 g, 4.45 mmol), 4-bromo-2-fluorobenzylamine [112734-22-2] (0.954 g, 4.67 mmol), Me-THF (15 mL), DCM (15 mL) and DIPEA (1.23 mL, 7.12 mmol). HATU (1.86 g, 4.90 mmol) was added portion wise and the reaction mixture was stirred at room temperature for 17 h. The mixture was diluted with EtOAc and water. The layers were separated and the organic layer was washed with brine (twice), dried over $MgSO_4$, filtered and evaporated *in vacuo.* The residue was solubilized in warm EtOAc. The solution was cooled to room temperature and to 0 °C. The suspension was filtered off and the solid was washed with cold EtOAc and then with $Et_2O$. The solid was dried *in vacuo* to afford 773 mg of intermediate **G1** as an off-white solid (42%).

Preparation of intermediate **G2**

A mixture of intermediate **G1** (740 mg, 1.80 mmol), *N*-boc-ethylenediamine (375 mg, 2.34 mmol) and $Cs_2CO_3$ (1.06 g, 3.24 mmol) in tert-Amyl alcohol (24 mL) and Me-THF (16 mL) was purged with $N_2$. Brettphos Pd G3 (82 mg, 0.090 mmol) and Brettphos (97 mg, 0.18 mmol) were added. The reaction mixture was purged again with $N_2$ and stirred for 17 h at 80 °C. The reaction mixture was cooled to room temperature. Celite® was added and the mixture was evaporated *in vacuo.* The residue was purified by preparative LC (irregular SiOH 15-40 μm, 40 g, mobile phase: heptane/EtOAc, gradient from 50:50 0:100) to give 444 mg of intermediate **G2** as a pale-yellow foam (50%).

Preparation of intermediate **G3**

Intermediate **G3** was prepared following the synthesis reported for the synthesis of intermediate **F4** starting from intermediate **G2** and affording 408 mg as a yellow solid (87%).

Preparation of intermediate **G4**

Intermediate **G4** was prepared following the procedure reported for the synthesis of intermediate **F5** starting from intermediate **G3** and affording 362 mg as a beige solid (94%).

Preparation of intermediate **G5**

Intermediate **G5** was prepared following the procedure reported for the synthesis of intermediate **F6** starting from intermediate **G4** and affording 343 mg as a yellow powder (Quant.).

Preparation of intermediate **G6**

A mixture of intermediate **G5** (283 mg, 0.592 mmol) and trimethyl orthoformate (194 μL, 1.78 mmol) in anhydrous DMF (3.7 mL) was stirred for 23 h at 60 °C. Additional amount of anhydrous DMF (3.7 mL) and trimethyl orthoformate (194 μL, 1.78 mmol) were added at room temperature and the reaction mixture was stirred at 60 °C for another 1.5 h. The reaction mixture was diluted with DCM and quenched with $K_2CO_3$ (10%, aq.). The layers were separated and the aqueous layer was extracted with DCM and MeOH (95/5) (twice). The combined organic layers were washed with water and brine, dried over $MgSO_4$, filtered and evaporated *in vacuo.* The crude mixture was purified by preparative LC (irregular SiOH 15-40 μm, 12 g, dry loading (Celite®), mobile phase: DCM/(DCM/MeOH, 80/20), gradient from 95:5 to 70:30) to give 156 mg of intermediate **G6** as a white solid (63%).

Preparation of Compound **16**

Under $N_2$ atmosphere, a mixture of intermediate **G6** (143 mg, 0.345 mmol) and $Et_3N$ (240 μL, 1.72 mmol) in anhydrous DCM (5 mL), anhydrous Me-THF (5 mL) and anhydrous 1,4-dioxane (5 mL) was heated at 40 °C. The reaction mixture was cooled to 0 °C and trifluoromethanesulfonic anhydride (0.517 mL, 0.517 mmol) was added dropwise. The mixture was stirred at 0 °C for 20 min and diluted with DCM. A small quantity of MeOH was added and $K_2CO_3$ (10%, aq.) was added. The layers were separated and the aqueous layer was extracted with DCM (twice). The combined organic layers were washed with water and brine, dried over $MgSO_4$, filtered and evaporated *in vacuo.* The crude mixture was purified by preparative LC (irregular SiOH 15-40 μm, 12 g, dry loading (Celite®), mobile phase: DCM/(DCM/MeOH, 80:20), gradient from 100:0 to 80/20). The residue was purified by reverse phase (stationary phase: YMC-actus Triart C18 10μm 30*150mm, mobile phase: $NH_4HCO_3$ (0.2% in water)/MeCN, gradient from 55:45 to 25:75) to give 84 mg of compound **16** as a white solid (45%).

**$^1$H NMR** (500 MHz, DMSO-$d_6$) δ ppm 9.05 (s, 1 H) 8.40 (t, *J*=5.8 Hz, 1 H) 7.66 (d, *J*=9.5 Hz, 1 H) 7.45 (dd, *J*=9.5, 2.1 Hz, 1 H) 7.36 (t, *J*=8.5 Hz, 1 H) 7.02 (m, 2 H) 7.32 (s, 1 H) 4.50 (d, *J*=5.8 Hz, 2 H) 4.07 (t, *J*=4.7 Hz, 2 H) 3.86 (t, *J*=4.7 Hz, 2 H) 2.96 (q, *J*=7.5 Hz, 2 H) 1.25 (t, *J*=7.5 Hz, 3 H).

**Synthesis of Compound 17**

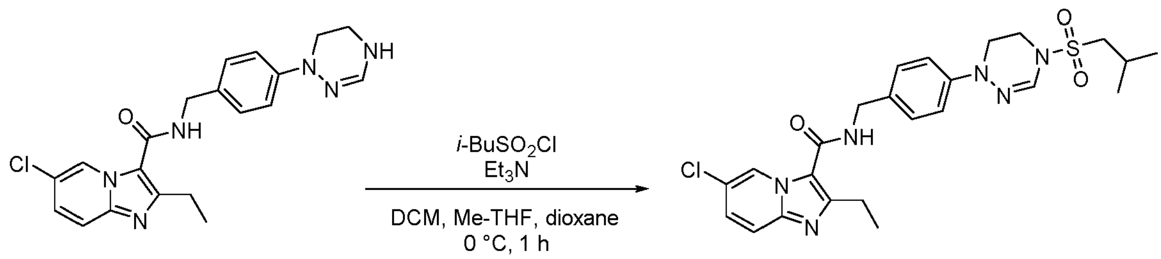


**Intermediate A6** **Compound 17**

Preparation of Compound **17**

Under $N_2$ atmosphere, a mixture of intermediate **A6** (180 mg, 0.454 mmol) and $Et_3N$ (315 µL, 2.27 mmol) in anhydrous Me-THF (7 mL), *anhydrous* 1,4-dioxane (7 mL) and anhydrous DCM (7 mL) was cooled to 0 °C. Isobutanesulfonyl chloride (88.8 µL, 0.680 mmol) was added dropwise. The reaction mixture was stirred for 1 h at 0 °C and diluted with DCM and quenched with $K_2CO_3$ (10%, aq.). The layers were separated and the aqueous layer was extracted with DCM and MeOH (95/5) (twice). The combined organic layers were dried over $MgSO_4$, filtered and evaporated *in vacuo.* The solid was purified by preparative LC (irregular SiOH 15-40 µm, 12 g, dry loading (Celite®), mobile phase: DCM/(DCM:MeOH, 80:20), gradient from 100:0 to 95:5) to give 124 mg of compound **17** as a slightly yellow solid (53%).

**$^1$H NMR** (500 MHz, $CDCl_3$) δ ppm 9.51 - 9.54 (m, 1 H) 7.51 - 7.55 (m, 1 H) 7.32 (d, *J*=8.7 Hz, 2 H) 7.29 (dd, *J*=9.5, 2.0 Hz, 1 H) 7.23 (s, 1 H) 7.18 (d, *J*=8.7 Hz, 2 H) 6.03 (br t, 1 H) 3.71 (t, *J*=4.6 Hz, 2 H) 3.00 (d, *J*=6.6 Hz, 2 H) 2.95 (q, *J*=7.6, 2 H) 2.32 (m, 1 H) 1.39 (t, *J*=7.6 Hz, 3 H) 1.15 (s, 3 H) 1.14 (s, 3 H).

**Synthesis of Compound 18**

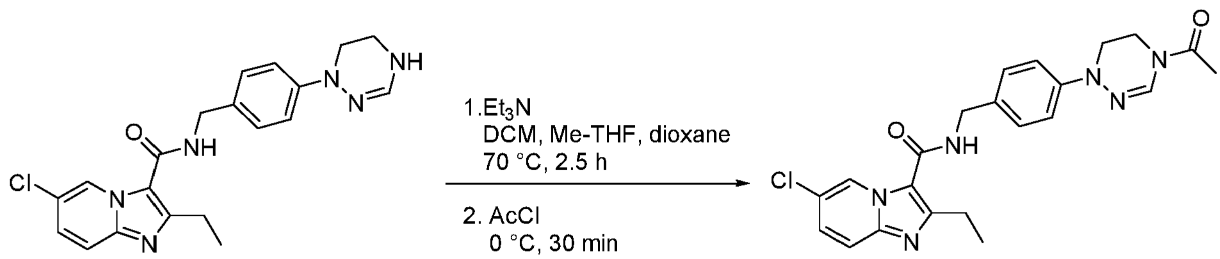


**Intermediate A6** **Compound 18**

Under $N_2$ atmosphere a mixture of intermediate **A6** (300 mg, 0.756 mmol) and $Et_3N$ (0.525 mL, 3.78 mmol) in anhydrous DCM (11.5 mL), anhydrous Me-THF (11.5 mL) and anhydrous 1,4-dioxane (11.5 mL) was stirred for 2.5 h at 70 °C. The mixture was cooled to room temperature and then to 0 °C. Acetyl chloride (53.9 µL, 0.756 mmol) was added dropwise and the reaction mixture was stirred for 30 min at 0 °C. The reaction mixture was diluted with DCM and quenched with MeOH and $K_2CO_3$ (10%, aq.). The layers were separated and the aqueous layer was extracted with DCM and MeOH (95/5) (twice). The combined organic layers were washed with brine, dried over $MgSO_4$, filtered and evaporated *in vacuo.* The residue was purified by preparative LC (irregular SiOH 15-40 µm, 12 g, dry loading (Celite®), mobile phase: DCM/(DCM/MeOH, 80/20), gradient from 95:5 to 85:15) to give 180 mg of compound **18** as a white solid (54%).

**$^1$H NMR** (500 MHz, DMSO-$d_6$) δ ppm rotamers: 9.08 (d, *J*=1.3 Hz, 1 H) 8.17 (br t, *J*=5.4 Hz, 1 H) 7.62 (d, *J*=9.8 Hz, 1 H) 7.58 (br s, 1 H) 7.41 (dd, *J*=9.5, 2.2 Hz, 1 H) 7.30 (d, *J*=8.8 Hz, 2 H) 7.20 (d, *J*=8.5 Hz, 2 H) 4.49 (d, *J*=6.0 Hz, 2 H) 3.86 (br s, 2 H) 3.66 (t, *J*=5.0 Hz, 2 H) 2.99 (q, *J*=7.6 Hz, 2 H) 2.25 (s, 3 H) 1.28 (t, *J*=7.6 Hz, 3 H).

**Synthesis of Compound 19**

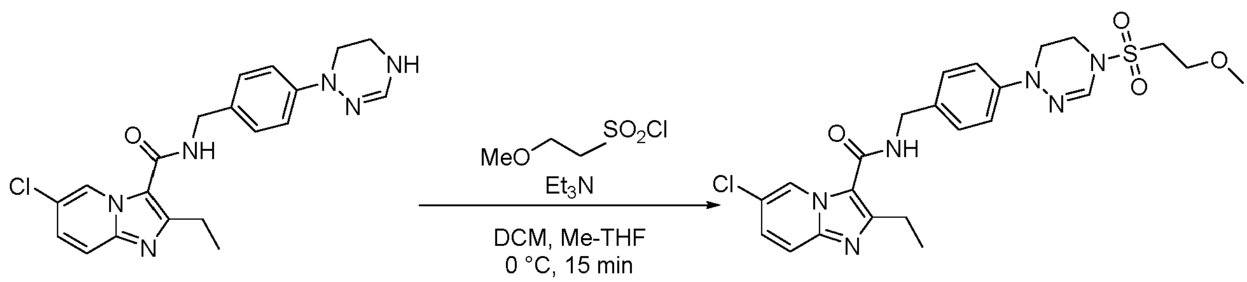


**Intermediate A6** **Compound 19**

To a mixture of intermediate **A6** (100 mg, 0.252 mmol) and $Et_3N$ (0.175 mL, 1.26 mmol) in anhydrous DCM (2.7 mL) and anhydrous Me-THF (2.7 mL) was added 2-methoxy-1-ethanesulfonyl chloride (88.3 µL, 0.756 mmol) at 0

°C and the reaction mixture was stirred at 0 °C for 15 min. The reaction was quenched with a small amount of MeOH and $K_2CO_3$ (10%, aq.) was added. The layers were separated and the aqueous layer was extracted with DCM (twice). The combined organic layers were washed with water (twice) and brine, dried over $MgSO_4$, filtered and evaporated *in vacuo.* The residue was purified by preparative LC (irregular SiOH 15-40 μm, 12 g, dry loading (Celite®), mobile phase: heptane/EtAOc, gradient from 55:45 to 0:100, then EtOAc/MeOH 99:1). The solid was triturated in MeCN, the supernatant was removed and the solid was dried under vacuum to give 53 mg of compound **19** as a white solid (41%).

**$^1$H NMR** (400 MHz, DMSO-$d_6$) δ ppm 9.06 (d, *J*=1.5 Hz, 1 H) 8.43 (t, *J*=5.9 Hz, 1 H) 7.66 (d, *J*=9.5 Hz, 1 H) 7.45 (dd, *J*=9.5, 2.1 Hz, 1 H) 7.28 (d, *J*=8.7 Hz, 2 H) 7.17 (d, *J*=8.7 Hz, 2 H) 7.14 (s, 1 H) 4.45 (d, *J*=5.9 Hz, 2 H) 3.84 (t, *J*=4.3 Hz, 2 H) 3.63 - 3.75 (m, 6 H) 3.24 (s, 3 H) 2.97 (q, *J*=7.5 Hz, 2 H) 1.25 (t, *J*=7.5 Hz, 3 H) 1.09 (t, *J*=7.0 Hz, 1 H).

## Synthesis of Compound 20

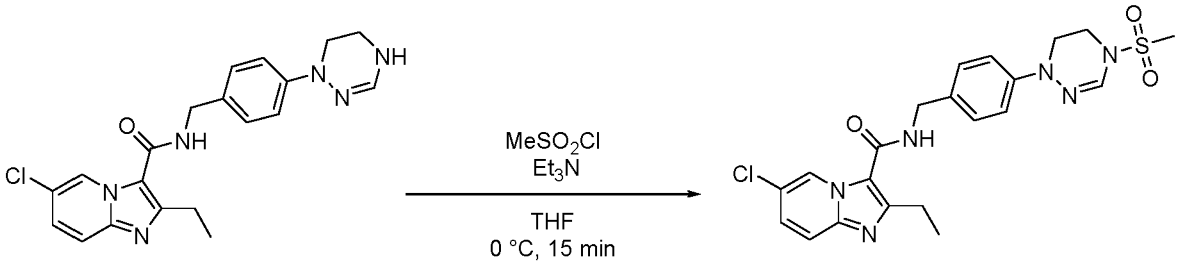


**Intermediate A6** **Compound 20**

A mixture of intermediate **A6** (120 mg, 0.302 mmol) and $Et_3N$ (210 μL, 1.51 mmol) in anhydrous THF (6 mL) was cooled to 0 °C. Methanesulfonyl chloride (46.8 μL, 0.605 mmol) was added dropwise and the reaction mixture was stirred at 0 °C for 15 min. Additional amount of methanesulfonyl chloride (23.4 μL, 0.302 mmol) was added dropwise at 0 °C and the reaction mixture was stirred for another 30 min at 0 °C. The reaction mixture was diluted with DCM and quenched with a small amount of MeOH and $K_2CO_3$ (10%, aq.) was added. The layers were separated and the aqueous layer was extracted with DCM (twice). The combined organic layers were washed with water and brine, dried over $MgSO_4$, filtered and evaporated *in vacuo.* The residue was purified by preparative LC (irregular SiOH 15-40 μm, 12 g, dry loading (Celite®), mobile phase: heptane/EtOAc, gradient from 30:70 to 0:100, then EtOAc/MeOH 99:1). The solid was triturated in EtOAc and the supernatant was removed to give 68 mg of compound **20** as a white solid (47%).

**$^1$H NMR** (400 MHz, DMSO-$d_6$) δ ppm 9.06 (d, *J*=1.6 Hz, 1 H) 8.43 (t, *J*=5.8 Hz, 1 H) 7.66 (d, *J*=9.5 Hz, 1 H) 7.45 (dd, *J*=9.4, 2.08 Hz, 1 H) 7.28 (d, *J*=8.6 Hz, 2 H) 7.19 (s, 1 H) 7.17 (d, *J*=8.8 Hz, 2 H) 4.46 (d, *J*=5.9 Hz, 2 H) 3.86 (t, *J*=5.1 Hz, 2 H) 3.70 (t, *J*=5.1 Hz, 2 H) 3.27 (s, 3 H) 2.97 (d, *J*=7.5 Hz, 2 H) 1.99 (s, 1 H) 1.25 (t, *J*=7.5 Hz, 3 H).

## Synthesis of Compound 21

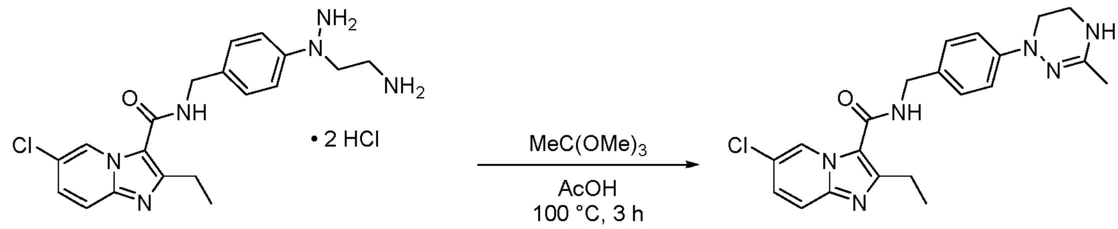


**Intermediate A5** **Intermediate H6**

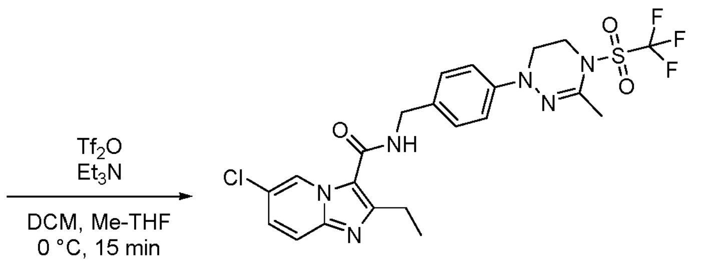


**Compound 21**

Preparation of intermediate **H6**

A mixture of intermediate **A5** (200 mg, 0.435 mmol) and trimethyl orthoacetate (166 μL, 1.31 mmol) in acetic acid (3.6 mL) was stirred for 3 h at 100 °C. The reaction mixture was evaporated in *vacuo.* The residue was diluted with DCM and $K_2CO_3$ (10 %, aq.) was added. The layers were separated and the aqueous layer was extracted with DCM and MeOH (95/5) (twice). The combined organic layers were dried over $MgSO_4$, filtered and evaporated *in vacuo.* The residue was purified by preparative LC (irregular SiOH 15-40 μm, 12 g, dry loading, mobile phase: DCM/MeOH, gradient from 100:0 to 95:5) to give 132 mg of intermediate **H6** as a yellow foam (77% purity, 57%).

Preparation of Compound **21**

To a mixture of intermediate **H6** (133 mg, 0.249 mmol) in anhydrous DCM (2.7 mL) and anhydrous Me-THF (2.5 mL) was added $Et_3N$ (0.17 mL, 1.3 mmol). The mixture was cooled to 0 °C and trifluoromethanesulfonic anhydride (0.75 mL, 0.75 mmol) was added dropwise. The reaction mixture was stirred at 0 °C for 15 min and quenched with a small amount of MeOH and $K_2CO_3$ (10 %, aq.). The layers were separated and the aqueous phase was extracted with DCM (twice). The combined organic extracts were washed with brine, dried over $MgSO_4$, filtered and evaporated *in vacuo.* The residue was purified by preparative LC (irregular SiOH 15-40 μm, 12 g, dry loading (Celite®), mobile phase: heptane/EtAOc, gradient from 80:20 to 0:100). A second purification was performed via reverse phase (stationary phase: YMC-actus Triart C18 10μm 30* 150mm, mobile phase: $NH_4HCO_3$ (0.2% in water)/MeCN, gradient from 40:60 to 10:90) to give 52 mg of compound **21** as an off-white solid (38%).

$^1$**H NMR** (500 MHz, DMSO-$d_6$) δ ppm 9.06 (d, *J*=1.6 Hz, 1 H) 8.44 (s, 1 H) 7.66 (d, *J*=9.5 Hz, 1 H) 7.45 (dd, *J*=9.6, 2.1 Hz, 1 H) 7.30 (d, *J*=8.8 Hz, 2 H) 7.16 (d, *J*=8.8 Hz, 2 H) 4.46 (d, *J*=6.0 Hz, 2 H) 4.00 (t, *J*=5.4 Hz, 2 H) 3.82 (t, *J*=5.4 Hz, 2 H) 2.97 (q, *J*=5.6 Hz, 2 H) 2.26 (s, 3 H) 1.25 (t, *J*=7.6 Hz, 3 H).

**Synthesis of Compound 22**

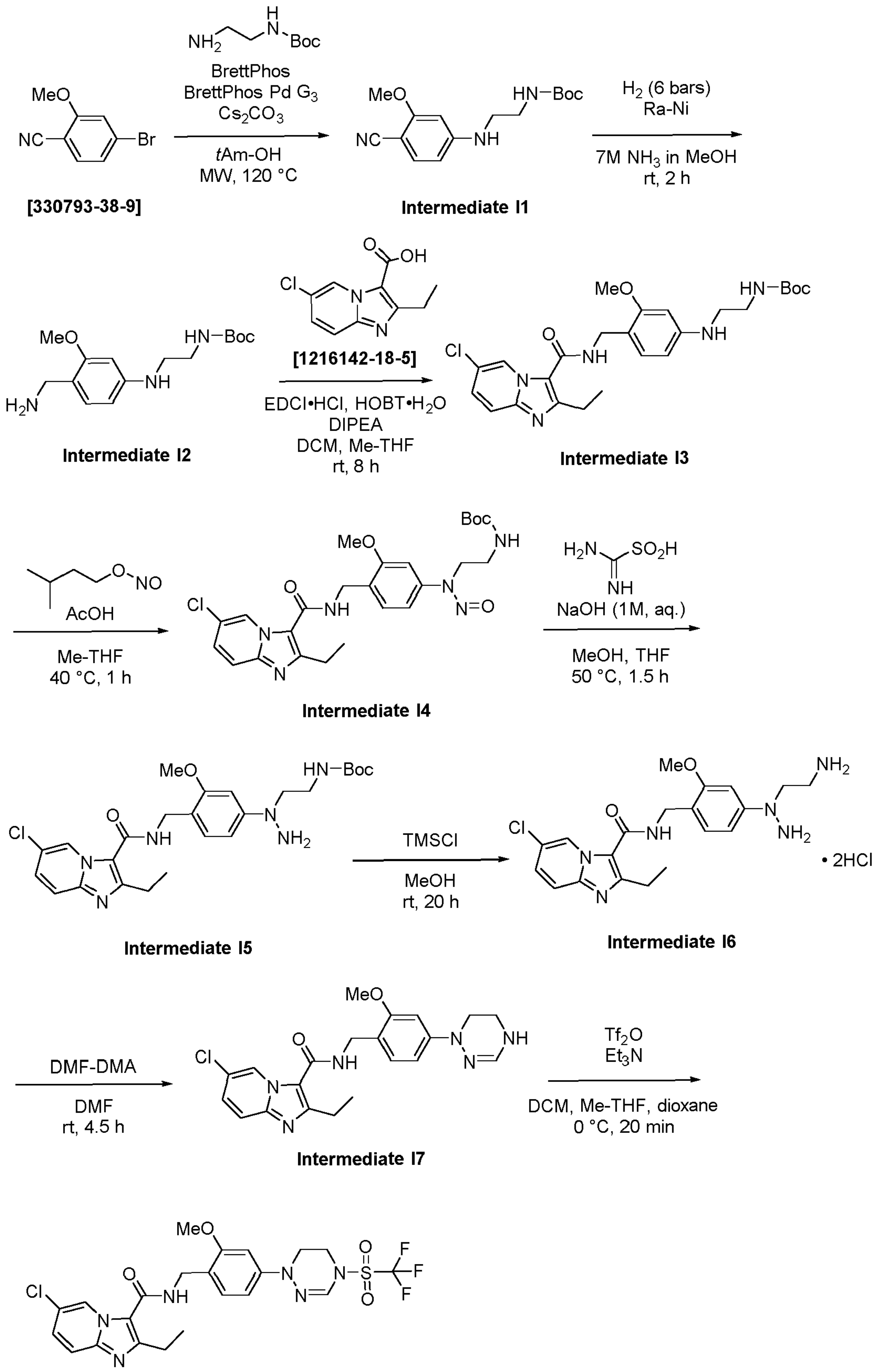


[330793-38-9]

Intermediate I1

Intermediate I2

Intermediate I3

Intermediate I4

Intermediate I5

Intermediate I6

Intermediate I7

**Compound 22**

Preparation of intermediate **I1**

A mixture of 4-bromo-2-methoxybenzonitrile [330793-38-9] (1.55 g, 7.31 mmol), N-boc-ethylenediamine (1.76 g, 11.0 mmol) and $Cs_2CO_3$ (4.76 g, 14.6 mmol) in anhydrous *tert*-amyl alcohol (46 mL) was purged with $N_2$. Brettphos Pd G3 (331 mg, 0.365 mmol) and Brettphos (392 mg, 0.731 mmol) were added and the reaction mixture was heated at 120 °C using a single mode microwave (Biotage Initiator60) for 1 h, and then for another 45 min. The two batches were filtered on a pad of Celite® and the filtrate was evaporated *in vacuo.* The residue was purified by preparative LC (irregular SiOH 15-40 μm, 120 g, dry loading (Celite®), mobile phase: heptane/EtAOc, gradient from 90:10 to 0:100) to give 1.64 g of intermediate **I1** (74%).

Preparation of intermediate **I2**

Intermediate **I2** was prepared following the procedure reported for the synthesis of intermediate **F2** starting from intermediate **I1** and affording 1.55 g of a grey oil (94%).

Preparation of intermediate **I3**

Intermediate **I3** was prepared following the procedure reported for the synthesis of intermediate **F3** starting from intermediate **I2** and affording 765 mg of a beige solid (62%).

Preparation of intermediate **I4**

Intermediate **I4** was prepared following the procedure reported for the synthesis of intermediate **F4** starting from intermediate **I3** and affording 724 mg of a yellow solid (90%).

Preparation of intermediate **I5**

Intermediate **I5** was prepared following the procedure reported for the synthesis of intermediate **F5** starting from intermediate **I4** and affording 692 mg of a beige foam (99%).

Preparation of intermediate **I6**

Intermediate **E6** was prepared following the procedure reported for the synthesis of intermediate **F6** starting from intermediate **I5** and affording 710 mg of a beige solid (Quant.).

Preparation of intermediate **I7**

A solution of intermediate **I6** (270 mg, 0.551 mmol) and *N,N*-dimethylformamide dimethyl acetal (73.8 μL, 0.551 mmol) in anhydrous DMF (3.4 mL) was stirred at room temperature for 4.5 h. The reaction mixture was diluted with DCM and quenched $K_2CO_3$ (10%, aq.). The layers were separated and the aqueous phase was extracted with DCM and MeOH (95/5) (twice). The combined organic layers were dried over $MgSO_4$, filtered and evaporated *in vacuo.* The residue was purified by preparative LC (irregular SiOH 15-40 μm, 12 g, dry loading (Celite®), mobile phase: DCM/(DCM:MeOH, 80/20), gradient from 95:5 to 85:15) to give 100 mg of intermediate **I7** as a white solid (42%).

Preparation of Compound **22**

Under $N_2$ atmosphere and at 0 °C, to a mixture of intermediate **I7** (92.0 mg, 0.216 mmol) and $Et_3N$ (150 μL, 1.08 mmol) in anhydrous DCM (3.1 mL), anhydrous Me-THF (3.1 mL) and anhydrous 1,4-dioxane (3.1 mL) was added dropwise trifluoromethanesulfonic anhydride (0.323 mL, 0.323 mmol). The reaction mixture was stirred at 0 °C for 10 min, and diluted with DCM and $K_2CO_3$ (10%, aq.). The layers were separated and the aqueous phase was extracted with DCM and MeOH (95/5) (twice). The combined organic extracts were dried over $MgSO_4$, filtered and evaporated *in vacuo.* The residue was purified by preparative LC (irregular SiOH 15-40 μm, 12 g, dry loading (Celite®), mobile phase: DCM/(DCM/MeOH, 95/5), gradient from 100:0 to 80/20). The solid was triturated in EtOAc. The supernatant was removed and the white solid was dried under vacuum for 1 h at 60 °C to give 28 mg of compound **22** (23%).

**$^1$H NMR** (400 MHz, DMSO-$d_6$) δ ppm 9.04 (d, *J*=1.5 Hz, 1 H) 8.23 (t, *J*=5.7 Hz, 1 H) 7.66 (d, *J*=9.7 Hz, 1 H) 7.45 (dd, *J*=9.5, 2.1 Hz, 1 H) 7.31 (s, 1 H) 7.19 (d, *J*=8.3 Hz, 1 H) 6.93 (d, *J*=2.0 Hz, 1 H) 6.70 (dd, *J*=8.3, 2.0 Hz, 1 H) 4.43 (d, *J*=5.7 Hz, 2 H) 4.07 (br d, *J*=4.6 Hz, 2 H) 3.86 (br d, *J*=5.3 Hz, 2 H) 3.84 (s, 3H) 2.96 (d, *J*=7.5 Hz, 2 H) 1.25 (t, *J*=7.5 Hz, 3 H).

**Synthesis of Compound 23**

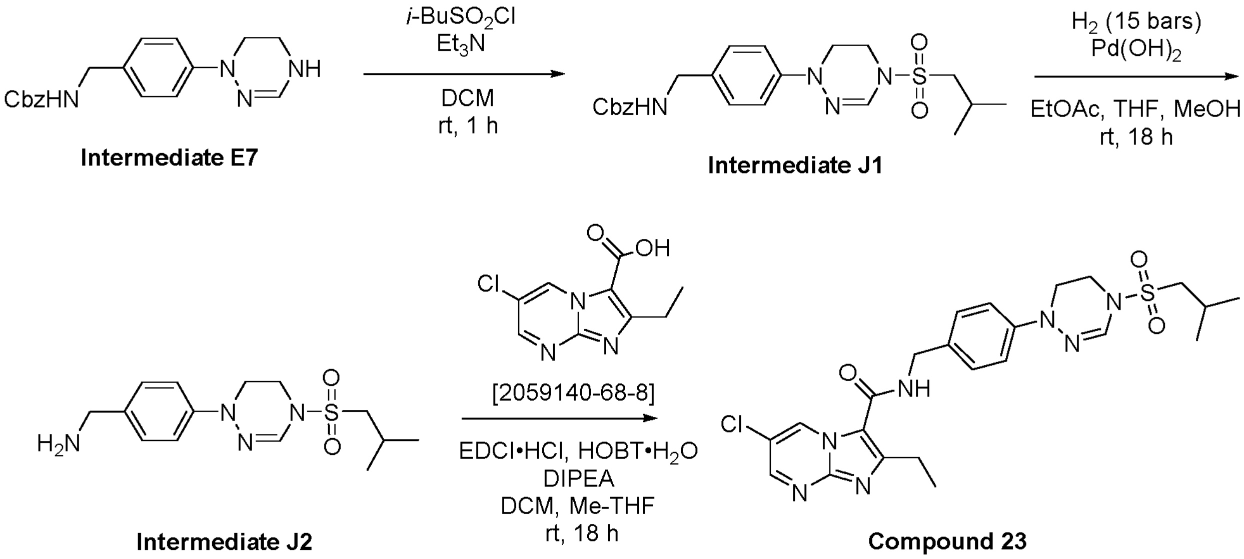


Preparation of intermediate **J1**

To a mixture of intermediate **E7** (400 mg, 1.23 mmol) and $Et_3N$ (0.857 mL, 6.17 mmol) in anhydrous DCM (18 mL) was added isobutanesulfonyl chloride (0.161 mL, 1.23 mmol) dropwise at 0 °C. The reaction mixture was stirred at room temperature for 1 h. The reaction was quenched with $NaHCO_3$ (sat., aq.). The layers were separated and the aqueous phase was extracted with DCM and MeOH (95/5) (twice). The combined organic extracts were dried over $MgSO_4$, filtered and evaporated *in vacuo.* The residue was purified by preparative LC (irregular SiOH 15-40 μm, 24 g, dry loading (Celite®), mobile phase: heptane/EtOAc, gradient from 100:0 to 0:100, then mobile phase EtOAc/MeOH, gradient from 100:0 to 95:5) to give 406 mg of intermediate **J1** as a green solid (74%).

Preparation of intermediate **J2**

A mixture of intermediate **J1** (406 mg, 0.913 mmol) and $Pd(OH)_2$ (264 mg, 0.941 mmol) in MeOH (20 mL), EtOAc (20 mL) and THF (5 mL) was stirred at room temperature under 15 bar of $H_2$ for 18 h. The reaction mixture was filtered off and rinsed with MeOH, EtOAc and THF. The filtrate was evaporated *in vacuo* to give 180 mg of intermediate **J2** as a yellow solid (60%).

Preparation of compound **23**

A mixture of 6-chloro-2-ethyl-imidazo[1,2-a]pyrimidine-3carboxylic acid [2059140-68-8] (113 mg, 0.501 mmol), intermediate **J2** (180 mg, 0.551 mmol), EDCI•HCl (96.0 mg, 0.501 mmol), $HOB_t$•$H_2O$ (76.7 mg, 0.501 mmol) and DIPEA (431 μL, 2.50 mmol) in DCM (10 mL) and Me-THF (6 mL) was stirred at room temperature for 18 h. The reaction mixture was diluted with DCM and washed with water (twice) and brine. The organic phase was dried over $MgSO_4$, filtered and evaporated *in vacuo.* The residue was purified by preparative LC (irregular SiOH 15-40 μm, 12 g, dry loading (Celite®), mobile phase: heptane/EtAOc, gradient from 90:10 to 0:100, then mobile phase: EtOAc/MeOH, gradient from 100:0 to 95:5) to give 101 mg of compound **23** as a slightly yellow solid (39%).

**$^1$H NMR** (500 MHz, DMSO-$d_6$) δ ppm 9.39 (d, *J*=2.8 Hz, 1 H) 8.67 (d, *J*=2.6 Hz, 1 H) 8.51 (t, *J*=6.0 Hz, 1 H) 7.28 (d, *J*=8.7 Hz, 2 H) 7.19 (s, 1 H), 7.17 (d, *J*=8.8 Hz, 3 H) 4.46 (d, *J*=6.0 Hz, 2 H) 3.86 (t, *J*=4.8 Hz, 2 H) 3.69 (t, *J*=4.9 Hz, 2 H) 3.32 (d, *J*=6.6 Hz, 3 H) 3.01 (q, *J*=7.5 Hz, 2 H) 2.13 (m, 1 H) 1.27 (t, *J*=7.6 Hz, 3 H) 1.06 (s, 3 H) 1.04 (s, 3 H).

**Synthesis of Compound 24**

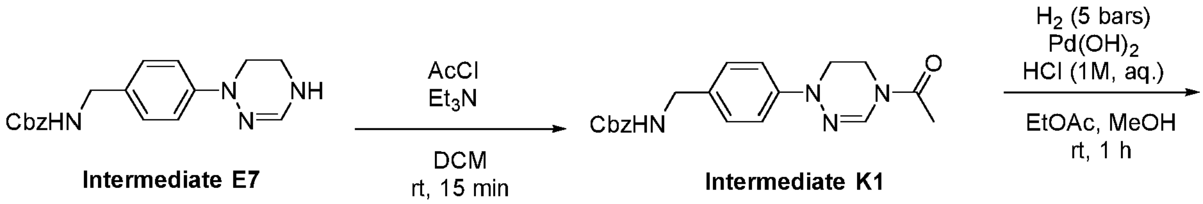


Intermediate E7 Intermediate K1

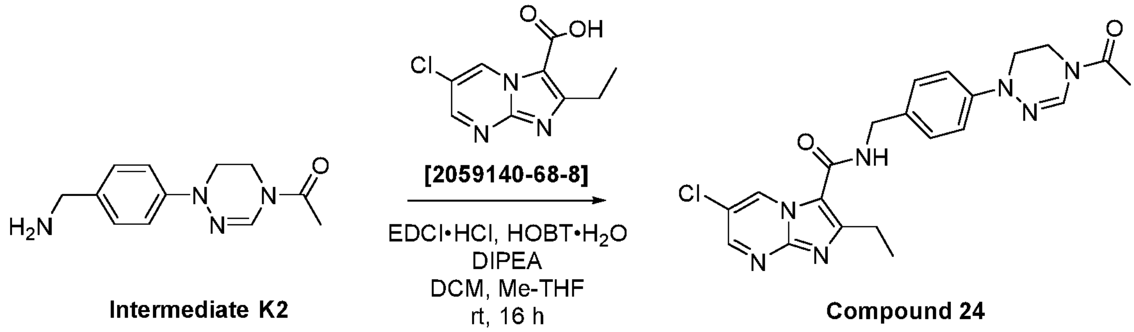


Intermediate K2 Compound 24

Preparation of intermediate **K1**

To a mixture of intermediate **E7** (550 mg, 1.70 mmol) and $Et_3N$ (1.18 mL, 8.48 mmol) in anhydrous DCM (24 mL) at 0 °C was added acetyl chloride (0.145 mL, 2.04 mmol) dropwise. The reaction mixture was stirred at room temperature for 15 min, and the reaction was quenched with NaHCOs (sat., aq.). The layers were separated and the aqueous phase was extracted with DCM and MeOH (95/5) (twice). The combined organic extracts were dried over $MgSO_4$, filtered, and evaporated *in vacuo.* The residue was triturated in EtOAc and the solid was collected by filtration to afford 320 mg of intermediate **K1** as a slightly yellow solid (52%).

Preparation of intermediate **K2**

A mixture of intermediate **K1** (256 mg, 0.698 mmol), $Pd(OH)_2$ (157 mg, 0.558 mmol) and HCl (1M in $H_2O$, 0.698 mL, 0.698 mmol) in MeOH (6.4 mL) and EtOAc (6.4 mL) was stirred at room temperature under 5 bars of $H_2$ for 1 h. The reaction mixture was filtered and rinsed with EtOAc and MeOH. The yellow solid was purified by preparative LC (irregular SiOH 15-40 μm, 12 g, dry loading (Celite®), mobile phase DCM/(DCM/MeOH/$NH_3$ aq., 80/20/0.5), gradient from 100:0 to 70:30) to give 130 mg of intermediate **K2** (75%).

Preparation of compound **24**

To a mixture of 6-chloro-2-ethyl-imidazo[1,2-a]pyrimidine-3-carboxylic acid [2059140-68-8] (98.5 mg, 0.436 mmol), intermediate **K2** (129 mg, 0.480 mmol) and DIPEA (752 μL, 4.36 mmol) in DCM (8.8 mL) and Me-THF (5.2 mL) were added EDCI•HCl (83.7 mg, 0.436 mmol) and $HOB_t$•$H_2O$ (66.8 mg, 0.436 mmol). The reaction mixture was stirred at room temperature for 16 h, filtered and the solid was washed with DCM to give 114 mg of compound **24** as a slightly yellow downy solid (59%).

**$^1$H NMR** (500 MHz, DMSO-$d_6$) δ ppm 9.38 (d, *J*=2.2 Hz, 1 H) 8.61 (d, *J*=2.5 Hz, 1 H) 8.26 (br t, *J*=6.0 Hz, 1 H) 7.56 (br s, 1 H) 7.28 (br d, *J*=8.5 Hz, 2 H) 7.18 (d, *J*=8.5 Hz, 2 H) 4.47 (d, *J*=5.7 Hz, 2 H) 3.84 (br s, 2 H) 3.64 (t, *J*=5.0 Hz, 2 H) 3.01 (q, *J*=7.6 Hz, 3 H) 2.23 (br s, 3 H) 1.28 (t, J=7.4 Hz, 3 H).

**Synthesis of Compound 25**

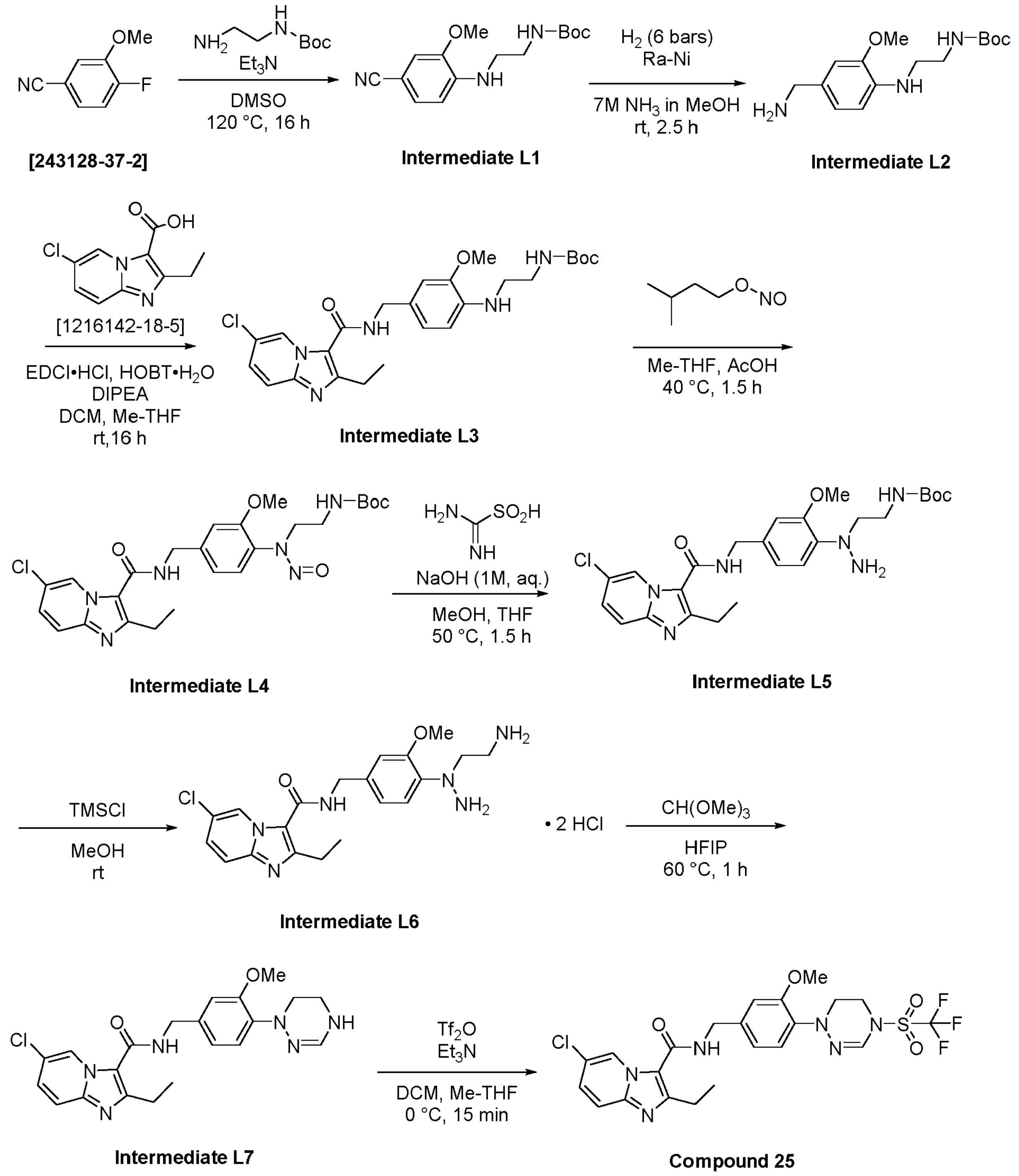


**[243128-37-2]** **Intermediate L1** **Intermediate L2** **Intermediate L3** **Intermediate L4** **Intermediate L5** **Intermediate L6** **Intermediate L7** **Compound 25**

Preparation of intermediate **L1**

To a mixture of 4-fluoro-3-methoxy-benzonitrile [243128-37-2] (4.88 g, 32.3 mmol) and *N*-boc-ethylenediamine (18.0 mL, 0.129 mol) in DMSO (58 mL) was added Et$_3$N (6.65 mL, 42.0 mmol). The reaction mixture was stirred at 120 °C for 16 h. The reaction mixture was cooled down and poured in brine. EtOAc was added. The layers were separated and the aqueous phase was extracted with EtOAc (twice). The combined organic extracts were washed with a mixture of water and brine (1/1) (3 times), dried over MgSO$_4$, filtered and evaporated *in vacuo.* The residue was purified by preparative LC (irregular SiOH 15-40 μm, 330 g, dry loading (Celite®), mobile phase: heptane/EtOAc, gradient from 100:0 to 30:70) to give 5.23 g of intermediate **L1** as a white solid (56%).

Preparation of intermediate **L2**

Intermediate **L2** was synthesized according to the procedure reported for the synthesis of intermediate **F2** starting from intermediate **L1** and affording 1.09 g of a green oil (Quant.).

Preparation of intermediate **L3**

To a mixture of 6-chloro-2-ethylimidazo[1,2-a]pyridine-3-carboxylic [1216142-18-5] (701 mg, 3.12 mmol), intermediate **L2** (1.01 g, 3.43 mmol) and DIPEA (2.69 mL, 15.6 mmol) in DCM (60 mL) and Me-THF (40 mL) were added EDCI•HCl (598 mg, 3.12 mmol) and HOBt•$H_2O$ (478 mg, 3.12 mmol). The reaction mixture was stirred at room temperature for 16 h and diluted with DCM and water. The layers were separated and the aqueous phase was extracted with DCM (twice). The combined organic extracts were washed with brine (twice), dried over $MgSO_4$, filtered and evaporated *in vacuo.* The residue was purified by preparative LC (irregular SiOH 15-40 $\mu$m, 80 g, dry loading (Celite®), mobile phase: heptane/EtOAc, gradient from 60:40 0:100) to give 1.078 g of intermediate **L3** as a yellow solid (69%).

Preparation of intermediate **L4**

Intermediate **L3** (1.08 g, 2.15 mmol) was solubilized in Me-THF (21 mL) and acetic acid (1.23 mL, 21.5 mmol). Isopentyl nitrite (1.44 mL, 10.7 mmol) was added dropwise and the reaction mixture was stirred at 40 °C for 1.5 h. The reaction mixture was diluted with EtOAc and NaHCOs (sat., aq.). The layers were separated. The organic phase was washed with NaHCOs (sat., aq.) (twice) and brine, dried over $MgSO_4$, filtered and evaporated *in vacuo.* The residue was triturated in pentane and the supernatant was removed to give a yellow solid which was dried under vacuum to afford 1.127 g of intermediate **L4** (99%).

Preparation of intermediate **L5**

Intermediate **L5** was prepared following the procedure reported for the synthesis of intermediate **F5** starting from intermediate **L4** and affording 1.07 g of an orange foam (97%).

Preparation of intermediate **L6**

Intermediate **L6** was prepared following the procedure reported for the synthesis of intermediate **F6** starting from intermediate **L5** and affording 1.10 g of a yellow powder (Quant.).

Preparation of intermediate **L7**

A mixture of intermediate **L6** (600 mg, 1.14 mmol) and trimethyl orthoformate (374 $\mu$L, 3.42 mmol) in HFIP (10.8 mL) was stirred at 60 °C for 1 h. The reaction mixture was diluted with EtOAc and quenched with $K_2CO_3$ (10%, aq.). The layers were separated and the organic phase was washed with $H_2O$ and brine, dried over $MgSO_4$, filtered and evaporated *in vacuo.* The residue was purified by preparative LC (irregular SiOH 15-40 $\mu$m, 25 g, dry loading (Celite®), mobile phase: DCM/(DCM/MeOH, 80/20), gradient from 100:0 to 50:50) to give 290 mg of intermediate **L7** as a slightly orange solid (60%).

Preparation of Compound **25**

To a mixture of intermediate **L7** (290 mg, 0.679 mmol) and $Et_3N$ (0.472 mL, 3.40 mmol) in anhydrous DCM (10 mL) and anhydrous Me-THF (10 mL) was added dropwise trifluoromethanesulfonic anhydride (0.815 mL, 0.815 mmol) at 0 °C. The reaction mixture was stirred at 0 °C for 15 min and diluted with DCM. A small amount of MeOH and $K_2CO_3$ (10%, aq.) were successively added. The layers were separated and the aqueous phase was extracted with DCM and MeOH (95/5) (twice). The combined organic extracts were washed with water and brine, dried over $MgSO_4$, filtered and evaporated. The residue was purified by preparative LC (irregular SiOH 15-40 $\mu$m, 25 g, dry loading (Celite®), mobile phase: heptane/EtOAc, gradient from 70:30 to 0: 100). The yellow solid was triturated in $Et_2O$, sonicated and collected by filtration to give 135 mg of compound **25** as a beige solid (36%).

**$^1$H NMR** (500 MHz, DMSO-$d_6$) $\delta$ ppm 9.06 (d, *J*=1.6 Hz, 1 H) 8.47 (br t, *J*=6.0 Hz, 1 H) 7.66 (d, *J*=9.5 Hz, 1 H) 7.46 (dd, *J*=9.5, 2.2 Hz, 1 H) 7.29 (s, 1 H) 7.21 (d, *J*=7.9 Hz, 1 H) 7.08 (s, 1 H) 6.96 (d, *J*=7.9 Hz, 1 H) 4.52 (d, *J*=6.0 Hz, 2 H) 4.06 (br t, *J*=4.4 Hz, 2 H) 3.82 (s, 3 H) 3.55 (br t, *J*=4.7 Hz, 2 H) 3.01 (d, *J*=7.6 Hz, 2 H) 1.27 (t, *J*=7.6 Hz, 3 H).

**Synthesis of Compound 26**

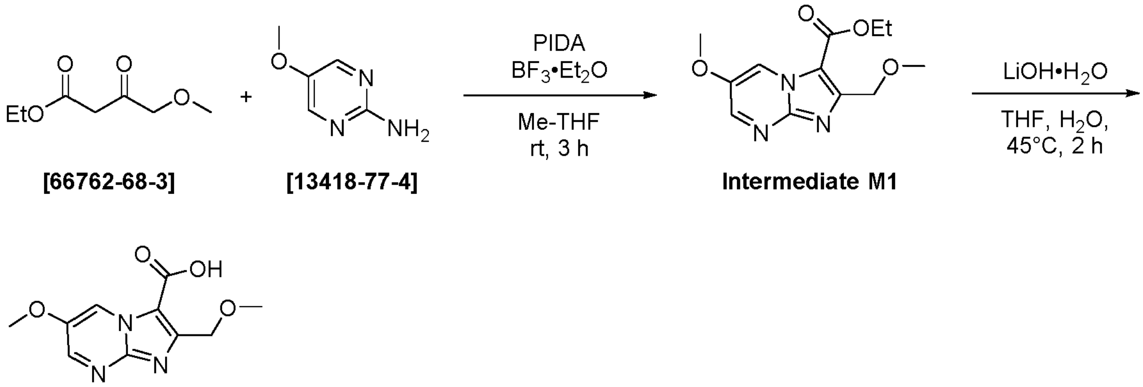


**[66762-68-3]** **[13418-77-4]** **Intermediate M1**

**Intermediate M2**

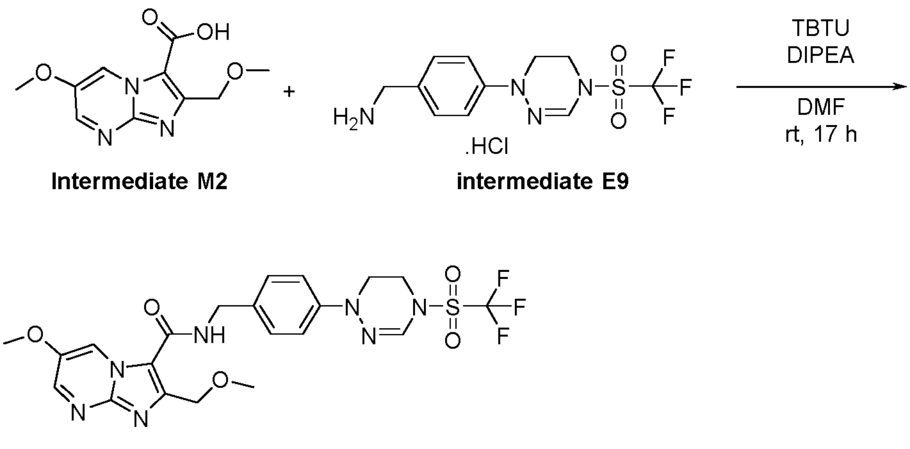


**Intermediate M2** **intermediate E9**

**Compound 26**

Preparation of intermediate **M1**

To a mixture of 2-amino-5-methoxypyrimidine [13418-77-4] (4.75 g, 38.0 mmol), ethyl-3-oxovaleraethyl-3-oxovalerate [4949-44-4] (9.48 mL, 66.4 mmol) and (diacetoxyiodo)benzene (iodobenzenediacteate) (12.2 g, 38.0 mmol) in anhydrous Me-THF (150 mL) was added boron trifluoride etherate (0.993 mL, 3.80 mmol) dropwise. The reaction mixture was stirred at room temperature for 3 h. The two batches were combined and the mixture was diluted with EtOAc. NaHCOs (sat., aq.) was added. The layers were separated and the organic phase was washed with brine, dried over $MgSO_4$, filtered and concentrated *in vacuo.* The residue was purified by preparative LC (irregular SiOH 15-40 μm, 330 g, liquid injection (DCM), mobile phase: heptane/EtOAc, gradient from 85:15 to 50:50) to give 4.94 g of intermediate **M1** as a yellow solid (26%).

Preparation of intermediate **M2**

To a solution of intermediate **M1** (500 mg, 2.01 mmol) in THF (10 mL) was added a solution of LiOH•$H_2O$ (253 mg, 6.02 mmol) in water (5 mL). The reaction mixture was stirred for 2 h at 45 °C, cooled to room temperature and HCl (1M, aq., 6 mL) was added followed by EtOAc. The layers were separated and the aqueous phase was extracted with DCM, then with a mixture of DCM and MeOH (95/5). The combined organic extracts were dried over $MgSO_4$, filtered and evaporated *in vacuo* to afford 80 mg of intermediate **M2** (18%).

Preparation of Compound **26**

To a mixture of intermediate **M2** (80 mg, 0.362 mmol) and intermediate **E9** (117 mg, 0.362 mmol) in DMF (2.44

mL) were successively added DIPEA (0.156 mL, 0.904 mmol) and TBTU (128 mg, 0.398 mmol). The reaction mixture was stirred at room temperature for 17 h. The reaction mixture was poured in EtOAc. The organic phase was washed with brine (twice), dried over $MgSO_4$, filtered and evaporated *in vacuo.* The residue was purified by preparative LC (irregular SiOH 15-40 μm, 24 g, liquid injection (DCM), mobile phase: heptane/EtOAc, gradient from 50:50 to 0:100) to give 78 mg of compound **26** as a white solid (41%).

**$^1$H NMR** (400 MHz, DMSO-$d_6$) δ ppm 9.40 (d, J=2.57 Hz, 1 H) 8.68 (d, J=2.69 Hz, 1 H) 8.53 (t, J=5.87 Hz, 1 H) 7.30 (d, J=8.68 Hz, 2 H) 7.15 (d, J=8.68 Hz, 2 H) 4.46 (d, J=5.87 Hz, 2 H) 4.06 - 4.18 (m, 2 H) 3.85 (s, 3 H) 3.69 - 3.78 (m, 2 H) 3.01 (q, J=7.54 Hz, 2 H) 1.27 (t, J=7.52 Hz, 3 H).

## Synthesis of Compound 27

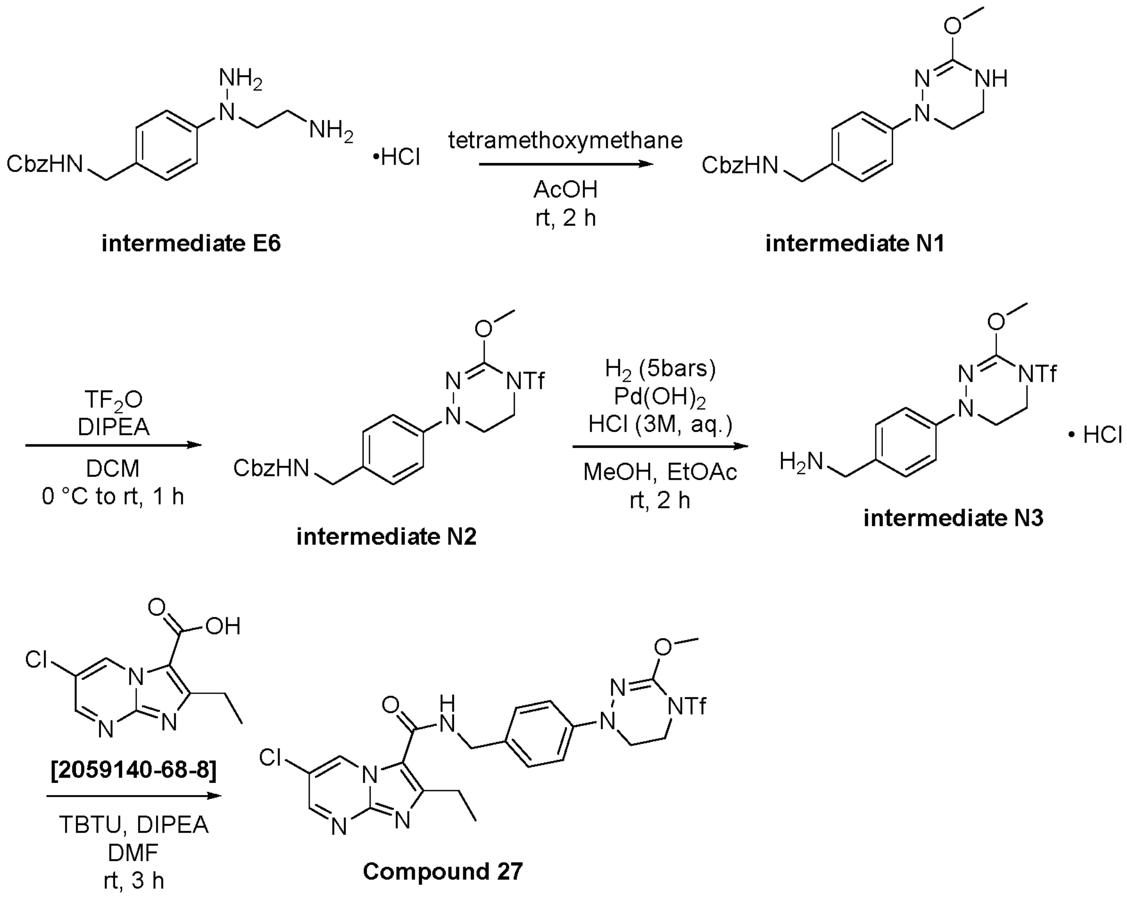


**intermediate E6**

**intermediate N1**

**intermediate N2**

**intermediate N3**

**Compound 27**

Preparation of intermediate **N1**

A solution of intermediate **E6** (3.00 g, 7.75 mmol) in acetic acid (30 mL) was treated with tetramethoxymethane (2.58 mL, 19.4 mmol) and stirred at room temperature for 2 h. The reaction mixture was poured in DCM and quenched with $K_2CO_3$ (10%, aq.). The layers were separated and the aqueous phase was extracted with DCM and MeOH (98/2). The combined organic extracts were dried over $MgSO_4$, filtered and evaporated *in vacuo.* The crude mixture was purified by preparative LC (irregular SiOH 15-40 μm, 80 g, liquid injection (DCM), mobile phase: heptane/EtOAc, gradient from 70:30 to 0:100) to give 1.09 g of intermediate **N1** as an oil (40%).

Preparation of intermediate **N2**

To a mixture of intermediate **N1** (1.00 g, 2.82 mmol) and DIPEA (0.972 mL, 5.64 mmol) in DCM (15 mL) was added a solution of $Tf_2O$ in DCM (1M in DCM, 2.96 mL, 2.96 mmol) dropwise over 10 min. The reaction mixture was stirred at room temperature for 30 min and diluted with DCM. The mixture was washed with $NaHCO_3$ (sat., aq.), dried over $MgSO_4$, filtered and evaporated *in vacuo.* The residue was purified by preparative LC (irregular SiOH 15-40 μm,

40 g, liquid injection (DCM), mobile phase: heptane/EtOAc, gradient from 80:20 to 40:60) to give 680 mg of intermediate **N2** as a white solid (50%).

Preparation of intermediate **N3**

In a steal bomb, a mixture of intermediate **N2** (630 mg, 1.30 mmol), $Pd(OH)_2$ (132 mg, 0.470 mmol) and HCl (3M in $H_2O$, 0.432 mL, 1.30 mmol) in MeOH (5 mL) and EtOAc (5 mL) was hydrogenated under 5 bars of $H_2$ at room temperature for 2 h. The mixture was filtered on a pad of Celite® to give 503 mg of intermediate **N3** as white solid (Quant.).

Preparation of Compound **27**

A mixture of intermediate **N3** (150 mg, 0.665 mmol), 6-chloro-2-ethyl-imidazo[1,2-a]pyrimidine-3-carboxylic acid [2059140-68-8] (284 mg, 0.731 mmol) and DIPEA (0.344 mL, 1.99 mmol) in DMF (4.5 mL) was treated with TBTU (235 mg, 0.731 mmol) and the reaction mixture was stirred at room temperature for 3 h. The reaction mixture was diluted with EtOAc, washed with water and brine, dried over $MgSO_4$, filtered and concentrated *in vacuo.* The residue was purified by preparative LC (irregular SiOH 40 μm, 24 g, liquid injection (DCM), mobile phase: heptane/EtOAc, gradient from 80:20 to 20:80). The white solid solubilized in warm EtOAc and the solution was cooled to room temperature, then to 0 °C. The suspension was filtered off, washed with $Et_2O$, and dried under vacuum to give a solid (71 mg). The filtrate was evaporated *in vacuo* and combined with the solid. The residue was solubilized in warm *i*-PrOH, and cooled to room temperature. The suspension was slowly concentrated under vacuum (120 mbar) to obtain a thick solution. After filtration, the solid was washed with $Et_2O$, and dried under vacuum to afford 135 mg of compound **27** as a white solid (36%).

**$^1$H NMR** (400 MHz, DMSO-*d*$_6$) δ ppm 8.94 (d, *J*=3.06 Hz, 1 H) 8.51 (d, *J*=3.06 Hz, 1 H) 8.40 (t, *J*=5.87 Hz, 1 H) 7.32 (d, *J*=8.68 Hz, 2 H) 7.28 (s, 1 H) 7.19 (d, *J*=8.68 Hz, 2 H) 4.48 (d, *J*=5.87 Hz, 2 H) 4.08 (t, *J*=4.65 Hz, 2 H) 3.86 (s, 3 H) 3.79 - 3.84 (m, 2 H) 2.99 (q, *J*=7.50 Hz, 2 H) 1.25 (t, *J*=7.52 Hz, 3 H).

**Synthesis of Compound 28**

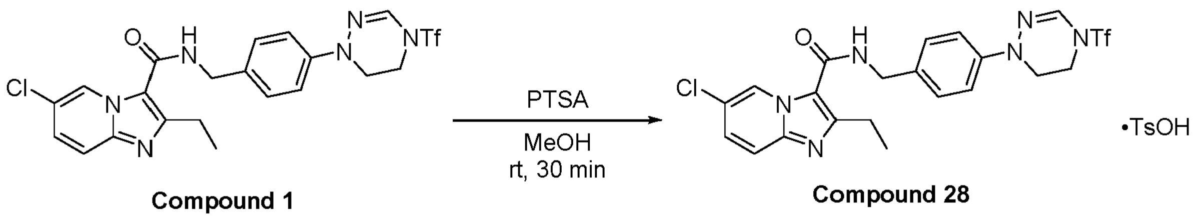


**Compound 1** **Compound 28**

PTSA (108 mg, 567 μmol) was added to a suspension of compound **1** (300 mg, 567 mmol) in MeOH (7.8 mL). After sonication, the solution was stirred at room temperature for 1 h and the solvent was removed under reduced pressure. The residue was triturated in $Et_2O$ and the solvent was removed under reduced pressure (operation repeated twice) to give 406 mg of compound **28** as an off-white solid (Quant.).

**$^1$H NMR** (400 MHz, DMSO-*d*$_6$) δ ppm 9.14 (s, 1 H) 8.80 (t, *J*=5.7 Hz, 1 H) 7.74 - 7.89 (m, 2 H) 7.47 (d, *J*=8.1 Hz, 2 H) 7.27 - 7.37 (m, 3 H) 7.19 (d, *J*=8.7 Hz, 2 H) 7.11 (d, *J*=7.8 Hz, 2 H) 4.49 (d, *J*=5.9 Hz, 3 H) 4.08 (t, *J*=4.4 Hz, 2 H) 3.83 (t, *J*=4.8 Hz, 2 H) 3.02 (q, *J*=7.5 Hz, 2 H) 2.29 (s, 3 H) 1.27 (t, *J*=7.5 Hz, 3 H).

**Synthesis of Compound 29**

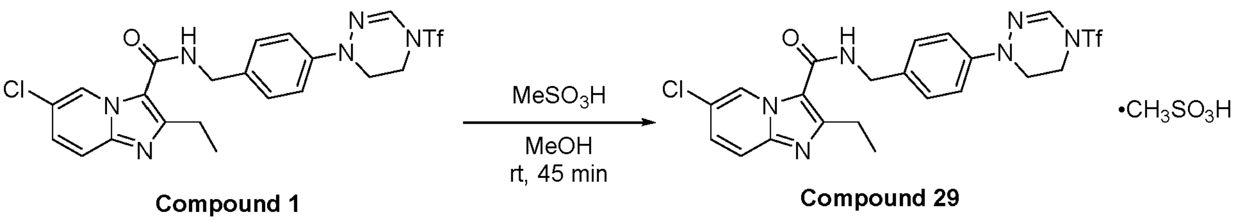


**Compound 1** **Compound 29**

A solution of MeSOsH in MeOH (9.1% v/v, 368 μL, 516 μmol) was added to a mixture of compound **1** (300

mg, 567 μmol) in MeOH (15 mL). The reaction mixture was stirred at room temperature for 45 min and evaporated to dryness. The residue was triturated in $Et_2O$ and the solvent was removed under reduced pressure. The solid was dried under reduced pressure to give 355 mg of compound **29** as an off-white solid (Quant.).

**$^1$H NMR** (400 MHz, DMSO-*$d_6$*) δ ppm 9.13 (s, 1 H) 8.74 (t, *J*=5.3 Hz, 1 H) 7.82 (d, *J*=9.4 Hz, 1 H) 7.73 (d, *J*=9.4 Hz, 1 H) 7.33 (m, *J*=8.7 Hz, 2 H) 7.29 (s, 1 H) 7.19 (m, *J*=8.7 Hz, 2 H) 4.49 (d, *J*=5.9 Hz, 2 H) 4.08 (t, *J*=4.6 Hz, 2 H) 3.83 (t, *J*=4.8 Hz, 2 H) 3.02 (q, *J*=7.5 Hz, 2 H) 2.32 (s, 3 H) 1.27 (t, *J*=7.5 Hz, 3 H).

**Synthesis of Compound 30**

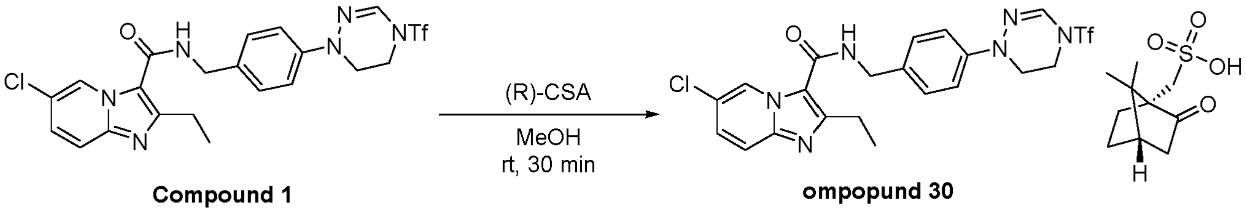


**Compound 1** **ompopund 30**

(1R)-(-)-Camphor-10-Sulfonic acid (110 mg, 473 μmol) was added to a solution of compound 1 (250 mg, 473 μmol) in anhydrous MeOH (5 mL). The reaction mixture was stirred at room temperature for 30 min and the solvent was removed under reduced pressure. The residue was triturated in $Et_2O$ and the solvent was removed under reduced pressure to give 359 mg of compound **30** as a white solid (Quant.).

**$^1$H NMR** (400 MHz, DMSO-*$d_6$*) δ ppm 9.12 (d, J=1.3 Hz, 1 H) 8.69 (t, *J*=5.3 Hz, 1 H) 7.80 (m, 1 H) 7.69 (m, 1 H) 7.33 (d, *J*=8.6 Hz, 2 H) 7.28 (s, 1 H) 7.19 (d, *J*=8.7 Hz, 2 H) 4.48 (d, *J*=5.7 Hz, 3 H) 4.08 (t, *J*=4.6 Hz, 2 H) 3.83 (t, *J*=4.8 Hz, 2 H) 3.01 (q, *J*=7.6 Hz, 2 H) 2.86 (d, J=14.7 Hz, 1 H) 2.65 - 2.75 (m, 1 H) 2.37 (d, *J*=14.7 Hz, 1 H) 2.23 (dt, *J*=18.1, 3.9 Hz, 1 H) 1.93 (t, *J*=4.5 Hz, 1 H) 1.83 - 1.91 (m, 1 H) 1.82 (s, 1 H) 1.77 (s, 1 H) 1.21 - 1.32 (m, 5 H) 1.05 (s, 3 H) 0.74 (s, 3 H).

**Synthesis of Compound 31**

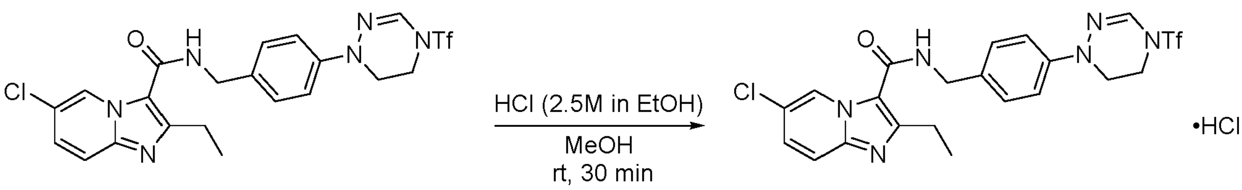


**Compound 1** **Compound 31**

A solution of HCl in EtOH (2.5M, 89 μL, 473 μmol) was added to a mixture of compound **1** (250 mg, 473 μmol) in MeOH (2.7 mL). The reaction mixture was stirred at room temperature for 30 min, then evaporated in vacuo to dryness. The residue was triturated in $Et_2O$ and the solvent was removed under reduced pressure to give 269 mg of compound **31** as a white solid (Quant.).

**$^1$H NMR** (400 MHz, DMSO-*$d_6$*) δ ppm 9.12 (s, 1 H) 8.71 (m, 1 H) 7.79 (d, *J*=9.4 Hz, 1 H) 7.68 (d, *J*=8.8 Hz, 1 H) 7.26 - 7.37 (m, 3 H) 7.19 (d, *J*=8.7 Hz, 2 H) 4.48 (d, *J*=5.9 Hz, 2 H) 4.08 (t, *J*=4.5 Hz, 2 H) 3.83 (t, *J*=4.8 Hz, 2 H) 3.01 (q, *J*=7.6 Hz, 2 H) 1.27 (t, *J*=7.5 Hz, 3 H).

**Synthesis of Compound 32**

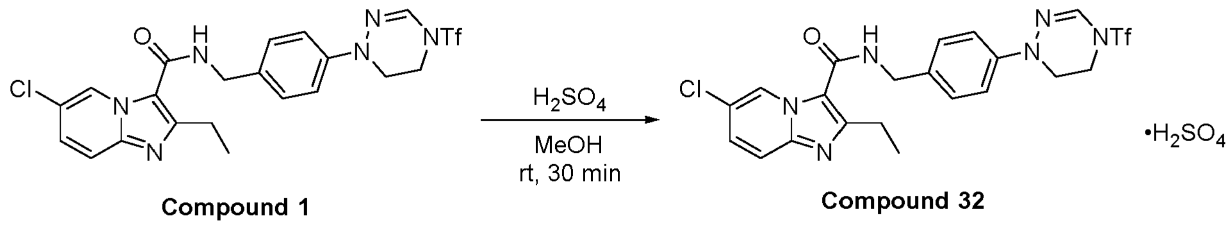


Compound 1 Compound 32

$H_2SO_4$ (13 μL, 238 μmol) was added to a solution of compound **1** (252 mg, 476 μmol) in MeOH (4.2 mL). The reaction mixture was stirred at room temperature for 30 min, then evaporated to dryness. The residue was triturated in $Et_2O$ and the solvent was removed under reduced pressure. The white solid was dried at 60 °C for 6 h under vacuum to give 271 mg of compound **32** as a white solid (98%).

**$^1$H NMR** (400 MHz, DMSO-$d_6$) δ ppm 9.11 (s, 1 H) 8.63 (t, *J*=5.5 Hz, 1 H) 7.76 (d, *J*=9.5 Hz, 1 H) 7.62 (d, *J*=9.8 Hz, 1 H) 7.26 - 7.36 (m, 3 H) 7.19 (d, *J*=8.7 Hz, 2 H) 4.48 (d, *J*=5.9 Hz, 2 H) 4.07 (t, *J*=4.7 Hz, 2 H) 3.83 (t, *J*=4.7 Hz, 2 H) 3.00 (q, *J*=7.5 Hz, 2 H) 1.26 (t, *J*=7.5 Hz, 3 H).

## Synthesis of Compound 33

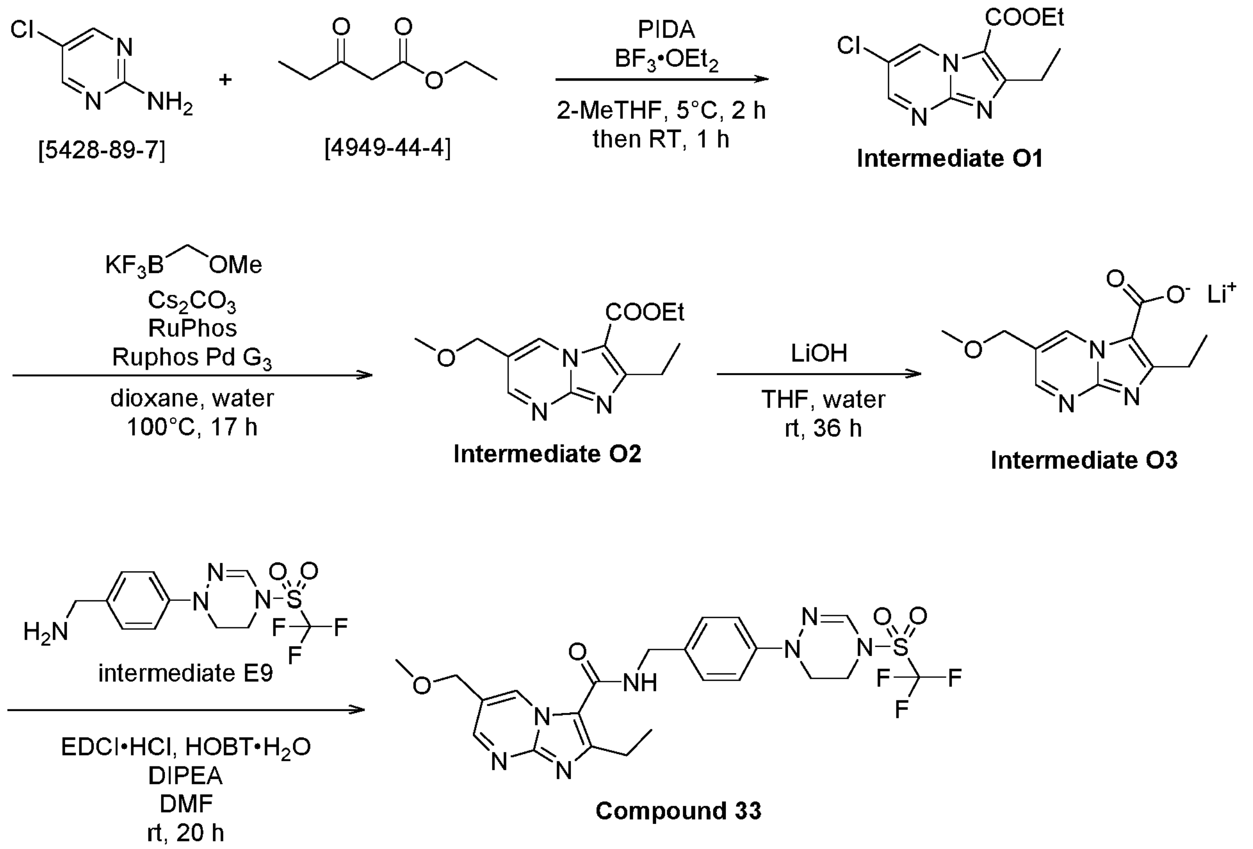


[5428-89-7] [4949-44-4] Intermediate O1

Intermediate O2 Intermediate O3

Compound 33

Preparation of intermediate **O1**

A 2 L round bottom flask equipped with a dropping funnel was charged at 5 °C with a solution of 2-amino-5-chloropyrimidine [5428-89-7] (10 g, 77 mmol) in Me-THF (350 L). Ethyl-3-oxovalerate [4949-44-4] (20 mL, 140 mmol) and (diacetoxyiodo)benzene (iodobenzene diacetate) (25 g, 78 mmol) were added. Boron trifluoride diethyl etherate (1 mL, 3.8 mmol) was added dropwise over 30 min and the solution was stirred at 5 °C for 2 h. The mixture was warmed to room temperature and stirred for 1 h. The mixture was filtered. EtOAc and NaHCOs (sat., aq.) were added to the filtrate. The organic layer was dried over $MgSO_4$, filtered and concentrated *in vacuo.* The crude mixture was purified by preparative LC (irregular SiOH, 15-40 μm, 330 g, liquid injection (DCM), mobile phase: heptane/EtOAc, gradient from

85:15 to 50:50) to give intermediate **O1** (2.98 g, 15%).

Preparation of intermediate **02**

A solution of intermediate **O1** (1.00 g; 3.94 mmol), potassium (methoxymethyl) trifluoroborate [910251-11-5] (1.80 g, 11.8 mmol) and $Cs_2CO_3$ (3.85 g, 11.8 mmol) in 1,4-dioxane (10 mL) and water (1.4 mL) was purged with nitrogen. RuPhos (184 mg, 0.394 mmol) and RuPhos Pd G3 (330 mg, 0.394 mmol) were added. The reaction mixture was purged again with nitrogen and stirred at 100 °C for 17 h. The reaction mixture was concentrated *in vacuo* and purified by preparative LC (irregular SiOH 15-40 μm, 40 g, liquid injection (DCM), mobile phase: heptane/EtOAc, gradient from 75:25 to 0:100). The residue was purified by reverse phase (stationary phase: YMC-actus Triart C18 10μm 30*150mm, mobile phase: (aq. $NH_4HCO_3$ 0.2%)/MeCN, gradient from 70:30 to 30:70) to give intermediate **O2** (212 mg, 20%) as a white solid.

Preparation of intermediate **03**

A mixture of intermediate **O2** (130 mg, 0.494 mmol) and LiOH (14 mg, 0.585 mmol) in THF (2.3 mL) and water (2.3 mL) was stirred at room temperature for 36 h. The reaction mixture was evaporated *in vacuo* to afford 168 mg of intermediate **O3** as a light-yellow gum. The crude product was used as such in next step.

Preparation of Compound **33**

To a mixture of intermediate **O3** (168 mg, 0.529 mmol) and DIPEA (0.275 mL, 1.59 mmol) in DMF (5 mL) were successively added $HOB_t•H_2O$ (83.0 mg, 0.542 mmol), EDCI•HCl (102 mg, 0.533 mmol) and intermediate **E9** (223 mg, 0.536 mmol). The reaction mixture was stirred at room temperature for 20 h. DCM and water were added. The layers were separated and the organic layer was washed with $NaHCO_3$ (sat., aq.) and brine (3 times), dried over $MgSO_4$, filtered and evaporated. The crude mixture was purified by preparative LC (irregular SiOH 15-40 μm, 24 g, dry loading (Celite®), mobile phase: heptane/(EtOAc/MeOH, 9/1), gradient from 90:10 to 0:100). The residue (175 mg) was purified by reverse phase (stationary phase: YMC-actus Triart C18 10 μm 30*150 mm, 40 g, dry loading (Celite®), mobile phase: (aq. $NH_4HCO_3$ 0.2%)/MeCN, gradient from 90:10 to 30:70). MeCN was evaporated and the product was extracted with DCM (twice). The organic layer was dried over $MgSO_4$, filtered and evaporated *in vacuo* to afford 154 mg of a white solid. The product was purified by reverse phase (stationary phase: YMC-actus Triart C18 10 μm 30*150 mm, 40 g, dry loading (Celite®), mobile phase: (aq. $NH_4HCO_3$ 0.2%)/MeCN, gradient from 60:40 to 45:55). MeCN was evaporated and the product was extracted with DCM (twice). The organic layer was dried over $MgSO_4$, filtered and evaporated *in vacuo.* The product was triturated in MeCN and EtOAc, filtered and dried under high vacuum at 50 °C for 16 h to afford compound **33** (119 mg, 42%) as a white solid.

**$^1$H NMR** (400 MHz, DMSO-$d_6$) δ ppm 9.27 (d, J=2.3 Hz, 1H) 8.60 (d, J=2.4 Hz, 1H) 8.50 (t, J=6.0 Hz, 1H) 7.27 - 7.34 (m, 3H) 7.19 (d, J=8.7 Hz, 2H) 4.53 (s, 2H) 4.47 (d, J=5.9 Hz, 2H) 4.03 - 4.12 (m, 2H) 3.79 - 3.86 (m, 2H) 3.34 (s, 3H) 3.00 (q, J=7.5 Hz, 2H) 1.27 (t, J=7.5 Hz, 3H).

**Synthesis of Compound 34**

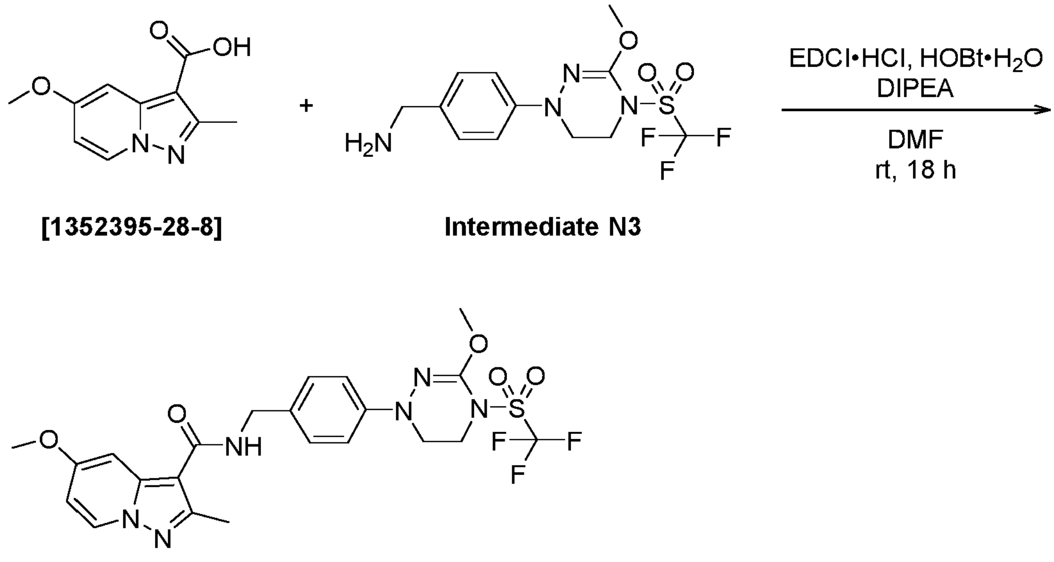


[1352395-28-8]

Intermediate N3

**Compound 34**

To a mixture of the 5-methoxy-2-methylpyrazolo[1,5-a]pyridine-3-carboxylic acid [1352395-28-8] (80 mg, 0.39 mmol), intermediate **N3** (151 mg, 0.39 mmol) and DIPEA (201 μL, 1.17 mmol) in DMF (5 mL) were added EDCI•HCl (74 mg, 0.39 mmol) and $HOB_t$•$H_2O$ (59 mg, 0.39 mmol). The reaction mixture was stirred at room temperature for 18 h and concentrated *in vacuo.* The residue was diluted in EtOAc and water. The layers were separated and the aqueous phase was extracted with EtOAc. The combined organic layers were dried over $MgSO_4$, filtered and concentrated. The residue (229 mg) was purified by reverse phase (stationary phase: YMC-actus Triart C18 10μm (30*150mm), mobile phase: (aq. $NH_4HCO_3$ 0.2%)/MeCN, gradient from 50:50 to 25:75) affording 118 mg of compound **34.**

$^1$**H NMR** (400 MHz, DMSO-$d_6$) δ ppm 8.49 (d, J=7.5 Hz, 1H) 7.85 (t, J=5.9 Hz, 1H) 7.22 - 7.29 (m, 3H) 7.14 (d, J=8.7 Hz, 2H) 6.62 (dd, J=7.5, 2.8 Hz, 1H) 4.41 (d, J=6.0 Hz, 2H) 4.07 - 4.12 (m, 2H) 3.84 (d, J=2.3 Hz, 6 H) 3.69 - 3.75 (m, 2H) 2.52 (s, 3H).

## Synthesis of Compound 35

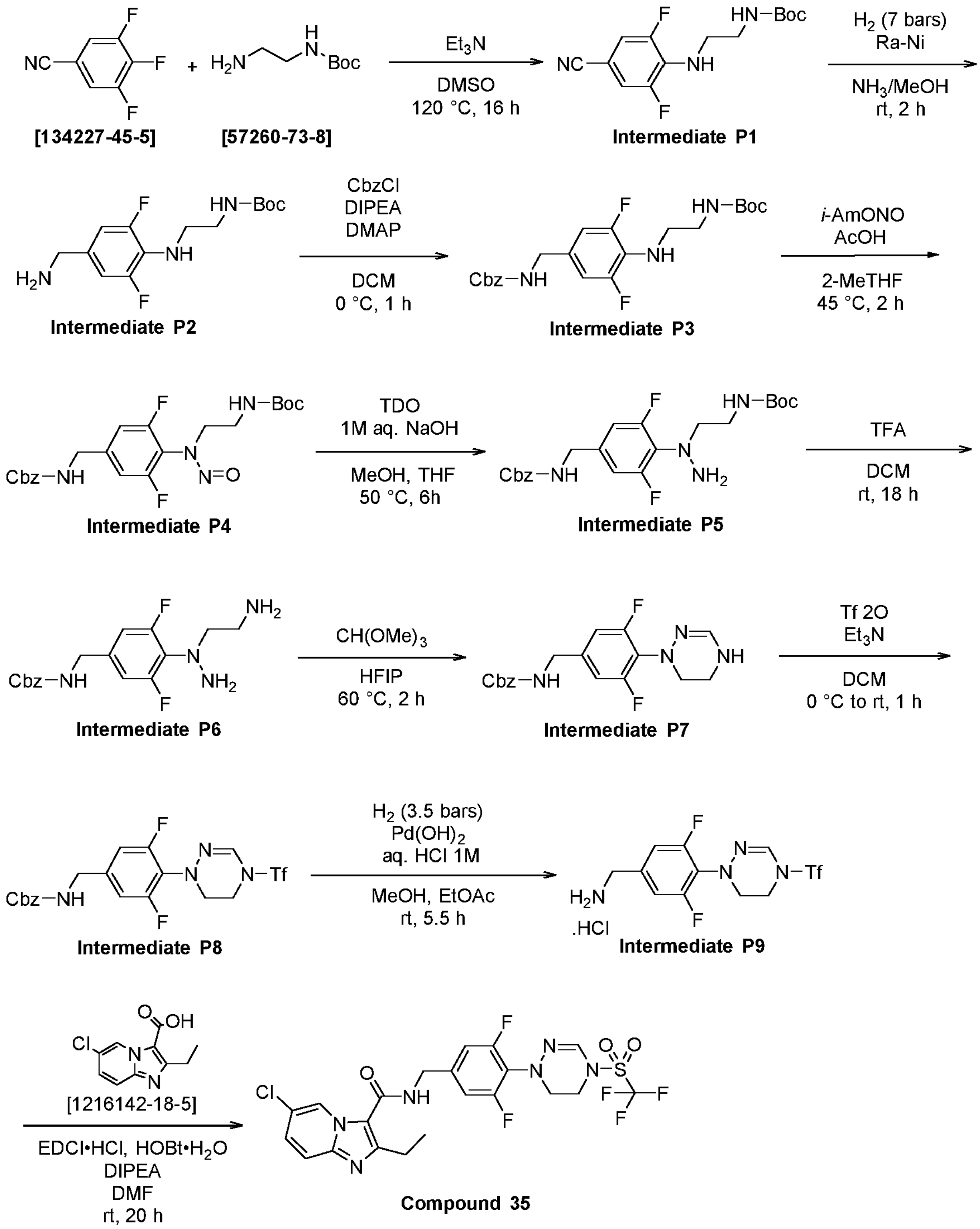


Preparation of intermediate **P1**

In a round bottom flask, a solution of 3,4,5-trifluorobenzonitrile [134227-45-5] (5 g, 31.8 mmol), N-boc-1,2-diaminoethane [57260-73-8] (5.2 mL, 32.8 mmol) and $Et_3N$ (17.7 mL, 127 mmol) in anhydrous DMSO (57 mL) was stirred at 120 °C for 16 h. The reaction mixture was cooled to room temperature and DMSO was evaporated with Genevac. EtOAc, water and NaCl were added. The layers were separated and the organic layer was washed with brine (3 times), dried over $MgSO_4$, filtered and evaporated *in vacuo.* The crude mixture was solubilized in EtOAc and SiOH was added. The dry loading was evaporated and washed with heptane (100 mL). The product was eluted with heptane/EtOAc (1:1, 3 x 100 mL). The filtrate was evaporated to afford 9.30 g of intermediate **P1** as a colorless oil which crystallized on standing (98%).

Preparation of intermediate **P2**

Intermediate **P2** was prepared following the synthesis reported for intermediate **E2,** starting from intermediate **P1** (31.3 mmol) and affording 9.3 g as a light blue gum (99%) which crystallized on standing.

Preparation of intermediate **P3**

Intermediate **P3** was prepared following the synthesis reported for intermediate **E3,** starting from intermediate **P2** (6.64 mmol) and affording 1.63 g as a colorless oil (56%) which crystallized on standing.

Preparation of intermediate **P4**

Intermediate **P4** was prepared following the synthesis reported for intermediate **E4,** starting from intermediate **P3** (3.74 mmol) and affording 1.91 g as a yellow oil (91%).

Preparation of intermediate **P5**

Intermediate **P5** was prepared following the synthesis reported for intermediate **E5,** starting from intermediate **P4** (3.74 mmol) and affording 1.69 g as a yellow oil (100%) which crystallized on standing.

Preparation of intermediate **P6**

A solution of intermediate **P5** (1.69 g, 3.75 mmol) in anhydrous DCM (35 mL) was treated with TFA (3.5 mL, 45.7 mmol) and the reaction mixture was stirred at room temperature for 18 h. The reaction mixture was evaporated *in vacuo* to give 3.42 g of intermediate **P6** as an orange gum.

Preparation of intermediate **P7**

Trimethylorthoformate (1.24 mL, 11.3 mmol) was added to a solution of intermediate **P6** (3.42 g, 3.78 mmol) in HFIP (35 mL) and the mixture was stirred at 60 °C for 2 h. The reaction mixture was cooled to room temperature, diluted with EtOAc and basified with NaHCOs (sat., aq.). The layers were separated and the aqueous layer was extracted with EtOAc (once). The combined organic layers were dried over $MgSO_4$, filtered and the solvent was removed under reduced pressure to give 2.0 g of intermediate **P7** as a yellow gum.

Preparation of intermediate **P8**

Triethylamine (1 mL,7.19 mmol) was added to a solution of intermediate **P7** (1.5 g, 2.83 mmol) in DCM (28 mL). The solution was then cooled to 0 °C (ice / water bath) and TfzO (1M in DCM, 3.4 mL, 3.4 mmol) was added dropwise over 5 min. The reaction mixture was stirred at 0 °C for 30 min. The mixture was slowly warmed to room temperature and stirred for 2 h. DCM, water and NaHCOs (10%, aq.) were added. The layers were separated, and the aqueous layer was extracted with DCM. The combined organic layers were dried over MgSO4, filtered and evaporated. The residue (1.61 g) was purified by preparative LC (irregular SiOH, 30 μm, 80 g, liquid injection (DCM), mobile phase: heptane/EtOAc, gradient from 95:5 to 50:550) to afford 317 mg of intermediate **P8** as an orange gum (23% over 3 steps).

Preparation of intermediate **P9**

In a steal bomb, a mixture of intermediate **P8** (317 mg, 0.644 mmol), palladium hydroxide, Pd 20% on carbon, nominally 50 % water (120 mg, 0.171 mmol) and HCl (1M, aq., 0.64 mL, 0.64 mmol) in EtOAc (3.2 mL) and MeOH (3.2 mL) was hydrogenated under 5 bars of $H_2$ at room temperature for 4 h. The mixture was filtered. An extra amount of palladium hydroxide, Pd 20% on carbon, nominally 50 % water (60 mg, 0.085 mmol) and HCl (1M, aq., 0.64 mL, 0.64 mmol) were added. The mixture was hydrogenated under 5 bars of $H_2$ at room temperature for 1.5 h. The reaction mixture was filtered and the filtrate was evaporated *in vacuo* to afford 269 mg of intermediate **P9** as an orange gum. The crude product was used as such in next step.

Preparation of Compound **35**

To a mixture of 6-chloro-2-ethylimidazo[1,2-a]pyridine-3-carboxylicacid [1216142-18-5] (80 mg, 0.356 mmol) and DIPEA (0.245 mL, 1.42 mmol) in DMF (3.5 mL) were successively added EDCI•HCl (72 mg, 0.376 mmol), HOBt•$H_2O$

(60 mg, 0.392 mmol) and intermediate **P9** (270 mg, 0.356 mmol). The reaction mixture was stirred at room temperature for 20 h. The crude mixture was taken-up in DCM and $NaHCO_3$ (sat., aq.) was added. The layers were separated and the organic layer was washed with brine (twice), dried over $MgSO_4$, filtered and evaporated *in vacuo*. The residue (409 mg) was purified by preparative LC (regular SiOH 30 μm, 24 g, mobile phase: heptane/(EtOAc/MeOH, 9/1), gradient from 80:20 to 20:80). A second purification was performed by reverse Phase (stationary phase: YMC-actus Triart C18 25 μm 30*150 mm, 40 g, dry loading (Celite®), mobile phase: (aq. $NH_4HCO_3$ 0.2%)/MeCN, gradient from 65:35 to 25:75). The desired fractions were combined and MeCN was evaporated. The product was extracted with DCM (3 times) and the organic layer was dried over $MgSO_4$, filtered and evaporated to give a colorless gum (81 mg). The product was triturated in pentane and $Et_2O$ (1/1), evaporated and dried under high vacuum at 50 °C for 5 h to afford 66 mg of compound **35** as a light-yellow solid (24%).

**$^1$H NMR** (400 MHz, DMSO-$d_6$) δ ppm 9.11 (m, 1H) 8.45 - 8.53 (m, 1H) 7.69 (d, *J*=9.4 Hz, 1H) 7.48 (dd, *J*=9.7, 1.8 Hz, 1H) 7.29 (s, 1H) 7.18 (d, *J*=9.5 Hz, 2H) 4.54 (d, *J*=5.6 Hz, 2H) 4.05 - 4.13 (m, 2H) 3.61 - 3.70 (m, 2H) 3.03 (q, *J*=7.4 Hz, 2H) 1.23 - 1.35 (t, *J*=7.4 Hz, 3 H).

## Synthesis of Compound 36

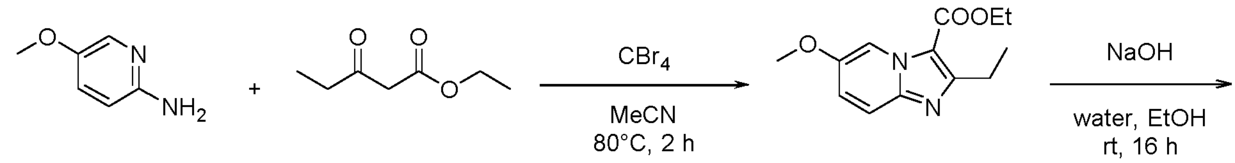


**[10167-97-2]** **[4949-44-4]** **Intermediate Q1**

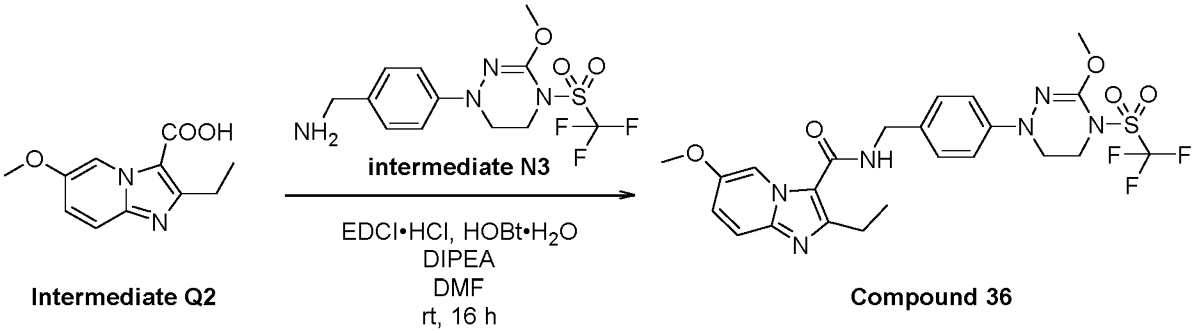


**Intermediate Q2** **Compound 36**

### Preparation of intermediate **Q1**

Carbone tetrabromide (16 g; 43.4 mmol) was added to a mixture of 2-amino-5-methoxypryridine [10167-97-2] (3 g, 24.2 mmol) and ethyl-3-oxovalerate[4949-44-4] (5.2 mL, 36.6 mmol) in MeCN (50 mL). The reaction mixture was heated at 80 °C for 2 h. The reaction mixture was cooled to room temperature and concentrated to dryness.

The residue (20 g) was purified by preparative LC (regular SiOH 30 μm, 330 g, dry loading (SiOH), mobile phase: heptane/EtOAc, gradient from 80:20 to 0:100) to give 1.89 g of intermediate **Q1** as a greenish solid (32%).

### Preparation of intermediate **Q2**

To a solution of intermediate **Q1** (1.89 g, 7.61 mmol) in water (20 mL) and EtOH (25 mL) was added NaOH (913 mg, 22.8 mmol). The reaction mixture was stirred at room temperature for 16 h. Additional quantity of NaOH (304 mg, 7.61 mmol) was added and the reaction mixture was stirred for 3 h. EtOH was concentrated. The mixture was acidified to pH 2-3 with HCl (1N). The white precipitate was filtered and washed with water and dried under high vacuum to give 750 mg of intermediate **Q2** as a white solid (45%).

### Preparation of compound **36**

To a mixture of intermediate **Q2** (150 mg, 0.681 mmol) and DIPEA (0.48 mL, 2.79 mmol) in DMF (7 mL) were successively added EDCI•HCl (174 mg, 0.908 mmol), HOBt•$H_2O$ (144 mg, 0.94 mmol) and intermediate **N3** (265 mg,

0.681 mmol). The reaction mixture was stirred at room temperature for 16 h and evaporated. The residue was taken-up in DCM and NaHCOs (sat., aq.) was added. The layers were separated and the organic layer was washed with water and brine (twice), dried over $MgSO_4$, filtered and evaporated. The crude mixture was purified by preparative LC (regular SiOH 30 μm, 24 g, liquid injection (DCM), mobile phase: heptane/(EtOAc/MeOH, 9/1), gradient from 80:20 to 20:80). The fractions containing product were combined and evaporated to afford a white solid (304 mg). The product was recrystallized from MeCN, filtered and dried under high vacuum at 50 °C for 3 h to afford 200 mg of compound **36** as a white solid (53%).

**$^1$H NMR** (400 MHz, DMSO-$d_6$) δ ppm 8.65 (d, J=2.2 Hz, 1H) 8.23 - 8.32 (m , 1H) 7.53 (d, J=9.5 Hz, 1H) 7.29 (d, J=8.7 Hz, 2H) 7.13 - 7.21 (m, 3H) 4.46 (d, J=5.9 Hz, 2H) 4.06 - 4.17 (m, 2H) 3.85 (s, 3H) 3.72 - 3.82 (m, 5H) 2.95 (q, J=7.5 Hz, 2H) 1.24 (t, J=7.5 Hz, 3 H).

## Synthesis of compound 37

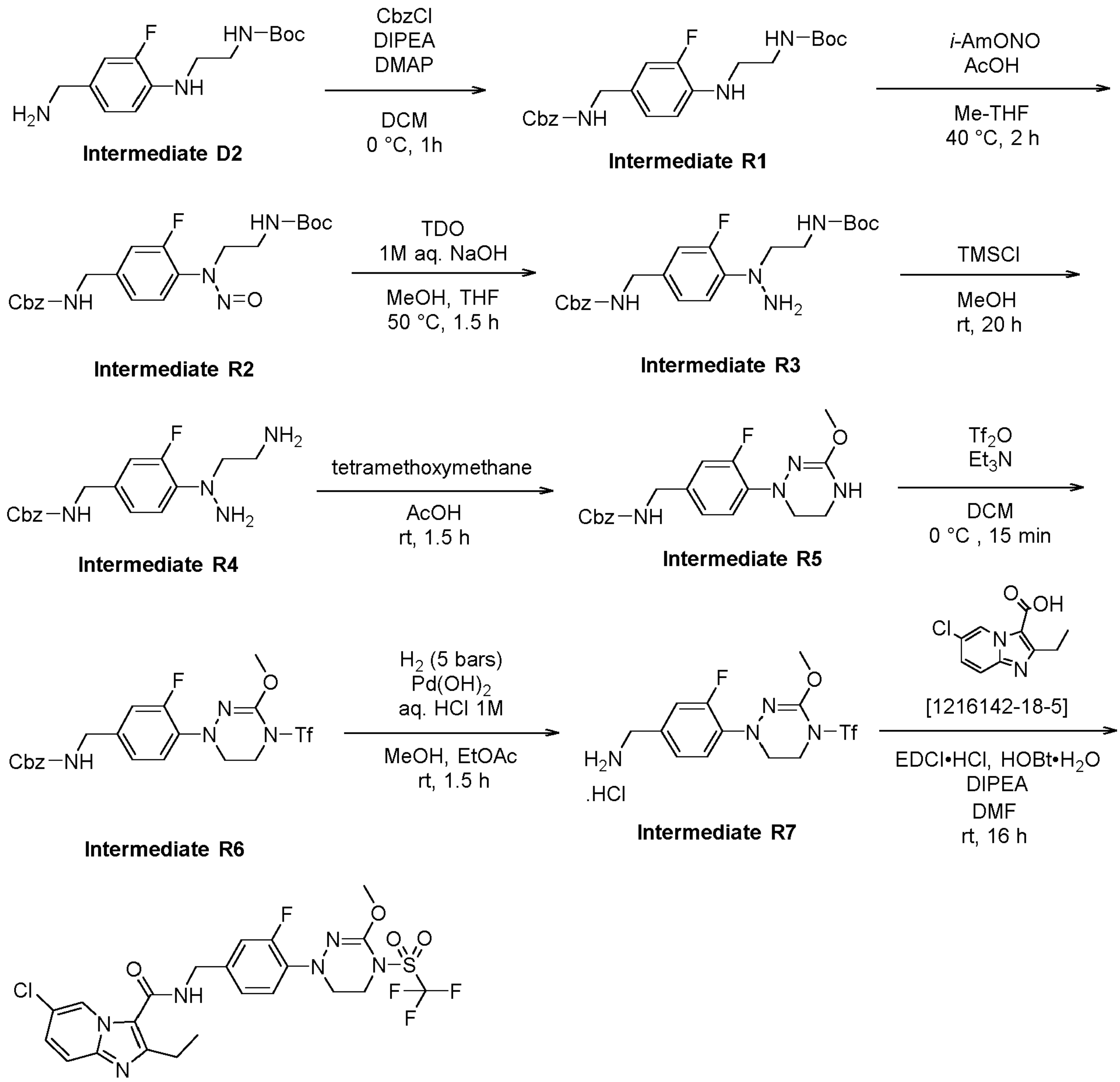


**Compound 37**

Preparation of intermediate **R1**

Intermediate **R1** was prepared following the synthesis reported for intermediate **E3,** starting from intermediate

**D2** (7.06 mmol) and affording 2.53 g as an off-white solid (86%).

Preparation of intermediate **R2**

Intermediate **R2** was prepared following the synthesis reported for intermediate **E4,** starting from intermediate **R1** (6.06 mmol) and affording, 3.2 g as a yellow oil used as such for next step without purification.

Preparation of intermediate **R3**

Intermediate **R3** was prepared following the synthesis reported for intermediate **E5,** starting from intermediate **R2** (6.06 mmol theorical) and affording 2.22 g as a yellow oil (87% over 2 steps).

Preparation of intermediate **R4**

To a solution of **intermediate R3** (2.22 g, 5.13 mmol) in MeOH (52 mL) was added dropwise TMSCl (5.2 mL, 41 mmol). The reaction mixture was stirred at room temperature for 20 h and concentrated *in vacuo.* $Et_2O$ was added to the residue and the gum was triturated. The solvent was removed under reduced pressure to give 2.06 g of intermediate **R4** as a pale green solid (99%).

Preparation of intermediate **R5**

A solution of intermediate **R4** (1.00 g, 2.47 mmol) in acetic acid (25 mL) was treated with tetramethoxymethane (0.82 mL, 6.17 mmol) and stirred at room temperature for 1 h. Additional amount of tetramethoxymethane (0.82 mL, 6.17 mmol) was added and the mixture was stirred at room temperature for 30 min. The reaction mixture was poured in DCM and water. The mixture was basified with $K_2CO_3$ powder and the layers were separated. The aqueous layer was extracted with DCM (once) and the combined organic layers were dried over $MgSO_4$, filtered and evaporated *in vacuo.* The residue (685 mg) was purified by preparative LC (irregular SiOH 40 μm, 24 g, liquid injection (DCM), mobile phase: DCM/MeOH, gradient from 100:0 to 85:15) to give 445 mg of intermediate **R5** as a colorless oil (48%).

Preparation of intermediate **R6**

Intermediate **R6** was prepared following the synthesis reported for intermediate **P8,** starting from intermediate **R5** (1.19 mmol) and affording 0.45 g as colorless oil (72%).

Preparation of intermediate **R7**

Intermediate **R7** was prepared following the synthesis reported for intermediate **P9,** starting from intermediate **R6** (0.61 mmol) and affording 0.24 g as colorless oil (96%).

Preparation of compound **37**

To a mixture of 6-chloro-2-ethylimidazo[1,2-a]pyridine-3-carboxylic acid [1216142-18-5] (87.3 mg, 0.388 mmol), intermediate **R7** (158 mg, 0.388 mmol) and DIPEA (0.335 mL, 1.94 mmol) in DMF (5.3 mL) were successively added EDCI•HCl (74.5 mg, 0.388 mmol) and $HOB_t$•$H_2O$ (59.5 mg, 0.388 mmol). The reaction mixture was stirred at room temperature for 16 h and evaporated *in vacuo.* The crude mixture was purified by preparative LC (irregular SiOH 15-40 μm, 12 g, dry loading (Celite®), mobile phase: heptane/EtOAc, gradient from 80:20 to 30:70). The desired fractions were combined and evaporated under vacuum. The product (163 mg) was sonicated in $Et_2O$ and filtered to give 118 mg of compound **37** as a white solid (53%).

**$^1$H NMR** (400 MHz, DMSO-$d_6$) δ ppm 9.09 (d, J=1.6 Hz, 1H) 8.47 (t, J=5.9 Hz, 1H) 7.68 (d, J=9.5 Hz, 1H) 7.42 - 7.50 (m, 2H) 7.16 - 7.25 (m, 2H) 4.49 (d, J=5.9 Hz, 2H) 4.07 - 4.15 (m, 2H) 3.83 (s, 3H) 3.53 - 3.61 (m, 2H) 3.00 (q, J=7.5 Hz, 2H) 1.27 (t, J=7.5 Hz, 3H).

**Synthesis of compound 38**

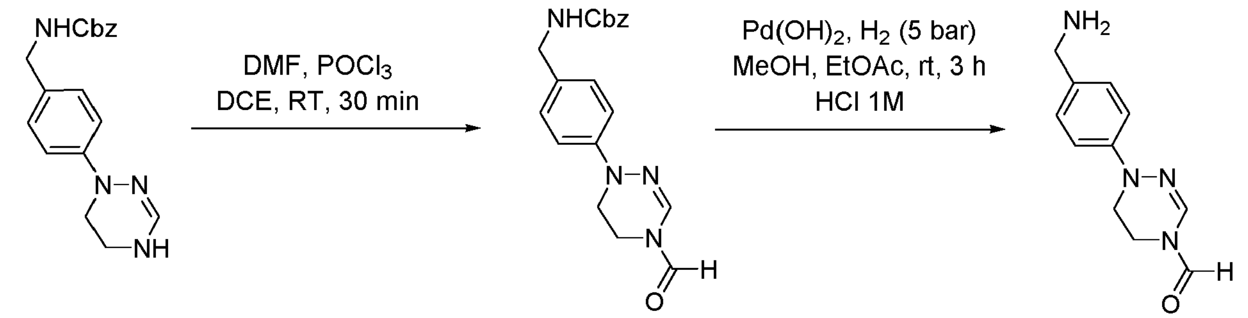


**Intermediate E7** **Intermediate S1** **Intermediate S2**

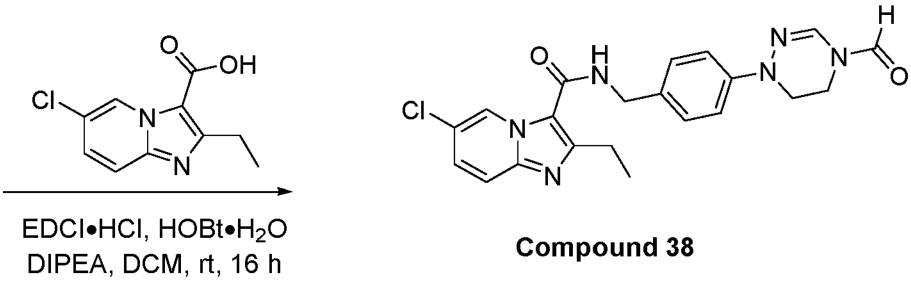


**Compound 38**

Preparation of intermediate S1

To a solution of DMF (103 $\mu$L, 1.33 mmol) in DCE (6.5 mL) at room temperature was added $POCl_3$ (123 $\mu$L, 1.33 mmol) and the mixture was stirred at room temperature for 30 min. Then the mixture was cooled down to 0°C and intermediate E7 (430 mg, 1.33 mmol) in DCE (6.5 mL) was added dropwise and the mixture was stirred at 0°C for 2 hours. Water and DCM were added. The aqueous layer was slowly basified with NaHCOs (s) to pH 8. The layers were separated, and the aqueous layer was extracted with DCM. The combined organic layers were washed with brine, dried over $MgSO_4$, filtered off and evaporated to afford 421 mg of intermediate S1 as a yellow solid. The crude was used as such in next step.

Preparation of intermediate S2

In a steal vessel, a mixture of intermediate S1 (421 mg, 1.20 mmol), palladium hydroxide (100 mg, 0.14 mmol) and HCl 1M in $H_2O$ (1.2 mL, 1.2 mmol) in MeOH (10.5 mL) and EtOAc (10.5 mL) was hydrogenated under 5 bar of $H_2$ at room temperature for 3 hours. The mixture was filtered on a pad of celite® to give 413 mg of intermediate S2 as a yellow solid. The crude was used as such in next step.

Preparation of compound 38

To a solution of 6-chloro-2-ethylimidazo[1,2-a]pyridine-3-carboxylicacid (CAS [1216142-18-5], 240 mg, 1.07 mmol) and diisopropylethylamine (0.75 mL, 4.35 mmol) in DCM (11 mL) were added EDCI•HCl (210 mg, 1.10 mmol) and $HOB_t$•$H_2O$ (170 mg, 1.11 mmol) then intermediate S2 (410 mg, 1.13 mmol) and the mixture was stirred at room temperature for 16 hours. DCM and water were added. The layers were separated, and the organic layer was washed with an aqueous saturated solution of $NaHCO_3$ and brine. The organic layer was dried over MgSO4, filtered and evaporated. The crude was purified by Reverse Phase (Stationary phase: YMC-actus Triart C18 10 $\mu$m 30*150 mm, 40 g, dry loading (on Celite®), mobile phase: Gradient from 80% (aq. $NH_4HCO_3$ 0.2%), 20% MeCN to 40% (aq. $NH_4HCO_3$ 0.2%), 60% MeCN). MeCN was evaporated and the product was extracted with DCM/MeOH (9:1) (3 times). The organic layer was dried over $MgSO_4$, filtered and evaporated to afford 176 mg of a light-yellow solid. It was purified by Reverse phase (Stationary phase: YMC-actus Triart C18 10 $\mu$m 30*150 mm, 40 g, dry loading (on Celite®), mobile phase: Gradient from 60% (aq. $NH_4HCO_3$ 0.2%), 40% MeCN to 45% (aq. $NH_4HCO_3$ 0.2%), 55% MeCN over 16 CV). All fractions were combined to obtain 139 mg as a yellow solid. It was purified by Reverse phase (Stationary phase: YMC-actus Triart C18 10$\mu$m 30* 150mm, liquid loading (DMSO), Mobile phase: Gradient from 70% (aq. $NH_4HCO_3$ 0.2%), 30% ACN to 50%

(aq. $NH_4HCO_3$ 0.2%), 50% ACN) to afford 39 mg as a white solid. It was solubilized in DCM/MeOH then combined with a previous fraction, evaporated and dried under high vacuum (50 °C, 2 h) to afford 68 mg as an off-white solid. It was co-evaporated in MeOH (5 times), then dried under high vacuum (50 °C, 6 h) to give 65 mg of compound **38** as an off-white solid (12%)

Major rotamer (84%) **$^1$H NMR** (500 MHz, DMSO-d6, 350K) $\delta$ ppm 9.07 (s, 1 H), 8.57 (s, 1 H), 8.15 (br t, J = 5.2 Hz, 1 H), 7.61 (d, J = 9.5 Hz, 1 H), 7.53 (s, 1 H), 7.39 (dd, J = 9.6, 2.0 Hz, 1 H), 7.28 (d, J = 8.5 Hz, 2 H), 7.19 (d, J = 8.5 Hz, 2 H), 4.47 (d, J = 6.0 Hz, 2 H), 3.78 (br t, J = 4.7 Hz, 2 H) 3.64 (br t, J = 4.8 Hz, 2 H), 2.97 (q, J = 7.6 Hz, 2 H), 1.26 (t, J = 7.6 Hz, 3 H). Minor rotamer (16%) **$^1$H NMR** (500 MHz, DMSO-d6, 350K) $\delta$ ppm 9.07 (s, 1 H), 8.57 (s, 1 H), 8.15 (br t, J = 5.2 Hz, 1 H), 7.61 (d, J = 9.5 Hz, 1 H), 7.53 (s, 1 H), 7.39 (dd, J = 9.6, 2.0 Hz, 1 H), 7.28 (d, J = 8.5 Hz, 2 H), 7.19 (d, J = 8.5 Hz, 2 H), 4.47 (d, J = 6.0 Hz, 2 H), 3.90 (m, 2 H) 3.73 (m, 2 H), 2.97 (q, J = 7.6 Hz, 2 H), 1.26 (t, J = 7.6 Hz, 3 H).

<u>Synthesis of compound 39</u>

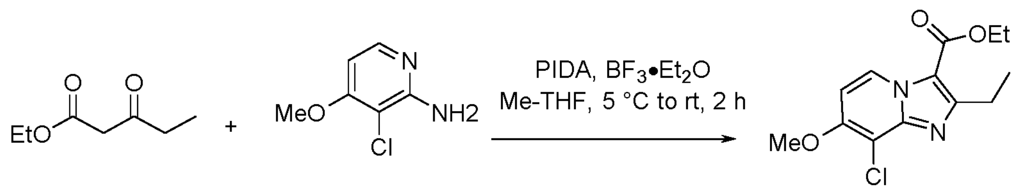


CAS [4949-44-4]　　CAS [1232431-05-8 ]　　**Intermediate T1**

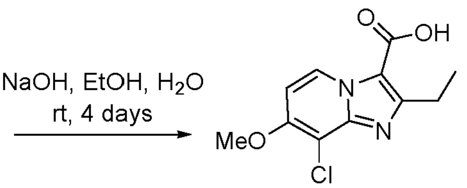


**Intermediate T2**

**Intermediate T2**

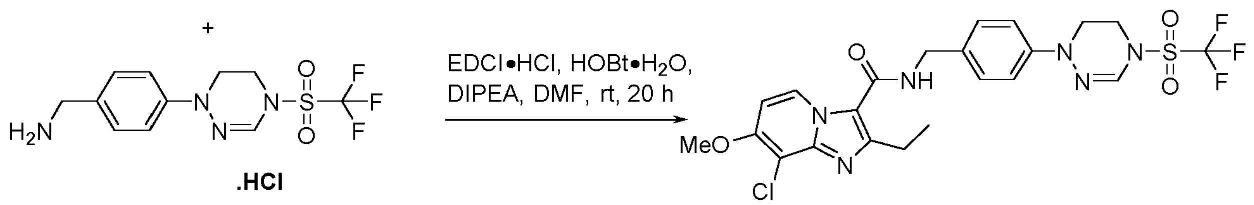


**.HCl**

**intermediate E9**　　**Compound 39**

<u>Preparation of intermediate T1</u>

To a solution of 3-chloro-4-methoxypyridine-2-amine (CAS [1232431-05-8], 0.2 g, 1.26 mmol) in 2-MeTHF (6 mL) at 5 °C under $N_2$ were added ethyl-3-oxovalerate (CAS [4949-44-4], 0.18 mL, 1.26 mmol) and iodobenzenediacetate ((diacetoxyiodo)benzene) (0.406 g, 1.26 mmol.), then borontrifluoride etherate (16.5 $\mu$L, 0.063 mmol) was added dropwise. The solution was stirred at the 5°C for 30 min then warmed to room temperature and stirred for 2 hours. An extra amount of ethyl-3-oxovalerate (0.09 mL, 0.63 mmol), iodobenzenediacetate (0.203 g, 0.63 mmol) and borontrifluoride etherate (16.5 $\mu$L, 0.063 mmol) were added, the mixture was purged with $N_2$ and stirred at rt for 1 hour. EtOAc and water were added. The layers were separated, and the organic layer was dried over $MgSO_4$, filtered off and concentrated. The crude was purified by preparative LC (regular SiOH, 30 $\mu$m, 24 g liquid loading (DCM), mobile phase: Heptane 95%, EtOAc 5% isocratic for 3 CV then gradient to Heptane 60%, EtOAc 40% over 12 CV) to afford 295 mg of intermediate T1 as a white solid (83%).

Preparation of intermediate T2

To a solution of intermediate T1 (270 mg, 0.96 mmol) in water (4.8 mL) and EtOH (4.8 mL) was added NaOH (115 mg, 2.88 mmol) and the mixture was stirred at room temeprature for 4 days. The mixture was evaporated to afford 371 mg of intermediate T2 as a light-yellow solid (purity 71%). The crude was used as such in next step.

Preparation of compound 39

To a solution of intermediate T2 (371 mg, 0.952 mmol) and diisopropyethylamine (0.50 mL, 2.90 mmol) in DMF (9.5 mL) were added $HOB_t{\bullet}H_2O$ (160 mg, 1.05 mmol) and EDCI•HCl (195 mg, 1.02 mmol) then intermediate E9 (400 mg, 0.959 mmol). The mixture was stirred at rt for 20 hours. The mixture was evaporated then taken-up in DCM and an aqueous saturated solution of $NaHCO_3$ was added. The organic layer was separated and washed with brine, dried over $MgSO_4$, filtered and evaporated to give an orange gum. The crude was purified by preparative LC (irregular SiOH, 15-40 μm, 50 g, liquid loading (in DCM), mobile phase gradient: from Heptane 75%, EtOAc/MeOH (9:1) 25% to Heptane 25%, EtOAc/MeOH (9:1) 75% over 12 CV). Clean fractions were combined and evaporated to afford 312 mg as a light-yellow solid. It was purified by Reverse Phase (Stationary phase: YMC-actus Triart C18 10 μm 30*150 mm, 40 g, dry loading (on Celite®), mobile phase: Gradient from 55% (aq. $NH_4HCO_3$ 0.2%), 45% MeCN to 5% (aq. $NH_4HCO_3$ 0.2%), 95% MeCN over 12 CV) to afford 286 mg as an off-white solid. It was sonicated in MeCN (suspension) then filtered off. The solid was dried under high vacuum (50 °C, 6 h) to afford 230 mg of compound 39 as a white solid (43%).

**$^1$H NMR** (400 MHz, DMSO-d6) δ ppm 8.94 (d, J = 7.7 Hz, 1 H), 8.35 (t, J = 5.9 Hz, 1 H), 7.26 - 7.35 (m, 3 H), 7.12 - 7.23 (m, 3 H), 4.45 (br d, J = 5.9 Hz, 2 H), 4.07 (br d, J = 4.4 Hz, 2 H), 3.99 (s, 3 H), 3.82 (t, J = 4.6 Hz, 2 H), 2.95 (q, J = 7.6 Hz, 2 H), 1.24 (t, J = 7.5 Hz, 3 H).

**Synthesis of compound 40 and compound 41**

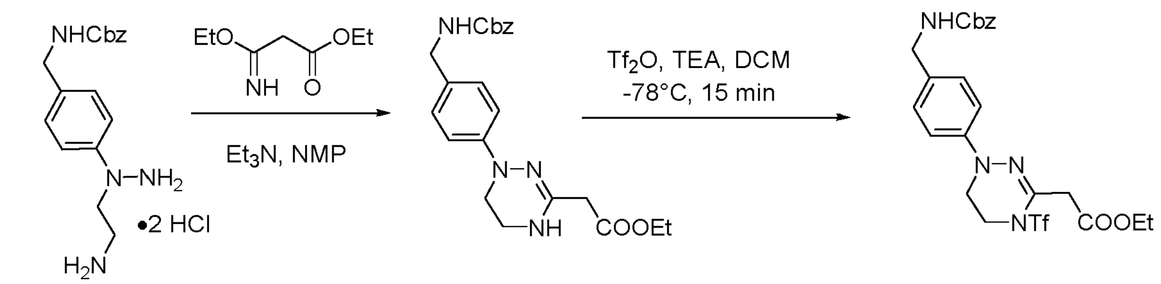


Intermediate E6 Intermediate U1 Intermediate U2

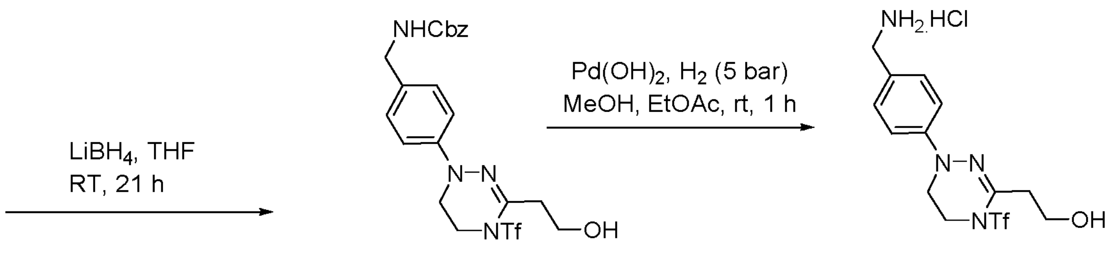


Intermediate U3 Intermediate U4

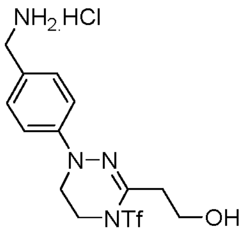

Intermediate U4

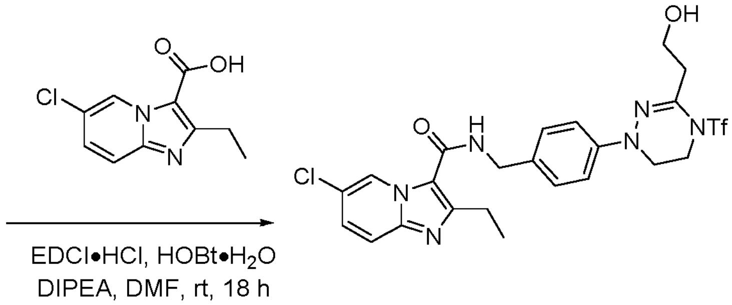


Compound 40

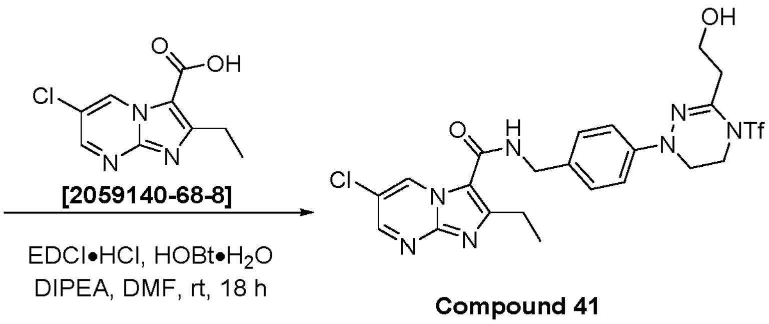


Compound 41

Preparation of intermediate U1

A mixture of intermediate E6 (1.00 g, 2.58 mmol), ethyl-3-ethoxy-3-iminopropanoate hydrochloride (CAS [2318-25-4], 2.17 g, 7.75 mmol) and triethylamine (1.08 mL, 7.75 mmol) in NMP (14 mL) was stirred for 18 h at 150 °C in a sealed tube. The reaction mixture was diluted with EtOAc and water. The aqueous phase was extracted with EtOAc (x3). The combined organic phases were washed with NaCl sat., dried over $MgSO_4$ and concentrated to give 1.85 g as a brown oil. It was diluted in EtOAc and washed with a diluted solution of NaCl. The organic layer was dried over $MgSO_4$ and concentrated to give 1.03 g of intermediate U1. The crude product was used as such in the next step based on the theoretical quantity.

Preparation of intermediate V2

At -78 °C, to a solution of intermediate U1 (900 mg, 2.19 mmol) and triethylamine (914 μL, 6.58 mmol) in dry DCM (45 mL) was added dropwise $Tf_2O$ 1M in DCM (3.1 mL, 3.1 mmol) and the reaction mixture was stirred for 15 min. The reaction mixture was diluted with DCM and water. The organic phase was dried over $MgSO_4$, filtered off and evaporated to give 1.0 g. The residue was purified by preparative LC (irregular SiOH 15-40 μm, 40 g, liquid loading (DCM), mobile phase gradient: (EtOAc/MeOH (90:10)) in heptane from 0 to 50% over 5 CV then isocratic for 5 CV) to give 456 mg of intermediate U2 as an orange-brown oil (38%).

Preparation of intermediate U3

Lithium borohydride (276 μL; 0.553 mmol) was added to a solution of intermediate U2 (150 mg; 0.276 mmol) in THF (5 mL) and the solution was stirred at room temperature for 15 hours. Further lithium borohydride (276 μL, 0.553 mmol) was added and the reaction mixture was stirred for 6 hours. The reaction mixture was diluted with EtOAc and water. The aqueous layer was extracted once again with EtOAc and the combined organic layers were washed with brine (3 times) dried over $MgSO_4$, filtered and evaporated to dryness to give 132 mg of intermediate U3 (95%) as a yellow residue.

Preparation of intermediate U4

Accordingly, intermediate U4 was prepared in the same way as intermediate S2 starting from intermediate U3 (0.132 g, 0.26 mmol) affording 0.11 g (quantitative).

Preparation of compound 40

To a solution of 6-chloro-2-ethylimidazo[1,2-a]pyridine-3-carboxylicacid (CAS [1216142-18-5],67 mg, 0.300 mmol), intermediate U4 (110 mg, 0.300 mmol), and diisopropylethylamine (155 µL, 0.901 mmol) in DMF (4 mL) were added EDCI•HCl (58 mg, 0.30 mmol) and $HOB_t$•$H_2O$ (46 mg, 0.30 mmol) and the reaction mixture was stirred at room temperature for 18 hours. The reaction mixture was concentrated. The residue was taken up in EtOAc and water. The organic layer was washed with NaCl sat, dried over $MgSO_4$, filtered off and concentrated to give 143 mg. The crude was purified by preparative LC (irregular SiOH 15-40 µm, 80 g, liquid loading (DCM), mobile phase gradient: (EtOAc/MeOH (90:10)) in heptane from 0 to 50% over 5 CV then isocratic for 5 CV) to give 100 mg as white solid. It was purified by reverse phase (spherical C18, 25 µm, 40 g YMC-ODS-25, dry loading (Celite®), mobile phase gradient: from 55% (aq. $NH_4HCO_3$ 0.2%), 45% MeCN to 75% (aq. $NH_4HCO_3$ 0.2%) MeCN) to give 19 mg and 59 mg of a residue which was co-evaporated with EtOH and MeCN affording 80 mg of compound 40 as a yellowish solid (combined yield: 57%).
**$^1$H NMR** (500 MHz, DMSO-d6) δ ppm 9.03 - 9.13 (m, 1 H) 8.41 (br t, J=6.0 Hz, 1 H) 7.66 (d, J=9.5 Hz, 1 H) 7.45 (dd, J=9.5, 1.9 Hz, 1 H) 7.32 (d, J=8.5 Hz, 2 H) 7.16 (d, J=8.5 Hz, 2 H) 4.66 (t, J=5.7 Hz, 1 H) 4.47 (d, J=6.0 Hz, 2 H) 3.96 (br t, J=5.0 Hz, 2 H) 3.84 (t, J=4.9 Hz, 2 H) 3.73 (q, J=6.6 Hz, 2 H) 2.98 (q, J=7.6 Hz, 2 H) 2.74 (t, J=6.9 Hz, 2 H) 1.26 (t, J=7.6 Hz, 3 H)

Preparation of compound 41

Accordingly, compound 41 was prepared in the same way as compound 40, starting from 6-chloro-2-ethylimidazo[1,2-a]-pyrimidine-3-carboxylic acid (CAS [2059140-68-8], 0.32 mmol) and intermediate U4 (0.32 mmol) affording 0.067 g (37%) as green-light solid.
**$^1$H NMR** (500 MHz, DMSO-d6) δ ppm 9.39 (d, J=2.5 Hz, 1 H) 8.68 (d, J=2.5 Hz, 1 H) 8.55 (t, J=5.8 Hz, 1 H) 7.31 (m, J=8.5 Hz, 2 H) 7.15 (m, J=8.5 Hz, 2 H) 4.70 (t, J=5.7 Hz, 1 H) 4.47 (d, J=6.0 Hz, 2 H) 3.95 (br t, J=4.9 Hz, 2 H) 3.79 - 3.88 (m, 2 H) 3.72 (q, J=6.6 Hz, 2 H) 3.01 (q, J=7.4 Hz, 2 H) 2.73 (t, J=6.8 Hz, 2 H) 1.27 (t, J=7.6 Hz, 3 H)

**Synthesis of compound 42**

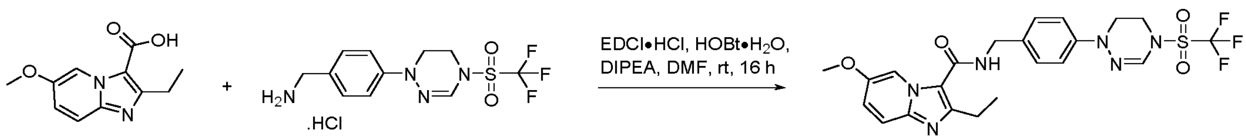


intermediate Q2 intermediate E9 Compound 42

To a solution of intermediate Q2 (125 mg, 0.568 mmol) in diisopropylethylamine (0.4 mL, 2.32 mmol) and DMF (6 mL) were added EDCI•HCl (145 mg, 0.756 mmol), $HOB_t$•$H_2O$ (120 mg, 0.784 mmol) then intermediate E9 (205 mg, 0.571 mmol). The mixture was stirred at room temperature for 16 hours. The reaction mixture was evaporated and taken-up in DCM and an aqueous saturated solution of NaHCOs. The layers were separated, and the organic layer was washed with water, brine (twice), dried over $MgSO_4$, filtered and evaporated. The crude was purified by preparative LC (regular SiOH, 30 µm, 24 g, liquid loading (DCM), mobile phase gradient: from Heptane 80%, EtOAc/MeOH (9:1) 20% to Heptane 20%, EtOAc/MeOH (9:1) 80% over 12 CV) to afford 166 mg as a white solid. It was recrystallized form MeCN then filtered off and dried under high vacuum to afford 107 mg of compound 42 as a white solid (36%).

**$^1$H NMR** (400 MHz, DMSO-d6) δ ppm 8.64 (d, *J* = 2.2 Hz, 1 H), 8.30 (t, *J* = 5.8 Hz, 1 H), 7.53 (d, *J* = 9.5 Hz, 1 H), 7.27 - 7.36 (m, 3 H), 7.14 - 7.22 (m, 3 H), 4.47 (d, *J* = 5.9 Hz, 2 H), 4.08 (br t, *J* = 4.5 Hz, 2 H), 3.83 (br t, *J* = 4.5 Hz, 2 H) 3.76 (s, 3 H), 2.95 (q, *J* = 7.5 Hz, 2 H), 1.24 (t, *J* = 7.5 Hz, 3 H).

**Synthesis of compound 43**

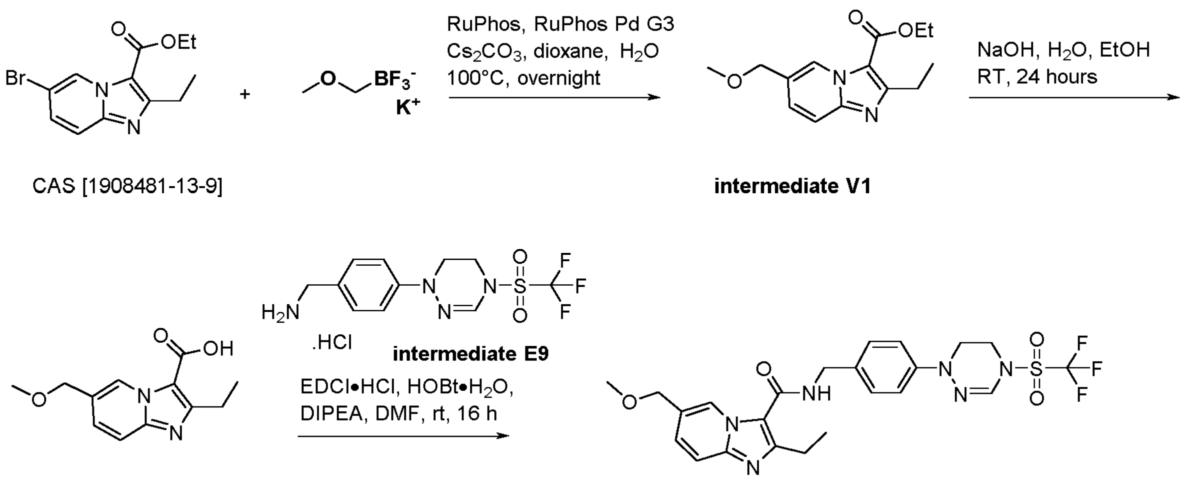


CAS [1908481-13-9]

**intermediate V1**

**intermediate E9**

**intermediate V2**

**Compound 43**

Preparation of intermediate V1

In a sealed tube, a suspension of imidazo[1,2-a]-pyridine-3-carboxylic acid, 6-bromo-2-ethyl-ethyl ester (CAS [1908481-13-9], 400 mg, 1.35 mmol), potassium (methoxymethyl)trifluoroborate (614 mg, 4.04 mmol) and cesium carbonate (1.32 g, 4.04 mmol) in 1,4-dioxane (3.44 mL) and water (0.49 mL) was purged with $N_2$. RuPhos (62.8 mg, 0.135 mmol) and RuPhos Pd G3 (113 mg, 0.135 mmol) were added, the mixture was purged again with $N_2$ then stirred at 100°C overnight. The mixture was filtered off then the filtrate was evaporated. The crude was purified by preparative LC (regular SiOH, 30 μm, 50 g, dry loading (on Celite®), mobile phase gradient: from heptane 90%, EtOAc/MeOH (9:1) 10% to Heptane 50%, EtOAc/MeOH (9:1) 50% over 12 CV) to obtain 317 mg of intermediate V1 as a colorless gum which crystallized on standing (66%).

Preparation of intermediate V2

To a solution of intermediate V1 (317 mg, 0.894 mmol) in water (4 mL) and EtOH (4 mL) was added NaOH (107 mg, 2.68 mmol) and the mixture was stirred at room temperature for 24 hours. The mixture was evaporated to afford 518 mg of intermediate V2 as a yellow gum. The crude was used as such in next step.

Preparation of compound 43

Accordingly, compound 43 was prepared in the same way as compound 42, starting from intermediate V2 (0.9 mmol) and intermediate E9 (0.84 mmol) affording 0.113 g (22%) as a white solid.

**$^1$H NMR** (500 MHz, DMSO-d6) δ ppm 8.93 (s, 1 H), 8.38 (t, J = 6.0 Hz, 1 H), 7.58 (d, J = 9.1 Hz, 1 H), 7.26 - 7.36 (m, 4 H), 7.19 (d, J = 8.5 Hz, 2 H), 4.43 - 4.51 (m, 4 H), 4.08 (br t, J = 4.6 Hz, 2 H), 3.83 (t, J = 4.7 Hz, 2 H), 3.30 (s, 3 H), 2.96 (q, J = 7.4 Hz, 2 H), 1.25 (t, J = 7.6 Hz, 3 H).

**Synthesis of compound 44**

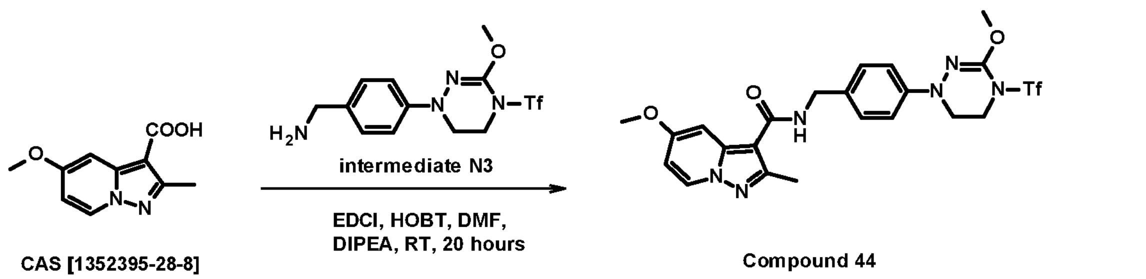


CAS [1352395-28-8]

intermediate N3

Compound 44

Accordingly, compound 44 was prepared in the same way as compound 42, starting from 5-methoxy-2-methylpyrrazolo[1,5-a]-pyridine-3-carboxylic acid (CAS [1352395-28-8], 0.37 mmol) and intermediate N3 (0.37 mmol) affording 0.19 g (42%) as a white solid.
**$^1$H NMR** (500 MHz, DMSO-$d_6$) δ ppm 8.51 (d, *J*=7.6 Hz, 1 H) 7.91 (t, *J*=6.0 Hz, 1 H) 7.43 (t, *J*=8.7 Hz, 1 H) 7.26 (d, *J*=2.8 Hz, 1 H) 7.12 - 7.23 (m, 2 H) 6.64 (dd, *J*=7.6, 2.8 Hz, 1 H) 4.44 (d, *J*=5.7 Hz, 2 H) 4.07 - 4.15 (m, 2 H) 3.86 (s, 3 H) 3.82 (s, 3 H) 3.53 - 3.60 (m, 2 H) 2.53 (s, 3 H)

**Synthesis of compound 45**

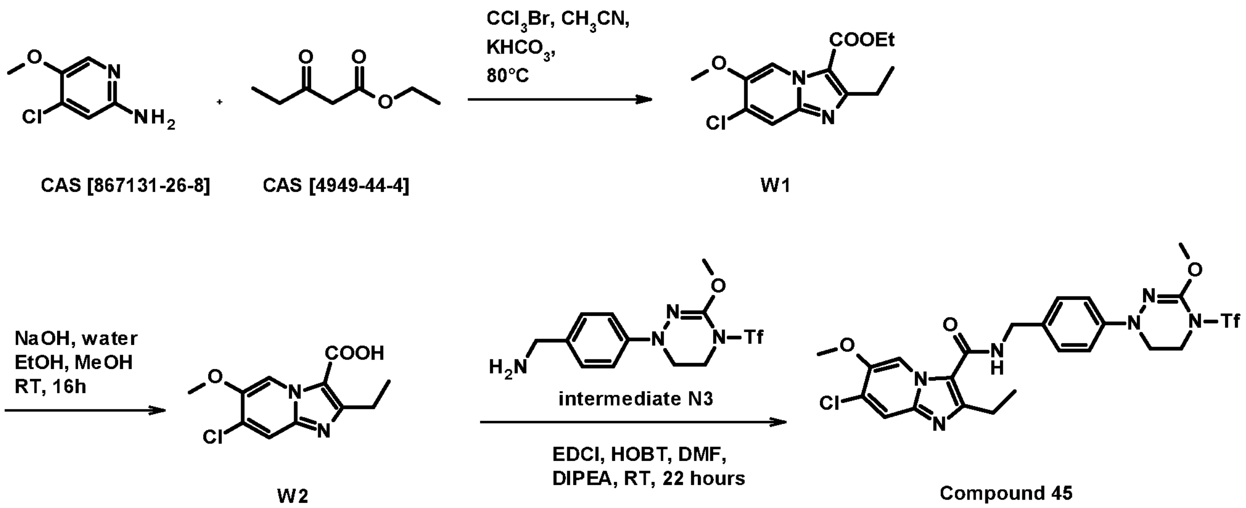


Preparation of intermediate W1

To a solution of 4-chloro-5-methoxypyridin-2-amine (CAS [867131-26-8], 500 mg, 3.15 mmol) in dry acetonitrile (7.5 mL) were added ethyl 3-oxovalerateethyl 3-oxovalerate (0.90 mL, 6.3 mmol), bromotrichloromethane (1.1 mL, 11 mmol) and potassium bicarbonate (947 mg, 9.46 mmol). The mixture was stirred at 80 °C for 16 hours. The reaction mixture was diluted in EtOAc and water. The organic layer was then washed with brine, dried over $MgSO_4$, filtered off and evaporated. The residue was purified by preparative LC (irregular SiOH 15-40 μm, 40 g, dry loading on celite®, mobile phase gradient: Heptane/EtOAc 95/5 to Heptane/EtOAc 40/60 in 15 CV) to give 458 mg of intermediate W1 as a yellow solid (51% yield).

Preparation of intermediate W2

A mixture of intermediate W1 (456 mg, 1.61 mmol) and NaOH (194 mg, 4.86 mmol) in water (8.1 mL), EtOH (8.1 mL) and MeOH (9.8 mL) was stirred at room temperature for 16 hours. The reaction mixture was evaporated. The residue was solubilized with MeOH and acidified with a 3N aqueous solution ofHCl. The solution was evaporated to give 726 mg of a yellow solid. DCM and MeOH were added to the yellow solid. The mixture was then filtered off and the filtrate was evaporated to give 443 mg of intermediate W2 as a beige solid (93% purity, quantitative).

Preparation of compound 45

Accordingly, compound 45 was prepared in the same way as compound 42, starting from intermediate W2 (0.46 mmol) and intermediate N3 (0.46 mmol) affording 0.19 g (69%) as a beige solid.
**$^1$H NMR** (400 MHz, DMSO-$d_6$) δ ppm 8.77 (s, 1 H) 8.32 (t, *J*=5.8 Hz, 1 H) 7.86 (s, 1 H) 7.29 (d, *J*=8.6 Hz, 2 H) 7.15 (d, *J*=8.7 Hz, 2 H) 4.46 (br d, *J*=5.7 Hz, 2 H) 4.10 (br t, *J*=4.8 Hz, 2 H) 3.87 (s, 3 H) 3.85 (s, 3 H) 3.74 (br t, *J*=4.8 Hz, 2 H) 2.95 (q, *J*=7.5 Hz, 2 H) 1.24 (t, *J*=7.5 Hz, 3 H)

**Synthesis of compound 46**

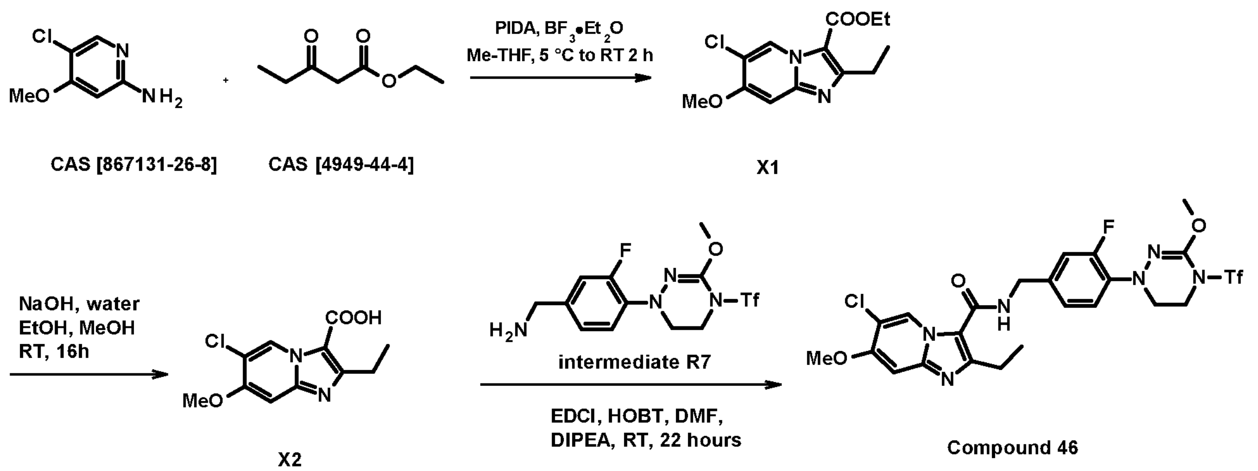


Preparation of intermediate X1

Accordingly, intermediate X1 was prepared in the same way as intermediate T1 starting from 5-chloro-4-methoxypyridin-2-amine CAS [662117-63-7] (6.31 mmol) affording 1.23 g (69%) as a light-yellow solid.

Preparation of intermediate X2

Accordingly, intermediate X2 was prepared in the same way as intermediate V2 starting from intermediate X1 (4.35 mmol) affording 0.83 g (75%) as a light-yellow solid.

Preparation of compound 46

Accordingly, compound 46 was prepared in the same way as compound compound 42, starting from intermediate X2 (0.45 mmol) and intermediate R7 (0.43 mmol) affording 0.14 g (48%) as a white solid.
**$^1$H NMR** (500 MHz, DMSO-d6) δ ppm 9.11 (s, 1 H), 8.27 (br t, J = 5.8 Hz, 1 H), 7.44 (t, J = 8.5 Hz, 1 H), 7.16 - 7.25 (m, 3 H), 4.47 (br d, J = 5.7 Hz, 2 H), 4.08 - 4.13 (m, 2 H), 3.95 (s, 3 H), 3.83 (s, 3 H), 3.54 - 3.59 (m, 2 H), 2.96 (q, J = 7.5 Hz, 2 H), 1.27 (t, J = 7.5 Hz, 3 H)

**Synthesis of compound 47**

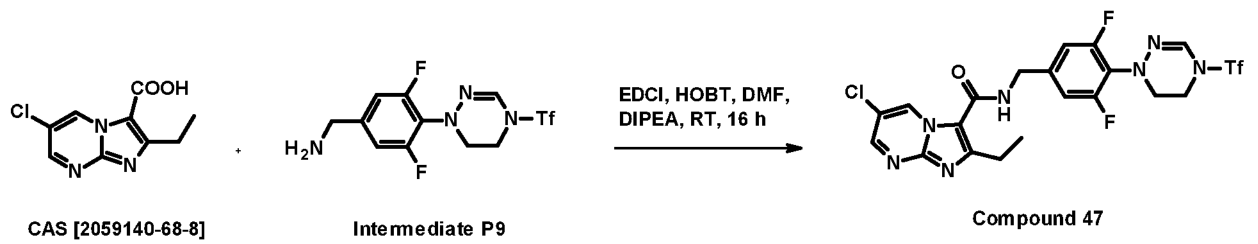


Accordingly, compound 47 was prepared in the same way as compound 42, starting from intermediate 6-Chloro-2-ethyl-imidazo[1,2-a]-pyrimidine-3-carboxylic acid CAS [2059140-68-8] (0.38 mmol) and intermediate P9 (0.31 mmol) affording 0.027 g (15%) as a white fluffy solid.
**$^1$H NMR** (400 MHz, DMSO-$d_6$) δ ppm 9.35 (d, *J* = 2.7 Hz, 1 H), 8.63 (d, *J* = 2.7 Hz, 1 H), 8.52 (t, *J* = 5.9 Hz, 1 H), 7.21 (s, 1 H), 7.12 (d, *J* = 9.4 Hz, 2 H), 4.46 (br d, *J* = 5.7 Hz, 2 H), 4.01 (br s, 2 H), 3.57 (br t, *J* = 4.3 Hz, 2 H), 2.98 (q, *J* = 7.5 Hz, 2 H), 1.23 (t, *J* = 7.5 Hz, 3 H)

**Synthesis of compound 48**

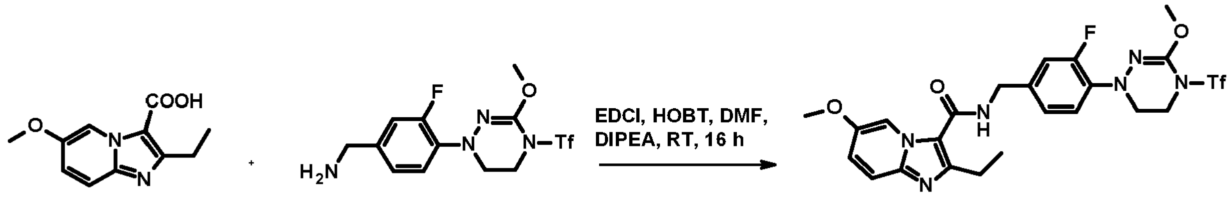


Intermediate Q2 Intermediate R7 Compound 48

Accordingly, compound 48 was prepared in the same way as compound 42, starting from intermediate Q2 (0.52 mmol) and intermediate R7 (0.51 mmol) affording 0.15 g (52%) as a white solid.
**$^1$H NMR** (500 MHz, DMSO-d6) δ ppm 8.67 (d, J = 2.2 Hz, 1 H), 8.31 (t, J = 5.8 Hz, 1 H), 7.54 (d, J = 9.8 Hz, 1 H), 7.45 (t, J = 8.7 Hz, 1 H), 7.15 - 7.25 (m, 3 H), 4.49 (d, J = 5.7 Hz, 2 H), 4.07 - 4.14 (m, 2 H), 3.83 (s, 3 H), 3.78 (s, 3 H), 3.54 - 3.60 (m, 2 H), 2.98 (q, J = 7.6 Hz, 2 H), 1.26 (t, J = 7.6 Hz, 3 H)

## Synthesis of compound 49

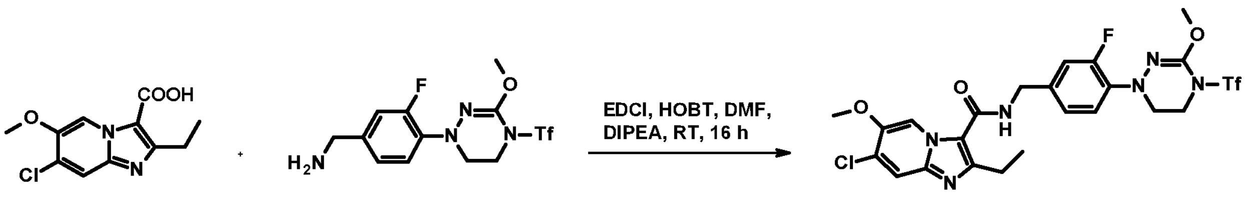


intermediate W2 intermediate R7 Compound 49

Accordingly, compound 49 was prepared in the same way as compound 42, starting from intermediate W2 (0.44 mmol) and intermediate R7 (0.44 mmol) affording 0.164 g (62%) as a white solid.
**$^1$H NMR** (500 MHz, DMSO-$d_6$) δ ppm 8.80 (s, 1 H) 8.36 (br t, *J*=5.8 Hz, 1 H) 7.87 (s, 1 H) 7.45 (t, *J*=8.5 Hz, 1 H) 7.15 - 7.26 (m, 2 H) 4.50 (br d, *J*=5.7 Hz, 2 H) 4.10 (br t, *J*=5.0 Hz, 2 H) 3.87 (s, 3 H) 3.82 (s, 3 H) 3.56 (br t, *J*=5.0 Hz, 2 H) 2.98 (q, *J*=7.6 Hz, 2 H) 1.26 (t, *J*=7.6 Hz, 3 H)

## Synthesis of compound 50

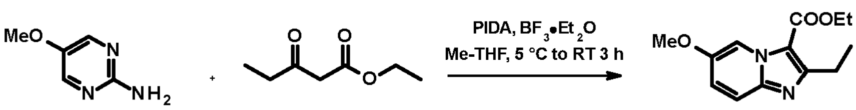


CAS [13418-77-4] CAS [4949-44-4] Y1

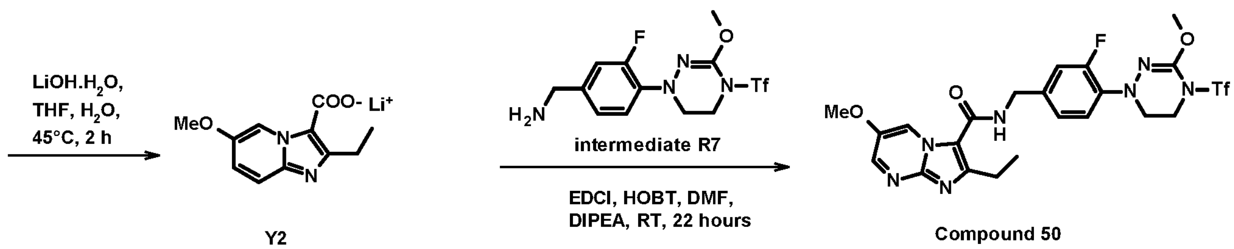


Y2 Compound 50

Preparation of intermediate Y1

Accordingly, intermediate Y1 was prepared in the same way as intermediate X1 starting from 2-amino-5-methoxypyrimidine CAS [13418-77-4] (75.92 mmol) affording 4.94 g (26%) as a yellow solid.

Preparation of intermediate Y2

To a solution of intermediate Y1 (150 mg, 0.602 mmol) in THF (3 mL) was added a solution of LiOH (75.8 mg, 1.81 mmol) in water (1.5 mL). The reaction mixture was stirred for 2 hours at 45 °C. The mixture was evaporated to afford 218 mg of intermediate Y2 as a yellow solid. The crude was used as such in next step.

Preparation of compound 50

Accordingly, compound 50 was prepared in the same way as compound 42, starting from intermediate Y2 (0.6 mmol) and intermediate R7 (0.55 mmol) affording 0.098 g (31%) as a white solid.
$^1$**H NMR** (400 MHz, DMSO-d6) δ ppm 8.96 (d, J = 2.9 Hz, 1 H), 8.52 (d, J = 2.9 Hz, 1 H), 8.41 (t, J = 5.9 Hz, 1 H), 7.45 (t, J = 8.6 Hz, 1 H), 7.15 - 7.26 (m, 2 H), 4.50 (d, J = 5.7 Hz, 2 H), 4.08 - 4.14 (m, 2 H), 3.86 (s, 3 H), 3.83 (s, 3 H), 3.53 - 3.59 (m, 2 H), 3.02 (q, J = 7.5 Hz, 2 H), 1.28 (t, J = 7.5 Hz, 3 H)

**Synthesis of compound 51 and compound 52**

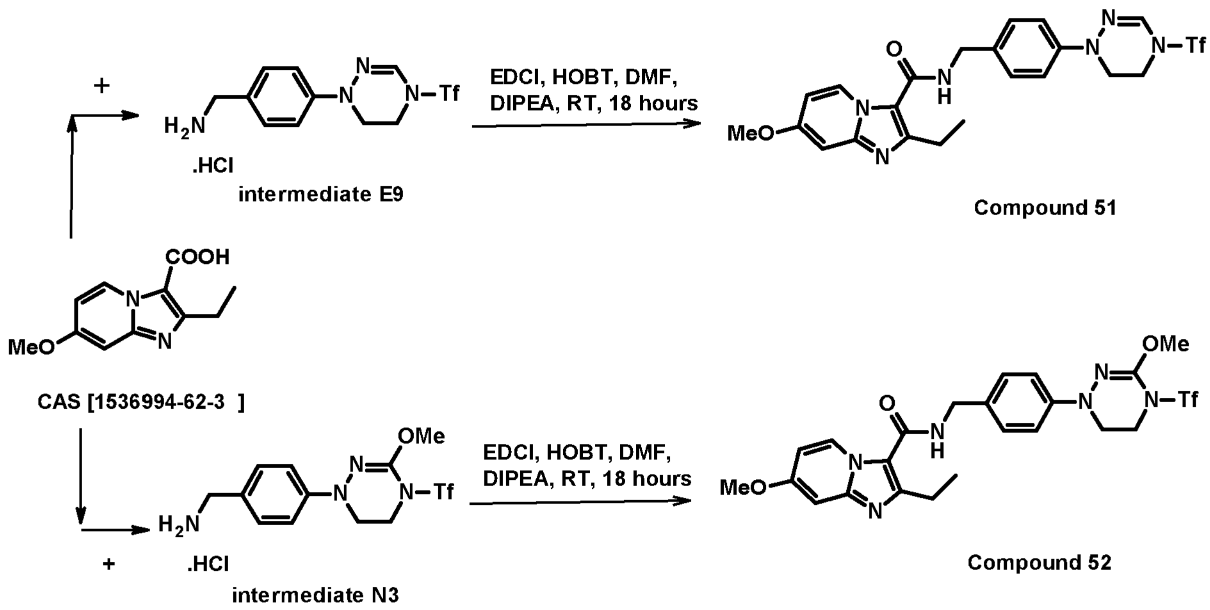


.HCl intermediate E9

Compound 51

CAS [1536994-62-3 ]

.HCl intermediate N3

Compound 52

Preparation of compound 51

Accordingly, compound 51 was prepared in the same way as compound 42, starting from 2-ethyl-7-methoxyimidazo[1,2-a]-pyridine-3-carboxylic acid (CAS [1536994-62-3], 0.46 mmol) and intermediate E9 (0.46 mmol) affording 0.195 g (72%) as a white solid.
$^1$**H NMR** (400 MHz, DMSO-$d_6$) δ ppm 8.83 (d, *J*=7.6 Hz, 1 H) 8.19 (t, *J*=5.9 Hz, 1 H) 7.25 - 7.34 (m, 3 H) 7.18 (d, *J*=8.7 Hz, 2 H) 7.00 (d, *J*=2.4 Hz, 1 H) 6.70 (dd, *J*=7.6, 2.6 Hz, 1 H) 4.44 (d, *J*=5.9 Hz, 2 H) 4.07 (br t, *J*=4.4 Hz, 2 H) 3.78 - 3.88 (m, 5 H) 2.92 (q, *J*=7.5 Hz, 2 H) 1.24 (t, *J*=7.5 Hz, 3 H)

Preparation of compound 52

Accordingly, compound 52 was prepared in the same way as compound 42, starting from 2-ethyl-7-methoxyimidazo[1,2-a]-pyridine-3-carboxylic acid (CAS [1536994-62-3], 0.46 mmol) and intermediate N3 (0.46 mmol) affording 0.178 g (69%) as a white solid.

**$^1$H NMR** (500 MHz, DMSO-$d_6$) δ ppm 8.84 (d, *J*=7.6 Hz, 1 H) 8.16 (t, *J*=6.0 Hz, 1 H) 7.28 (d, *J*=8.7 Hz, 2 H) 7.14 (d, *J*=8.7 Hz, 2 H) 6.99 (d, *J*=2.5 Hz, 1 H) 6.70 (dd, *J*=7.7, 2.7 Hz, 1 H) 4.43 (d, *J*=5.7 Hz, 2 H) 4.10 (br t, *J*=5.0 Hz, 2 H) 3.84 (m, 6 H) 3.73 (br t, *J*=5.0 Hz, 2 H) 2.91 (q, *J*=7.6 Hz, 2 H) 1.25 (t, *J*=7.6 Hz, 3 H)

**Synthesis of compound 53**

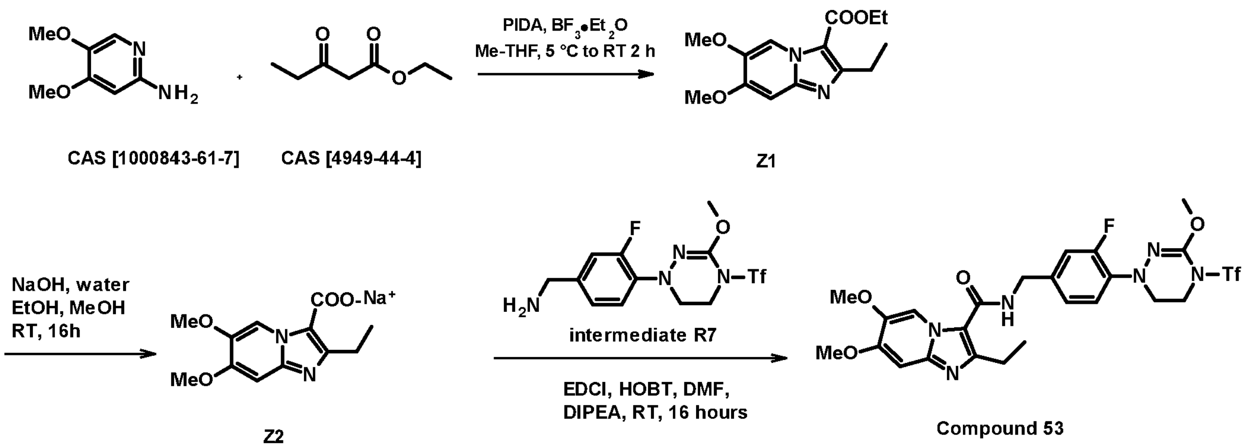


CAS [1000843-61-7] CAS [4949-44-4] Z1

Z2 Compound 53

Preparation of intermediate Z1

Accordingly, intermediate Z1 was prepared in the same way as intermediate X1 starting from 4,5-dimethoxy-pyridin-2-ylamine CAS [1000843-61-7] (1.3 mmol) affording 0.135 g (37%) as a light-yellow solid

Preparation of intermediate Z2

Accordingly, intermediate Z2 was prepared in the same way as intermediate X2 starting from intermediate Z1 (0.49 mmol) affording 0.209 g (63%) as a light-yellow solid.

Preparation of compound 53

Accordingly, compound 53 was prepared in the same way as compound 42, starting intermediate Z2 (0.48 mmol) and intermediate R7 (0.4 mmol) affording 0.149 g (39% over last 2 steps) as a white solid.
**$^1$H NMR** (400 MHz, DMSO-d6) δ ppm 8.67 (s, 1 H), 8.11 (t, J = 5.8 Hz, 1 H), 7.44 (t, J = 8.6 Hz, 1 H), 7.15 - 7.23 (m, 2 H), 7.05 (s, 1 H), 4.47 (d, J = 5.7 Hz, 2 H), 4.07 - 4.14 (m, 2 H), 3.87 (s, 3 H), 3.83 (s, 3 H), 3.76 (s, 3 H), 3.53 - 3.59 (m, 2 H), 2.95 (q, J = 7.5 Hz, 2 H), 1.25 (t, J = 7.5 Hz, 3 H)

**Synthesis of compound 54**

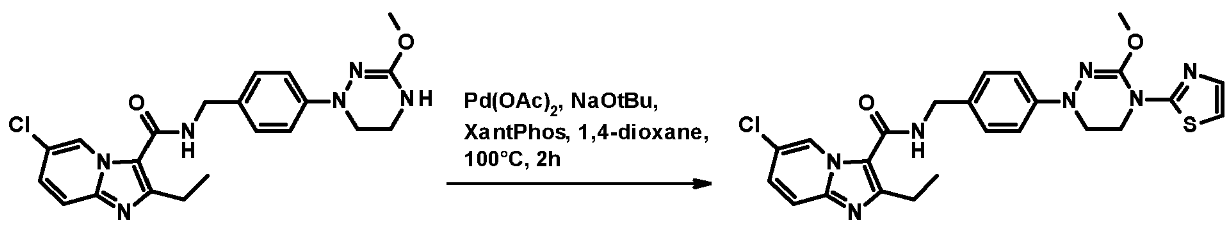


Intermediate C1 Compound 54

A mixture of intermediate C1 (190 mg, 0.445 mmol), 2-bromothiazole (48.1 μL, 0.534 mmol) and sodium tert-butoxide (214 mg, 2.23 mmol) in dry 1,4-dioxane (5 mL) was purged with $N_2$ (3 times). XantPhos (51.5 mg, 89.0 μmol) and $Pd(OAc)_2$ (9.99 mg, 44.5 μmol) were added and the mixture was purged with $N_2$ (3 times). The reaction mixture was stirred at 100 °C for 2 hours. The reaction mixture was diluted with EtOAc/MeOH (95/5) and water. The aqueous layer was extracted with EtOAc (twice). The combined organic layers were washed with brine, dried over $MgSO_4$, filtered off and evaporated to give a yellow solid. The solid was purified by preparative LC (regular SiOH 30 μm, 25 g, dry loading (celite®), mobile phase gradient: DCM 100% to DCM/(DCM: MeOH 80:20) 90/10 in 15 CV). The fractions containing product were combined and evaporated under vacuum to give a pale-yellow solid. The solid was triturated in $Et_2O$, filtered off, washed with $Et_2O$ and then dried under vacuum to give 153 mg of compound 54 as a white solid (67% yield).
**$^1$H NMR** (500 MHz, DMSO-$d_6$) δ ppm 9.08 (d, *J*=1.5 Hz, 1 H) 8.42 (t, *J*=5.9 Hz, 1 H) 7.66 (d, *J*=9.6 Hz, 1 H) 7.45 (dd, *J*=9.5, 2.1 Hz, 1 H) 7.40 (d, *J*=3.7 Hz, 1 H) 7.27 (d, *J*=8.7 Hz, 2 H) 7.22 (d, *J*=8.7 Hz, 2 H) 7.17 (d, *J*=3.7 Hz, 1 H) 4.46 (d, *J*=5.8 Hz, 2 H) 4.20 (t, *J=5.1* Hz, 2 H) 3.92 (s, 3 H) 3.67 (t, *J*=5.1 Hz, 2 H) 2.98 (q, *J*=7.6 Hz, 2 H) 1.26 (t, *J*=7.6 Hz, 3 H)

**Synthesis of compound 55**

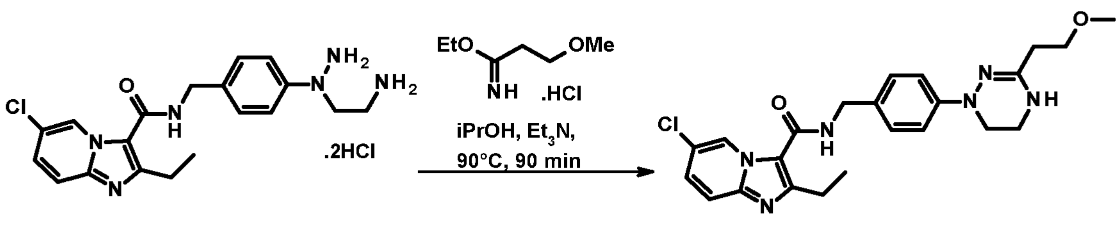


**Intermediate A5** **Intermediate AA1**

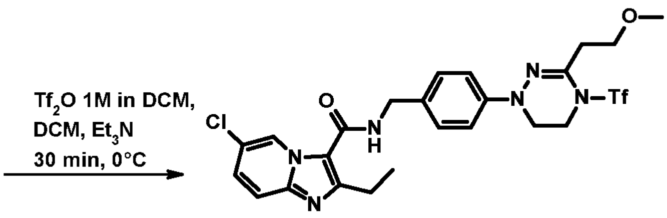


**Compound 55**

Preparation of intermediate AA1

In a sealed tube, a mixture of intermediate A5 (300 mg, 0.652 mmol), 3-methoxypropionimidic acid ethyl ester hydrochloride (328 mg, 1.96 mmol) and triethylamine (272 μL, 1.96 mmol) in 2-propanol (6 mL) was stirred for 1.5 h at 90 °C. After cooling to room temperature, the reaction mixture was concentrated. The residue was taken up in EtOAc and aqueous solution of NaHCOs (1%) was added. After separation, the aqueous phase was extracted with EtOAc (twice). The combined organic layers were dried over $MgSO_4$, filtered off and concentrated to give 280 mg of intermediate AA1 as a light-yellow oil which crystallized on standing (94%).

Preparation of compound 55

Triethylamine (0.281 mL, 2.02 mmol) was added to a solution of intermediate AA1 (230 mg, 0.506 mmol) in dry DCM (4.6 mL). The solution was then cooled at 0 °C (ice / water bath). A 1M solution of $Tf_2O$ (1.01 mL, 1.01 mmol) was added dropwise and the reaction mixture was stirred at 0 °C for 30 min. DCM and an aqueous solution of NaHCOs (10%) were added. The layers were separated, and the aqueous layer was extracted with DCM. The combined organic layers were dried over $MgSO_4$, filtered off and evaporated to obtain a brown gum which was purified by preparative LC (regular SiOH, 30 μm, 24 g, liquid loading (DCM), mobile phase gradient: from Heptane 90%, EtOAc/MeOH (9:1) 10% to Heptane 25%, EtOAc/MeOH (9:1) 75% over 12 CV). Fractions containing product were combined and evaporated to give 208 mg as a yellow solid. It was purified by Reverse phase (Stationary phase: YMC-actus Triart C18 25 μm 30*150 mm, 40 g, dry loading (Celite®) Mobile phase: Gradient from 60%
(aq. $NH_4HCO_3$ 0.2%), 40% MeCN to 100% MeCN over 12 CV). Fractions containing product were combined

and evaporated to afford 175 mg as a yellow solid. It was purified by preparative LC (regular SiOH, 30 μm, 24 g, liquid loading (DCM), mobile phase gradient: from Heptane 90%, EtOAc/MeOH (9:1) 10% to Heptane 25%, EtOAc/MeOH (9:1) 75% over 12 CV). Fractions containing product were combined and evaporated to give 146 mg as a white solid. This one was purified by Reverse phase (Stationary phase: YMC-actus Triart C18 25 μm 30* 150 mm, 40 g, dry loading (Celite®) Mobile phase: Gradient from 60% (aq. $NH_4HCO_3$ 0.2%), 40% MeCN/MeOH (1:1) to 15% (aq. $NH_4HCO_3$ 0.2%), 85% MeCN/MeOH (1:1) over 14 CV). Fractions containing product were combined and evaporated to afford 129 mg as a white solid. It was purified by achiral SFC (Stationary phase: diethylaminopropyl 5μm 150x21.2mm, Mobile phase: 90% $CO_2$, 10% MeOH). Fractions containing product were combined and evaporated to afford 94 mg as a white solid. This one was sonicated in MeCN (10 mL) and evaporated (3 times) then MeCN (5 mL) was added, the product was filtered and dried under high vacuum (50 °C, 2 h) to afford 84 mg of compound 55 as a white solid (28%)

**$^1$H NMR** (400 MHz, DMSO-d6) δ ppm 9.07 (d, J = 1.5 Hz, 1 H), 8.44 (br t, J = 5.7 Hz, 1 H), 7.67 (d, J = 9.4 Hz, 1 H), 7.45 (dd, J = 9.4, 2.1 Hz, 1 H), 7.32 (m, J = 8.7 Hz, 2 H), 7.16 (m, J = 8.7 Hz, 2 H), 4.47 (br d, J = 5.9 Hz, 2 H), 3.90 - 4.00 (m, 2 H), 3.81 - 3.89 (m, 2 H), 3.66 (t, J = 6.7 Hz, 2 H), 3.26 - 3.29 (m, 3 H), 2.98 (q, J = 7.5 Hz, 2 H), 2.82 (t, J = 6.7 Hz, 2 H), 1.26 (t, J = 7.5 Hz, 3 H)

The following compounds were prepared in accordance with the procedures described herein:

Compound 56

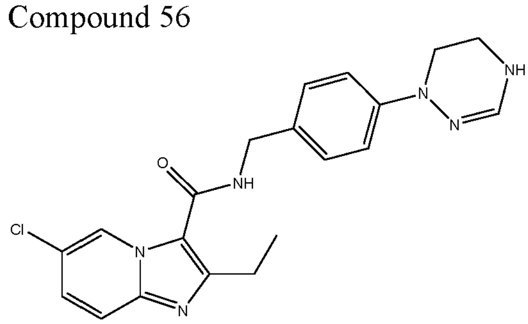

Compound 57

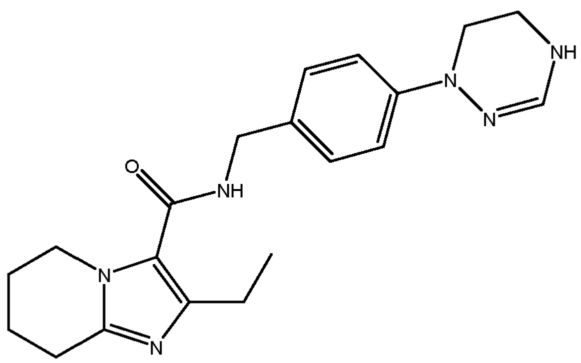

Compound 58

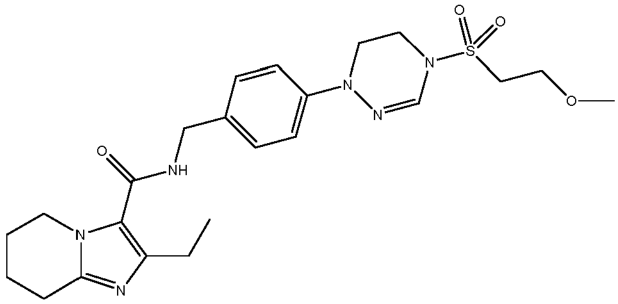

Compound 59

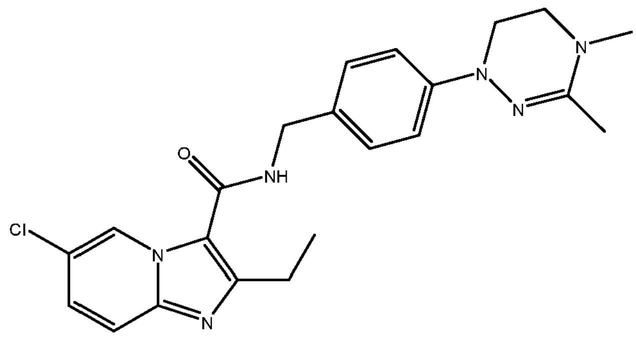

Compound 60

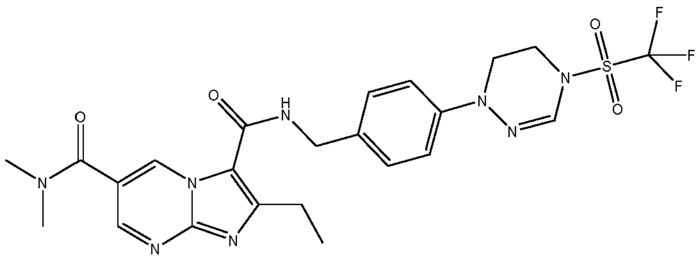

Compound 61

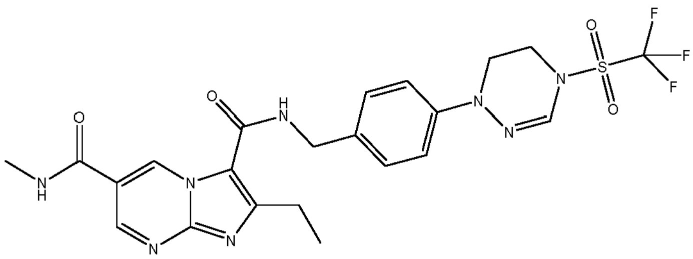

Compound 62

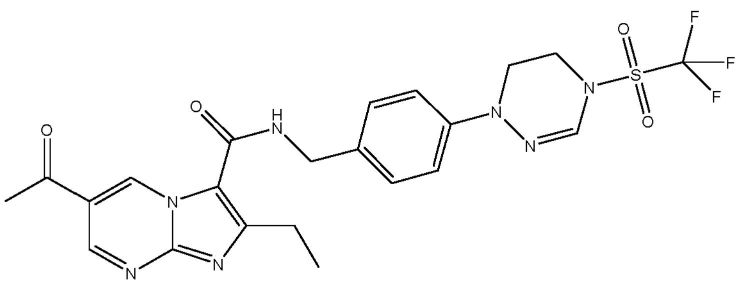

Compound 63

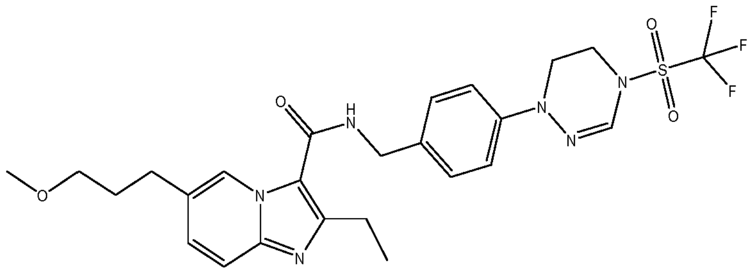

Compound 64

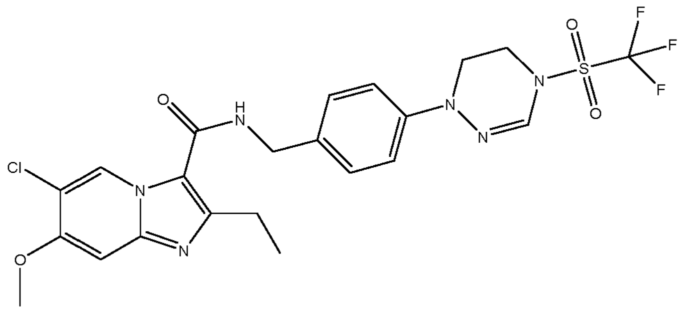

Compound 65

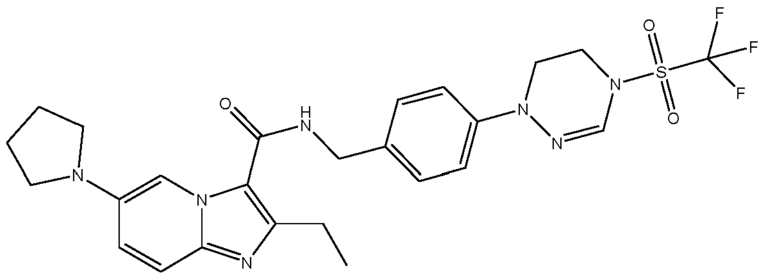

Compound 66

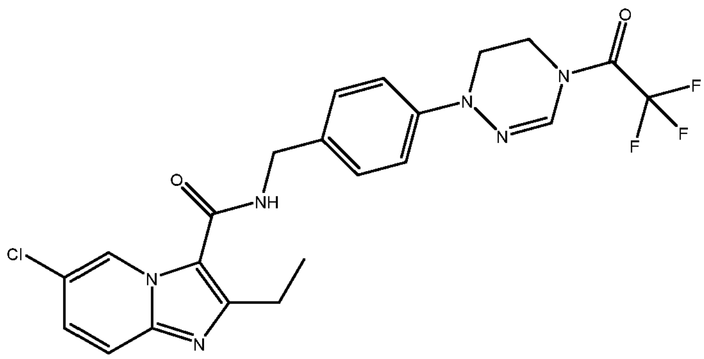

Compound 67

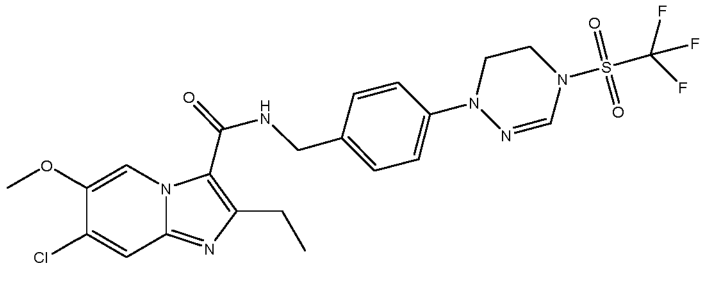

Compound 68

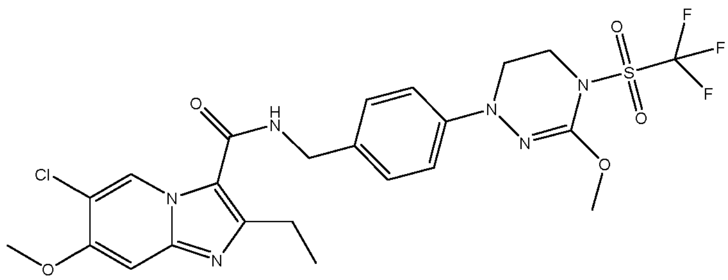

Compound 69

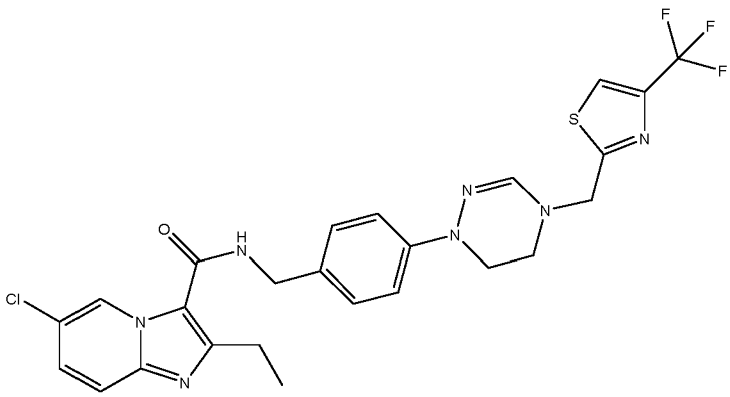

Compound 70

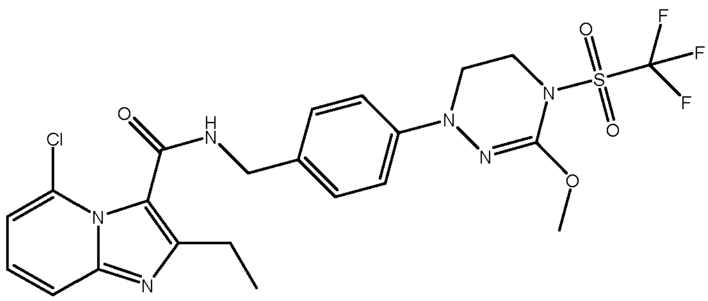

Compound 71

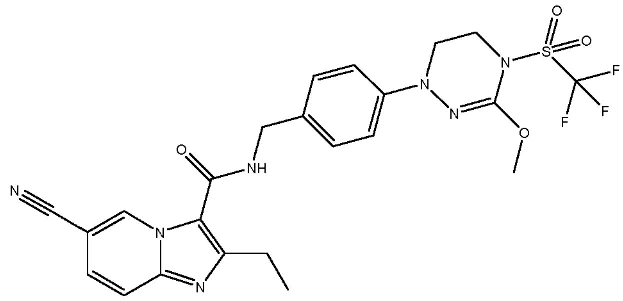

Compound 72

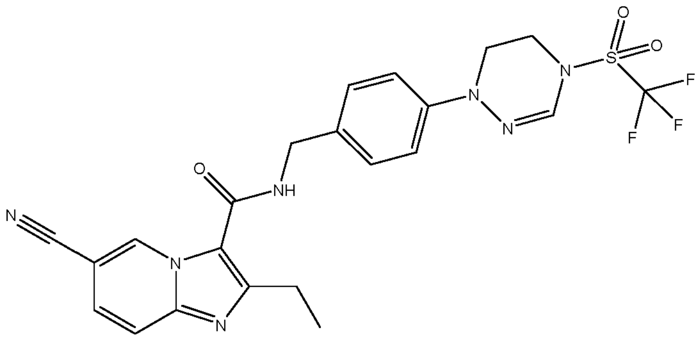

**Synthesis of compound 73**

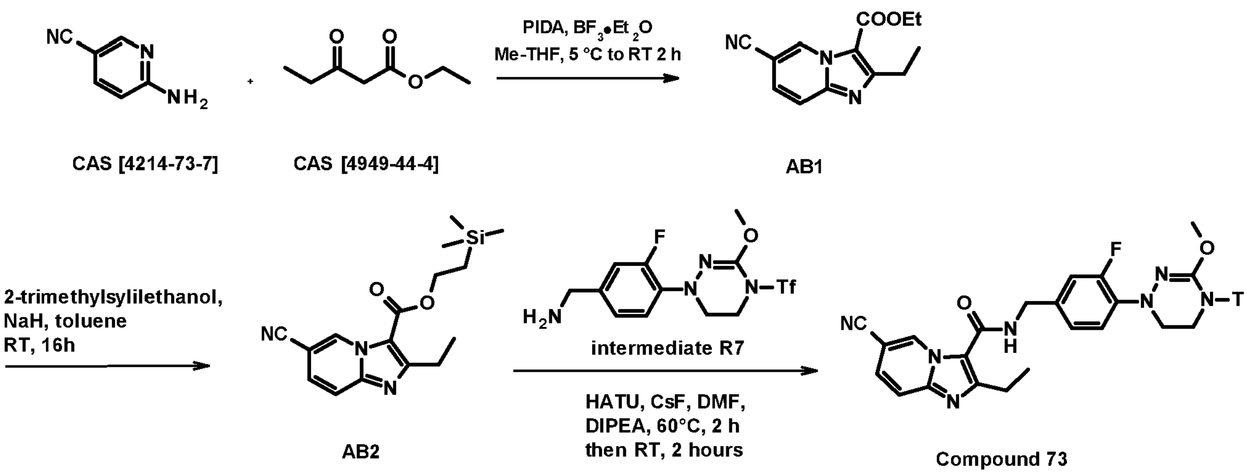


CAS [4214-73-7] CAS [4949-44-4] AB1

AB2 Compound 73

Preparation of intermediate AB1

To a solution of 2-amino-5-cyanopyridine (CAS [4214-73-7]; 5 g, 42.0 mmol) in Me-THF (200 mL) at 5 °C were added iodobenzene diacetate (13.5 g, 41.9 mmol) and ethyl-3-oxovalerate (10 mL, 70.1 mmol). Then boron trifluoride etherate (550 µL, 2.10 mmol) was added dropwise. The solution was stirred at 5°C for 1 h. The mixture was warmed to room temperature and stirred for 2 hours. EtOAc and a sat. solution of NaHCOs were added. The layers were separated, and the aqueous layer was extracted with EtOAc. The combined organic layers were washed with brine (twice), dried over $MgSO_4$, filtered off then evaporated to give 26 g of a brown liquid (which crystallized on standing). The crude product was purified by preparative LC (irregular SiOH, 15-40 µm, 330 g, Grace, dry loading (Celite®), mobile phase gradient: from heptane 85%, EtOAc 15% to heptane 30%, EtOAc 70%) to afford 3.14 g of the intermediate AB1 as a yellow solid (30%).

Preparation of intermediate AB2

Under Nitrogen, NaH 60% (0.677 g; 16.9 mmol) was added to a solution of 2-(trimethylsilyl)ethanol (2.43 mL; 16.9 mmol) in dry toluene (50 mL) at 0 °C. The reaction mixture was stirred at 0 °C for 15 min then intermediate AB1 (0.823 g; 3.38 mmol) was added and the reaction mixture was stirred for 16 h warming to room temperature. The reaction mixture was hydrolyzed with a aqueous saturated solution of NH4Cl and extracted with EtOAc. The aqueous layer was extracted with EtOAc (twice). The combined organic layers were dried over $MgSO_4$, filtered, evaporated to dryness and purified by preparative LC (Regular SiOH, 30-40 μm, 40 g, loading (DCM), mobile phase gradient: Heptane / EtOAc from 100:0 to 50:50). The fractions containing product were evaporated to give 559 mg of intermediate AB2 as a white solid (52%).

Preparation of compound 73

Cesium fluoride (289 mg, 1.90 mmol) was added to a solution of intermediate AB2 (200 mg, 0.634 mmol) in F (8.4 mL) and the reaction mixture was stirred at 60 °C for 2 h. Then diisopropylethylamine (139 μL, 0.817 mmol) and HATU (267 mg, 0.701 mmol) were added and the reaction mixture was stirred at room temperature for 15 min (the reaction mixture turned to brown). Intermediate R7 (266 mg, 0.634 mmol) was added and the reaction mixture was stirred at room temperature for 2 hours.

The reaction mixture was diluted with EtOAc, and the organic layer was washed with an aqueous solution of NaHCO3 1%, then with water and brine, dried over $MgSO_4$, filtered off and concentrated. DCM and MeOH were added to the residue. The mixture was filtered. The precipitate was dried under vacuum at 50 °C to give 160 mg of a crude product as a white solid.

The crude product was heated to reflux with EtOAc (15 mL) for 20 min then slowly cooled down to room temperature for 18 hours with slowly stirring.

The solid was filtered, rinced with cooled EtOAc and dried under vacuum at 60 °C to give 128 mg of compound 73 as white solid (36%).

1H NMR (400 MHz, DMSO-d6) δ ppm 9.50 (s, 1 H) 8.63 (t, J=5.9 Hz, 1 H) 7.78 (d, J=9.3 Hz, 1 H) 7.66 (dd, J=9.3, 1.7 Hz, 1 H) 7.45 (t, J=8.6 Hz, 1 H) 7.13 - 7.31 (m, 2 H) 4.51 (d, J=5.87 Hz, 2 H) 4.06 - 4.19 (m, 2 H) 3.53 - 3.62 (m, 2 H) 3.02 (q, J=7.50 Hz, 2 H) 1.28 (t, J=7.46 Hz, 3 H).

**Synthesis of compound 74**

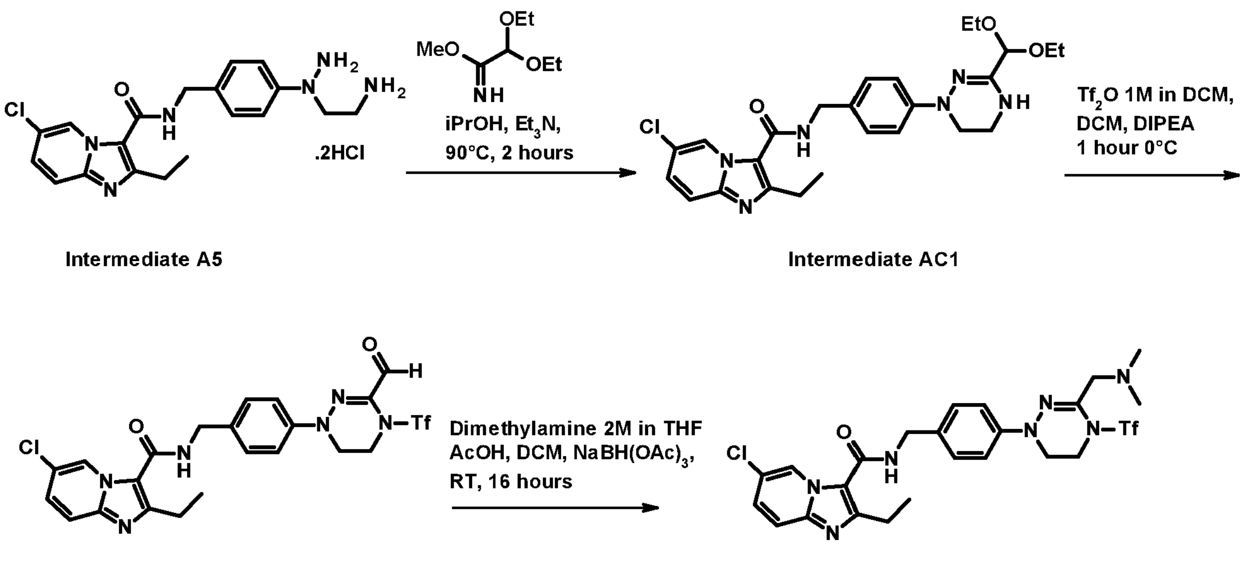


Intermediate A5 — Intermediate AC1

Intermediate AC1 — Compound 74

paration of intermediate AC1

A mixture of intermediate A5 (500 mg, 1.09 mmol), methyl-2,2-diethoxyacetimidate (526 mg, 3.26 mmol) and triethylamine (453 μL, 3.26 mmol) in iPrOH (9.4 mL) was stirred for 2 h at 90 °C. After cooling to room temperature, the

reaction mixture was concentrated. The residue was taken up in EtOAc and water. After separation, the aqueous phase was extracted with EtOAc (once). The combined organic layers were washed with brine, dried over $MgSO_4$, filtered off and concentrated. The residue was purified by preparative LC (irregular SiOH 15-40 μm, 80 g, liquid loading (DCM), mobile phase gradient: EtOAc in heptane from 20 to 80% then isocratic). Fractions containing product were combined and evaporated to give 343 mg of intermediate AC1 as a white solid (63%).

Preparation of intermediate AC2

Diisopropylethylamine (0.311 mL, 1.80 mmol) was added to a solution of intermediate AC1 (300 mg, 0.601 mmol) in DCM (5.5 mL). The solution was then cooled at 0 °C (ice / water bath). A 1M solution of $Tf_2O$ in DCM (0.721 mL, 1.2 eq., 0.721 mmol) was added dropwise and the reaction mixture was stirred at 0 °C for 1 h. An extra amount of a 1M solution of $Tf_2O$ in DCM (0.721 mL, 1.2 eq., 0.721 mmol) was added and the mixture was stirred at 0 °C for 1 hour. A saturated aqueous solution of $NaHCO_3$ and DCM were added. The layers were separated, and the aqueous layer was extracted with DCM. The combined organic layers were dried over $MgSO_4$, filtered off and evaporated to afford a brown gum. This crude product was purified by preparative LC (regular SiOH, 30 μm, 24 g, liquid loading (DCM), mobile phase gradient: from DCM 100% to DCM 85%, MeOH/AcOH (9:1) 15%) to afford 94 mg of intermediate AC2 as an orange gum.

Preparation of compound 74

To a solution of intermediate AC2 (94 mg, 0.17 mmol) in AcOH (29 μL, 0.51 mmol) and DCM (1.5 mL) was added a 2M solution of dimethylamine in THF (0.25 mL, 0.51 mmol) and the mixture was stirred at room temperature for 6 hours. Then, sodium triacetoxyborohydride (71.5 mg, 0.34 mmol) was added and the mixture was stirred at room temperature for 16 hours. A saturated aqueous solution of NaHCOs was added carefully then the layers were separated. The aqueous layer was extracted with DCM (twice) then the combined organic layers were dried over $MgSO_4$, filtered off and evaporated. The crude product was purified by preparative LC (regular SiOH, 30 μm, 12 g, liquid loading (DCM), mobile phase gradient: from heptane 80%, EtOAc/MeOH (9:1) 20% to Heptane 15%, EtOAc/MeOH (9:1) 85%). Fractions containing product were combined and evaporated to give 68 mg as a light-yellow oil which was purified by Reverse phase (Stationary phase: YMC-actus Triart C18 25 μm 30*150 mm, 12 g, dry loading (Celite®) Mobile phase: Gradient from 55% (aq. $NH_4HCO_3$ 0.2%), 45% MeCN to 100% MeCN). Fractions containing product were combined and evaporated to afford a colorless oil which was triturated in $Et_2O$, dried under high vacuum (50 °C, 1 h) to afford 40 mg of compound 74 as a white solid (40%).

$^1H$ NMR (400 MHz, DMSO-$d_6$) δ ppm 9.06 (d, *J*=1.0 Hz, 1 H) 8.44 (br t, *J*=5.8 Hz, 1 H) 7.67 (d, *J*=9.7 Hz, 1 H) 7.45 (dd, *J*=9.4, 1.8 Hz, 1 H) 7.33 (br d, *J*=8.6 Hz, 2 H) 7.19 (br d, *J*=8.6 Hz, 2 H) 4.47 (br d, *J*=5.5 Hz, 2 H) 3.90 (br dd, *J*=16.6, 4.2 Hz, 4 H) 2.97 (q, *J*=7.5 Hz, 2 H) 2.19 (s, 7 H) 1.26 (t, *J*=7.5 Hz, 4 H).

**Synthesis of compound 75**

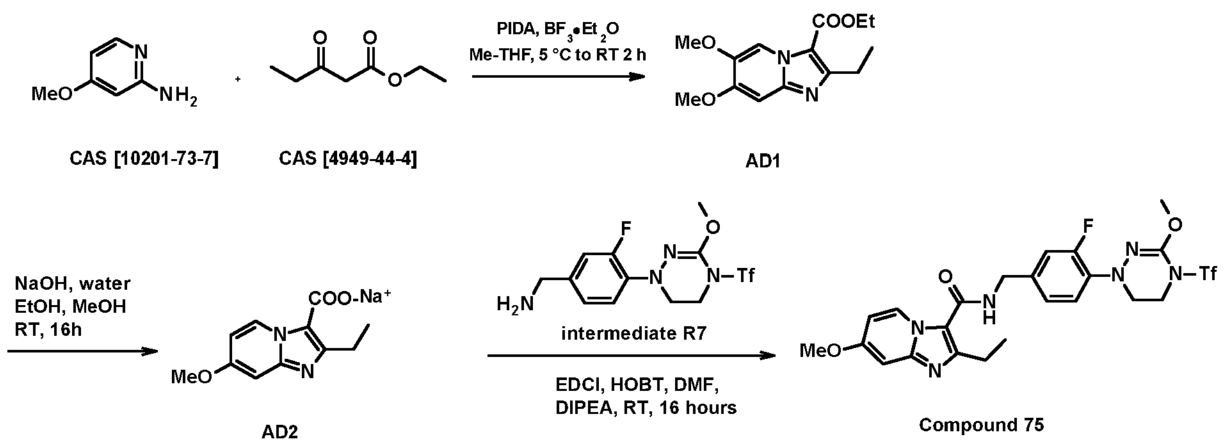


Preparation of intermediate AD1

Carbon tetrabromide (26.9 g, 81.0 mmol) was added to a solution of 2-amino-4-methoxypyridine [CAS:

10201-73-7] (5.02 g, 40.4 mmol) and ethyl-3-oxovalerate (8.69 mL, 60.8 mmol) in MeCN (85 mL) and the reaction mixture was stirred at 80 °C for 4 hours. The reaction mixture was evaporated until dryness then purified by preparative LC (regular SiOH, 30 μm, 330 g, dry loading (Celite®), mobile phase gradient: from Heptane/EtOAc 95/5 to EtOAc) to give 669 mg of intermediate AD1(16%).

Preparation of intermediate AD2

To a mixture of intermediate AD1 (1.55 g, 6.24 mmol) in water (20 mL) and EtOH (20 mL) was added NaOH (752 mg, 18.8 mmol) and the mixture was stirred at room temperature for 2 days. The reaction mixture was evaporated to give 2.16 g of intermediate AD2 (Quant.)

Preparation of compound 75

A mixture of intermediate AD2 (138 mg, 0.397 mmol), intermediate R7 (160 mg, 397 μmol), EDCI•HCl (99.1 mg, 0.517 mmol), HOBt (79.1 mg, 0.517 mmol) and diisopropylethylamine (205 μL, 1.19 mmol) in DMF (6 mL) was stirred at room temperature for 20 hours.

The residue was dissolved in EtOAc and water. The aqueous layer was extracted with EtOAc (twice). The combined organic layers were dried over $MgSO_4$, filtered off and evaporated to give an orange oil. The oil was purified by preparative LC (regular SiOH 30 μm, 12 g, dry loading (celite®), mobile phase gradient: Heptane/EtOAc 70/30 to EtOAc 100%). The fractions containing product were combined and evaporated under vacuum to give a yellow solid which was triturated in $Et_2O$. The supernatant was removed by pipette and the solid was dried under vacuum to give 124 mg of a white solid which was co-evaporated in $Et_2O$ (3 times) to give 120 mg of comound 75 as a white solid (46% yield).

$^1H$ NMR (400 MHz, DMSO-$d_6$) δ ppm 8.86 (d, *J*=7.7 Hz, 1 H) 8.21 (br t, *J*=5.8 Hz, 1 H) 7.44 (t, *J*=8.5 Hz, 1 H) 7.12 - 7.26 (m, 2 H) 7.01 (d, *J*=2.3 Hz, 1 H) 6.71 (dd, *J*=7.6, 2.5 Hz, 1 H) 4.47 (br d, *J*=5.9 Hz, 2 H) 4.07 - 4.15 (m, 2 H) 3.84 (d, J=8.2 Hz, 6 H) 3.52 - 3.61 (m, 2 H) 2.94 (q, *J*=7.5 Hz, 2 H) 1.26 (t, *J*=7.5 Hz, 3 H).

**Synthesis of Compound 76**

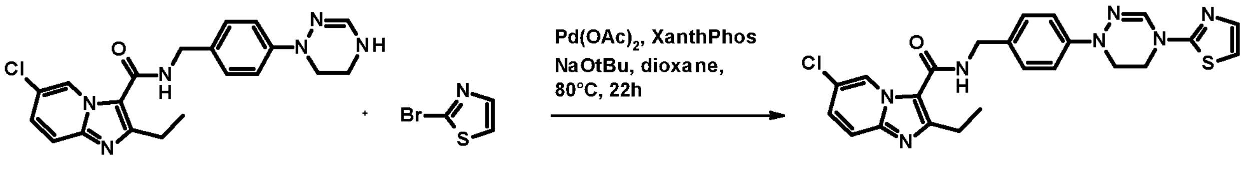


Intermediate A6 compound 76

A mixture of intermediate A6 (30.0 mg, 75.6 μmol), 2-Bromothiazole (8.18 μL, 90.7 μmol) and NaOtBu (36.3 mg, 0.378 mmol) in dry 1,4-dioxane (1.3 mL) was purged with $N_2$ (3 times). XanthPhos (8.7 mg, 15 μmol) and Palladium II acetate (1.7 mg, 7.6 μmol) were then added and the mixture was purged with $N_2$ (3 times). The reaction mixture was stirred at 80 °C for 22 hours. The reaction mixture was diluted with EtOAc/MeOH and water. The aqueous layer was extracted with EtOAc (twice). The combined organic layer was washed with brine, dried over $MgSO_4$, filtered off and evaporated to give a brown solid. The solid was purified by preparative LC (regular SiOH 30 μm, 12 g, dry loading (celite®), mobile phase gradient: DCM 100% to DCM/(DCM: MeOH 80:20) 30/70). The fractions containing product were combined and evaporated under vacuum to give 17 mg of compound 76 as yellow solid (47% yield).

$^1H$ NMR (500 MHz, DMSO-$d_6$) δ ppm 9.07 (d, *J*=1.4 Hz, 1 H) 8.45 (t, *J*=5.9 Hz, 1 H) 7.63 - 7.69 (m, 2 H) 7.45 (dd, *J*=9.5, 2.0 Hz, 1 H) 7.39 (d, *J*=3.5 Hz, 1 H) 7.26 (dd, *J*=36.7, 8.7 Hz, 2 H) 7.16 (d, *J*=3.5 Hz, 1 H) 4.46 (d, *J*=5.6 Hz, 2 H) 4.00 (t, *J*=5.0 Hz, 2 H) 3.78 (t, *J*=5.0 Hz, 2 H) 2.98 (q, *J*=7.5 Hz, 2 H)1.26 (t, *J*=7.5 Hz, 4 H).

The following compound was also prepared in accordance with the procedures described herein:

Compound 77

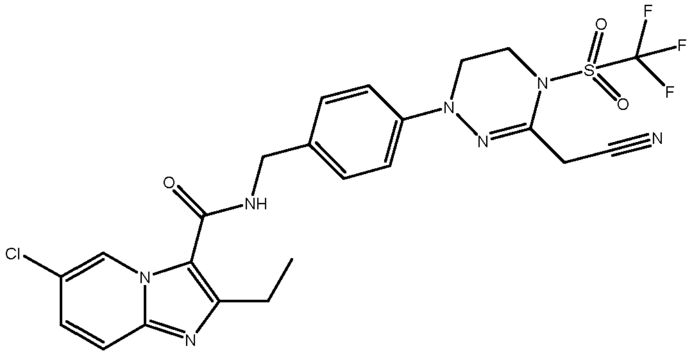

B. Further procedures

**Synthesis of compound 127**

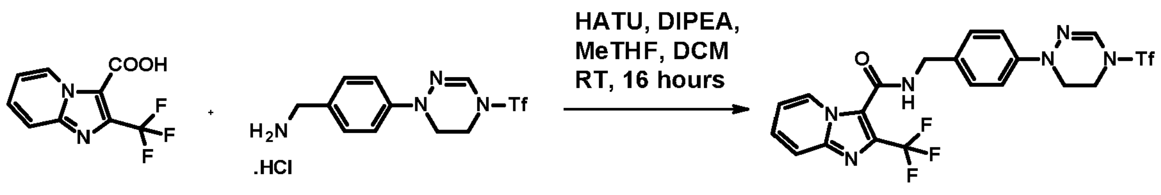


CAS [73221-19-9] Intermediate E9 compound 127

HATU (0.099 g, 0.26 mmol) was added to a solution of 2-(Trifluoromethyl)-imidazo[1,2-A]pyridine-3-carboxylic acid (CAS [73221-19-9], 0.052 g, 0.23 mmol) and DIPEA (0.097 mL, 0.56 mmol) in dry Me-THF (1.52 mL) and DCM (0.51 mL) under $N_2$. The solution was stirred at room temperature for 15 min. Then intermediate E9 (0.08 g, 0.25 mmol) was added and the reaction mixture was stirred at room temperature for 16 hours. The solvent was evaporated then the residue was diluted in ethyl acetate, washed with a saturated aqueous solution of $NaHCO_3$, water then brine. The organic layer was dried over $MgSO_4$, filtered and evaporated in vacuo to give a yellow oil, 0.167 g. Purification was carried out by flash chromatography over silica gel (12 g, irregular SiOH 25-40μM, DCM/MeOH from 100/0 to 97/3). Pure fractions were collected and evaporated affording a colorless oil which crystallized on standing, 0.102 g. A purification was performed via Reverse phase (Stationary phase: YMC-actus Triart C18 10μm 30*150mm, Mobile phase: Gradient from 40% $NH_4HCO_3$ 0.2%, 60% ACN to 10% $NH_4HCO_3$ 0.2%, 90% ACN). Pure fractions were collected and evaporated affording 0.037 g as white foam. It was triturated with DIPE and a few Heptane, the precipitate was filtered off and dried under vacuum at 60°C affording compound 127 as white powder, 0.032 g (26%).
$^1$H NMR (500 MHz, DMSO-$d_6$) δ ppm 9.23 (br s, 1H), 8.53 (br d, *J*=6.4 Hz, 1H), 7.79 (br d, *J*=8.9 Hz, 1H), 7.55 (br t, *J*=7.5 Hz, 1H), 7.25 - 7.37 (m, 3H), 7.20 (br d, *J*=8.1 Hz, 3H), 4.42 - 4.56 (m, 2H), 4.08 (br s, 2H), 3.84 (br s, 2H)

**Synthesis of compound 128**

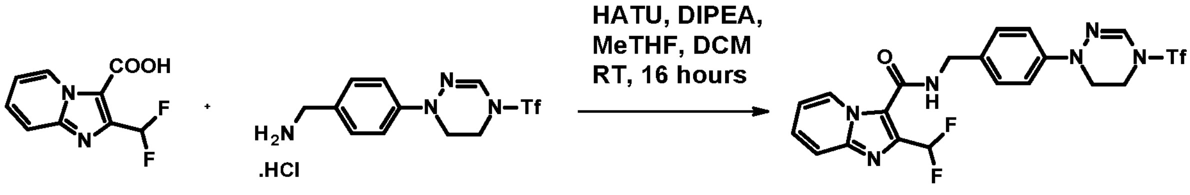


CAS [2059954-47-9] Intermediate E9 compound 128

Accordingly, compound 128 was prepared in the same way as compound 127 starting from 2-(Difluoromethyl)-imidazo[1,2-A]pyridine-3-carboxylic acid (CAS [2059954-47-9], 0.23 mmol) and intermediate E9 affording a white powder, 0.045 g (39%).
$^1$H NMR (500 MHz, DMSO-$d_6$) δ ppm 8.96 (br t, *J*=5.6 Hz, 1H), 8.79 (d, *J*=7.0 Hz, 1H), 7.76 (d, *J*=9.0 Hz, 1H), 7.52 (t, *J*=7.8 Hz, 1H), 7.25 - 7.45 (m, 4H), 7.20 (d, *J*=8.7 Hz, 2H), 7.16 (td, *J*=6.9, 1.1 Hz, 1H), 4.48 (d, *J*=5.6 Hz, 2H), 4.08 (br t, *J*=4.5 Hz, 2H), 3.84 (t, *J*=4.8 Hz, 2H)

**Synthesis of compound 137**

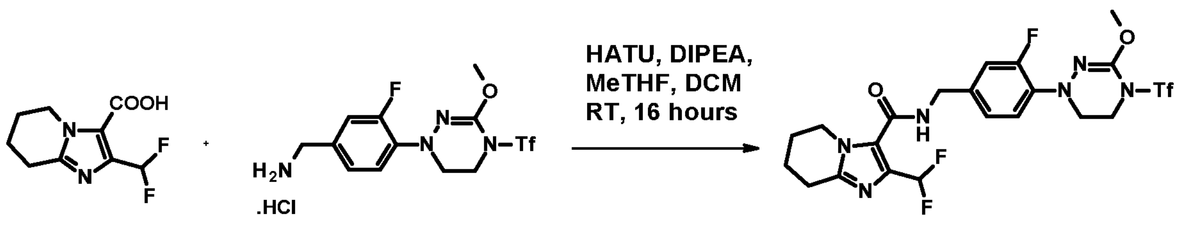


CAS [2060043-79-8] Intermediate R7 compound 137

HATU (0.093 g, 0.24 mmol) was added to a solution of 2-(Difluoromethyl)-SH,6H,7H,8H-imidazo[1,2-A]pyridine-3-carboxylic acid (0.046 g, 0.21 mmol) and DIPEA (0.091 mL, 0.53 mmol) in dry Me-THF (1.43 mL) and DCM (0.48 mL) under $N_2$. The solution was stirred at room temperature for 15 min. Then intermediate R7 (0.095 g, 0.23 mmol) was added and the reaction mixture was stirred at room temperature for 16 hours. The solvent was evaporated then the residue was diluted in ethyl acetate, washed with a saturated aqueous solution of $NaHCO_3$, water then brine. The organic layer was dried over $MgSO_4$, filtered and evaporated in vacuo to give a yellow oil, 0.271 g. Purification was carried out by flash chromatography over silica gel (12 g, irregular SiOH 25-40μM, DCM/MeOH from 100/0 to 97/3). Pure fractions were collected and evaporated affording 0.112 g as colourless oil which crystalized on standing. It was triturated with DIPE and a few Heptane, the precipitate was filtered off and dried under vacuum at 60°C affording compound 137 as white powder, 0.096 g (79%).
$^1$H NMR (500 MHz, DMSO-$d_6$) δ ppm 8.77 (br t, *J*=5.6 Hz, 1H), 7.44 (t, *J*=8.6 Hz, 1H), 7.10 - 7.19 (m, 2H), 6.95 (t, *J*=54.3 Hz, 1H), 4.40 (br d, *J*=5.8 Hz, 2H), 4.06 - 4.15 (m, 2H), 4.02 (br t, *J=5.5* Hz, 2H), 3.83 (s, 3H), 3.54 - 3.60 (m, 2H), 2.78 (br t, *J*=6.3 Hz, 2H), 1.89 (br d, *J*=4.6 Hz, 2H), 1.83 (br d, *J=5.5* Hz, 2H)

**Synthesis of compound 79**

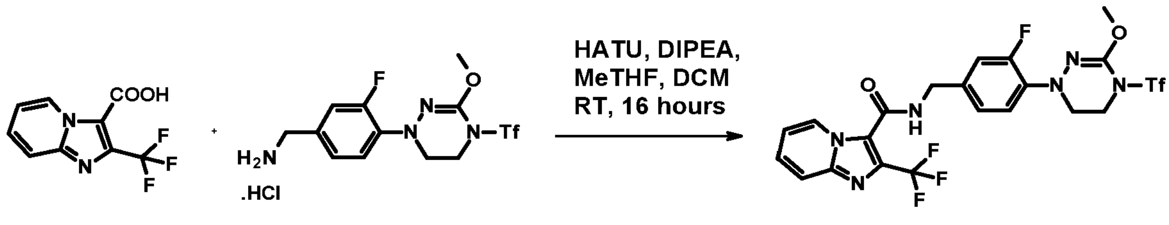


CAS [73221-19-9] Intermediate R7 compound 79

Accordingly, compound 79 was prepared in the same way as compound 137 starting from 2-(Trifluoromethyl)-imidazo[1,2-A]pyridine-3-carboxylic acid (CAS [73221-19-9], 0.21 mmol) and intermediate R-7 (0.23 mmol) affording a white powder, 0.09 g (70%).
$^1$H NMR (500 MHz, DMSO-d6) δ ppm 9.27 (t, J=5.8 Hz, 1 H), 8.57 (d, J=6.9 Hz, 1 H), 7.80 (d, J=9.2 Hz, 1 H), 7.40 - 7.62 (m, 2 H), 7.14 - 7.27 (m, 3 H), 4.47 - 4.56 (m, 2 H), 4.08 - 4.14 (m, 2 H), 3.84 (s, 3 H), 3.52 - 3.63 (m, 2 H)

**Synthesis of compound 132**

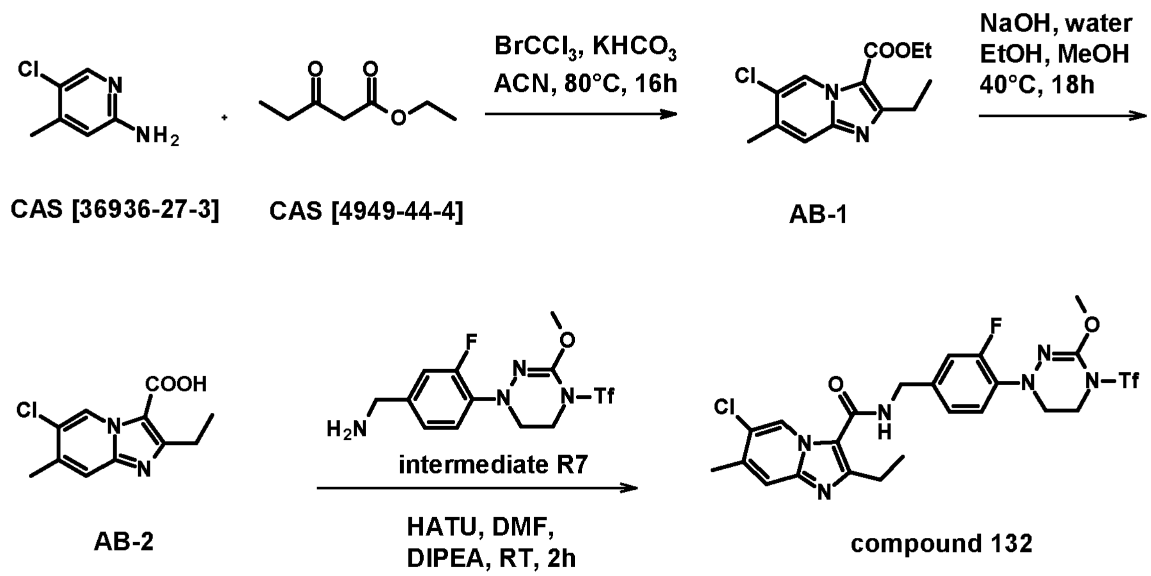


CAS [36936-27-3] CAS [4949-44-4] AB-1

AB-2 compound 132

Preparation of intermediate AB-1

In a sealed tube, to a solution of 2-amino-5-chloropicoline (CAS [36936-27-3], 1.00 g, 7.01 mmol) in ACN (12 mL) were added Ethyl-ethyl 3-oxovalerate (CAS [4949-44-4], 2.00 mL, 14.0 mmol), bromotrichloromethane (2.40 mL, 24.4 mmol) and potassium bicarbonate (2.12 g, 21.2 mmol). The mixture was stirred at 80 °C for 16 h. EtOAc and water were added. The organic layer was washed with brine, dried ($MgSO_4$), evaporated and purified by preparative LC (irregular SiOH, 15-40 μm, 80 g, mobile phase gradient: from heptane / EtOAc 90:10 to 10:90)The fractions containing product were combined and evaporated to afford 0.95 g of intermediate AB-1 as an orange solid (51%).

Preparation of intermediate AB-2

To a mixture of intermediate AB-1 (180 mg, 0.675 mmol) in water (2.2 mL) and EtOH (2.2 ml) was added NaOH (81 mg, 2.03 mmol) and the mixture was stirred at 40 °C for 18 h.

The reaction mixture was evaporated to give 270 mg g of intermediate AB-2 (Quant. purity 65%).

Preparation of compound 132

A mixture of intermediate AB-2 (150 mg, 0,374 mmol, purity 65%), intermediate R7 (151 mg, 0,374 mmol), HATU (157 mg, 0.414 mmol), DIPEA (82 μL, 0.48 mmol) and DMF (2.3 mL) was stirred at room temperature for 2 h. The reaction mixture was diluted with EtOAc, and the organic layer was washed with an aqueous solution of NaHCOs 1%, then with water and brine, dried over MgSO4, filtered off, concentrated and purified by preparative LC (irregular SiOH, 15-40 μm, 40 g Grace, loading (DCM), mobile phase gradient: from Heptane/EtOAc: 50/50 to 0/100 in 7 CV then EtOAc 100% in 7 CV). The fractions containing product were combined and evaporated to give 116 mg as a white solid. It was purified by preparative LC (spherical C18 25 μm, 40 g YMC-ODS-25, (MeOH/MeCN), mobile phase gradient 0.2% aq. $NH_4^+HCO_3^-$ / MeCN from 70:30 to 0:100). The fraction containing product were combined and evaporated to give 86 mg of compound 132 as white solid (39%).

1H NMR (400 MHz, DMSO-d6) δ ppm 9.12 (s, 1 H), 8.35 (t, J=5.9 Hz, 1 H), 7.64 (s, 1 H), 7.45 (t, J=8.6 Hz, 1 H), 7.11 - 7.27 (m, 2 H), 4.48 (d, J=5.9 Hz, 2 H), 4.11 (br t, J=5.2 Hz, 2 H), 3.83 (s, 3 H), 3.57 (br t, J=4.9 Hz, 2 H), 2.99 (q, J=7.5 Hz, 2 H), 2.40 (s, 3 H), 1.26 (t, J=7.5 Hz, 3 H)

**Synthesis of compound 141**

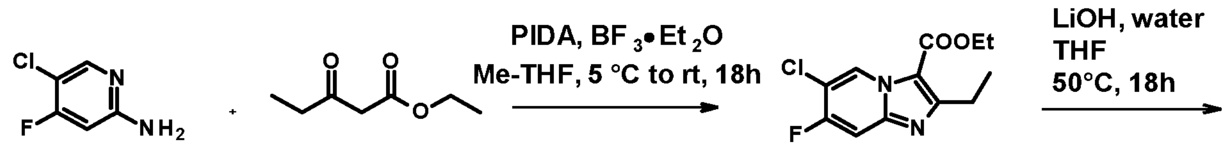


CAS [36936-27-3] CAS [4949-44-4] AC-1

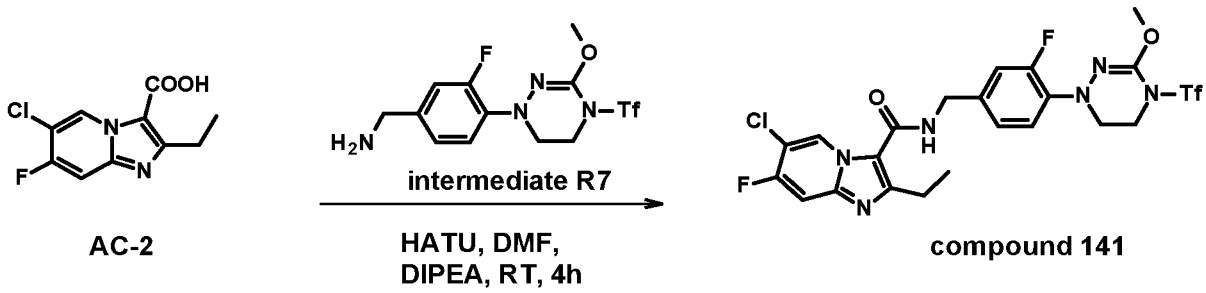


AC-2 compound 141

Preparation of intermediate AC-1

To a solution of 5-Chloro-4-fluoro-2-pyridinamine (CAS [1393574-54-3], 250 mg, 1.71 mmol) in Me-THF (8 mL) at 5 °C were added iodobenzene diacetate (550 mg, 1.71 mmol) and ethyl-ethyl 3-oxovalerate (0.4 mL, 2.80 mmol). Then Boron trifluoride etherate (25 μL, 95.5 μmol) was added dropwise. The solution was stirred at 5°C for 1 h. The mixture was warmed to room temperature and stirred for 18. EtOAc and water were added. The organic layer was washed with brine, dried (MgSO4),evaporated and purified by prepartive LC (irregular SiOH, 15-40 μm, 40 g, grace, loading (DCM) mobile phase gradient: from heptane / EtOAc 90:10 to 10:90 over 10 CV) to afford 119 mg of intermediate AC-1 as a pale brown solid (P1; 26%)

Preparation of intermediate AC-2

A mixture of intermediate AC-1 (200 mg, 0.739 mmol), Lithium hydroxide (177 mg, 7.39 mmol), water (3.2 mL) and THF (4.4 mL) was stirred at 50 °C for 18h. EtOAc and aq. $KHSO_4$ 10% was added. The organic layer was dried ($MgSO_4$) and evaporated to give 179 mg of intermediate AC-2 as yellow solid (Quant.).

Preparation of compound 141

Accordingly, compound 141 was prepared in the same way as compound 132 starting from intermediate AC-2 (0.78 mmol) and intermediate R7 affording 0.127 g (27%) as a white powder.
1H NMR (400 MHz, DMSO-d6) δ ppm 9.24 (d, J=7.3 Hz, 1 H), 8.45 (br t, J=5.8 Hz, 1 H), 7.79 (d, t, J=9.9 Hz, 1 H), 7.45 (t, t, J=8.7 Hz, 1 H), 7.12 - 7.27 (m, 2 H), 4.49 (d, t, J=5.9 Hz, 2 H), 4.11 (t, t, J=4.9 Hz, 2 H), 3.83 (s, 3 H), 3.57 (t, t, J=4.9 Hz, 2 H), 2.99 (q, t, J=7.5 Hz, 2 H), 1.27 (t, J=7.5 Hz, 3 H)

**Synthesis of compound 158**

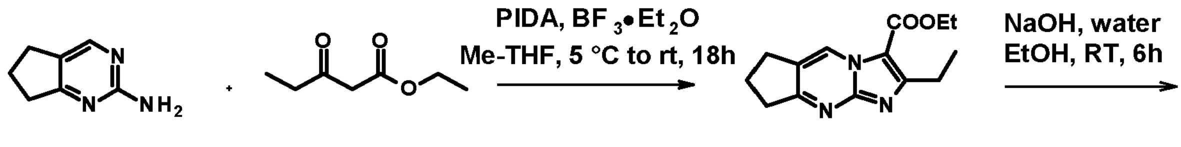


CAS [108990-72-3] CAS [4949-44-4] AD-1

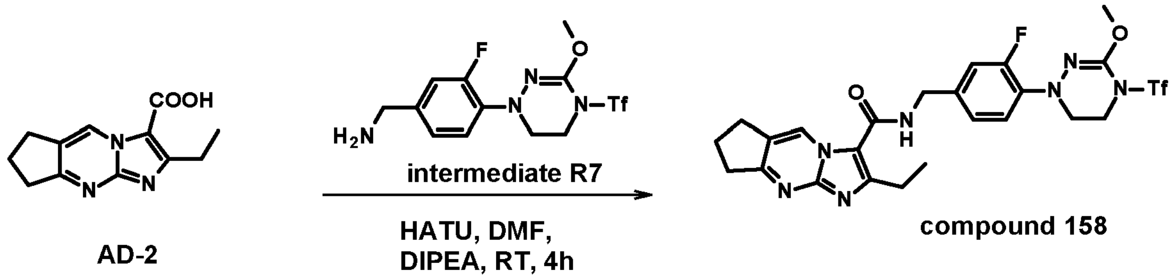


AD-2

compound 158

Preparation of intermediate AD-1

Accordingly, compound AD-1 was prepared in the same way as compound AC-1 starting from 6,7-dihydro-5h-cyclopenta[d]pyrimidin-2-amine (CAS [108990-72-3], 7.4 mmol) affording 0.726 g (38%).

Preparation of intermediate AD-2

Accordingly, compound AD-2 was prepared in the same way as compound AB-2 starting from AD-1 (0.77 mmol) affording 0.446 g (44%).

Preparation of compound 158

Accordingly, compound 158 was prepared in the same way as compound 132 starting from intermediate AD-2 (0.77 mmol) and intermediate R7 affording 0.145 g (32%) as a white powder.
1H NMR (500 MHz, DMSO-d6) δ ppm 9.10 (s, 1 H), 8.39 (t, J=6.0 Hz, 1 H), 7.44 (t, J=8.5 Hz, 1 H), 7.12 - 7.26 (m, 2 H), 4.47 (d, J=5.9 Hz, 2 H), 4.10 (t, J=4.8 Hz, 2 H), 3.83 (s, 3 H), 3.56 (t, J=4.8 Hz, 2 H), 2.89 - 3.03 (m, 6 H), 2.05 - 2.16 (m, 2 H), 1.26 (t, J=7.6 Hz, 3 H)

**Preparation of compound 193**

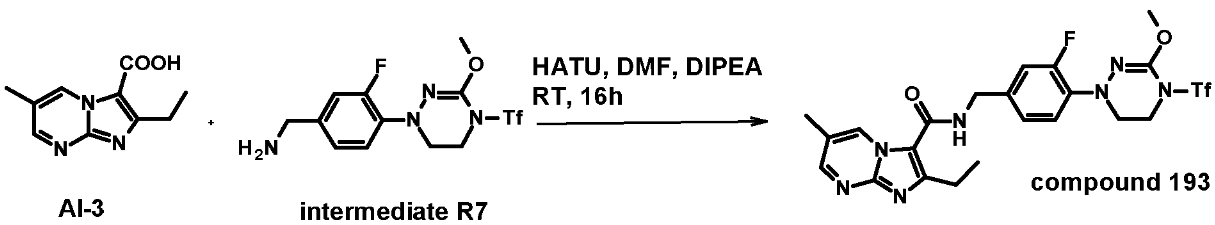


AI-3 intermediate R7 compound 193

Accordingly, compound 193 was prepared in the same way as compound 158 starting from intermediate AI-3 (0.44 mmol) and intermediate R-7 (0.37 mmol) affording a white solid, 0.108 g (52%).
$^1$H NMR (400 MHz, DMSO) d 9.19 - 9.10 (m, 1H), 8.51 (d, J = 2.4 Hz, 1H), 8.44 (t, J = 5.9 Hz, 1H), 7.44 (t, J = 8.6 Hz, 1H), 7.26 - 7.14 (m, 2H), 4.49 (d, J = 5.9 Hz, 2H), 4.14 - 4.03 (m, 2H), 3.83 (s, 3H), 3.59 - 3.53 (m, 2H), 3.01 (q, J = 7.5 Hz, 2H), 2.34 (d, J = 0.6 Hz, 3H), 1.28 (t, J = 7.5 Hz, 3H).

**Preparation of compound 194**

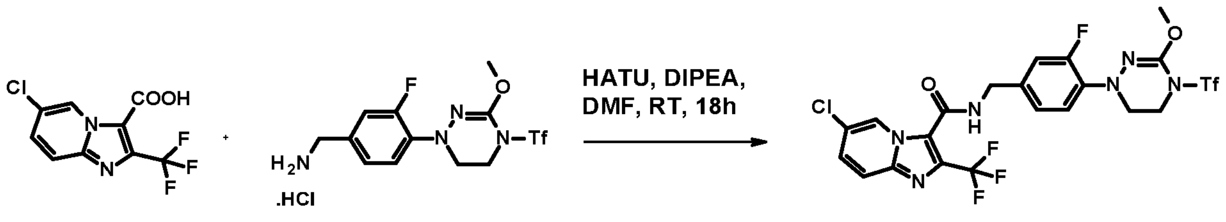


CAS [874830-60-1] Intermediate R-7 compound 194

Accordingly, compound 194 was prepared in the same way as compound 158 starting from 6-Chloro-2-(trifluoromethyl)imidazo[1,2-a]pyridine-3-carboxylic acid (CAS [874830-60-1] (0.7 mmol) and intermediate R-7 (0.47 mmol) affording a white solid, 0.110 g (39%).

$^1$H NMR (400 MHz, DMSO) d 9.23 (t, J = 5.8 Hz, 1H), 8.35 (s, 1H), 7.70 (d, J = 9.3 Hz, 1H), 7.52 - 7.37 (m, 2H), 7.19 (m, 2H), 4.51 (d, J = 5.8 Hz, 2H), 4.17 - 4.07 (m, 2H), 3.84 (s, 3H), 3.63 - 3.55 (m, 2H), 2.34 (s, 3H).

## Preparation of compound 204

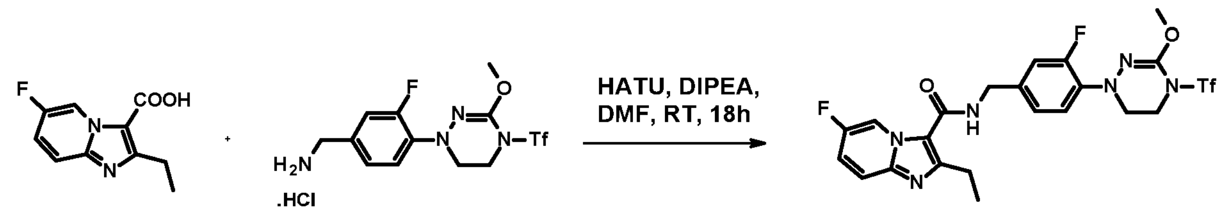


CAS [1368682-64-7] Intermediate R-7 compound 204

Accordingly, compound 204 was prepared in the same way as compound 158 starting from 2-ethyl-6-fluoroimidazo[1,2-a]pyridine-3-carboxylic acid (CAS [1368682-64-7], 0.84 mmol) and intermediate R-7 (0.7 mmol) affording a white solid, 0.132 g (34%).

$^1$H NMR (400 MHz, DMSO) d 9.09 - 9.01 (m, 1H), 8.40 (t, J = 5.9 Hz, 1H), 7.73 - 7.64 (m, 1H), 7.53 - 7.41 (m, 2H), 7.25 - 7.14 (m, 2H), 4.49 (d, J = 5.9 Hz, 2H), 4.15 - 4.05 (m, 2H), 3.83 (s, 3H), 3.61 - 3.51 (m, 2H), 3.00 (q, J = 7.5 Hz, 2H), 1.27 (t, J = 7.5 Hz, 3H).

## Preparation of compound 206

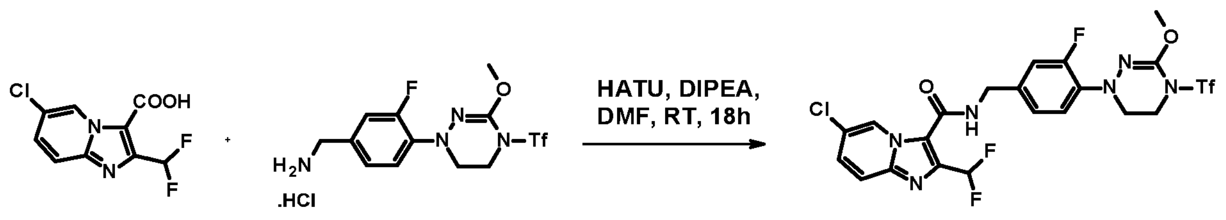


intermediate AM-2 Intermediate R-7 compound 206

Accordingly, compound 206 was prepared in the same way as compound 158 starting from intermediate AM-2 (0.61 mmol) and intermediate R-7 (0.47 mmol) affording a beige powder, 0.07 g (24%).

$^1$H NMR (400 MHz, DMSO) d 9.02 (t, J = 5.7 Hz, 1H), 8.92 (d, J = 1.7 Hz, 1H), 7.83 (d, J = 9.6 Hz, 1H), 7.61 (dd, J = 9.6, 2.0 Hz, 1H), 7.52 - 7.16 (m, 4H), 4.51 (d, J = 5.7 Hz, 2H), 4.13 - 4.07 (m, 2H), 3.83 (s, 3H), 3.60 - 3.55 (m, 2H).

**Preparation of compound 209**

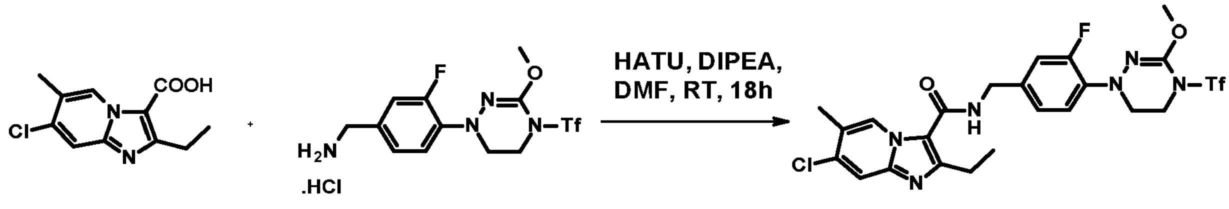


intermediate AQ-2    Intermediate R-7    compound 209

Accordingly, compound 209 was prepared in the same way as compound 158 starting from intermediate AQ-2 (0.56 mmol) and intermediate R-7 (0.4 mmol) affording a white powder, 0.142 g (59%).

$^1H$ NMR (400 MHz, DMSO) d 8.95 (s, 1H), 8.41 (t, J = 5.9 Hz, 1H), 7.80 (s, 1H), 7.44 (t, J = 8.6 Hz, 1H), 7.26 - 7.14 (m, 2H), 4.48 (d, J = 5.9 Hz, 2H), 4.15 - 4.06 (m, 2H), 3.83 (s, 3H), 3.60 - 3.52 (m, 2H), 2.97 (q, J = 7.5 Hz, 2H), 2.32 (s, 3H), 1.26 (t, J = 7.5 Hz, 3H).

**Preparation of compound 210**

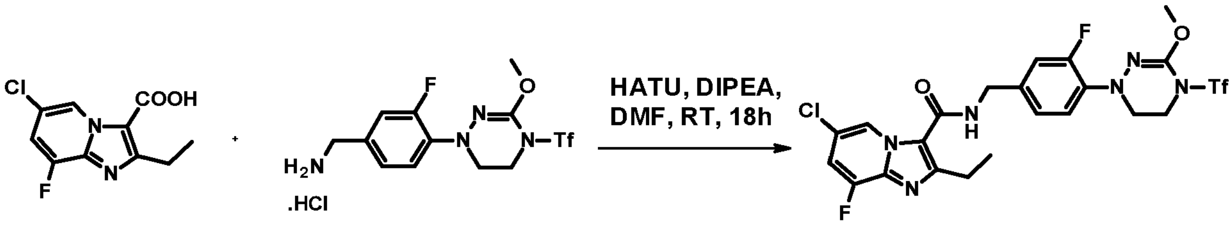


intermediate AL-2    Intermediate R-7    compound 210

Accordingly, compound 210 was prepared in the same way as compound 158 starting from intermediate AL-2 (0.55 mmol) and intermediate R-7 (0.4 mmol) affording a white solid, 0.161 g (68%).

$^1H$ NMR (400 MHz, DMSO) d 8.92 (d, J = 1.4 Hz, 1H), 8.60 (t, J = 5.9 Hz, 1H), 7.62 (dd, J = 10.6, 1.6 Hz, 1H), 7.45 (t, J = 8.6 Hz, 1H), 7.26 - 7.15 (m, 2H), 4.50 (d, J = 5.8 Hz, 2H), 4.15 - 4.06 (m, 2H), 3.83 (s, 3H), 3.61 - 3.52 (m, 2H), 3.01 (q, J = 7.5 Hz, 2H), 1.27 (t, J = 7.5 Hz, 3H).

**Preparation of intermediate AA-3**

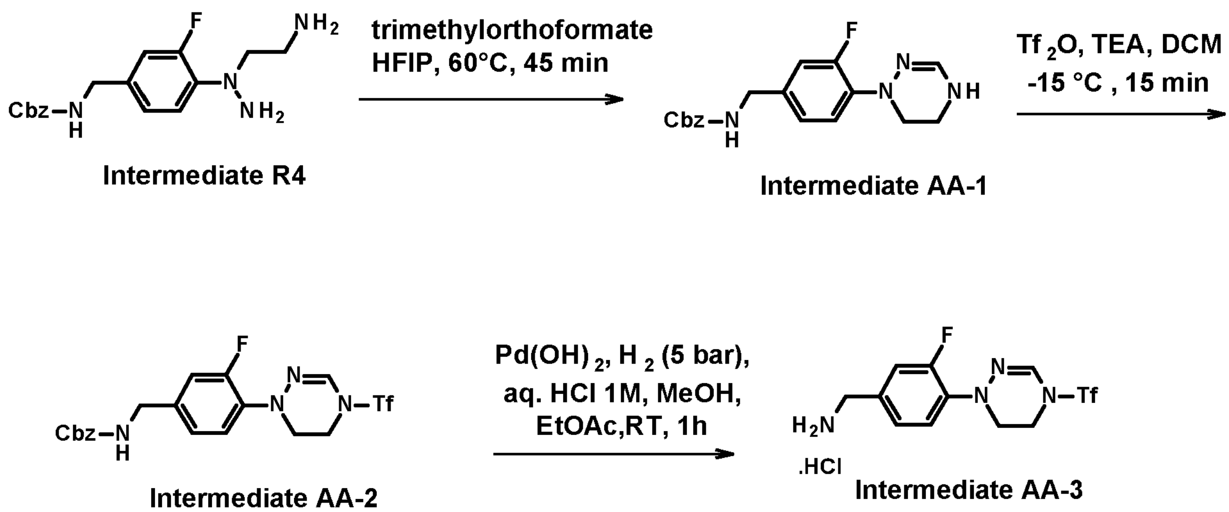


Intermediate R4

Intermediate AA-1

Intermediate AA-2

Intermediate AA-3

Preparation of intermediate AA-1

A solution of intermediate R4 (19.6 g, 48.4 mmol) and Trimethylorthoformate (15.9 mL, 145 mmol) in HFIP (490 mL) was stirred at 60 °C for 45 min. The reaction mixture was evaporated. The residue was diluted in DCM and a 10 % aq. solution of $K_2CO_3$ was added. The aqueous layer was extracted twice with DCM/MeOH (95/5). The combined organic layers were dried on $MgSO_4$, filtered off and evaporated. The crude (m=25.6 g) was purified by preparative LC (regular SiOH 30 µm, 330 g, dry loading (celite®), mobile phase gradient: from Heptane 75%, EtOAc/MeOH (9:1) 25% to Heptane 25%, EtOAc/MeOH (9:1). Fractions containing product were combined and evaporated to give 14.61 g of intermediate AA-1 as a colorless oil which crystallized on standing (85%).

Preparation of intermediate AA-2

To a solution of intermediate AA-1 (14.6 g, 42.7 mmol) and DIPE (22.1 mL, 128 mmol) in dry DCM (340 mL) at -5 °C (ice/NaCl solid) was added dropwise TfzO 1M in DCM (47 mL, 47 mmol) over 15 min using a dropping funnel and stirring was continued for 5 min. The reaction mixture was quenched with a saturated aqueous solution of $NaHCO_3$. The layers were separated, and the aqueous layer was extracted with DCM (twice). The combined organic layer was dried over $MgSO_4$, filtered off and concentrated. The crude (m= 36.4 g) was purified by preparative LC (regular SiOH, 30 µm, 120 g, dry loading (celite®), mobile phase gradient: Heptane/EtOAc 90/10 to 70/30). The fractions containing product were combined and evaporated under vacuum to give 10.18 g of intermediate AA-2 as a white solid (50%).

Preparation of intermediate AA-3

In a steal bomb, a mixture of intermediate AA-2 (10.2 g, 21.5 mmol), Palladium hydroxide 20% on carbon nominally 50% water (3.01 g, 2.15 mmol) and aqueous HCl 3M (7.15 mL, 7.15 mmol) in MeOH (150 mL) and EtOAc (150 mL) was hydrogenated under 5 bar of $H_2$ at room temperature for 1 h. The mixture was filtered on a pad of celite® and washed with MeOH. The filtrate was evaporated then co-evaporated with MeOH (twice) to give 7.86 g of intermediate AA-3.

**Synthesis of compound 163**

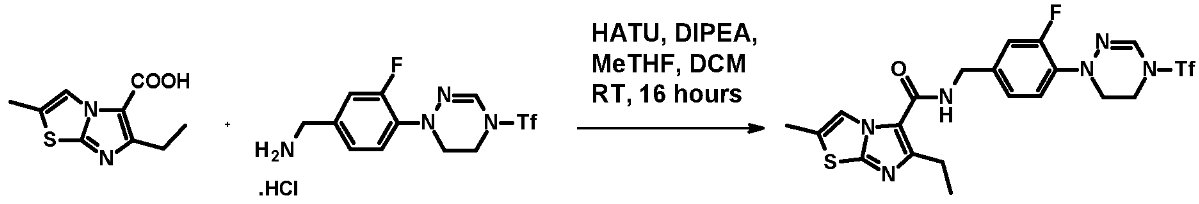


CAS [1131613-58-5] Intermediate AA-3 compound 163

HATU (0.083 g, 0.22 mmol) was added to a solution of 6-ethyl-2-methylimidazo[2,1-b][1,3]thiazole-5-carboxylic acid (CAS [1131613-58-5], 0.04 g, 0.19 mmol) and DIPEA (0.082 mL, 0.48 mmol) in dry Me-THF (1.28 mL) and DCM (0.43 mL) under $N_2$. The solution was stirred at room temperature for 15 min. Then intermediate AA-3 (0.083 g, 0.22 mmol) was added and the reaction mixture was stirred at room temperature for 16 hours. The solvent was evaporated then the residue was diluted in ethyl acetate, washed with a saturated aqueous solution of $NaHCO_3$, water then brine. The organic layer was dried over $MgSO_4$, filtered and evaporated in vacuo to give a colorless oil. Purification was carried out by flash chromatography over silica gel (12 g, irregular SiOH 25-40μM, DCM/MeOH from 100/0 to 97/3). Pure fractions were collected and evaporated affording a white foam, 0.096 g. It was triturated with DIPE and a few Heptane, the precipitate was filtered off and dried under vacuum at 60°C affording compound 163 as white powder, 0.088 g, 86%.
$^1$H NMR (500 MHz, DMSO-d$_6$) δ ppm 8.14 (br t, *J*=5.8 Hz, 1H), 7.90 (s, 1H), 7.38 (s, 1H), 7.32 (t, *J*=8.5 Hz, 1H), 7.20 (br d, *J*=13.1 Hz, 1H), 7.16 (br d, *J*=8.2 Hz, 1H), 4.44 (br d, *J*=6.0 Hz, 2H), 4.10 (br s, 2H), 3.59 - 3.68 (m, 2H), 2.88 (q, *J*=7.5 Hz, 2H), 2.42 (s, 3H), 1.22 (t, *J*=7.5 Hz, 3H)

**Synthesis of compound 147**

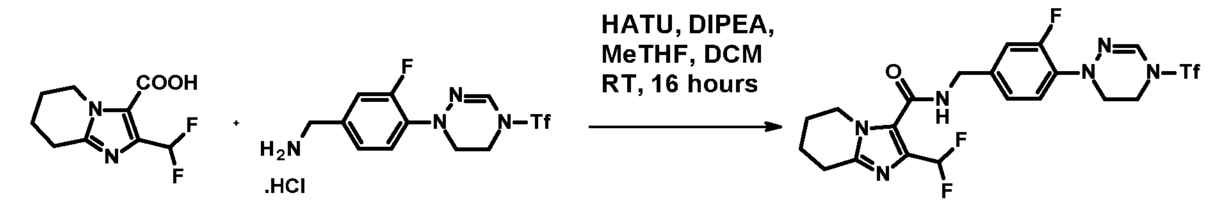


CAS [2060043-79-8] Intermediate AA-3 compound 147

Accordingly, compound 147 was prepared in the same way as compound 163 starting from 2-(Difluoromethyl)-5H,6H,7H,8H-imidazo[1,2-A]pyridine-3-carboxylic acid (CAS [2060043-79-8], 0.19 mmol) and intermediate AA-3 affording a white powder, 0.08 g (77%).
$^1$H NMR (400 MHz, DMSO-*d*$_6$) δ ppm 8.79 (br t, *J*=5.6 Hz, 1H), 7.38 (s, 1H), 7.33 (t, *J*=8.6 Hz, 1H), 7.07 - 7.23 (m, 2H), 6.95 (t, *J*=54.2 Hz, 1H), 4.41 (br d, *J*=5.9 Hz, 2H), 4.10 (br s, 2H), 4.02 (br t, *J*=*5.5* Hz, 2H), 3.65 (br t, *J*=4.6 Hz, 2H), 2.68 - 2.91 (m, 2H), 1.89 (br d, *J*=4.3 Hz, 2H), 1.83 (br d, *J*=5.3 Hz, 2H)

**Synthesis of compound 159**

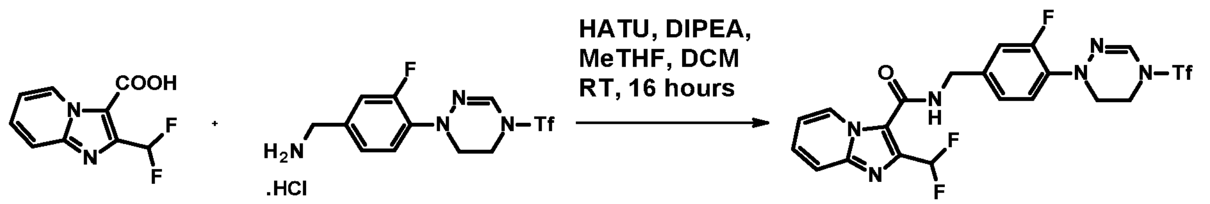


CAS [2059954-47-9] Intermediate AA-3 compound 159

Accordingly, compound 159 was prepared in the same way as compound 163 starting from 2-(Difluoromethyl)-imidazo[1,2-A]pyridine-3-carboxylic acid (CAS [2059954-47-9], 0.19 mmol) and intermediate AA-3 affording a white powder, 0.084 g (82%).
$^1$H NMR (400 MHz, DMSO-d$_6$) δ ppm 9.00 (br s, 1H), 8.81 (br d, *J*=7.0 Hz, 1H), 7.77 (d, *J*=9.0 Hz, 1H), 7.08 - 7.59 (m, 7H), 4.52 (br s, 2H), 4.10 (br s, 2H), 3.66 (br t, *J*=4.5 Hz, 2H)

**Synthesis of compound 135**

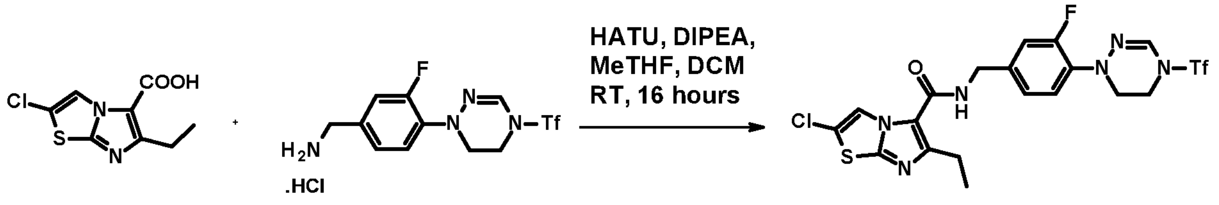


CAS [2089471-58-7] Intermediate AA-3 compound 135

Accordingly, compound 135 was prepared in the same way as compound 163 starting from 2-Chloro-6-ethyl-2-methylimidazo[2,1-b][1,3]thiazole-5-carboxylic acid (CAS [2089471-58-7], 0.21 mmol) and intermediate AA-3 affording a white powder, 0.056 g (49%).
$^1$H NMR (500 MHz, DMSO-$d_6$) δ ppm 8.31 (m, 1H), 8.28 (br t, *J*=5.8 Hz, 1H), 7.38 (m, 1H), 7.33 (br t, *J*=8.5 Hz, 1H), 7.21 (br d, *J*=13.4 Hz, 1H), 7.16 (br d, *J*=8.2 Hz, 1H), 4.45 (br d, *J*=5.8 Hz, 2H), 4.10 (br s, 2H), 3.64 (br t, *J*=4.4 Hz, 2H), 2.89 (q, *J*=7.4 Hz, 2H), 1.22 (br t, *J*=7.5 Hz, 3H)

## Synthesis of compound 152

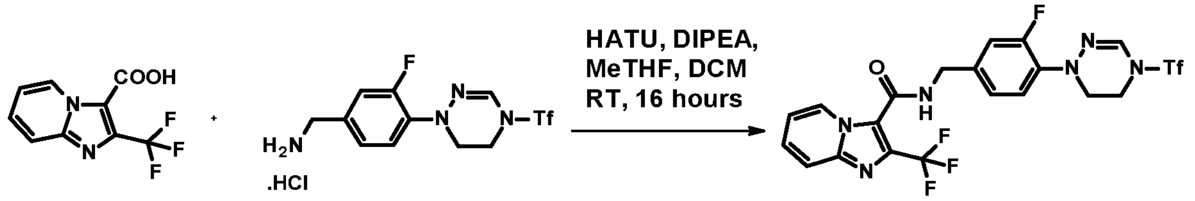


CAS [73221-19-9] Intermediate AA-3 compound 152

Accordingly, compound 152 was prepared in the same way as compound 163 starting from 2-(Trifluoromethyl)-imidazo[1,2-A]pyridine-3-carboxylic acid (CAS [73221-19-9], 0.92 mmol) and intermediate AA-3 affording a white powder, 0.418 g (82%).
$^1$H NMR (500 MHz, DMSO-$d_6$) δ ppm 9.29 (t, *J*=5.8 Hz, 1H), 8.57 (d, *J*=6.9 Hz, 1H), 7.80 (d, *J*=9.2 Hz, 1H), 7.56 (ddd, *J*=9.1, 6.9, 1.1 Hz, 1H), 7.39 (s, 1H), 7.36 (t, *J*=8.5 Hz, 1H), 7.22 - 7.26 (m, 1H), 7.18 - 7.22 (m, 2H), 4.53 (d, *J*=5.8 Hz, 2H), 4.11 (br t, *J*=4.3 Hz, 2H), 3.67 (t, *J*=4.7 Hz, 2H)

## Synthesis of compound 124

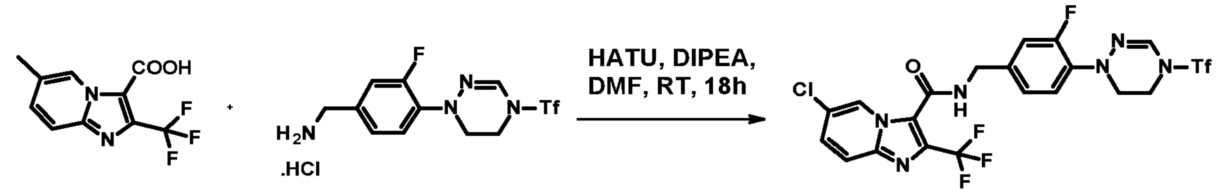


CAS [874830-60-1] Intermediate AA-3 compound 124

To a solution of 6-Chloro-2-(trifluoromethyl)imidazo[1,2-a]pyridine-3-carboxylic acid (CAS [874830-60-1], 100 mg, 0.378 mmol) and DIPEA (0.306 mL, 1.80 mmol) in DMF (1.7 mL) was added HATU (164 mg, 0.432 mmol). After 10 min of stirring, intermediate AA-3 (137 mg, 0.360 mmol) was added and the reaction mixture was stirred at room temperature for 18 h. The brown paste was purified by preparative LC (regular SiOH 30 μm, 25 g, dry loading (celite®), mobile phase gradient: Heptane/EtOAc 90/10 to 30/70). The fractions containing product were combined and evaporated to give 216 mg as a yellow solid. It was triturated in $Et_2O$. The mixture was filtered off. The solid was rinsed with $Et_2O$, collected and dried under vacuum to give 172 mg as a white solid. It was dissolved in EtOAc and evaporated (3 times) to give 158 mg as a white solid. It was coevaporated with MeCN (3 times) and dried under vacuum to give 143 mg of

compound 124 as a white solid (50%).

1H NMR (400 MHz, DMSO-d6) δ ppm 9.28 (br s, 1 H), 8.75 (m, 1 H), 7.87 (d, J=9.4 Hz, 1 H), 7.65 (dd, J=9.4, 1.8 Hz, 1 H), 7.31 - 7.41 (m, 2 H), 7.15 - 7.30 (m, 2 H), 4.54 (br d, J=4.1 Hz, 2 H), 4.10 (br t, J=4.0 Hz, 2 H), 3.67 (br t, J=4.6 Hz, 2 H)

## Synthesis of compound 129

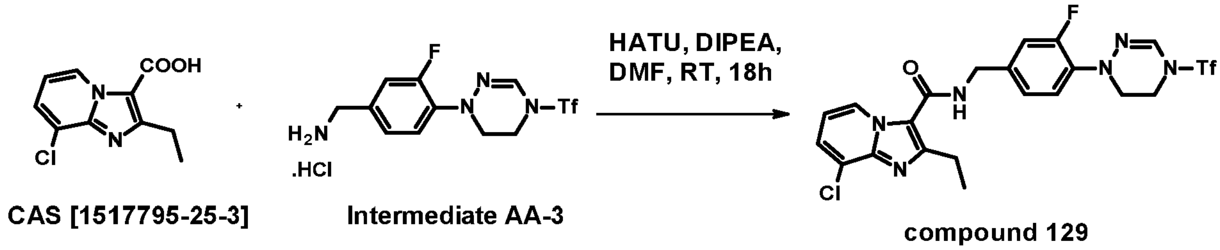


**CAS [1517795-25-3]** **Intermediate AA-3** **compound 129**

Accordingly, compound 129 was prepared in the same way as compound 124 starting from 8-chloro-2-ethyl-imidazo[1,2-a]pyridine-3-carboxylic acid (CAS [1517795-25-3], 0.6 mmol) and intermediate AA-3 affording 0.136 g (41%) as white powder.

$^{1}$H NMR (400 MHz, DMSO-d6) δ ppm 8.90 (br d, J=6.9 Hz, 1 H), 8.59 (br t, J=5.6 Hz, 1 H), 7.59 (br d, J=7.5 Hz, 1 H), 7.30 - 7.46 (m, 2 H), 7.15 - 7.29 (m, 2 H), 7.01 (br t, J=7.1 Hz, 1 H), 4.50 (d, J=5.9 Hz, 2 H), 4.10 (br t, J=4.4 Hz, 2 H), 3.65 (br t, J=4.9 Hz, 2 H), 3.01 (q, J=7.5 Hz, 2 H), 1.27 (br t, J=7.6 Hz, 3 H)

## Synthesis of compound 133

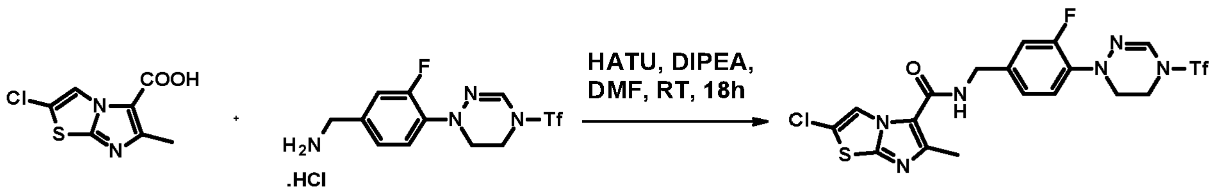


**CAS [2089471-57-6]** **Intermediate AA-3** **compound 133**

Accordingly, compound 133 was prepared in the same way as compound 124 starting from 2-chloro-6-methyl-imidazo[2,1-b]thiazole-5-carboxylic acid (CAS [2089471-57-6], 0.52 mmol) and intermediate AA-3 affording 0.142 g (51%) as white solid.

$^{1}$H NMR (400 MHz, DMSO-d6) δ ppm 8.31 (s, 1 H), 8.25 (br t, J=5.9 Hz, 1 H), 7.38 (br s, 1 H), 7.33 (t, J=8.5 Hz, 1 H), 7.14 - 7.25 (m, 2 H), 4.45 (br d, J=5.9 Hz, 2 H), 4.10 (br t, J=4.5 Hz, 2 H), 3.64 (br t, J=4.8 Hz, 2 H), 2.52 (s, 1H)

## Synthesis of compound 136

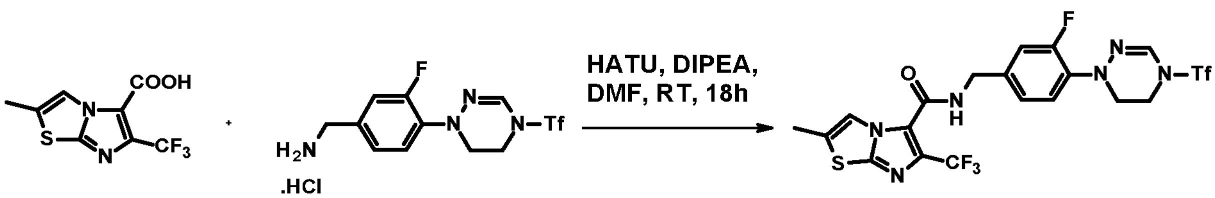


**CAS [1369332-25-1]** **Intermediate AA-3** **compound 136**

Accordingly, compound 136 was prepared in the same way as compound 124 starting from 2-Methyl-6-(trifluoromethyl)imidazo[2,1-b]thiazole-5-carboxylic acid (CAS [1369332-25-1], 0.58 mmol) and intermediate AA-3 affording

0.173 g (56%) as white powder.
$^{1}H$ NMR (500 MHz, DMSO-d6) δ ppm 8.99 (br t, J=4.3 Hz, 1 H), 7.86 (br s, 1 H), 7.39, (m, 1H), 7.35 (br t, J=8.5 Hz, 1 H), 7.14 - 7.24 (m, 2 H), 4.47 (br d, J=5.5 Hz, 2 H), 4.11 (m, 2 H), 3.67 (br t, J=4.3 Hz, 2 H), 2.48 (br s, 3 H)

## Synthesis of compound 164

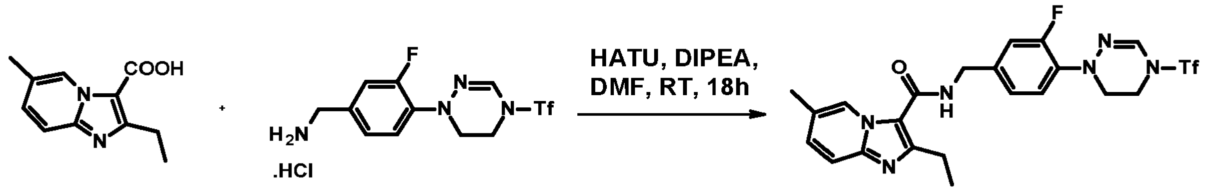


CAS [1216036-36-0] Intermediate AA-3 compound 164

Accordingly, compound 164 was prepared in the same way as compound 124 starting from 2-ethyl-6-methyl-imidazo[1,2-a]pyridine-3-carboxylic acid (CAS [1216036-36-0], 0.64 mmol) and intermediate AA-3 affording 0.11 g (33%) as a white solid.
$^{1}H$ NMR (400 MHz, DMSO-d6) δ ppm 8.75 - 8.84 (br s, 1 H), 8.37 (t, J=6.0 Hz, 1 H), 7.52 (d, J=8.9 Hz, 1 H), 7.32 - 7.41 (m, 2 H), 7.17 - 7.28 (m, 3 H), 4.50 (br d, J=5.9 Hz, 2 H), 4.11 (br t, J=4.2 Hz, 2 H), 3.66 (t, J=4.7 Hz, 2 H), 2.98 (q, J=7.5 Hz, 2 H), 2.31 (s, 3 H), 1.37 (t, J=7.5 Hz, 3H)

## Synthesis of compound 157

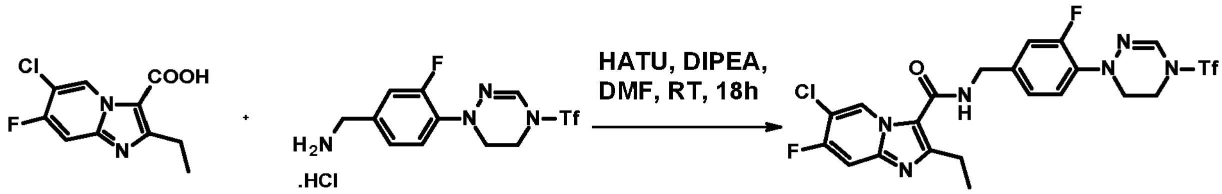


Intermediate AC-2 Intermediate AA-3 compound 157

Accordingly, compound 157 was prepared in the same way as compound 124 starting from intermediate AC-2 (0.78 mmol) and intermediate AA-3 affording 0.106 g (24%) as white powder.
$^{1}H$ NMR (400 MHz, DMSO-d6) δ ppm 9.23 (d, J=7.3 Hz, 1 H), 8.42 - 8.53 (m, 1 H), 7.80 (d, J=9.7 Hz, 1 H), 7.29 - 7.40 (m, 2 H), 7.17 - 7.28 (m, 2 H), 4.50 (d, J=5.9 Hz, 2 H), 4.07 - 4.13 (m, 2 H), 3.65 (br t, J=4.6 Hz, 2 H), 2.99 (q, J=7.5 Hz, 2 H), 1.27 (t, J=7.5 Hz, 3 H)

## Synthesis of compound 154

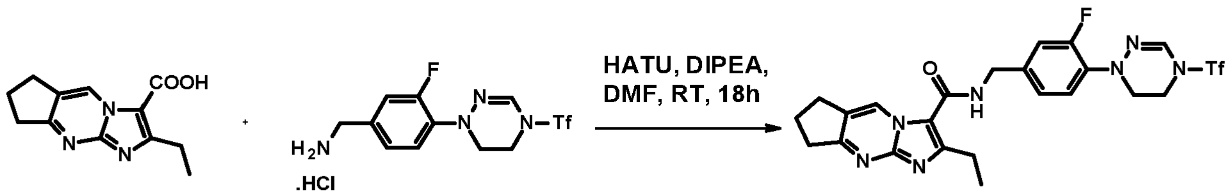


Intermediate AD-2 Intermediate AA-3 compound 154

Accordingly, compound 154 was prepared in the same way as compound 124 starting from intermediate AD-2 (0.78 mmol) and intermediate AA-3 affording 0.092 g (21%) as white solid.

$^1H$ NMR (400 MHz, DMSO-d6) δ ppm 9.23 (d, J=7.3 Hz, 1 H), 8.42 - 8.54 (br t, J=5.9 Hz, 1 H), 7.80 (d, J=9.8 Hz, 1 H), 7.30 - 7.41 (m, 2 H), 7.16 - 7.28 (m, 2 H), 4.50 (br d, J=5.9 Hz, 2 H), 4.10 (br t, J=4.9 Hz, 2 H), 3.65 (br t, J=4.7 Hz, 2 H), 2.99 (br q, J=7.4 Hz, 2 H), 1.27 (br t, J=7.5 Hz, 3 H)

## Synthesis of compound 156

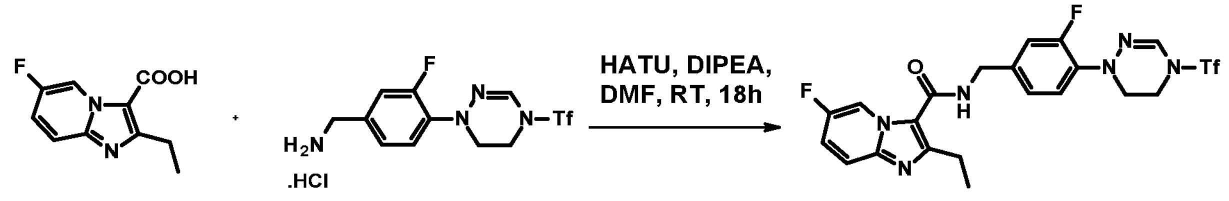


CAS [1368682-64-7] Intermediate AA-3 compound 156

Accordingly, compound 156 was prepared in the same way as compound 124 starting from 2-ethyl-6-fluoroimidazo[1,2-a]pyridine-3-carboxylic acid (CAS [1368682-64-7], 0.27 mmol) and intermediate AA-3 affording a white solid, 0.096 g (68%).

$^1H$ NMR (400 MHz, DMSO-d6) δ ppm 8.99 - 9.12 (m, 1 H), 8.41 (br t, J=7.5 Hz, 1 H), 7.65 - 7.77 (m, 1 H), 7.44 - 7.57 (m, 1 H), 7.32 - 7.40 (m, 2 H), 7.18 - 7.28 (m, 2 H), 4.51 (br t, J=5.9 Hz, 2 H), 4.11 (br t, J=4.5 Hz, 2 H), 3.66 (t, J=4.6 Hz, 2 H), 3.01 (q, J=7.5 Hz, 2 H), 1.28 (br t, J=7.5 Hz, 3 H)

## Synthesis of compound 153

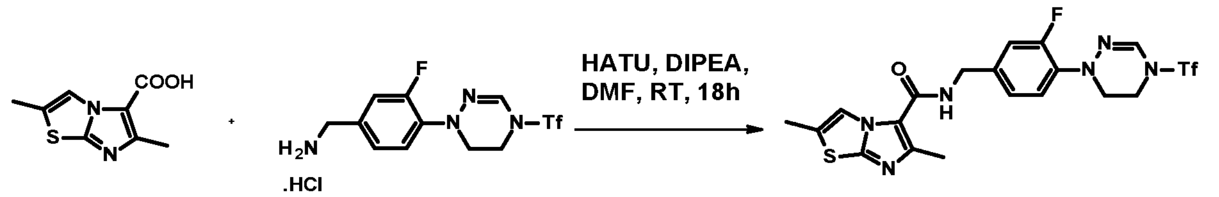


CAS [1007875-19-5] Intermediate AA-3 compound 153

Accordingly, compound 153 was prepared in the same way as compound 124 starting from 2,6-dimethylimidazo[2,1-b][1,3]thiazole-5-carboxylic acid (CAS [1007875-19-5], 0.67 mmol) and intermediate AA-3 affording a white solid, 0.138 g (42%).

$^1H$ NMR (500 MHz, DMSO-d6) δ ppm 8.11 (t, J=6.0 Hz, 1 H), 7.84 - 7.95 (m, 1 H), 7.38 (br s, 1 H), 7.32 (br t, J=8.7 Hz, 1 H), 7.14 - 7.23 (m, 2 H), 4.45 (d, J=6.0 Hz, 2 H), 4.10 (br t, J=4.4 Hz, 2 H), 3.64 (br t, J=4.9 Hz, 2 H), 2.51 ( s, 3H), 2.41 (d, J=1.2 Hz, 3 H)

## Synthesis of compound 146

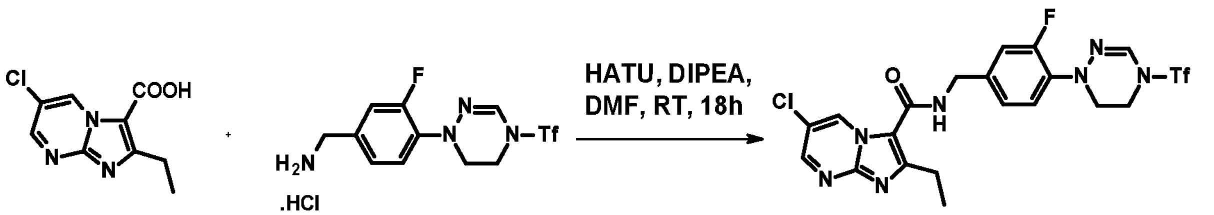


CAS [2059140-68-8] Intermediate AA-3 compound 146

Accordingly, compound 146 was prepared in the same way as compound 124 starting from 6-chloro-2-ethylimidazo[1,2-a]pyrimidine-3-carboxylic acid (CAS [2059140-68-8], 0.26 mmol) and intermediate AA-3 affording a white solid, 0.154 g (74%).

$^1$H NMR (400 MHz, DMSO-d6) δ ppm 9.41 (d, J=2.7 Hz, 1 H), 8.69 (d, J=2.7 Hz, 1 H), 8.58 (m, 1 H), 7.31 - 7.40 (m, 2 H), 7.18 - 7.28 (m, 2 H), 4.51 (m, 2 H), 4.10 (br t, J=4.5 Hz, 2 H), 3.65 (br t, J=4.8 Hz, 2 H), 3.04 (br q, J=7.5 Hz, 2 H), 1.29 (br t, J=7.5 Hz, 3 H)

**Synthesis of compound 175**

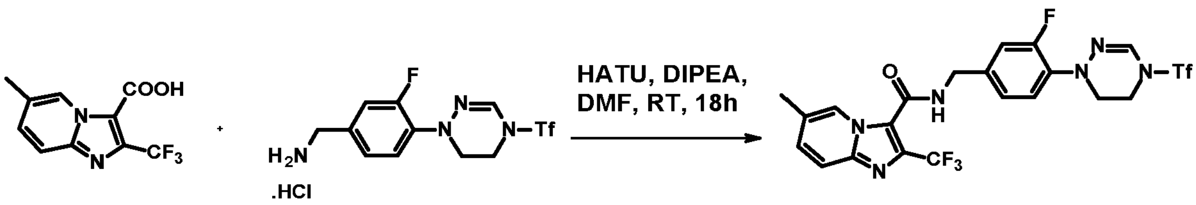


CAS [874830-67-8] Intermediate AA-3 compound 175

Accordingly, compound 175 was prepared in the same way as compound 124 starting from 6-methyl-2-(trifluoromethyl)imidazo[1,2-a]pyridine-3-carboxylic acid (CAS [874830-67-8], 0.53 mmol) and intermediate AA-3 affording 0.117 g (53%) as white powder.

$^1$H NMR (400 MHz, DMSO-d6) δ ppm 9.08 (s, 1H), 7.66 (d, J=9.2 Hz, 1H), 7.44 (t, J=8.4 Hz, 1H), 7.32 (dd, J=9.2, 1.6 Hz, 1H), 7.19 (s, 1H), 7.17 - 7.08 (m, 2H), 6.63 (br s, 1H), 4.64 (d, J=5.7 Hz, 2H), 4.13 - 4.04 (m, 2H), 3.74 - 3.65 (m, 2H), 2.41 (s, 3H).

**Synthesis of compound 125**

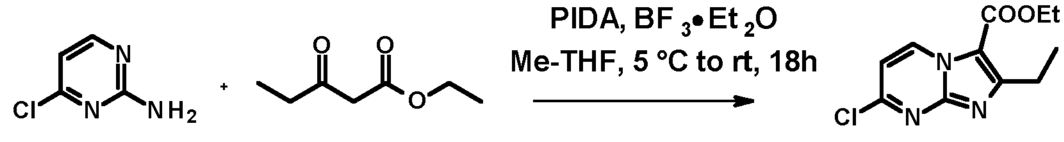


CAS [3993-78-0] CAS [4949-44-4] AE-1

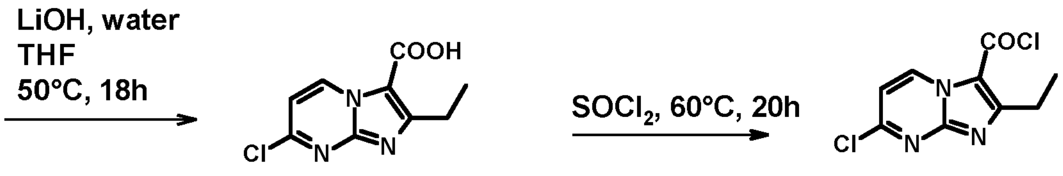


AE-2 AE-3

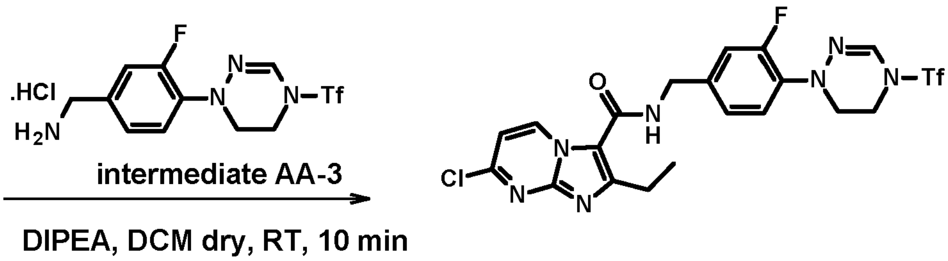


compound 125

Preparation of intermediate AE-1

Accordingly, intermediate AE-1 was prepared in the same way as intermediate AC-1 starting from 2-amino-4-chloropyrimidine (CAS [3993-78-0], 15.4 mmol) affording 0.94 g (26%).

Preparation of intermediate AE-2

Accordingly, intermediate AE-2 was prepared in the same way as intermediate AC-2 starting from intermediate AE-1 (1.25 mmol) affording 0.26 g (92%).

Preparation of intermediate AE-3

A mixture of intermediate AE-2 (175 mg, 0.776 mmol) in thionyl chloride (4.4 mL) was stirred at 60 °C for 20 h. The reaction mixture was evaporated to give 0.288 g as a brown paste. (The purity was calculated to give a quantitative yield).

Preparation of compound 125

A mixture of intermediate AE-3 (288 mg, 0.779 mmol) and intermediate AA-3 (295 mg, 0.779 mmol) and DIPEA (0.331 mL, 1.95 mmol) in dry DCM (4.8 mL) was stirred at room temperature for 10 min. Water was added. The aqueous layer was extracted with DCM (once). The combined organic layers were washed with brine, dried over $MgSO_4$, filtered off and evaporated to give 0.4 g as a brown foam. It was purified by preparative LC (regular SiOH 30 μm, 25 g, dry loading (celite®), mobile phase gradient: Heptane/EtOAc 90/10 to 50/50). The fraction containing products were combined and evaporated to give 0.229 g of a yellow foam. The yellow foam was sonicated in $Et_2O$. The precipitate was filtered off to give 146 mg of compound 125 as a white solid (33%).
$^1H$ NMR (500 MHz, DMSO-d6) δ ppm 9.29 (d, J=7.2 Hz, 1 H), 8.53 - 8.61 (m, 1 H), 7.38 (br s, 1 H), 7.34 (br t, J=8.7 Hz,

1 H), 7.17 - 7.28 (m, 3 H), 4.49 (br d, J=5.9 Hz, 2 H), 4.08 - 4.12 (m, 2 H), 3.65 (br t, J=4.9 Hz, 2 H), 3.01 (br q, J=7.4 Hz, 2 H), 1.27 (br t, J=7.4 Hz, 3 H)

**Synthesis of compound 130**

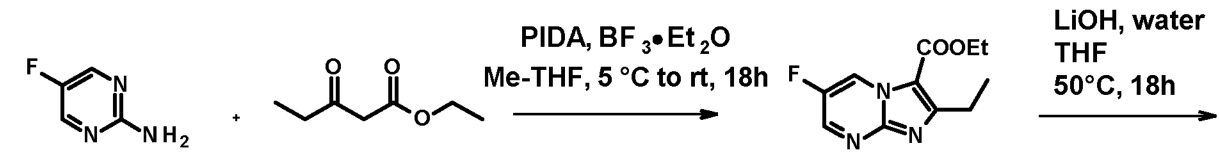


CAS [1683-85-8] CAS [4949-44-4] AF-1

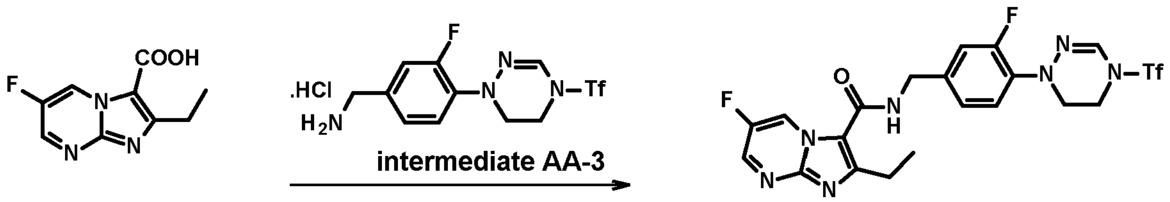


AF-2

HATU, DIPEA, DMF, RT, 18h

compound 130

Preparation of intermediate AF-1

Accordingly, intermediate AF-1 was prepared in the same way as intermediate AC-1 starting from 2-amino-5-fluoropyrimidine (CAS [1683-85-8], 17.68 mmol) affording 1.18 g (27%).

Preparation of intermediate AF-2

To a solution of intermediate AF-1 (1.1 g, 4.64 mmol) in EtOH (24 mL) and water (24 mL) was added potassium carbonate (3.2 g, 23.2 mmol) and the mixture was heated at 65°C and stirred for 3 h. (Alternative conditions re depicted in the scheme above.) The mixture was acidified to pH=1 with HCl 3M (no precipitation occurred) then evaporated in vacuo. The residue was taken up with EtOH/water (1:1), sonicated then filtered off (precipitate only contained $K_2CO_3$) and the filtrate was concentrated and then coevaporated twice with DCM to give 0.92 g of intermediate AF-2 as a brown solid (95%). The crude was used as such.

Preparation of compound 130

Accordingly, compound 130 was prepared in the same way as compound 124 starting from intermediate AF-2 (0.96 mmol) and intermediate AA-3 affording a white solid, 0.194 g (39%).
$^1$H NMR (400 MHz, DMSO-d6) δ ppm 9.39 - 9.48 (m, 1 H), 8.77 - 8.89 (m, 1 H), 8.50 - 8.59 (m, 1 H), 7.17 - 7.42 (m, 4 H), 4.52 (br d, J=4.4 Hz, 2 H), 4.07 - 4.13 (m, 2 H), 3.62 - 3.68 (m, 2 H), 3.05 (br q, J=7.2 Hz, 2 H), 1.29 (br t, J=7.5 Hz, 3 H)

**Synthesis of compound 131**

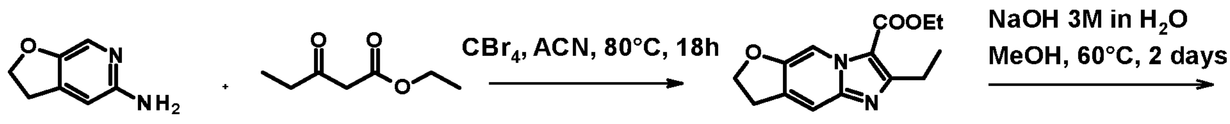


CAS [108990-72-3] CAS [4949-44-4] AG-1

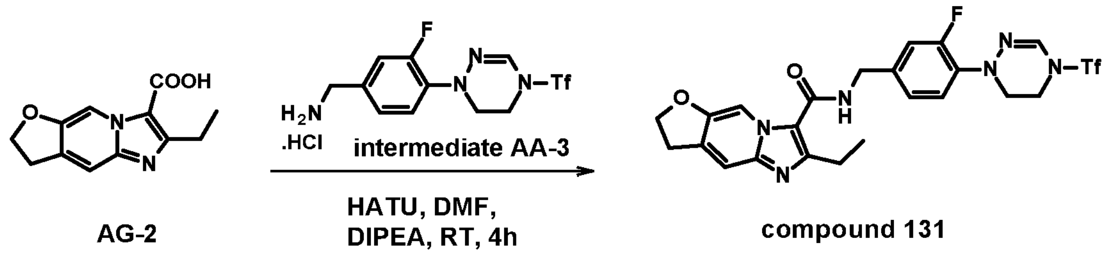


AG-2 compound 131

Preparation of intermediate AG-1

To a solution of 2H,3H-furo[2,3-c]pyridin-5-amine (CAS [1785357-12-1], 500 mg, 3.67 mmol) in ACN (8.4 mL) were added ethyloxovalerate (1.05 mL, 7.35 mmol) and boron tetrabromide (2.44 g, 7.35 mmol) and the reaction mixture was stirred at 80 °C for 18 h. The reaction mixture was diluted with EtOAc and the organic layer was washed with water and brine, dried over $MgSO_4$, filtered off, concentrated and purified by preparative LC (irregular SiOH, 15-40 μm, 40 g, liquid loading (DCM), mobile phase gradient: from Heptane/EtOAc: 100/0 to 0/100 in 10 CV then EtOAc 100% for 5 CV). The fractions containing product were combined and evaporated to give 0.21 g of intermediate AG-1 (22%).

Preparation of intermediate AG-2

A mixture of intermediate AG-1 (186 mg, 0.715 mmol), aqueous NaOH 3M (1.19 mL, 3.57 mmol) and MeOH (2 mL) was stirred 60 °C for 2 days. The mixture was evaporated to give 0.33 g of intermediate AG-2 (purity was estimated to give a quantitative yield).

Preparation of compound 131

Accordingly, compound 131 was prepared in the same way as compound 124 starting from intermediate AG-2 (0.71 mmol) and intermediate AA-3 affording a white solid, 0.09 g (23%).
$^1$H NMR (400 MHz, DMSO-d6) δ ppm 8.50 (s, 1 H), 8.19 - 8.32 (m, 1 H), 7.47 (s, 1 H), 7.38 (br s, 1 H), 7.29 - 7.36 (m, 1 H), 7.14 - 7.25 (m, 2 H), 4.61 (t, J=8.2 Hz, 2 H), 4.47 (br d, J=5.7 Hz, 2 H), 4.09 (br t, J=4.3 Hz, 2 H), 3.65 (t, J=4.7 Hz, 2 H), 3.25 - 3.32 (m, 2 H), 2.94 (q, J=7.5 Hz, 2 H), 1.24 (t, J=7.5 Hz, 3 H)

**Synthesis of compound 134**

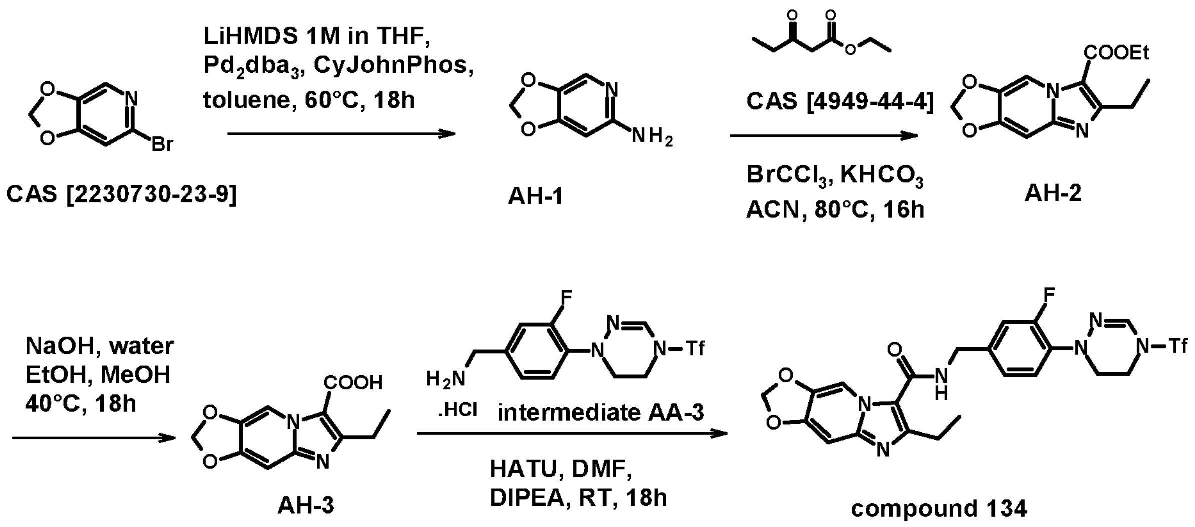


Preparation of intermediate AH-1

A solution of 6-bromo-1,3-Dioxolo[4,5-c]-pyridine (CAS [2230730-23-9], 3.87 g, 19.2 mmol) in dry toluene (100 mL) was c with $N_2$ (3 times). $Pd_2(dba)_3$ (1.75 g, 1.92 mmol) and CyJohnPhos (2.80 g, 7.66 mmol) were added and the reaction mixture was degassed with $N_2$ (3 times). LiHMDS (1.0M in THF) (23 mL, 23 mmol) was then added dropwise at room temperature and the reaction mixture was stirred at 60 °C for 18 h. The reaction mixture was diluted in EtOAc, water and acidified with an aqueous solution of HCl (1N). The aqueous layer was extracted with EtOAc (twice). The aqueous layer was then basified with a solution of NaOH (3M) and extracted with EtOAc (3 times). The combined organic layers were dried over $MgSO_4$, filtered off and evaporated to give 1.84 g of intermediate AH-1 as a brown solid (70%).

Preparation of intermediate AH-2

Accordingly, intermediate AH-2 was prepared in the same way as intermediate AB-1 starting from intermediate AH-1 (3.62 mmol) affording 0.165 g (17%).

Preparation of intermediate AH-3

Accordingly, intermediate AH-3 was prepared in the same way as intermediate AB-2 starting from intermediate AH-2 (0.95 mmol) affording 0.421 g (purity was estimated to give a quantitative yield).

Preparation of compound 134

Accordingly, compound 134 was prepared in the same way as compound 124 starting from intermediate AH-3 (0.45 mmol) and intermediate AA-3 affording a white solid, 0.194 g (84%).
$^1H$ NMR (400 MHz, DMSO-d6) δ ppm 8.62 (br s, 1 H), 8.24 (t, J=6.0 Hz, 1 H), 7.38 (s, 1 H), 7.34 (t, J=8.6 Hz, 1 H), 7.14 - 7.24 (m, 2 H), 7.08 (s, 1 H), 6.16 (br s, 2 H), 4.47 (br d, J=5.8 Hz, 2 H), 4.07 - 4.12 (m, 2 H), 3.65 (br t, J=4.6 Hz, 2 H), 2.91 (q, J=7.5 Hz, 2 H), 1.23 (t, J=7.5 Hz, 3 H)

**Synthesis of compound 161**

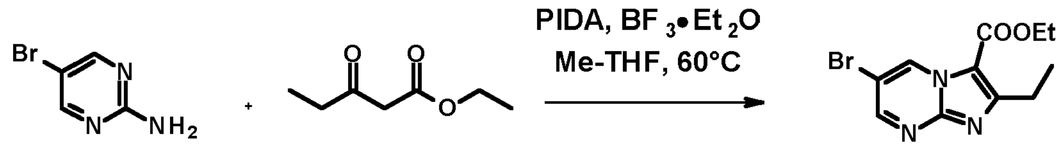


CAS [7752-82-1] CAS [4949-44-4] AI-1

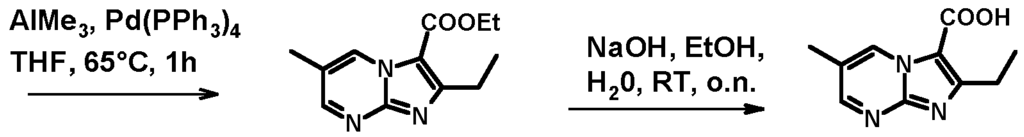


AI-2 AI-3

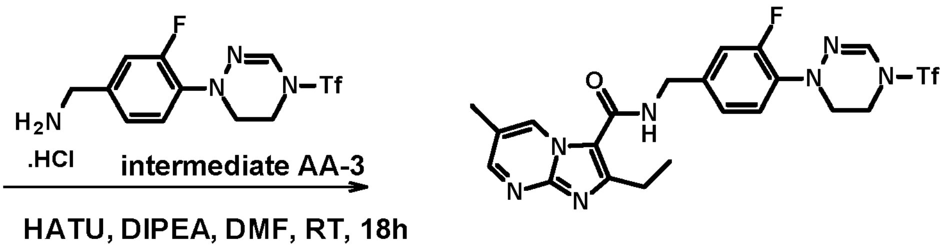


compound 161

Preparation of intermediate AI-1

2-amino-5-bromopyrimidine (10.0 g; 57.5 mmol) was suspended in dry 2-MeTHF (250 mL). ethyl 3-oxovalerate (8.2 mL, 57.5 mmol, 1 eq.) and iodobenzene diacetate (18.5 g, 57.5 mmol, 1 eq.) were added. boron trifluoride etherate (0.75 mL, 2.87 mmol, 0.05 eq.) was then added dropwise and the reaction mixture was stirred at 60 °C for 1.5 hours. An extra amount of ethyl ethyl 3-oxovalerate (4.10 mL, 28.7 mmol, 0.5 eq.), iodobenzene diacetate (9.25 g, 28.7 mmol, 0.5 eq.) and boron trifluoride etherate (0.75 mL, 2.87 mmol, 0.05 eq.) were added at room temperature and the mixture was stirred at 60 °C for 1h . The mixture was cooled down to room temperature then EtOAc and water were added. The organic layer was separated and washed with a saturated solution of NaHCOs (twice), then with brine (twice). The organic layer was dried over $MgSO_4$, filtered off and concentrated to give 19.7 g as a brown oil. The crude was purified by preparative LC (irregular SiOH, 15-40 μm, 330 g, dry loading (SiOH), mobile phase gradient: from DCM 100% to DCM 85%, EtOAc 15%) to give intermediate AI-1, 9.03 g as yellow crystals (53%).

Preparation of intermediate AI-2

In a sealed tube under $N_2$, to a solution of intermediate AI-1 (500 mg, 1.68 mmol) and $Pd(PPh_3)_4$ (96.9 mg, 0.084 mmol) in THF (12 mL) degassed under N2 was added trimethylaluminum 2m in Hexanes (2 eq., 1.68 mL, 3.35 mmol). The mixture was purged again with N2 and was heated at 65 °C for 1 h. An extra amount of trimethylaluminum 2m in Hexanes (1 eq., 0.839 mL, 1.68 mmol) was added and the mixture was stirred at 65 °C for 1 h. The mixture was diluted with DCM, cooled down to 0 °C and 1 mL of water was added carefully. The mixture was stirred at room temperature overnight then $MgSO_4$ was added. After 30 min under stirring, the mixture was filtered over a plug of celite® and evaporated to give 412 mg of as an orange gum. The crude was purified by preparative LC (regular SiOH, 30 μm, 40 g, dry loading (celite®), mobile phase eluent: Heptane 95%, EtOAc 5% to Heptane 50%, EtOAc 50%). Fractions containing product were combined and concentrated to obtain intermediate AI-2, 354 mg of as a yellow gum (90%).

Preparation of intermediate AI-3

To a solution of intermediate AI-2 (120 mg, 0.514 mmol) in water (1 mL) and EtOH (4 mL) was added NaOH

(62 mg, 1.55 mmol) and the mixture was stirred at room temperature overnight. The mixture was evaporated then co-evaporated with EtOH to give intermediate AI-3, 190 mg as a yellow solid. The crude was used as such in next step.

Preparation of compound 161

A mixture of intermediate AI-3 (190 mg, 0.518 mmol), HATU (280 mg, 0.736 mmol), DIPEA (0.163 mL, 0.958 mmol) and DMF (2.5 mL) was stirred at room temperature for 15 min then intermediate AA-3 (180 mg, 0.473 mmol) was added and stirring was continued over 3 days. DMF was evaporated. The residue was taken-up in DCM and water then washed with a saturated aqueous solution of $NaHCO_3$ (twice), brine (twice), dried over $MgSO_4$, filtered off and concentrated. The crude (m= 378 mg) was purified by preparative LC (regular SiOH, 30 μm, 24 g, mobile phase gradient: from Heptane 85%, EtOAc/MeOH (9:1) 15% to Heptane 25%, EtOAc/MeOH (9:1) 75). Fractions containing product were combined and concentrated to afford 277 mg as a white solid. The solid was recrystallized from EtOAc, filtered off and dried under high vacuum to afford 162 mg of compound 161 as a white solid (54%).
$^1H$ NMR (400 MHz, DMSO-d6) δ ppm 9.15 (d, J=1.2 Hz, 1 H), 8.52 (br d, J=2.3 Hz, 1 H), 8.44 - 8.49 (m, 1 H), 7.38 (br s, 1 H), 7.34 (m, J=8.6 Hz, 1 H), 7.17 - 7.27 (m, 2 H), 4.50 (br d, J=5.9 Hz, 2 H), 4.07 - 4.13 (m, 2 H), 3.65 (br t, J=4.6 Hz, 2 H), 3.01 (q, J=7.5 Hz, 2 H), 2.34 (br s, 3 H), 1.28 (t, J=7.5 Hz, 3 H)

**Synthesis of compounds 162, 148 & 151**

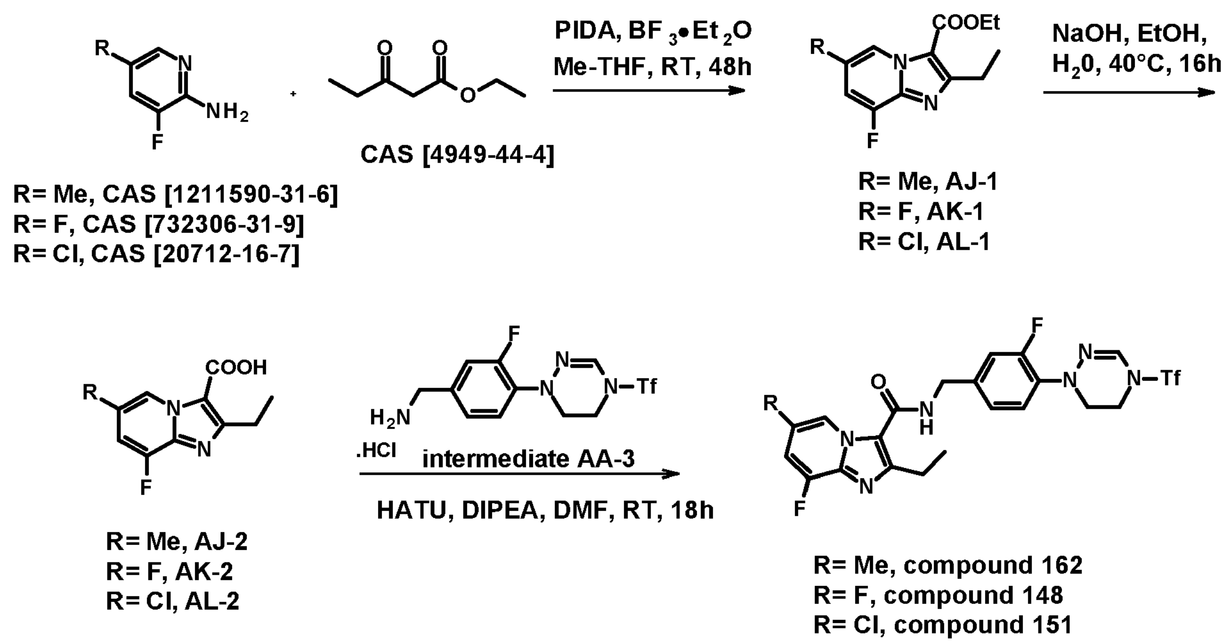


Preparation of intermediate AJ-1

The reaction was performed in anhydrous conditions under nitrogen atmosphere.

To a solution of 3-Fluoro-5-methylpyridin-2-amine (2.00 g, 15.9 mmol) in 2-MeTHF (60 mL) at 5 °C under $N_2$ were added Ethyl propionyl acetate (3.60 mL, 24.8 mmol), Iodobenze diacetate (7.80 g, 24.2 mmol) and Boron trifluoride diethyl etherate (200 μL, 1.62 mmol). The reaction was stirred 1 h at 5°C then at room temperature for 48 h. EtOAc (200 mL) and water (200 mL) were added. The layers were separated, and the organic layer was washed with a saturated aqueous solution of NaHCOs (200 mL), brine (2 x 100 mL), dried over $Na_2SO_4$, filtered and evaporated to afford 4.92 g as a brown paste. The crude was purified via preparative LC (SiOH, 120 g, 50 μm, Eluent: Cyclohexane/EtOAc, from 95:05 to 50:5), fractions containing product were collected, evaporated and triturated with pentane (2 x 20 mL) to afford 1.68 g of intermediate AJ-1 as a white solid (42%).

Preparation of intermediate AJ-2

To a solution of intermediate AJ-1 (500 mg, 2.00 mmol) in water (12.5 mL) and EtOH (12.5 mL) was added

NaOH (275 mg, 6.880 mmol). The reaction mixture was stirred for 16 h at 40 °C. The crude was washed with DCM (30 mL) and with EtOAc (30 mL), the aqueous phase was acidified with an aqueous solution of HCl (3N) until pH = 2. The formed precipitate was recuperated using a sintered glass under vacuum, washed with water (2 x 2 mL) and dried in a vacuum chamber at 50 °C overnight to afford 415 mg of intermediate AJ-2 as an off-white solid (93%).

Preparation of compound 162

Accordingly, compound 162 was prepared in the same way as compound 161 starting from intermediate AJ-2 (0.36 mmol) and intermediate AA-3 affording 0.113 g (48%) as white solid.
$^1$H NMR (400 MHz, DMSO-d6) δ ppm 8.61 (br s, 1 H), 8.53 (br t, J=5.9 Hz, 1 H), 7.31 - 7.40 (m, 2 H), 7.17 - 7.27 (m, 3 H), 4.50 (d, J=5.9 Hz, 2 H), 4.10 (br t, J=4.5 Hz, 2 H), 3.65 (br t, J=4.5 Hz, 2 H), 2.98 (q, J=7.5 Hz, 2 H), 2.31 (s, 3 H), 1.26 (t, J=7.5 Hz, 3 H)

Preparation of intermediate AK-1

Accordingly, intermediate AK-1 was prepared in the same way as intermediate AJ-1 starting from 2-Amino-3,5-difluoropyridine (CAS [732306-31-9], 15.37 mmol) affording 0.89 g (23%) as white solid.

Preparation of intermediate AK-2

Accordingly, intermediate AK-2 was prepared in the same way as intermediate AJ-2 starting from intermediate AK-1 (1.97 mmol) giving 0.345 g (78%).

Preparation of compound 148

Accordingly, compound 148 was prepared in the same way as compound 161 starting from intermediate AK-2 (0.35 mmol) and intermediate AA-3 affording 0.189 g (82%) as white solid.
$^1$H NMR (500 MHz, DMSO-d6) δ ppm 8.92 (dd, J=4.7, 1.8 Hz, 1 H), 8.58 (t, J=5.9 Hz, 1 H), 7.64 - 7.74 (m, 1 H), 7.38 (br s, 1 H), 7.35 (t, J=8.5 Hz, 1 H), 7.18 - 7.27 (m, 2 H), 4.50 (d, J=5.9 Hz, 2 H), 4.10 (br t, J=4.7 Hz, 2 H), 3.65 (t, J=4.9 Hz, 2 H), 3.01 (q, J=7.5 Hz, 2 H), 1.27 (t, J=7.6 Hz, 3 H)

Preparation of intermediate AL-1

Accordingly, intermediate AL-1 was prepared in the same way as intermediate AJ-1 starting from 2-Amino-5-chloro-3-fluoropyridine (CAS [20712-16-7], 17.06 mmol) affording 0.52 g (11%) as white solid.

Preparation of intermediate AL-2

Accordingly, intermediate AL-2 was prepared in the same way as intermediate AJ-2 starting from intermediate AL-1 (1.77 mmol) giving 0.26 g (60%).

Preparation of compound 151

Accordingly, compound 151 was prepared in the same way as compound 161 starting from intermediate AL-2 (0.43 mmol) and intermediate AA-3 affording 0.104 g (38%) as white solid.
$^1$H NMR (400 MHz, DMSO-d6) δ ppm 8.92 (d, J=1.0 Hz, 1 H), 8.58 - 8.67 (m, 1 H), 7.63 (dd, J=10.6, 1.4 Hz, 1 H), 7.31 - 7.40 (m, 2 H), 7.17 - 7.28 (m, 2 H), 4.51 (br d, J=5.6 Hz, 2 H), 4.07 - 4.13 (m, 2 H), 3.65 (t, J=4.6 Hz, 2 H), 3.01 (q, J=7.4 Hz, 2 H), 1.27 (t, J=7.4 Hz, 3 H)

**Synthesis of compounds 145 & 144**

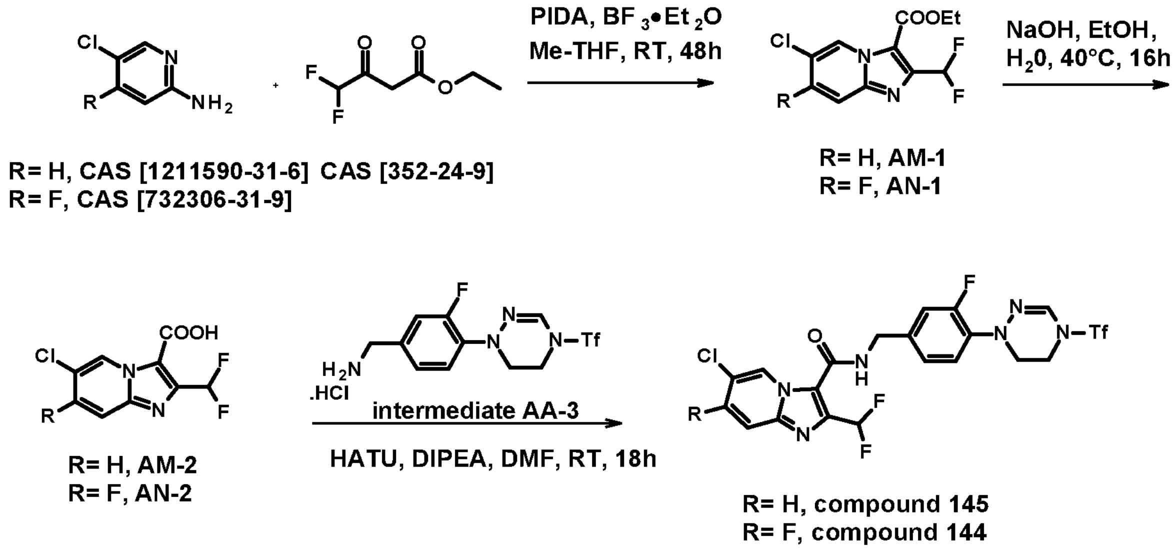


R= H, CAS [1211590-31-6] CAS [352-24-9]
R= F, CAS [732306-31-9]

R= H, AM-1
R= F, AN-1

R= H, AM-2
R= F, AN-2

R= H, compound 145
R= F, compound 144

Preparation of intermediate AM-1

Accordingly, intermediate AM-1 was prepared in the same way as AJ-1 starting from 2-amino-5-chloropyridine (CAS [1072-98-6], 3.89 mmol) and Ethyl 4,4-difluoro-3-oxobutyrate (CAS [352-24-9]) giving 0.248 g (23%) as white solid.

Preparation of intermediate AM-2

Accordingly, intermediate AM-2 was prepared in the same way as intermediate AJ-2 starting from intermediate AM-1 (0.73 mmol) giving 0.175 g (96%).

Preparation of compound 145

Accordingly, compound 145 was prepared in the same way as compound 161 starting from intermediate AM-2 (0.39 mmol) and intermediate AA-3 affording 0.164 g (64%) as white solid.
$^1$H NMR (500 MHz, DMSO-d6) δ ppm 9.04 (s, 1 H), 8.88 - 8.96 (m, 1 H), 7.83 (dd, J=9.6, 1 Hz, 1 H), 7.61 (dd, J=9.6, 2.1 Hz, 1 H), 7.46 - 7.47 (m, 1 H), 7.33 - 7.40 (m, 2 H), 7.19 - 7.30 (m, 2 H), 4.51 - 4.54 (m, 2 H), 4.08 - 4.12 (m, 2 H), 3.66 (br t, J=4.9 Hz, 2H)

Preparation of intermediate AN-1

Accordingly, intermediate AN-1 was prepared in the same way as AJ-1 starting from 5-Chloro-4-fluoropyridin-2-amine (CAS [1393574-54-3], 6.82 mmol) and Ethyl 4,4-difluoro-3-oxobutyrate (CAS [352-24-9]) giving 0.57 g (28%) as white solid.

Preparation of intermediate AN-2

Accordingly, intermediate AN-2 was prepared in the same way as intermediate AJ-2 starting from intermediate AN-1 (0.85 mmol) giving 0.145 g (64%).

Preparation of compound 144

Accordingly, compound 144 was prepared in the same way as compound 161 starting from intermediate AM-2 (0.41 mmol) and intermediate AA-3 affording 0.204 g (72%) as white solid.
$^1$H NMR (500 MHz, DMSO-d6) δ ppm 9.09 (d, J=7.2 Hz, 1 H), 9.03 - 9.07 (m, 1 H), 7.98 (d, J=9.6 Hz 1 H), 7.20 - 7.40 (m, 4 H), 4.52 (br d, J=4.6 Hz, 2 H), 4.09 - 4.13 (m, 2 H), 3.65 - 3.68 (m, 2 H), 2.53 (br s, 1 H)

**Synthesis of compound 138, 139 & 140 and compound 143**

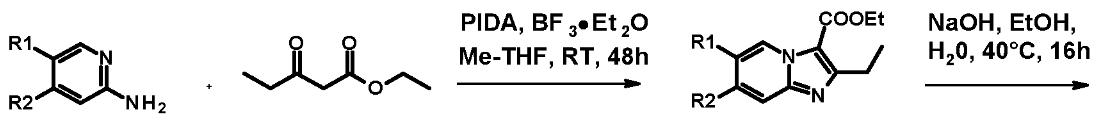


R1= Me, R2= Br, CAS [1033203-32-5]
R1= Me, R2= Me, CAS [57963-11-8]
R1= Me, R2= Cl, CAS [1033203-31-4]
R1= Cl, R2= Br, CAS [1187449-01-9]

R1= Me, R2= Br, AO-1
R1= Me, R2= Me, AP-1
R1= Me, R2= Cl, AQ-1
R1= Cl, R2= Br, AR-1

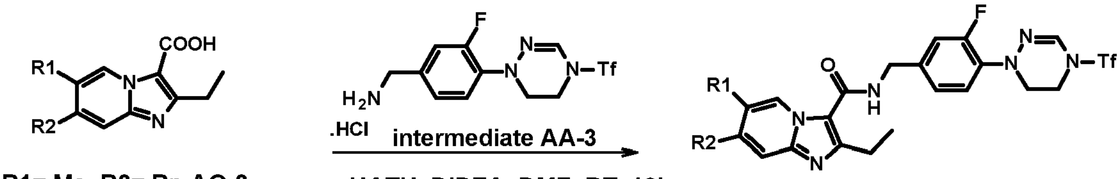


HATU, DIPEA, DMF, RT, 18h

R1= Me, R2= Br, AO-2
R1= Me, R2= Me, AP-2
R1= Me, R2= Cl, AQ-2
R1= Cl, R2= Br, AR-2

R1= Me, R2= Br, AO-3
R1= Me, R2= Me, compound 139
R1= Me, R2= Cl, compound 140
R1= Cl, R2= Br, AR-3

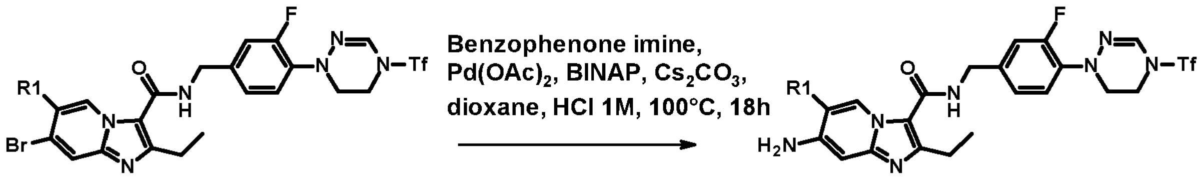


R1= Me, R2= Br, AO-3
R1= Cl, R2= Br, AR-3

R1= Me, compound 138,
R1= Cl, compound 143

Preparation of intermediate AO-1

Accordingly, intermediate AO-1 was prepared in the same way as AJ-1 starting from 4-bromo-5-methylpyridin-2-amine (CAS [1033203-32-5], 5.35 mmol) and ethyl 3-oxovalerate (CAS [4949-44-4]) giving 0.88 g (50%) as white solid.

Preparation of intermediate AO-2

Accordingly, intermediate AO-2 was prepared in the same way as intermediate AJ-2 starting from intermediate AO-1 (0.48 mmol) giving 0.205 g (78%).

Preparation of intermediate AO-3

Accordingly, intermediate AO-3 was prepared in the same way as compound 161 starting from intermediate AO-2 (0.49 mmol) and intermediate AA-3 affording 0.27 g (71%) as white solid.

Preparation of compound 138

A mixture of intermediate AO-3 (210 mg, 0.347 mmol), benzophenone imine (116 µL, 0.694 mmol), cesium carbonate (226 mg, 0.694 mmol) and 1,4-dioxane (1.75 mL) was purged with $N_2$, $Pd(OAc)_2$ (3.9 mg, 0.017 mmol) and BINAP (21.6 mg, 0.0347 mmol) were added. The mixture was purged with $N_2$ and stirred at 100 °C for 18 h. The mixture was filtered over a pad of celite® and the cake was washed with EtOAc. The organic layer was concentrated then the residue was stirred in 1,4-dioxane (2.5 ml) and aqueous HCl 1M (2.5 mL) at room temperature for 16 h. The mixture was diluted with EtOAc and slowly quenched with a saturated aqueous solution of $NaHCO_3$. The layers were separated, and the aqueous layer was extracted with EtOAc (twice). The organic layers were combined, dried over $MgSO_4$, filtered off and evaporated. The residue was purified by preparative LC (regular SiOH, 30 µm, 24 g, mobile phase eluent: from Heptane 90%, EtOAc/MeOH/aq. $NH_3$ (90:9.5:0.5) 10% to Heptane 20%, EtOAc/MeOH/aq. $NH_3$ (90:9.5:0.5) 80%). Fractions containing product were combined and concentrated to obtain 0.125 g as a white solid. This solid was recrystallized

from EtOAc, filtered off and dried under high vacuum to obtain 97 mg of compound 138 as a white solid (52%).
$^1$H NMR (400 MHz, DMSO-d6) δ ppm 8.61 - 8.70 (m, 1 H), 7.89 (t, J=6.0 Hz, 1 H), 7.38 (s, 1 H), 7.32 (t, J=8.5 Hz, 1 H), 7.14 - 7.22 (m, 2 H), 6.46 - 6.47 (m, 1 H), 5.69 - 5.72 (m, 2 H), 4.44 (br d, J=5.8 Hz, 2 H), 4.10 (br t, J=4.3 Hz, 2 H), 3.64 (t, J=4.6 Hz, 2 H), 2.87 (q, J=7.5 Hz, 2 H), 2.08 (s, 3 H), 1.21 (t, J=7.5 Hz, 3 H)

Preparation of intermediate AP-1

Accordingly, intermediate AP-1 was prepared in the same way as AJ-1 starting from 4,5-dimethylpyridin-2-amine (CAS [57963-11-8], 4.09 mmol) and ethyl 3-oxovalerate (CAS [4949-44-4]) giving 0.73 g (72%) as white solid.

Preparation of intermediate AP-2

Accordingly, intermediate AP-2 was prepared in the same way as intermediate AJ-2 starting from intermediate AP-1 (0.81 mmol) giving 0.3 g (quantitative).

Preparation of compound 139

Accordingly, compound 139 was prepared in the same way as compound 161 starting from intermediate AP-2 (0.49 mmol) and intermediate AA-3 affording 0.142 g (58%) as white solid.
$^1$H NMR (500 MHz, DMSO-d6) δ ppm 8.78 (br s, 1 H), 8.24 (t, J=5.9 Hz, 1 H), 7.38 (s, 2 H), 7.34 (t, J=8.5 Hz, 1 H), 7.16 - 7.25 (m, 2 H), 4.48 (d, J=5.9 Hz, 2 H), 4.10 (br t, J=4.7 Hz, 2 H), 3.65 (t, J=4.5 Hz, 2 H), 2.95 (q, J=7.5 Hz, 2 H), 2.30 (s, 3 H), 2.22 (s, 3 H), 1.25 (t, J=7.5 Hz, 3 H)

Preparation of intermediate AQ-1

Accordingly, intermediate AQ-1 was prepared in the same way as AJ-1 starting from 4-chloro-5-methylpyridin-2-amine (CAS [1033203-31-4], 7.01 mmol) and ethyl 3-oxovalerate (CAS [4949-44-4]) giving 0.39 g (20%) as white solid.

Preparation of intermediate AQ-2

Accordingly, intermediate AQ-2 was prepared in the same way as intermediate AJ-2 starting from intermediate AQ-1 (0.45 mmol) giving 0.15 g (quantitative).

Preparation of compound 140

Accordingly, compound 140 was prepared in the same way as compound 161 starting from intermediate AQ-2 (0.45 mmol) and intermediate AA-3 affording 0.23 g (68%) as white powder.
$^1$H NMR (500 MHz, DMSO-d6) δ ppm 8.95 (s, 1 H), 8.45 (br t, J=5.9 Hz, 1 H), 7.81 (br s, 1 H), 7.38 (br s, 1 H), 7.34 (t, J=8.5 Hz, 1 H), 7.17 - 7.26 (m, 2 H), 4.50 (d, J=5.9 Hz, 2 H), 4.10 (br t, J=4.4 Hz, 2 H), 3.65 (t, J=4.7 Hz, 2 H), 2.97 (q, J=7.3 Hz, 2 H), 2.32 (s, 3 H), 1.26 (t, J=7.4 Hz, 3 H)

Preparation of intermediate AR-1

Accordingly, intermediate AR-1 was prepared in the same way as AJ-1 starting from 4-bromo-5-chloropyridin-2-amine (CAS [1187449-01-9], 9.64 mmol) and ethyl 3-oxovalerate (CAS [4949-44-4]) giving 0.655 g (21%).

Preparation of intermediate AR-2

Accordingly, intermediate AR-2 was prepared in the same way as intermediate AJ-2 starting from intermediate AR-1 (2.05 mmol) giving 0.94 g (quantitative).

Preparation of intermediate AR-3

Accordingly, intermediate AR-3 was prepared in the same way as compound 161 starting from intermediate AR-2 (2.06 mmol) and intermediate AA-3 affording 0.42 g (33%) as an off-white solid.

Preparation of compound 143

Accordingly, compound 143 was prepared in the same way as compound 138 starting from intermediate AR-3 (0.4 mmol) giving 0.08 g (33%) as white solid.
$^1H$ NMR (400 MHz, DMSO-d6) $\delta$ ppm 9.03 (s, 1 H), 8.01 (t, J=5.7 Hz, 1 H), 7.38 (s, 1 H), 7.33 (t, J=8.6 Hz, 1 H), 7.15 - 7.24 (m, 2 H), 6.63 (br s, 1 H), 6.12 (br s, 2 H), 4.45 (d, J=5.9 Hz, 2 H), 4.07 - 4.12 (m, 2 H), 3.64 (t, J=4.5 Hz, 2 H), 2.90 (q, J=7.5 Hz, 2 H), 1.22 (t, J=7.5 Hz, 3 H)

**Synthesis of compound 126**

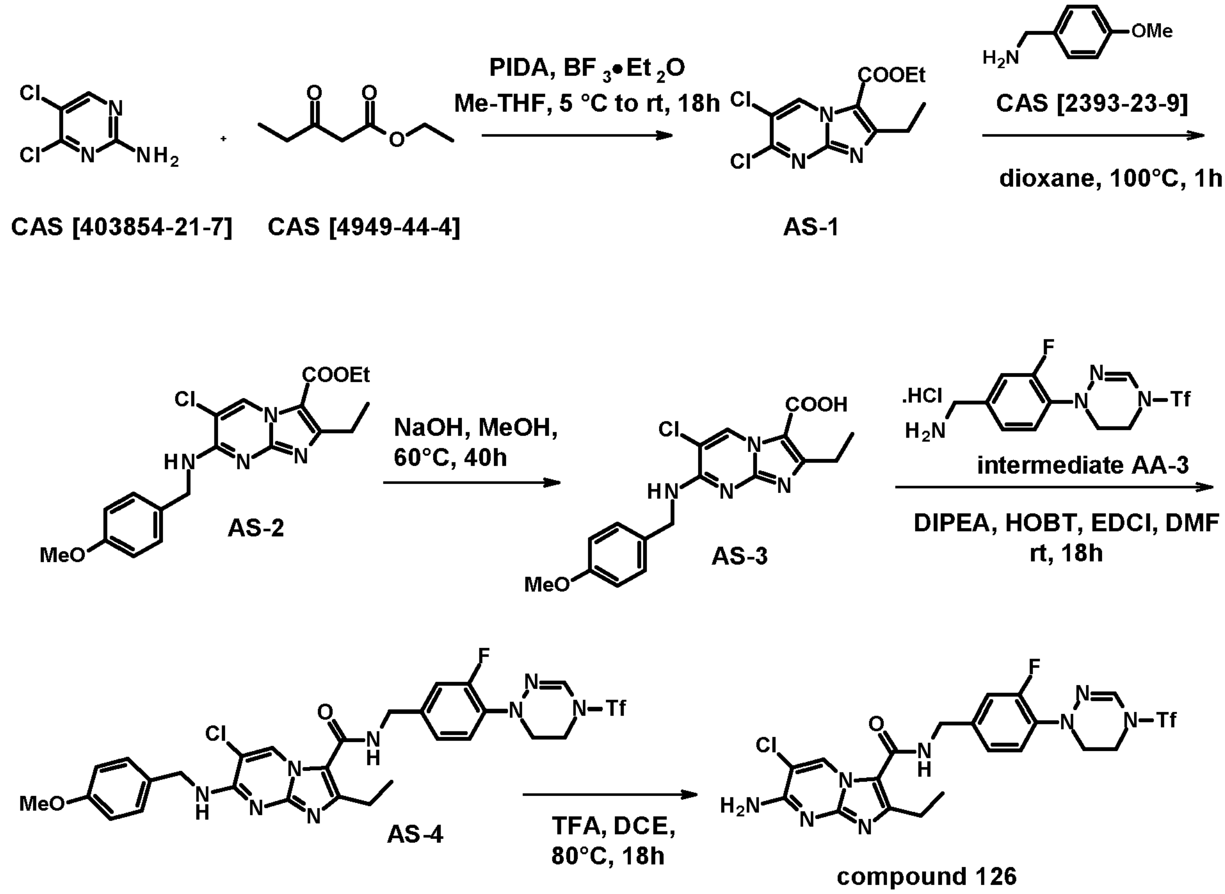


Preparation of intermediate AS-1

To a solution of 4,5-dichloropyrimidin-2-amine (CAS [403854-21-7], 12.5 g, 76.2 mmol) in Me-THF (315 mL) at 0 °C were added iodobenzene diacetate (73.7 g, 229 mmol) and ethyl 3-oxovalerate (16.5 mL, 116 mmol). Then boron trifluoride etherate (1.92 mL, 15.2 mmol) was added dropwise. The mixture was stirred at 5 °C for 1 h and then at room temperature for 16 h. Extra boron trifluoride etherate (1.92 mL, 15.2 mmol) was added dropwise and the reaction mixture was stirred at room temperature for 28 h. EtOAc and water were added. The organic layer was washed with brine, dried over $MgSO_4$ and evaporated to give a brown oil. The oil was purified by preparative LC (irregular SiOH, 15-40 $\mu$m, 330 g, gradient: Heptane 100% to heptane/EtOAc 75/25). The fractions containing product were combined and evaporated to give a yellow mixture which was triturated in pentane. The supernatant was removed by pipette and the residue was dried under vacuum to give 1.16 g of intermediate AS-1 as a white solid (5%). The supernatant was evaporated to give a yellow mixture. The supernatant was removed by pipette to give 5.02 g of intermediate AS-1 as a yellow paste (32%).

Preparation of intermediate AS-2

A mixture of intermediate AS-1 (5.02 g, 5.58 mmol, purity 32%), 4-methoxybenzylamine (CAS [2393-23-9],

2.19 mL, 16.7 mmol) and 1,4-dioxane (16 mL) was stirred at 100 °C for 1 h. The mixture was evaporated and purified by preparative LC (irregular SiOH, 15-40 μm, 120 g, dry loading (celite®), mobile phase gradient: from Heptane/EtOAc: 70/30 to 30/70). The fractions containing product were combined and evaporated to give 1.6 g of intermediate AS-2 (74%).

Preparation of intermediate AS-3

A mixture of intermediate AS-2 (0.900 g, 2.31 mmol), NaOH (278 mg, 6.94 mmol) and MeOH (9.2 mL) was stirred at 60 °C for 40 h. The mixture was evaporated to give 1.05 g of intermediate AS-3 (quantitative).

Preparation of intermediate AS-4

A mixture of intermediate AS-3 (1.05 g, 2.30 mmol, purity 84%), EDCI.HCl (0.8783 g, 4.61 mmol), HOBT.$H_2$0 (0.706 mg, 4.61 mmol), DIPEA (1.19 ml, 6.91 mmol) and DMF (35 mL) was stirred at 50 °C for 30 min. Intermediate AA-3 (865 mg, 2.42 mmol) was added and the mixture was stirred at room temperature for 18 h. The reaction mixture was diluted with EtOAc and the organic layer was washed with water and brine, dried over $MgSO_4$, filtered off, concentrated and purified by preparative LC (irregular SiOH, 15-40 μm, 120 g, mobile phase gradient: from heptane/EtOAc 50/50 to 0/100). The fractions containing product were combined and evaporated to give 560 mg of intermediate AS-4 (36%).

Preparation of compound 126

A mixture of intermediate AS-4 (560 mg, 0.820 mmol), TFA (4.5 mL) and DCE (4.5 mL) was stirred at 80 °C for 20 h. The mixture was evaporated and purified by preparative LC (spherical C18 25 μm, 120 g YMC-ODS-25, liquid loading (DMSO), mobile phase gradient 0.2% aq. $NH_4^+HCO_3^-$ / MeCN from 75:25 to 20:80). The fractions containing product were evaporated to give 204 mg as white solid. and 350 mg of impure desired product. This second fraction was purified by preparative LC (spherical C18 25 μm, 120 g YMC-ODS-25, liquid loading (DMSO), mobile phase gradient 0.2% aq. $NH_4^+HCO_3^-$ / MeCN from 75:25 to 20:80). The fractions containing product were evaporated to give 65 mg as white solid. Fractions of pure compounds were solubilized with EtOAc at reflux. The mixture was slowly cooled to room temperature with a slow stirring. The precipitate was filtered to give 0.355 g of compound 126 as a white solid (93%).
$^1$H NMR (500 MHz, DMSO-d6) δ ppm 9.06 (s, 1H), 8.12 (t, J=6.0 Hz, 1H), 6.99 - 7.64 (m, 6H), 4.45 (d, J=6.0 Hz, 2H), 4.09 (br d, J=5.2 Hz, 2H), 3.64 (t, J=4.7 Hz, 2H), 2.87 (q, J=7.4 Hz, 2H), 1.21 (t, J=7.5 Hz, 3H)

**Synthesis of compound 155**

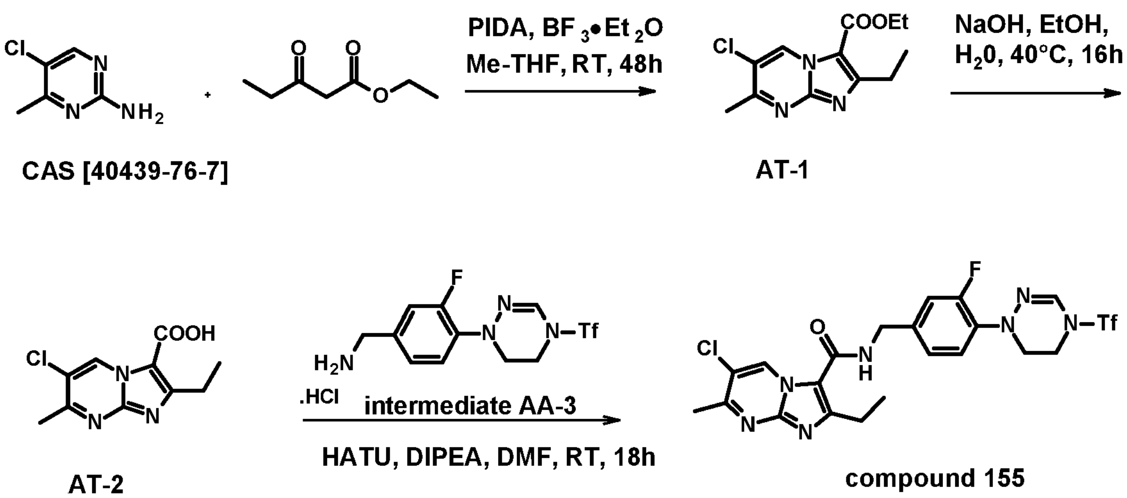


CAS [40439-76-7] AT-1 AT-2 compound 155

Preparation of intermediate AT-1

Accordingly, intermediate AT-1 was prepared in the same way as AJ-1 starting from 5-chloro-4-methylpyrimidin-2-amine (CAS [40439-76-7], 6.96 mmol) and ethyl 3-oxovalerate (CAS [4949-44-4]) giving 0.37 g (20%) as white solid.

Preparation of intermediate AT-2

Accordingly, intermediate AT-2 was prepared in the same way as intermediate AJ-2 starting from intermediate AT-1 (0.37 mmol) giving 0.165 g (quantitative).

Preparation of compound 155

Accordingly, compound 155 was prepared in the same way as compound 161 starting from intermediate AT-2 (0.38 mmol) and intermediate AA-3 affording 0.055 g (26%) as white powder.
$^1$H NMR (500 MHz, DMSO-$d_6$) δ ppm 9.35 (br s, 1 H), 8.48 (t, *J*=6.1 Hz, 1 H), 7.30 - 7.40 (m, 2 H), 7.16 - 7.28 (m, 2 H), 4.50 (br d, *J*=5.6 Hz, 2 H), 4.06 - 4.13 (m, 2 H), 3.65 (br t, *J*=4.5 Hz, 2 H), 3.01 (q, *J*=7.5 Hz, 2 H), 2.62 (s, 3 H), 1.27 (t, *J*=7.5 Hz, 3 H)

**Synthesis of compound 150**

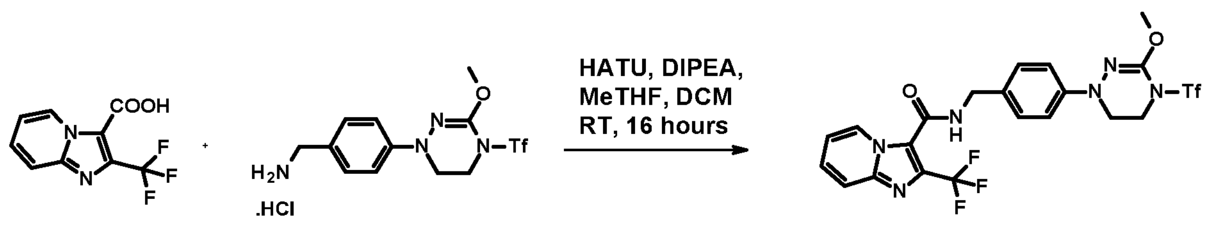


**CAS [73221-19-9]** **Intermediate N3** **compound 150**

HATU (0.097 g, 0.26 mmol) was added to a solution of 2-(Trifluoromethyl)-imidazo[1,2-A]pyridine-3-carboxylic acid (CAS [73221-19-9], 0.051 g, 0.22 mmol) and DIPEA (0.096 mL, 0.56 mmol) in dry Me-THF (1.5 mL) and DCM (0.5 mL) under $N_2$. The solution was stirred at room temperature for 15 min. Then intermediate N3 (0.095 g, 0.24 mmol) was added and the reaction mixture was stirred at room temperature for 16 hours. The solvent was evaporated then the residue was diluted in ethyl acetate, washed with a saturated aqueous solution of NaHCOs, water then brine. The organic layer was dried over MgSO4, filtered and evaporated in vacuo to give a yellow oil, 0.314 g. Purification was carried out by flash chromatography over silica gel (12 g, irregular SiOH 25-40μM, DCM/MeOH from 100/0 to 97/3). Pure fractions were collected and evaporated affording 0.119 g as white foam. It was triturated with DIPE and a few Heptane, the precipitate was filtered off and dried under vacuum at 60°C affording compound 150 as white powder, 0.103 g (82%).
$^1$H NMR (500 MHz, DMSO-$d_6$) δ ppm 9.21 (br t, *J*=5.3 Hz, 1H), 8.53 (br d, *J*=6.7 Hz, 1H), 7.79 (br d, *J*=9.0 Hz, 1H), 7.55 (br t, *J*=7.8 Hz, 1H), 7.29 (br d, J=8.4 Hz, 2H), 7.13 - 7.22 (m, 3H), 4.47 (br d, *J*=5.5 Hz, 2H), 4.07 - 4.15 (m, 2H), 3.86 (s, 3H), 3.76 (br t, *J*=4.6 Hz, 2H)

**Synthesis of compound 88**

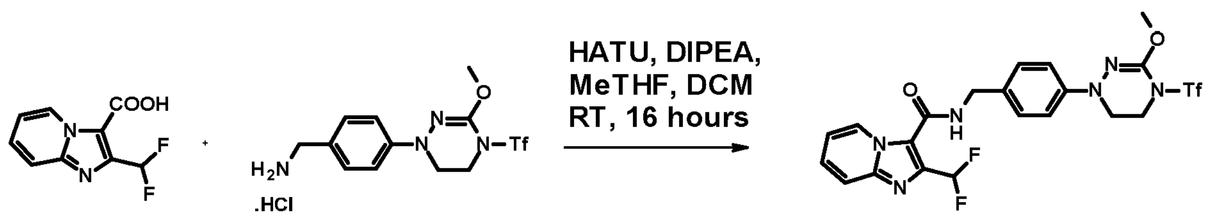


**CAS [2059954-47-9]** **Intermediate N3** **compound 88**

Accordingly, compound 88 was prepared in the same way as compound 150 starting from 2-(Difluoromethyl)-imidazo[1,2-A]pyridine-3-carboxylic acid (CAS [2059954-47-9], 0.23 mmol) and intermediate N3 affording a white powder, 0.104 g (86%).
$^1$H NMR (500 MHz, DMSO-$d_6$) δ ppm 8.94 (br t, *J*=5.1 Hz, 1H), 8.79 (d, *J*=7.0 Hz, 1H), 7.76 (d, *J*=9.0 Hz, 1H), 7.52 (t,

*J*=7.9 Hz, 1H), 7.19 - 7.43 (m, 3H), 7.14 - 7.19 (m, 3H), 4.47 (br d, *J*=5.2 Hz, 2H), 4.07 - 4.14 (m, 2H), 3.85 (s, 3H), 3.71 - 3.79 (m, 2H)

**Preparation of compound 200**

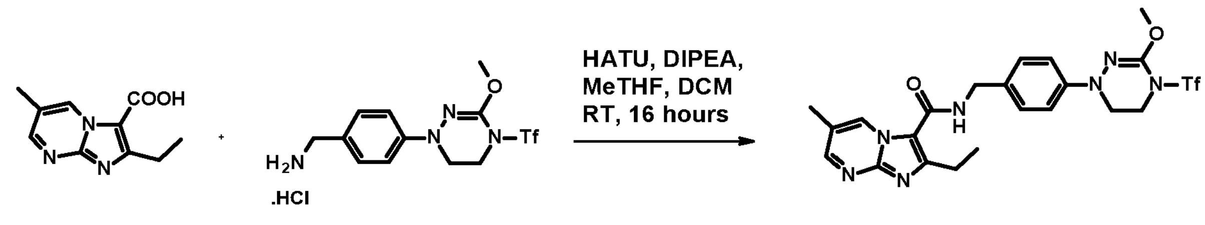


**intermediate AI-3** **Intermediate N3** **compound 200**

Accordingly, compound 200 was prepared in the same way as compound 150 starting from intermediate AI-3 (0.64 mmol) and intermediate N3 (0.51 mmol) affording a white powder, 0.085 g (31%).

$^1H$ NMR (400 MHz, DMSO) d 9.15 - 9.11 (m, 1H), 8.51 (d, J = 2.3 Hz, 1H), 8.41 (t, J = 5.9 Hz, 1H), 7.29 (d, J = 8.7 Hz, 2H), 7.15 (d, J = 8.7 Hz, 2H), 4.45 (d, J = 5.8 Hz, 2H), 4.15 - 4.06 (m, 2H), 3.85 (s, 3H), 3.76 - 3.70 (m, 2H), 2.98 (q, J = 7.5 Hz, 2H), 2.34 (s, 3H), 1.26 (t, J = 7.5 Hz, 3H).

**Synthesis of compound 169 & compound 180**

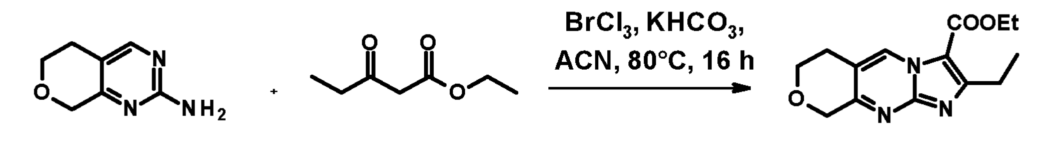


**CAS [1781072-41-0]** **CAS [4949-44-4]** **AU-1**

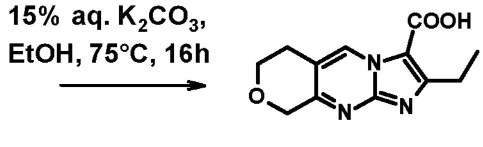


**AU-2**

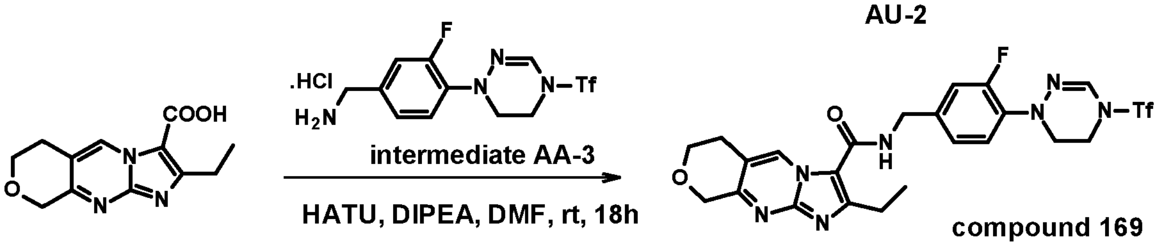


**compound 169**

**AU-2**

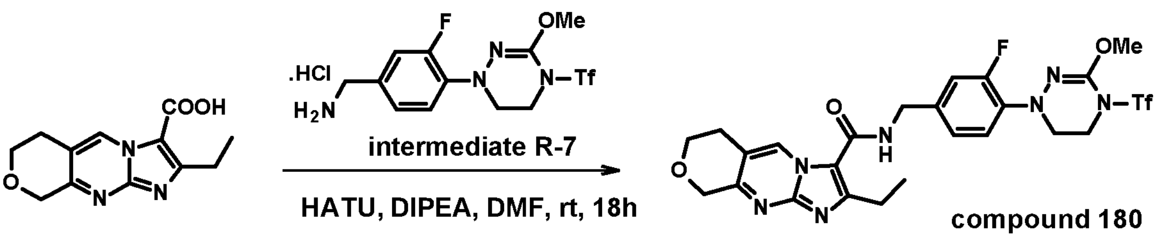


**compound 180**

**AU-2**

Preparation of intermediate AU-1

In a screw top vial, a mixture of Ethyl propionylacetate (0.105 g, 0.73 mmol), SH,6H,8H-pyrano[3,4-d]pyrimidin-2-amine (CAS [1781072-41-0], 0.11 g, 0.73 mmol), Potassium hydrogen carbonate (0.08 g, 0.8 mmol) and Bromotrichloromethane (0.143 mL, 1.45 mmol) in Acetonitrile (12 mL) at room temperautre was stirred at 80 °C for 16 hours. Additional Ethyl propionylacetate (0.105 g, 0.73 mmol), Potassium hydrogen carbonate (0.08 g, 0.8 mmol) and Bromotrichloromethane (0.143 mL, 1.45 mmol) were added to the mixture and it was stirred at 80 °C for 24 hours. Then, the mixture was diluted with EtOAc and washed with sat. NaHCOs aq. solution (3x). The organic layer was dried over $MgSO_4$, filtered and concentrated in vacuo. The crude was purified by flash column chromatography over silica gel (12g, EtOAc/Heptane from 0/100 to 100/0). The desired fractions were collected, and the solvent evaporated in vacuo to give intermediate AU-1 as a yellow sticky solid (0.084 g, 42%).

Preparation of intermediate AU-2

In a screw top vial, Potassium carbonate 15% aqueous solution (0.8 mmol, 0.87 mmol) was added over a solution of intermediate AU-1 in EtOH (4 mL) at room temperature. The reaction mixture was heated at 75 °C and stirred for 36h. Then, HCl 2M aq. solution was added until pH 3, and the solvent was evaporated in vacuo to yield intermediate AU-2 as an orange solid, that was used in the next step without further purification (0.18 g, quantitative)/

Preparation of compound 169

Accordingly, compound 169 was prepared in the same way as compound 161 starting from intermediate AU-2 (0.41 mmol) and intermediate AA-3 affording 0.051 g (28%) as white powder.

1H NMR (400 MHz, CDCl3) δ ppm 9.54 (s, 1H), 7.44 (t, J=8.5 Hz, 1H), 7.19 (s, 1H), 7.16 - 7.05 (m, 2H), 6.18 (br t, J=5.6 Hz, 1H), 4.84 (s, 2H), 4.64 (d, J=5.8 Hz, 2H), 4.13 - 4.05 (m, 2H), 4.02 (t, J=5.7 Hz, 2H), 3.71 - 3.63 (m, 2H), 3.05 - 2.89 (m, 4H), 1.45 (t, J=7.5 Hz, 3H).

Preparation of compound 180

Accordingly, compound 180 was prepared in the same way as compound 161 starting from intermediate AU-2 (0.081 mmol) and intermediate R-7 affording 0.012 g (30%) as white powder.

$^1H$ NMR (400 MHz, CDCl3) δ ppm 9.54 (s, 1H), 7.46 (t, J=8.6 Hz, 1H), 7.10 (m, 2H), 6.17 (br t, J=5.5 Hz, 1H), 4.84 (s, 2H), 4.63 (d, J=5.8 Hz, 2H), 4.15 - 4.05 (m, 2H), 4.02 (t, J=5.7 Hz, 2H), 3.89 (s, 3H), 3.65 - 3.55 (m, 2H), 3.07 - 2.92 (m, 4H), 1.45 (t, J=7.5 Hz, 3H).

**Synthesis of compound 177**

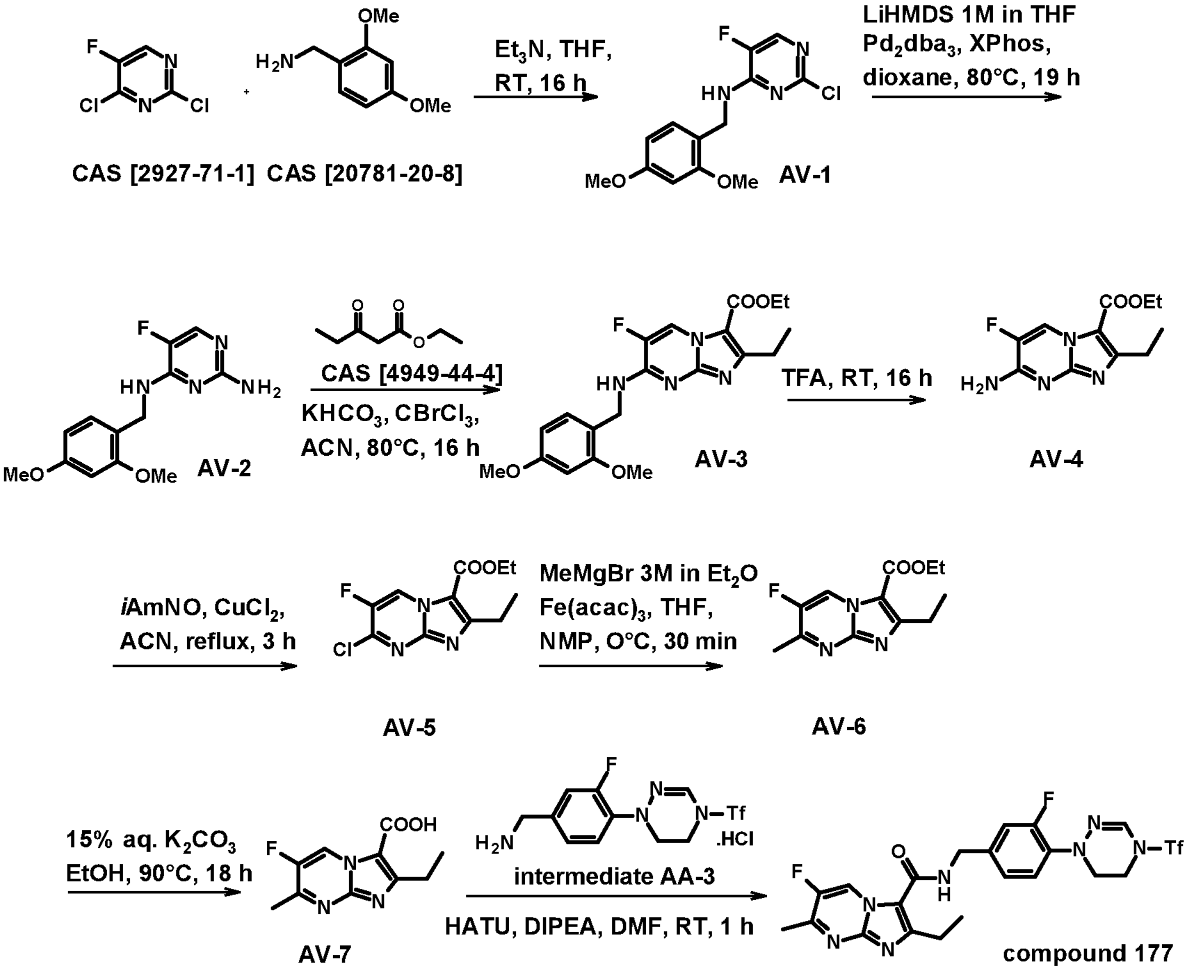

CAS [2927-71-1] CAS [20781-20-8]
AV-1
AV-2
AV-3
AV-4
AV-5
AV-6
AV-7
compound 177

Preparation of intermediate AV-1

The reaction was divided in two batches of 1.5 g each one.

2,4-Dimethoxybenzylamine (CAS [20781-20-8], 2.97 mL, 19.76 mmol) was added dropwise to a solution of 2,4-Dichloro-5-fluoropyrimidine (CAS [2927-71-1], 3g, 17.97 mmol) and triethylamine (3 mL, 21.5 mmol) in THF dry in a round bottom flask under nitrogen at 0 °C. The reaction mixture was allowed to warm to room temperature for 16 h. The mixture was diluted with saturated aqueous NaHCOs solution and extracted with EtOAc. The organic layer was separated, dried with $MgSO_4$, filtered and the solvents were evaporated in vacuo. The crude product was purified by flash column chromatography over silica gel (80 g, ethyl acetate in heptane from 100/0 to 20/80). The desired fractions were collected and concentrated in vacuo to yield intermediate AV-1 as a beige solid, 4.8 g (85%).

Preparation of intermediate AV-2

The reaction was divided in two batches of 2.4 g each one.

Tris(dibenzylideneacetone)dipalladium (0) (0.7 g, 0.77 mmol) and XPhos (0.73 g, 1.53 mmol) were added to a solution of AV-1 (4.32 g, 15.32 mmol) in dry dioxane (31 mL) while nitrogen was bubbling in a glass pressure bottle. Then lithium bis(trimethylsilyl)amide solution, 1M in THF (33.7 mL, 33.7 mmol) was added dropwise and the resulting solution was heated at 80 °C for 3 h. Tris(dibenzylideneacetone)dipalladium(0) (0.7 g, 0.77 mmol), XPhos (0.73 g, 1.53 mmol) and lithium bis(trimethylsilyl)amide solution, 1M in THF (33.7 mL, 33.7 mmol) were added while nitrogen was bubbling and the reaction mixture was heated at 80 °C for 16 h. The reaction was acidified with HCl 1N solution and stirred for 30 min. Then the result was extracted with EtOAc. The aqueous layer was neutralized with 1N NaOH solution and extracted with DCM. The organic layer was separated, dried ($MgSO_4$), filtered and the solvents were evaporated in vacuo to yield intermediate AV-2 as a brown solid, 3.4 g (76%).

Preparation of intermediate AV-3

The reaction was set up in 2 batches with the same quantity of reactive AV-2.

Potassium bicarbonate (0.6 g, 6.04 mmol) and Ethyl propionylacetate (0.89 mL, 6.04 mmol) were added to a solution of AV-2 (1.12 g, 4.02 mmol) in ACN (8.1 mL) in a screw top vial at rt. Then, Bromotricloromethane (1.19 mL, 12.07 mmol) was added at room temperature and the mixture was stirred at 80 °C for 16h. The batches were mixed to be worked out together. The mixture was diluted with water and extracted with EtOAc. The organic layer was dried (MgSO4), filtered and concentrated in vacuo. The crude was purified by flash chromatography column over silica gel (25 g; EtOAc in Heptane 0/100 to 35/65). The desired fractions were collected and concentrated in vacuo to yield intermediate AV-3 as a yellow foam solid, 0.42 g (22%).

Preparation of intermediate AV-4

TFA (9.64 mL, 128.43 mmol) was added to AV-3 (1.06 g, 2.37 mmol) in a round bottom flask at 0 °C. The mixture was stirred at room temperature for 16 h. The mixture was neutralized with sat. aqueous $NaHCO_3$ solution and extracted with DCM. The organic layer was washed with water and concentrated in vacuo. The result was triturated with DIPE and the solid was filtered to yield intermediate AV-4 as a beige solid, 0.6 g (95%).

Preparation of intermediate AV-5

Isoamylnitrite (CAS [110-46-3], 0.46 mL, 3.38 mmol) and copper (II) chloride (0.318 g, 2.36 mmol) were added to a suspension of AV-4 (0.6 g, 2.25 mmol) in dry ACN (36 mL) in a round bottom flask at room temperature. The mixture was stirred at reflux for 3 h. Water was added and the mixture was extracted with EtOAc. The organic layer was separated, dried ($MgSO_4$), filtered and the solvents were evaporated in vacuo. The crude was purified by flash chromatography column over silica gel (12 g; EtOAc in Heptane 0/100 to 10/90). The desired fractions were collected and concentrated in vacuo to yield intermediate AV-5 as a white solid, 0.315 g (51%).

Preparation of intermediate AV-6

Iron (III) acetylacetonate (0.051 g, 0.14 mmol) was added to a solution of AV-5 (0.39 g, 1.41 mmol) in dry THF (8 mL) and NMP (0.7 mL) in a round bottom flask under nitrogen at 0 °C. Then methylmagnesium bromide solution 3.0 M in diethyl ether (0.71 mL, 2.12 mmol) was added dropwise, and the reaction mixture was stirred at 0 °C for 30 min. TLC showed complete conversion. The reaction was quenched with saturated aqueous $NH_4Cl$ solution. The mixture was extracted with ethyl acetate. The organic layer was separated, dried over $MgSO_4$, filtered and the solvents were evaporated in vacuo. The crude product was purified by flash column chromatography over silica gel (12 g; EtOAc in heptane 0/100 to 15/75). The desired fractions were collected and concentrated in vacuo to yield a white solid, intermediate AV-6, 0.325 g (91%).

Preparation of intermediate AV-7

15% aqueous potassium carbonate (0.88 mL, 0.96 mmol) was added to a solution of AV-6 (0.152 g, 0.6 mmol) in EtOH (2 mL) in a screw top vial at room temperature. The mixture was stirred at 90 °C for 18 h. 15% aqueous potassium carbonate (0.88 mL, 0.96 mmol) was added to a reaction mixture. The mixture was stirred at 90 °C for 2 h. Then, 1M aqueous HCl solution was added until pH 7. The mixture was concentrated in vacuo to yield intermediate AV-7 as a white solid (0.188 g, quantitative).

Preparation of compound 177

Intermediate AA-3 (0.158 g, 0.4 mmol) was added to a solution of AV-7 (0.187 g, 0.6 mmol), HATU (0.198 g, 0.52 mmol), and DIPEA (0.42 mL, 2.4 mmol) in dry DMF (5 mL) in a round bottom flask at room temperature. The mixture was stirred at room temperature for 1 h. Saturated aqueous $NaHCO_3$ solution was added and the mixture was extracted with EtOAc (x3). The combined organic layers were dried over $MgSO_4$, filtered and concentrated in vacuo. The crude product was purified by flash column chromatography over silica gel (12 g; (DCM/MeOH 9:1) in DCM 0/100 to 10/90). The desired fractions were collected and concentrated in vacuo. The result was triturated with DIPE and the solid was filtered to yield compound 177 as a beige solid, 0.092 g (41%).

$^1H$ NMR (400 MHz, DMSO-d6) $\delta$ ppm 9.32 (d, J=5.5 Hz, 1H), 8.44 (br t, J=5.9 Hz, 1H), 7.38 (s, 1H), 7.34 (t, J=8.6 Hz, 1H), 7.25 (br d, J=13.2 Hz, 1H), 7.20 (br d, J=8.3 Hz, 1H), 4.50 (d, J=5.8 Hz, 2H), 4.17 - 4.02 (m, 2H), 3.72 - 3.58 (m, 2H), 3.02 (q, J=7.5 Hz, 2H), 2.56 (d, J=2.7 Hz, 3H), 1.28 (t, J=7.5 Hz, 3H).

Synthesis of compound 142 and compound 181

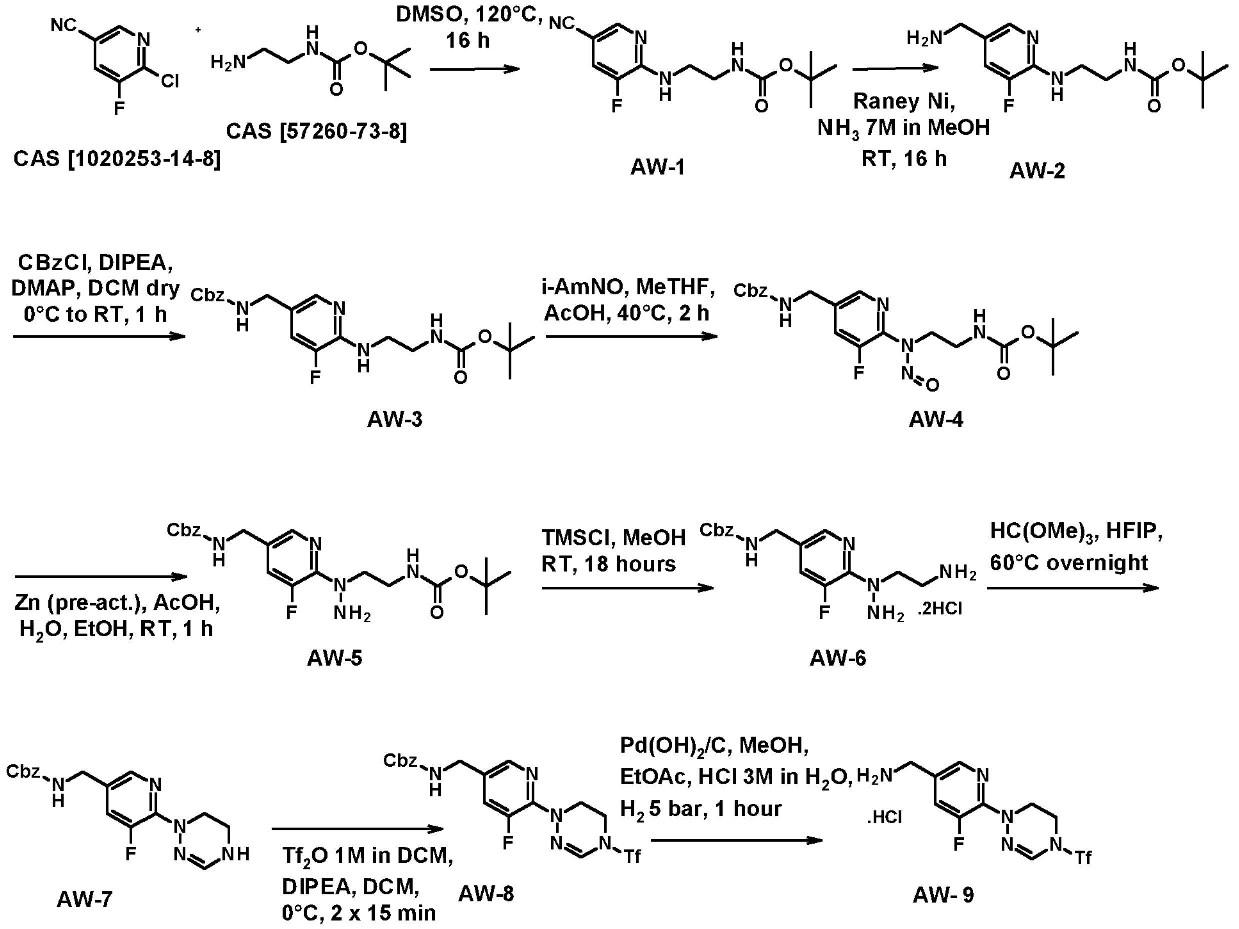


Preparation of intermediate AW-1

A solution of 6-chloro-5-fluoronicotinonitrile (CAS [1020253-14-8], 13.57 g, 86.68 mmol), n-boc-1,2-diaminoethane (CAS [57260-73-8], 17.8 mL, 113 mmol) and $Et_3N$ (48.2 mL, 347 mmol) in dry DMSO (155 mL) was stirred at 120 °C for 16 h. EtOAc and water were added to the reaction mixture. The layers were separated, and the organic layer was washed with brine (5 times), dried over MgSO4, filtered off and evaporated to give an orange solid. The solid was purified by preparative LC (regular SiOH 30 μm, 330 g, liquid loading (DCM), mobile phase gradient: Heptane/EtOAc 95/5 to Heptane/EtOAc 40/60). The fractions containing product were combined and evaporated to give 22.55 g of intermediate AW-1 as a yellow solid (93% yield).

Preparation of intermediate AW-2

To a solution of AW-1 (3.2 g, 11.42 mmol) in $NH_3$ (7M in MeOH) (179 mL), purged with nitrogen, was added Raney Nickel (5.3 g, 91.3 mmol) then the reaction mixture was hydrogenated under atmospheric pressure at room temperature for 16 hours. The mixture was filtered through a pad of Celite® and the Celite® was rinsed with MeOH and the filtrate was concentrated in vacuo. The residue was diluted in DCM, $MgSO_4$ was added. The mixture was filtered through a pad of Celite®, the Celite® was washed with DCM and the filtrate was evaporated in vacuo to give of mmotte_8598_1, 3.18 g, as colourless oil (96%).

Preparation of intermediate AW-3

A round-bottom flask was charged with a solution of AW-2 (3.18 g, 10.96 mmol), DIPEA (2.17 mL, 12.6 mmol) and DMAP (0.04 g, 0.33 mmol) in dry DCM (68.2 mL). The reaction mixture was connected to a nitrogen flow then cooled

down to 0 °C. Benzylchloroformate (1.72 mL, 12.06 mmol) was added dropwise. The reaction mixture was then stirred at 0 °C for 1h. The reaction mixture was quenched by addition of water and stirred for 10 minutes at room temperature. The aqueous layer was extracted with DCM (twice). The combined organic layer was dried over MgSO4, filtered off and evaporated to give 5.38 g as crude. Purification was carried out by flash chromatography over silica gel (120 g, irregular SiOH 25-40μM, DCM/MeOH from 100/0 to 97/3). Pure fractions were collected and evaporated affording intermediate AW-3 as pale beige solid, 3.54 g (77%).

Preparation of intermediate AW-4

AW-3 (3.54 g, 8.46 mmol) was solubilized at 40°C in Me-THF (65 mL) and AcOH (4.84 mL, 84.59 mmol). Then isoamylnitrite (5.68 mL, 42.3 mmol) was added dropwise and the mixture was stirred at 40°C for 2 hours. The solution was diluted in EtOAc (60 mL) and water (30 mL), washed with a saturated solution of NaHCOs (twice), brine, dried on $MgSO_4$ and evaporated to give 4.67 g as pale-yellow oil. Purification was carried out by flash chromatography over silica gel (80 g, irregular SiOH 25-40μM, DCM/MeOH from 100/0 to 97/3). Pure fractions were collected and evaporated affording intermediate AW-4 as a yellow oil, 3.99 g (97% with 92% purity, used as such for next step).

Preparation of intermediate AW-5

Zinc, dust (4.29 g, 65.63 mmol) was added to a solution of AW-4 (3.99 g, 8.2 mmol) and AcOH (7 mL, 123.05 mmol) in EtOH (170.9 mL) and water (42.7 mL) at room temperature. The mixture was stirred at room temperature for 1.5 hour. Water was added, the aqueous layer was extracted 3 times with DCM, the combined organic layers were dried over $MgSO_4$ and concentrated under reduced pressure giving a colourless oil, 4.12 g. Purification was carried out by flash chromatography over silica gel (80 g, irregular SiOH 25-40μM, DCM/MeOH from 100/0 to 97/3). Pure fractions were collected and evaporated affording intermediate AW-5, 1.88 g as colourless oil (50%).

Preparation of intermediate AW-6

To a solution of AW-5 (1.88 g, 4.08 mmol) in MeOH (40.2 mL) was added dropwise TMSCl (4.14 mL, 32.61 mmol). The reaction mixture was stirred at room temperature for 18 hours. The reaction mixture was concentrated in vacuo to give intermediate AW-6, 1.45 g (80%), used as such for next step.

Preparation of intermediate AW-7

A solution of AW-6 (1.45 g, 3.21 mmol) and B (1.41 mL, 12.85 mmol) in C (32.4 mL) was stirred at 70 °C overnight. The reaction mixture was evaporated. The residue was diluted in DCM and a 10 % aq. solution of $K_2CO_3$. The aqueous layer was extracted twice with DCM/MeOH (95/5). The combined organic layers were dried on $MgSO_4$, filtered off and evaporated to give a yellow solid. Purification was carried out by flash chromatography over silica gel (12g, irregular SiOH 25-40μM, DCM/MeOH from 100/0 to 90/10). Pure fractions were collected and evaporated affording intermediate AW-7 as colorless oil, 0.58 g, used as such for next step.

Preparation of intermediate AW-8

To a solution of AW-7 (0.58 g, 1.69 mmol) and DIPEA (0.87 mL, 5.07 mmol) in dry DCM (14.6 mL), cooled at 5°C in an ice bath, was added dropwise $Tf_2O$ 1M in DCM (1.69 mL, 1.69 mmol). The reaction mixture was stirred at 5°C for 15 min. The reaction mixture was immediately quenched with a saturated solution of $NaHCO_3$. The aqueous layer was extracted with DCM (twice). The combined organic layer was washed with brine (once), dried over $MgSO_4$, filtered off and evaporated. Purification was carried out by flash chromatography over silica gel (24 g, irregular SiOH 25-40μM, DCM/MeOH from 100/0 to 97/3). Pure fractions were collected and evaporated affording intermediate AW-8, as pale-yellow oil which crystalized on standing, 0.59 g (73%).

Preparation of intermediate AW-9

In a steal bomb, a mixture of AW-8 (0.59 g, 1.24 mmol), palladium hydroxide 20% on carbon nominally 50% water (0.17 g, 0.12 mmol) and aqueous HCl 3M (0.41 mL, 1.24 mmol) in MeOH (8.7 mL) and EtOAc (8.7 mL) was hydrogenated under 3 bar of $H_2$ at room temperature for 3 hours. The mixture was filtered on a pad of celite® and washed with MeOH. The filtrate was evaporated then co-evaporated with MeOH (twice) to give intermediate AW-9, 0.484 g (90%) as pale beige powder.

## Preparation of compound 142

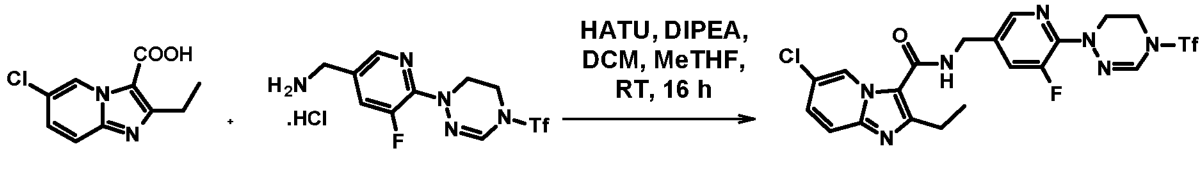


CAS [1216142-18-5] AW- 9 compound 142

HATU (0.15 g, 0.4 mmol) was added to a solution of 6-Chloro-2-ethylimidazo[1,2-a] pyridine-3-carboxylic acid (CAS [1216142-18-5], 0.078 g, 0.35 mmol) and DIPEA (0.21 mL, 1.21 mmol) in dry Me-THF (2.8 mL) and dry DCM (2 mL) under $N_2$ flow. The solution was stirred at room temperature for 15 min. Then AW-9 (0.118 g, 0.35 mmol) was added and the reaction mixture was stirred at room temperature for 16 hours. The solvent was evaporated then the residue was diluted in ethyl acetate, washed with a saturated aqueous solution of NaHCOs, water then brine. The organic layer was dried over MgSO4, filtered and evaporated in vacuo to give a brown residue. Purification was carried out by flash chromatography over silica gel (40 g, irregular SiOH 25-40μM, solid deposit on celite®, DCM/MeOH from 100/0 to 97/3). Pure fractions were collected and evaporated affording a pale-yellow powder, 0.512 g. A purification was performed via achiral SFC (Stationary phase: Whelk-O1 (S,S) 5μm 250*30mm, Mobile phase: 60% $CO_2$, 40% mixture of MeOH/DCM 80/20 v/v+0.3% $iPrNH_2$ ). Pure fractions were collected and evaporated affording a white solid, 0.31 g. This was triturated with DIPE and a few Heptane, the precipitate was filtered off and dried under vacuum at 60°C giving compound 142 as white powder, 0.29 g (47%).
$^1H$ NMR (500 MHz, DMSO-d6) δ ppm 9.09 (d, J=1.4 Hz, 1 H), 8.46 (t, J=5.8 Hz, 1 H), 8.13 (br s, 1 H), 7.63 - 7.75 (m, 2 H), 7.47 (dd, J=9.4, 2.1 Hz, 1 H), 7.37 (s, 1 H), 4.51 (br d, J=5.8 Hz 2 H), 4.13 (br t, J=4.5 Hz, 2 H), 3.92 (t, J=4.8 Hz, 2 H), 2.99 (q, J=7.5 Hz, 2 H), 1.26 (t, J=7.5 Hz, 3 H)

## Preparation of compound 181

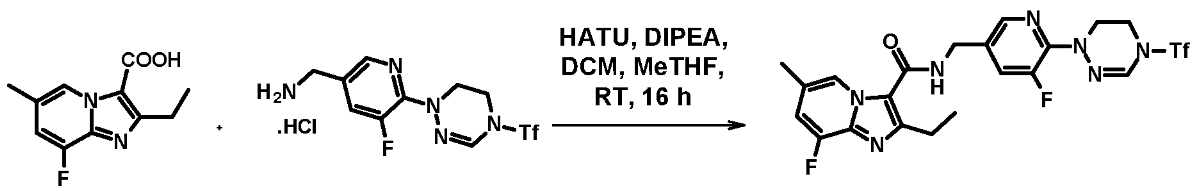


AJ-2 AW- 9 compound 181

AW-9 (0.09 g, 0.24 mmol) was added to a solution of AJ-2 (0.099 g, 0.38 mmol), HATU (0.12 g, 0.31 mmol) and DIPE (0.25 mL, 1.43 mmol) in dry DMF (5 mL) in a round bottom flask at room temperature. The mixture was stirred at room temperature for 16 h. The mixture was diluted with an aqueous saturated $NaHCO_3$ solution and extracted with DCM. The organic layer was separated, dried ($MgSO_4$), filtered and the solvents concentrated in vacuo to yield a brown oil. The crude product was triturated with DCM and the solid was filtered and dried in vacuo to yield a white solid, compound 181, 0.059 g (45%).

$^1H$ NMR (400 MHz, DMSO-d6) δ ppm 8.62 (s, 1H), 8.51 (br t, J=5.8 Hz, 1H), 8.13 (s, 1H), 7.69 (dd, J=12.7, 1.7 Hz, 1H), 7.37 (s, 1H), 7.22 (dd, J=11.7, 0.9 Hz, 1H), 4.51 (d, J=5.8 Hz, 2H), 4.17 - 4.10 (m, 2H), 3.96 - 3.89 (m, 2H), 2.97 (q, J=7.5 Hz, 2H), 2.31 (s, 3H), 1.26 (t, J=7.5 Hz, 3H).

## Preparation of compound 201

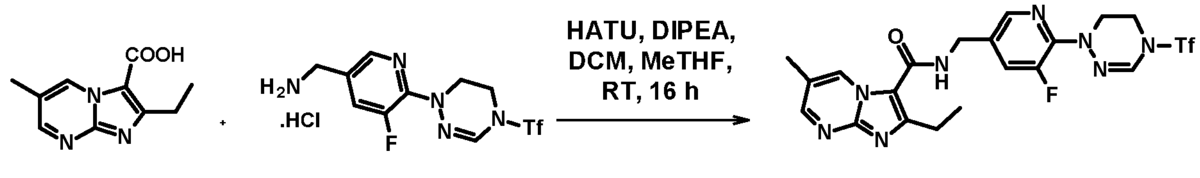


intermediate AI-3 AW- 9 compound 201

Accordingly compound 201 was prepared in the same way as compound 142 starting from intermediate AI-3 (0.64 mmol) and intermediate AW-9 (0.4 mmol) affording a white solid, 0.063 g (30%).

$^{1}$HNMR (400 MHz, DMSO) d 9.19 - 9.12 (m, 1H), 8.51 (d, J = 2.4 Hz, 1H), 8.44 (t, J = 5.8 Hz, 1H), 8.13 (s, 1H), 7.69 (dd, J = 12.7, 1.7 Hz, 1H), 7.36 (s, 1H), 4.51 (d, J = 5.8 Hz, 2H), 4.13 (t, J = 4.6 Hz, 2H), 3.96 - 3.87 (m, 2H), 3.00 (q, J = 7.5 Hz, 2H), 2.34 (s, 3H), 1.27 (t, J = 7.5 Hz, 3H).

## Synthesis of compound 213

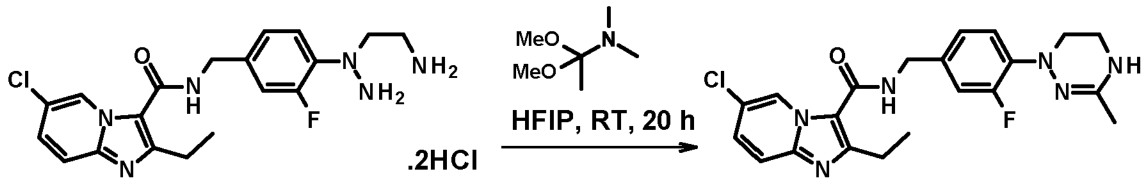


intermediate D6 intermediate AX-1

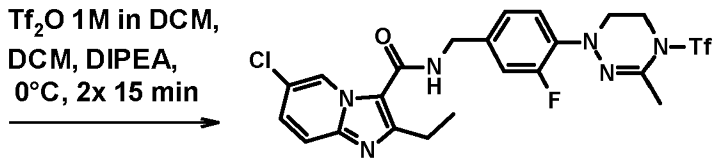


compound 213

## Preparation of intermediate AX-1

N,N Dimethylacetamide dimethyl acetal (0.2 mL; 1.26 mmol) was added to a solution of intermediate D6 (0.3 g; 0.63 mmol) in HFIP (10.8 mL) and the mixture was stirred at room temperature for 20 h. The reaction mixture was diluted with EtOAc and treated with an aqueous saturated solution of NaHCOs. The layers were separated, and the aqueous layer was extracted with EtOAc. The combined organic layers were dried over $MgSO_4$, filtered and the solvent was removed under reduced pressure to give a colorless oil. Purification was carried out by flash chromatography over silica gel (24 g, irregular SiOH 25-40μM, DCM/MeOH from 95/5 to 90/10). Pure fractions were collected and evaporated affording intermediate AX-1 as colorless oil, 0.176 g (65%).

## Preparation of compound 213

To a solution of intermediate AX-1 (0.139 g,0.32 mmol) and DIPEA (0.17 mL, 0.97 mmol) in dry DCM (2.8 mL), cooled at 5°C in an ice bath, was added dropwise $Tf_2O$ 1M in DCM (0.32 mL, 0.32 mmol). The reaction mixture was stirred at 5°C for 15 min. The reaction mixture was immediately quenched with a saturated solution of NaHCOs. The aqueous layer was extracted with DCM (twice). The combined organic layer was washed with brine (once), dried over $MgSO_4$ and filtered off to give a crude. Dry DCM (2.8 mL) was added to the crude, the solution was cooled down to 5°C then DIPEA (0.056 mL, 0.32 mmol) was added, followed by $Tf_2O$ 1M in DCM (0.13 mL, 0.13 mmol). The reaction mixture was stirred at 5°C for 15 min. The reaction mixture was immediately quenched with a saturated solution of NaHCOs. The aqueous layer was extracted with DCM (twice). The combined organic layer were washed with brine (once), dried

over $MgSO_4$ and filtered off to give 0.217 g as an oil. Purification was carried out by flash chromatography over silica gel (12 g, irregular SiOH 25-40μM, DCM/MeOH from 100/0 to 97/3). Pure fractions were collected and evaporated affording compound 213, as beige powder, 0.093 g (51%). Purification was carried out by flash chromatography over silica gel (12 g, irregular SiOH 25-40μM, DCM/MeOH from 100/0 to 97/3). Pure fractions were collected and evaporated affording compound 213, as beige powder, 0.075 g (41%). This one was crystallized from DIPE/Heptane, triturated, filtered off and dried under vacuum at 60°C affording compound 213 as white powder, 0.063 g (35%).

$^1H$ NMR (500 MHz, DMSO-$d_6$) δ ppm 9.04 - 9.11 (m, 1 H), 8.47 (t, *J*= 5.9 Hz, 1 H), 7.64 - 7.72 (m, 1 H), 7.46 (dd, *J*=9.5, 2.1 Hz, 1 H), 7.29 - 7.38 (m, 1 H), 7.13 - 7.27 (m, 2 H), 5.12 - 5.18 (m, 1 H), 4.49 (d, *J*=6.0 Hz, 2 H), 3.95 - 4.06 (m, 2 H), 3.67 - 3.77 (m, 2 H), 3.01 (q, *J*=7.5 Hz, 2 H), 2.25 (s, 3 H), 1.22 - 1.31 (t, *J*=7.5 Hz, 3 H).

## Synthesis of intermediate AY-3

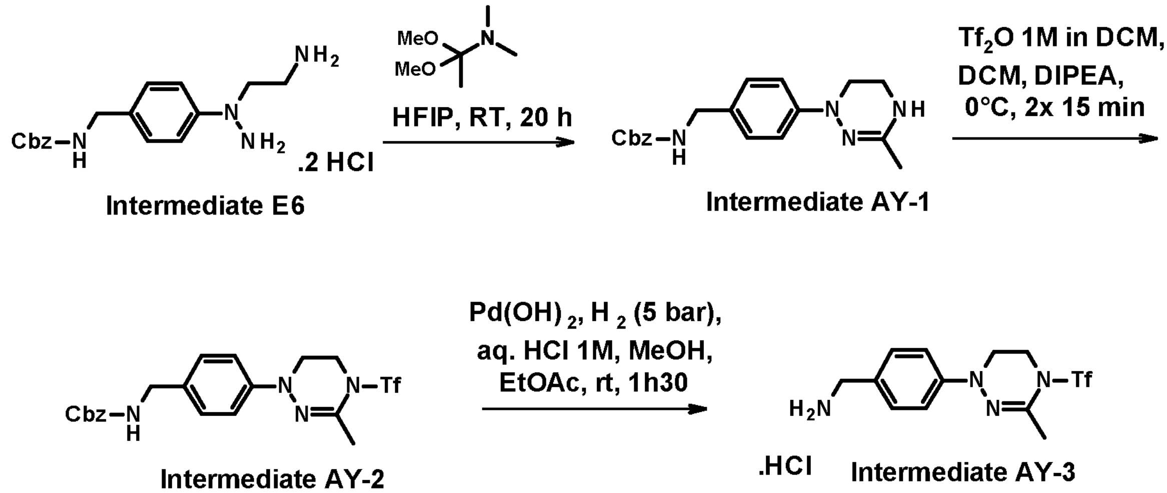


.2 HCl

Intermediate E6

Intermediate AY-1

Intermediate AY-2

.HCl Intermediate AY-3

## Preparation of intermediate AY-1

N,N Dimethylacetamide dimethyl acetal (1.68 mL; 10.33 mmol) was added to a solution of intermediate E6 (2 g; 5.16 mmol) in HFIP (88 mL) and the mixture was stirred at room temperature for 20 h. The reaction mixture was diluted with EtOAc and treated with an aqueous saturated solution of NaHCOs. The layers were separated, and the aqueous layer was extracted with EtOAc. The combined organic layers were dried over $MgSO_4$, filtered and the solvent was removed under reduced pressure. The residue was purified by preparative LC (irregular SiOH 40 μm, 40 g, from DCM/MeOH 95/5 to 90/10) to give 442 mg of intermediate AY-1 as a colorless residue which crystallized on standing (25%).

## Preparation of intermediate AY-2

Accordingly, intermediate AY-2 was prepared in the same way as compound 213 starting from AY-1 (1.31 mmol), yielding a beige powder, 0.388 g (63%).

## Preparation of intermediate AY-3

In a steal bomb, a mixture of AY-2 (0.39 g, 0.82 mmol), palladium hydroxide 20% on carbon nominally 50% water (0.12 g, 0.082 mmol) and aqueous HCl 1M (0.82 mL, 0.82 mmol) in MeOH (5.8 mL) and EtOAc (5.8 mL) was hydrogenated under 5 bar of Hz at room temperature for 1.5 hour. The mixture was filtered on a pad of celite and washed with MeOH. The filtrate was evaporated to give intermediate AY-3, 0.32 g (96%, purity 92%), used as such for next step.

## Preparation of compound 214

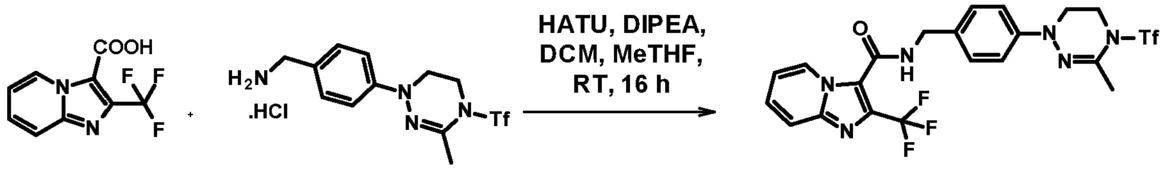


CAS [73221-19-9] AY-3 compound 214

Accordingly, compound 214 was prepared in the same way as compound 181 starting from 2-(Trifluoromethyl)-imidazo[1,2-A]pyridine-3-carboxylic acid (CAS [73221-19-9], 0.34 mmol) and intermediate AY-3 (0.39 mmol) yielding a white powder, 0.098 g (52%).
$^1$H NMR (500 MHz, DMSO-$d_6$) δ ppm 9.17 - 9.29 (m, 1 H), 8.48 - 8.58 (m, 1 H), 7.73 - 7.83 (m, 1 H), 7.49 - 7.60 (m, 1 H), 7.30 (br d, J=8.2 Hz, 2 H), 7.13 - 7.24 (m, 3 H), 4.42 - 4.52 (m, 2 H), 4.01 (br s, 2 H), 3.84 (br d, *J*=4.3 Hz, 2 H), 2.27 (s, 3 H)

## Preparation of compound 215

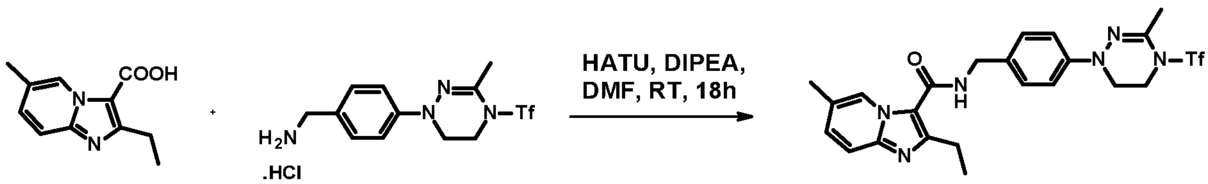


CAS [1216036-36-0] Intermediate AY-3 compound 215

Accordingly, compound 215 was prepared in the same way as compound 181 starting from 2-ethyl-6-methyl-imidazo[1,2-a]pyridine-3-carboxylic acid (CAS [1216036-36-0], 0.34 mmol) and intermediate AY-3 (0.39 mmol) affording a white powder, 0.129 g (72%).
$^1$H NMR (500 MHz, DMSO-d6) δ ppm 8.77 (s, 1 H), 8.29 - 8.36 (m, 1 H), 7.47 - 7.54 (m, 1 H), 7.27 - 7.33 (m, 2 H), 7.21 - 7.25 (m, 1 H), 7.14 - 7.19 (m, 2 H), 4.41 - 4.49 (m, 2 H), 4.06 - 4.09 (m, 1 H), 3.96 - 4.05 (m, 2 H), 3.79 - 3.84 (m, 2 H), 2.90 - 3.02 (m, 2 H), 2.31 (s, 3H) 2.26 (s, 3H), 1.20 - 1.30 (m, 3 H)

## Preparation of compound 217

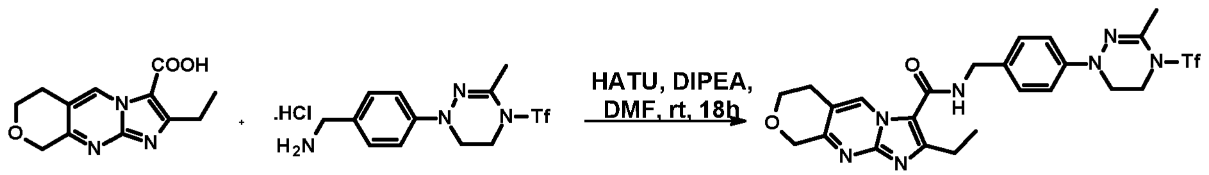


AU-2 intermediate AY-3 compound 217

Accordingly, compound 217 was prepared in the same way as compound 181 starting from intermediate AU-2 (0.31 mmol) and intermediate AY-3 yielding a white foam, 0.018 g (10%).
$^1$H NMR (500 MHz, DMSO-$d_6$) δ ppm 9.17 (s, 1 H), 8.40 (t, J=6.0 Hz, 1 H), 7.27 - 7.35 (m, 2 H), 7.12 - 7.21 (m, 2 H), 4.69 - 4.77 (m, 2 H), 4.41 - 4.49 (m, 2 H), 3.98 - 4.04 (m, 2 H), 3.91 - 3.97 (m, 2 H), 3.79 - 3.84 (m, 2 H), 2.95 - 3.01 (m, 2 H), 2.89 - 2.94 (m, 2 H), 2.25 (s, 3 H), 1.22 - 1.29 (m, 4 H)

## Preparation of compound 218

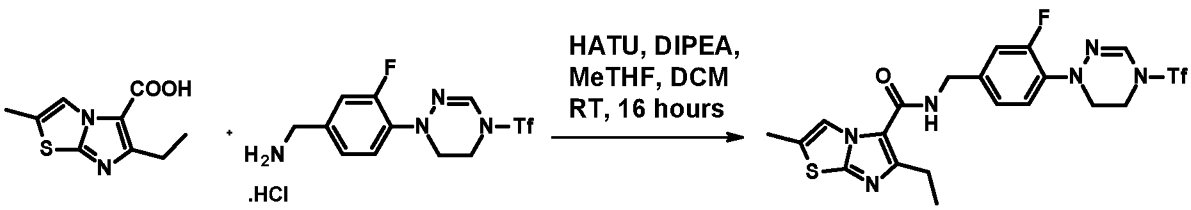


CAS [1131613-58-5] Intermediate AA-3 compound 163

Accordingly compound 218 was prepared in the same way as compound 181 starting from 6-ethyl-2-methyl-imidazo[2,1-b][1,3]thiazole-5-carboxylic acid (CAS [1131613-58-5], 0.29 mmol) and intermediate AY-3 yielding a white foam, 0.059 g (38%).

$^1$H NMR (500 MHz, DMSO-$d_6$) δ ppm 8.09 (t, *J*=6.0 Hz, 1 H), 7.80 - 7.91 (m, 1 H), 7.21 - 7.32 (m, 2 H), 7.08 - 7.19 (m, 2 H), 4.40 (d, *J*=6.0 Hz, 2 H), 4.00 (t, *J*=4.9 Hz, 2 H), 3.81 (t, *J*=4.9 Hz, 2 H), 2.85 (q, *J*=7.5 Hz, 2 H), 2.40 - 2.46 (m, 3 H), 2.22 - 2.28 (m, 3 H), 1.20 (t, *J*=7.5 Hz, 3H)

## Synthesis of compound 216

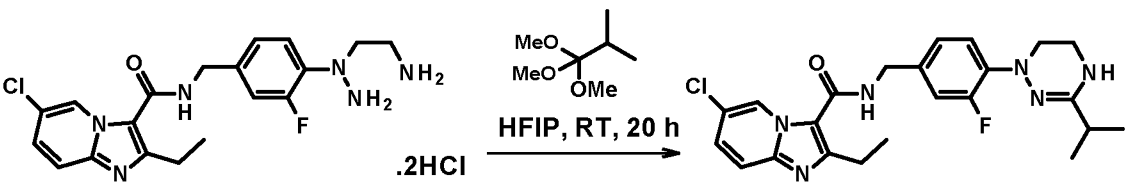


intermediate D6 intermediate AZ-1

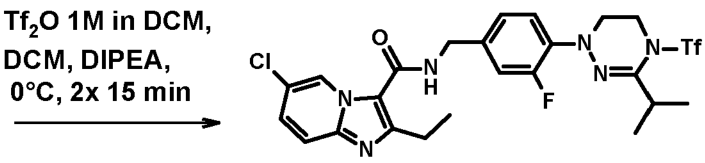


compound 216

## Preparation of intermediate AZ-1

Trimethyl Orthoisobutyrate (0.2 mL; 1.26 mmol) was added to a solution of intermediate D (0.3 g; 0.63 mmol) in HFIP (10.8 mL) and the mixture was stirred at room temperature for 20 h. The reaction mixture was diluted with EtOAc and treated with an aqueous saturated solution of $NaHCO_3$. The layers were separated, and the aqueous layer was extracted with EtOAc. The combined organic layers were dried over $MgSO_4$, filtered and the solvent was removed under reduced pressure to give an oil. Purification was carried out by flash chromatography over silica gel (4 g, irregular SiOH, DCM/MeOH from 95/5 to 85/15). Pure fractions were collected and evaporated affording intermediate AZ-1 as colourless oil, 0.105 g (37%).

## Preparation of compound 216

To a solution of AZ-1 (0.11 g, 0.23 mmol) and DIPEA (0.12 mL, 0.69 mmol) in dry DCM (2 mL), cooled at 5°C in a ice bath, was added dropwise $Tf_2O$ 1M in DCM (0.23 mL, 0.23 mmol). The reaction mixture was stirred at 5°C for 15 min. The reaction mixture was immediately quenched with a saturated solution of NaHCOs. The aqueous layer was extracted with DCM (twice). The combined organic layer were washed with brine (once), dried over MgSO4 and filtered off and evaporated. DCM (2 mL) was added to the residue, the solution was cooled down to 5°C then DIPEA (0.04 mL,

0.23 mmol) was added, followed by $Tf_2O$ 1M in DCM (0.092 mL, 0.092 mmol). The reaction mixture was stirred at 5°C for 15 min. The reaction mixture was immediately quenched with a saturated solution of NaHCOs. The aqueous layer was extracted with DCM (twice). The combined organic layer were washed with brine (once), dried over MgSO4 and filtered off to give 0.725 g. A purification was carried out by flash chromatography over silica gel (4 g, iregular SiOH 25-40μM, Heptane/EtOAc from 90/10 to 70/30). Pure fractions were collected and evaporated affording a beige powder, 0.06 g. This one was triturated with DIPE and a few Heptane, the precipitate was filtered off and dried under vacuum at 60°C affording compound 216 as white powder, 0.040 g.

$^1H$ NMR (500 MHz, DMSO-$d_6$) δ ppm 9.03 - 9.18 (m, 1 H), 8.47 (br t, *J*=5.5 Hz, 1 H), 7.63 - 7.73 (m, 1 H), 7.43 - 7.50 (m, 1 H), 7.30 - 7.38 (m, 1 H), 7.16 - 7.27 (m, 2 H), 4.50 (br d, J=5.6 Hz, 2 H), 3.87 - 3.94 (m, 2 H), 3.80 (br s, 2 H), 2.93 - 3.05 (m, 3 H), 1.24 - 1.32 (m, 3 H), 1.14 - 1.21 (m, 6 H)

**Synthesis of intermediate BA-3**

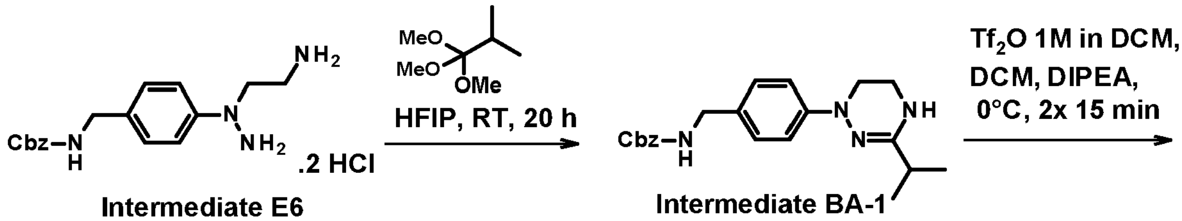


Intermediate E6

Intermediate BA-1

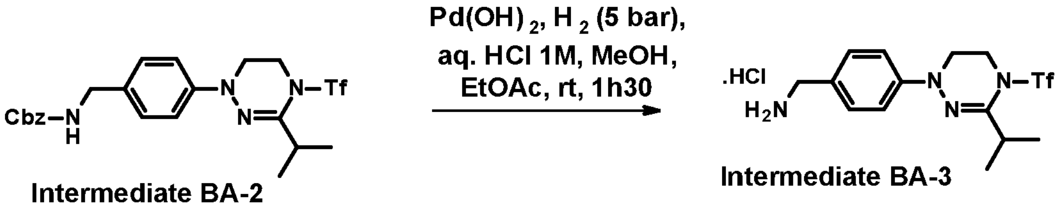


Intermediate BA-2

Intermediate BA-3

**Preparation of intermediate BA-1**

According, intermediate BA-1 was prepared in the same way as AZ-1 starting from intermediate E6 (6.45 mol) yielding a colorless oil, 1.82 g (77%).

**Preparation of intermediate BA-2**

Accordingly, intermediate BA-2 was prepared in the same way as compound 216 starting from BA-1 (4.97 mmol), yielding a beige powder, 1.58 g (58%).

**Preparation of intermediate BA-3**

According, intermediate BA-3 was prepared in the same way as AY-3 starting from intermediate BA-2 (3.17 mol) yielding a beige solid, 1.39 g (91%, purity around 90%, used as such for next step).

The following compounds are/were also prepared in accordance with the methods described herein:

Compound 191

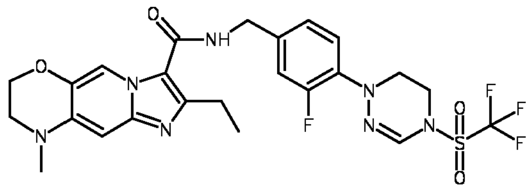

Compound 195

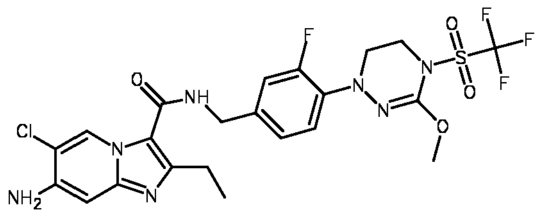

Compound 205

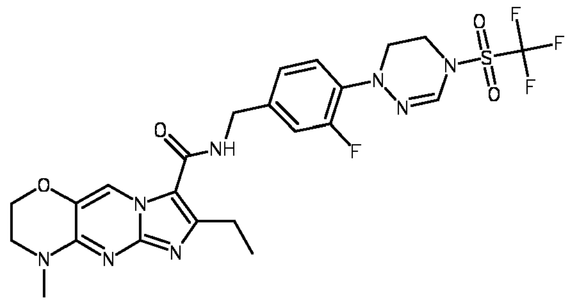

Compound 208

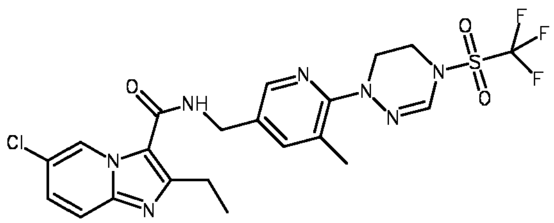

Compound 211

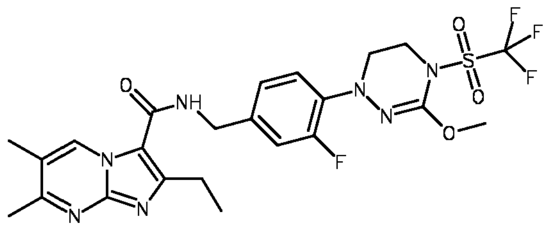

Compound 212

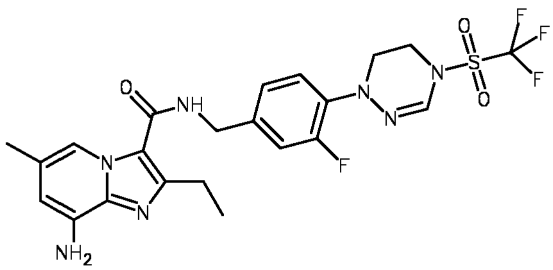

Compound 219

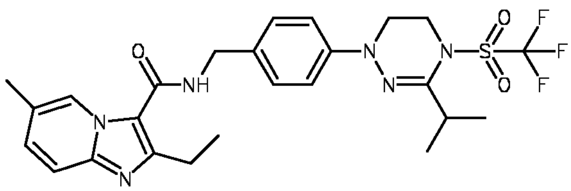

Compound 107

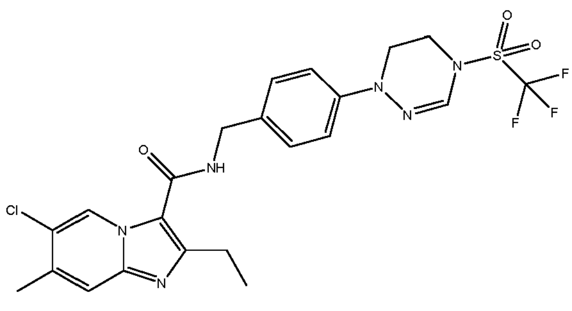

Compound 93

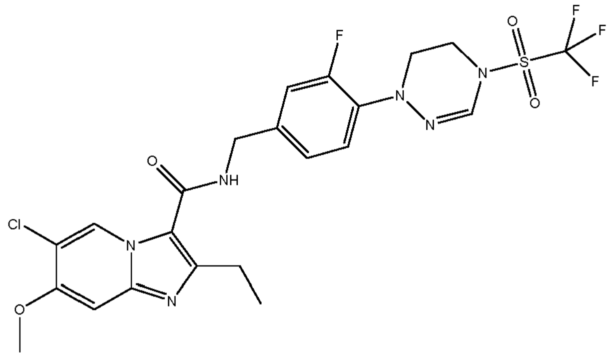

Compound 116

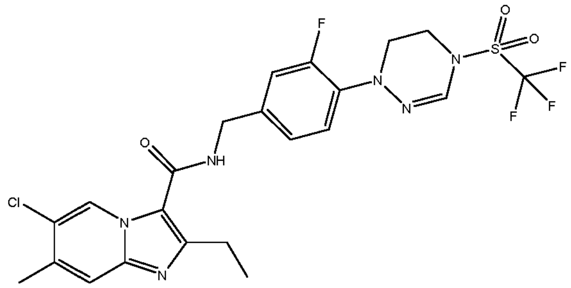

Compound 108

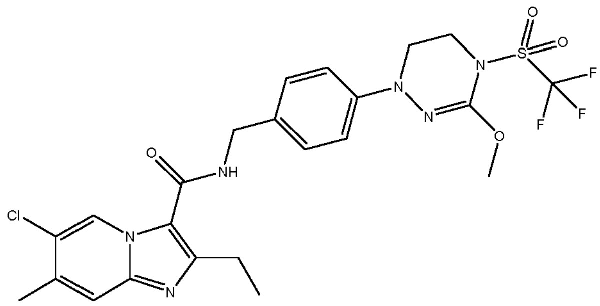

Compound 120

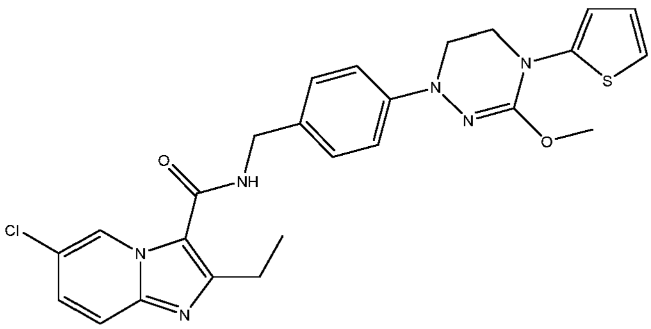

Compound 92

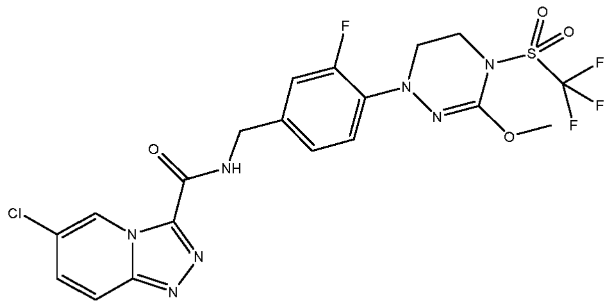

Compound 94

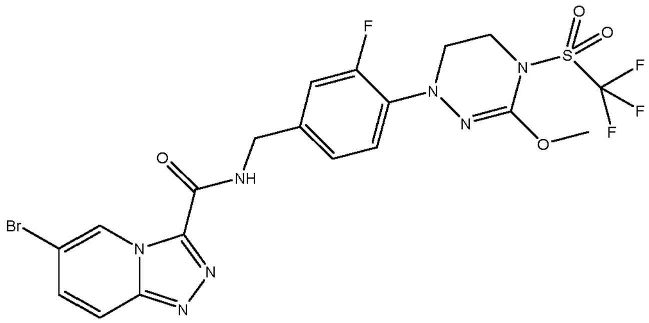

Compound 110

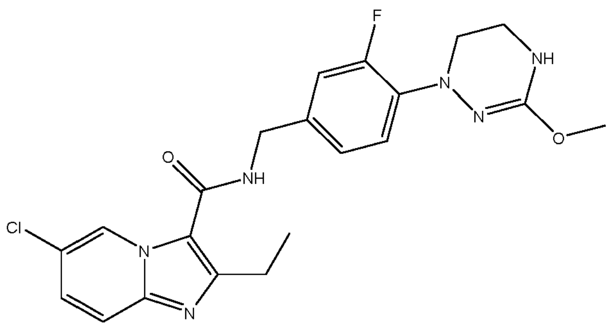

Compound 96

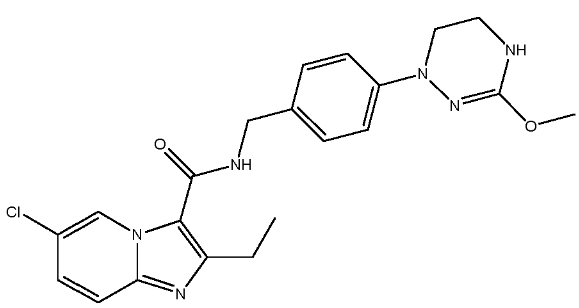

Compound 91

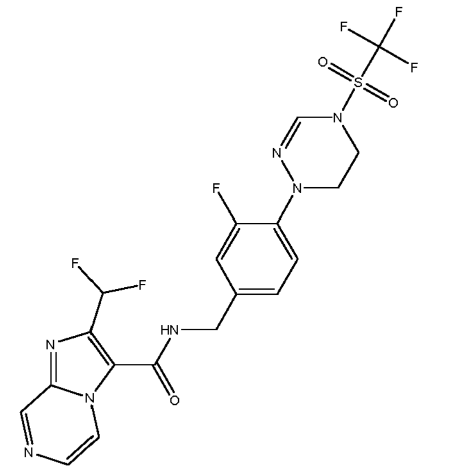

Compound 99

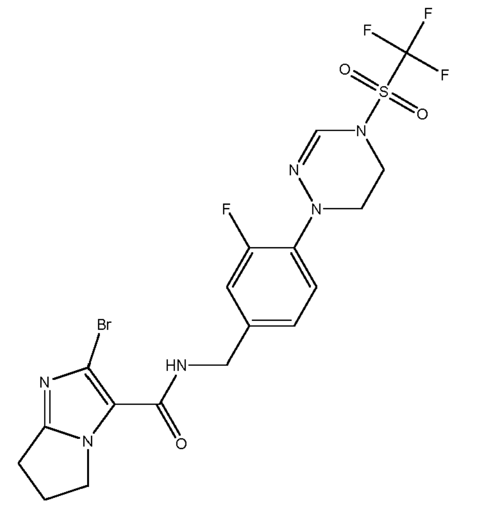

Compound 123

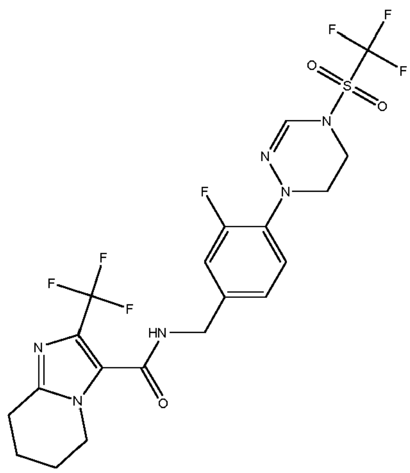

Compound 122

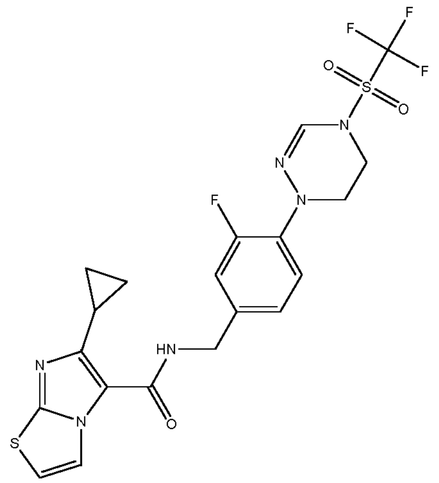

Compound 103

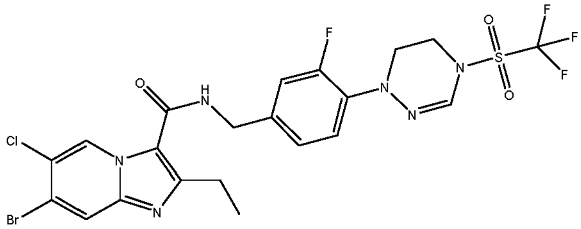

Compound 118

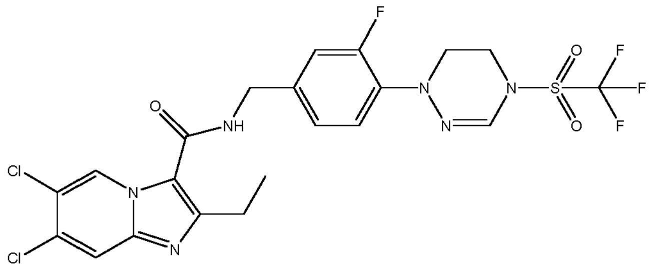

Compound 119

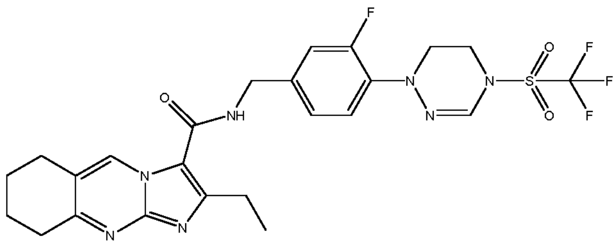

Compound 86

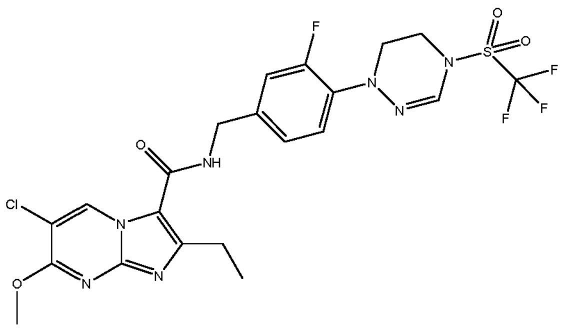

Compound 115

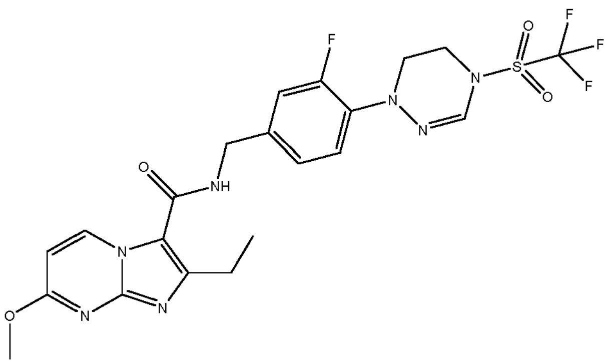

Compound 111

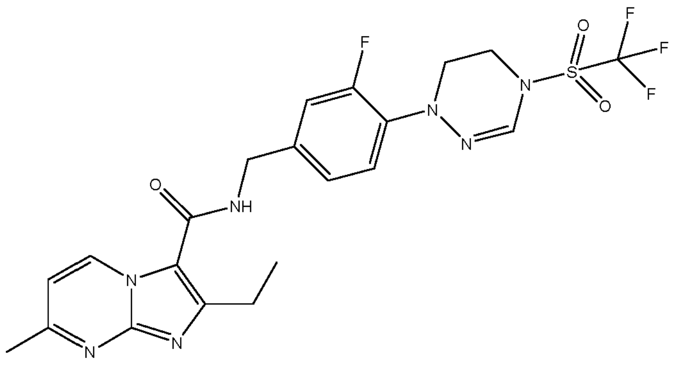

Compound 98

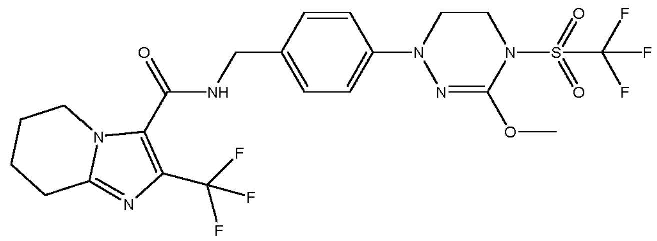

Compound 109

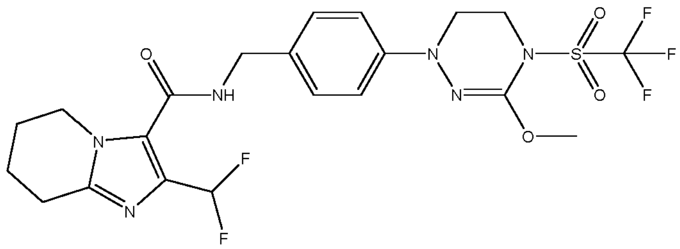

Compound 149

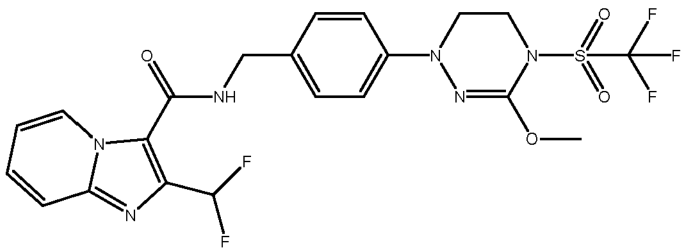

Compound 101

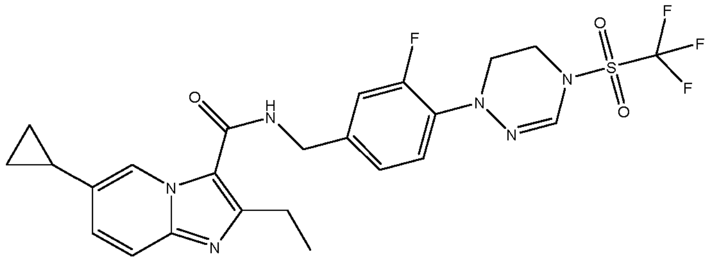

Compound 104

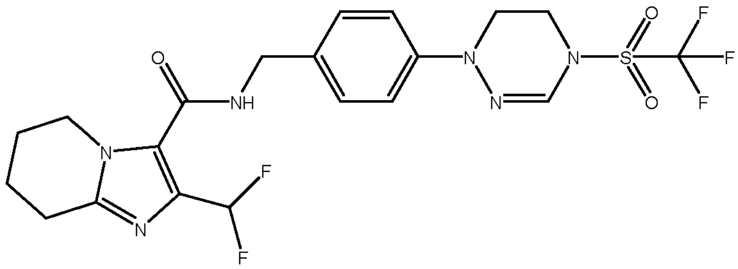

Compound 87

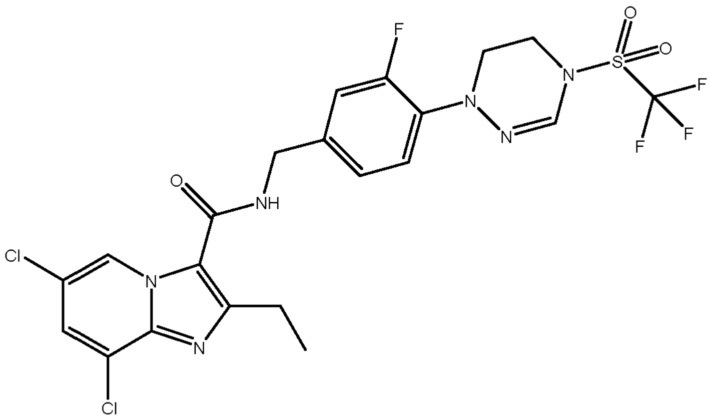

Compound 112

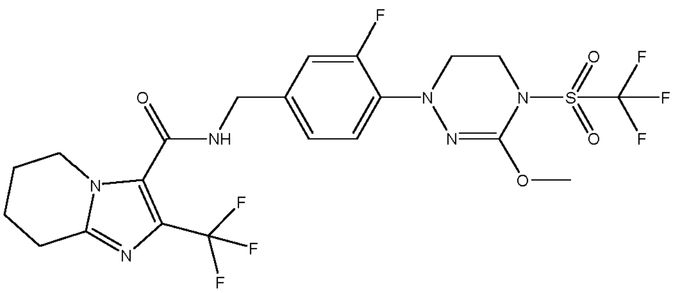

Compound 160

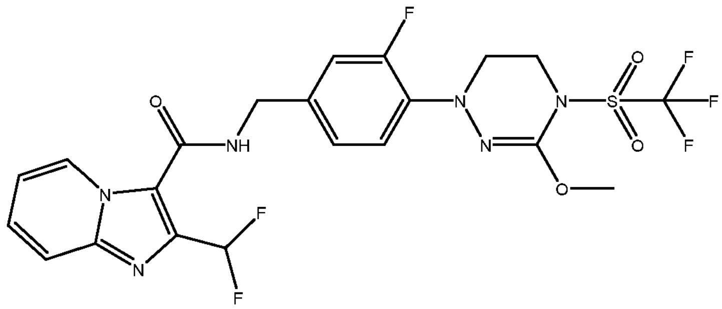

Compound 113

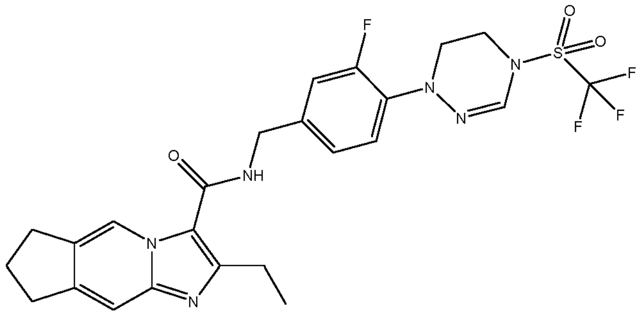

Compound 85

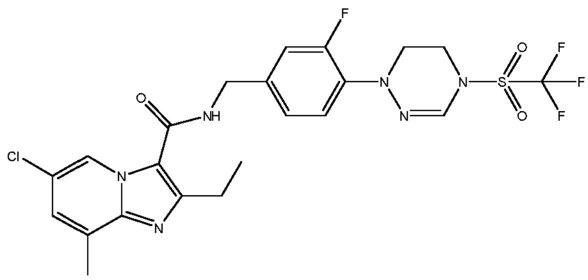

Compound 95

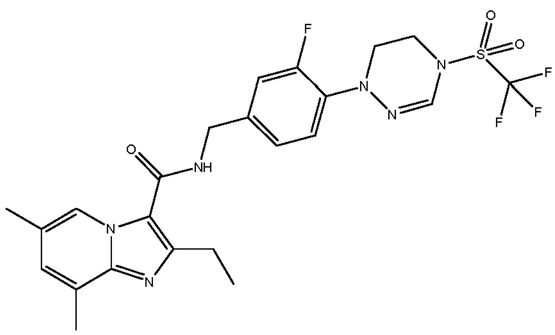

Compound 114

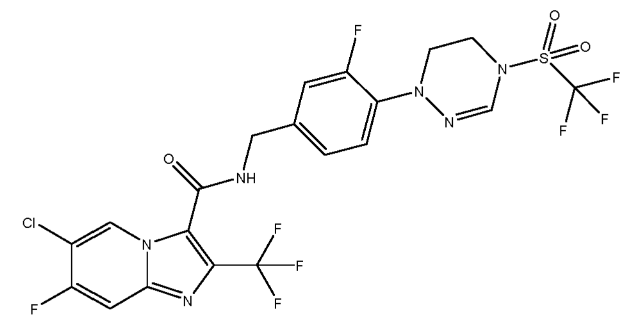

Compound 117

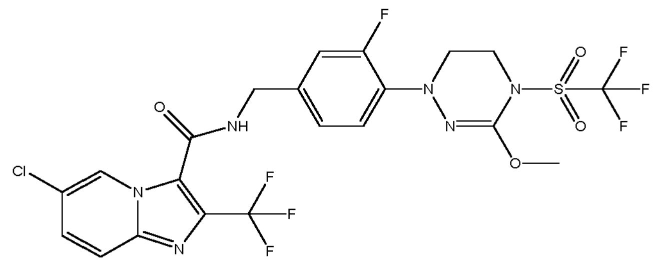

Compound 102

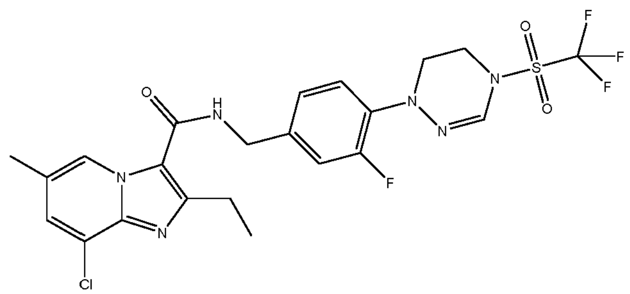

Compound 89

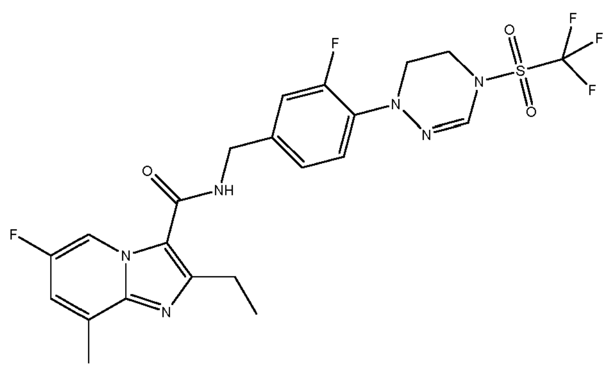

Compound 105

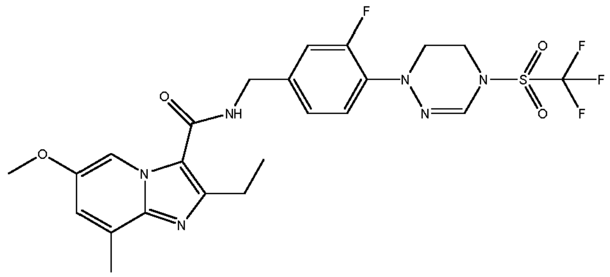

Compound 106

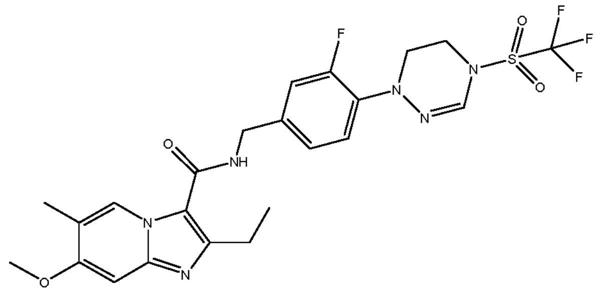

Compound 100

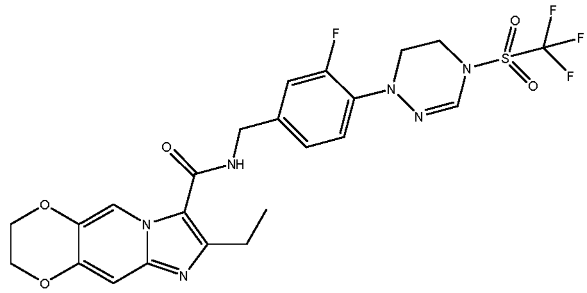

Compound 90

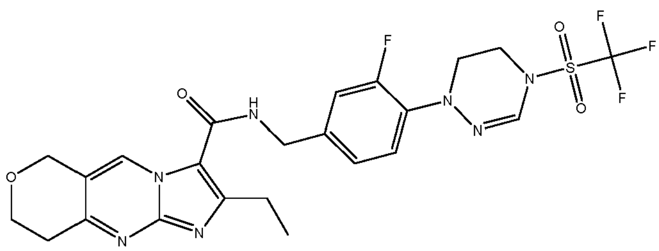

Compound 97

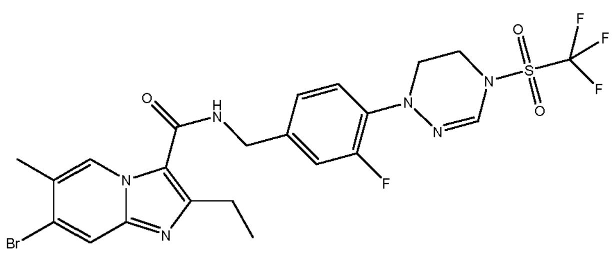

Compound 83

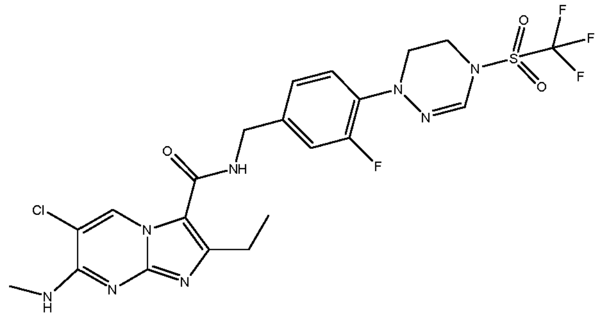

Compound 121

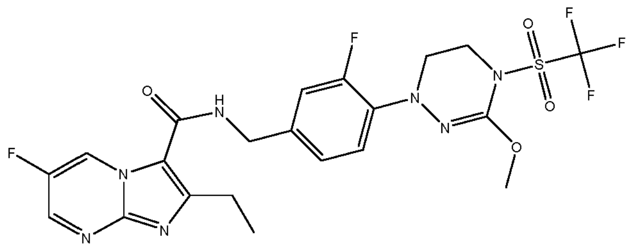

Compound 80

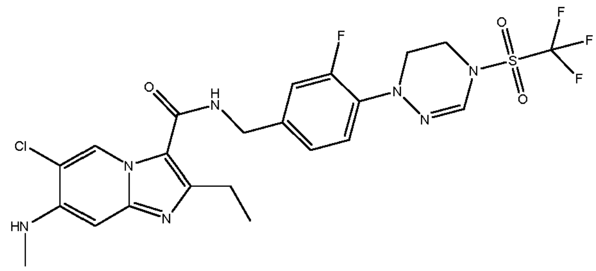

Compound 84

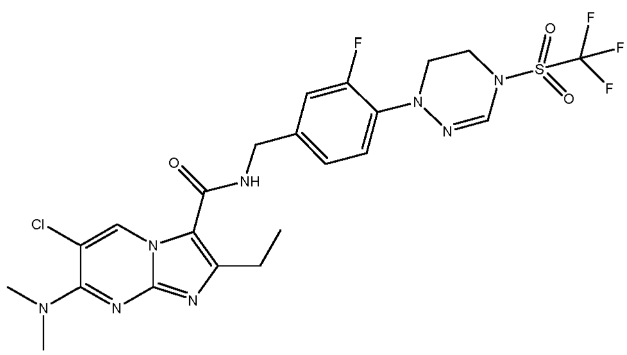

Compound 81

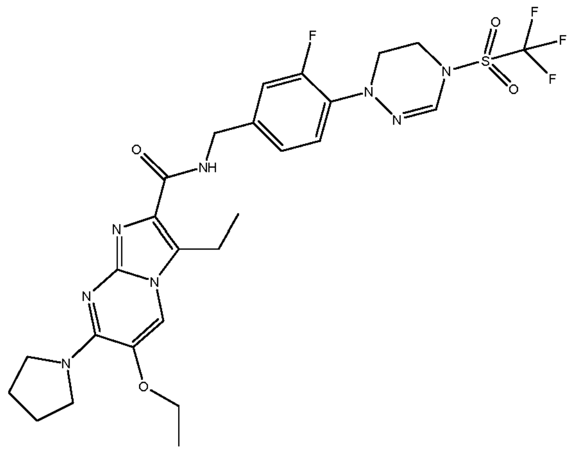

Compound 82

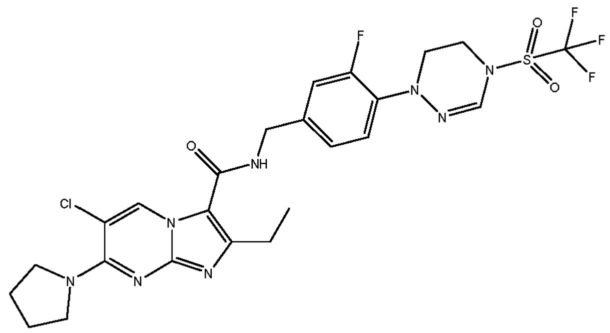

Compound 165

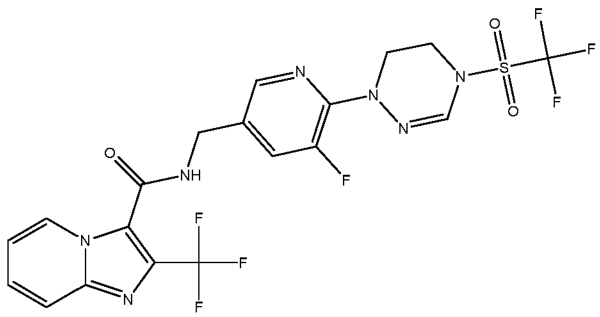

Compound 166

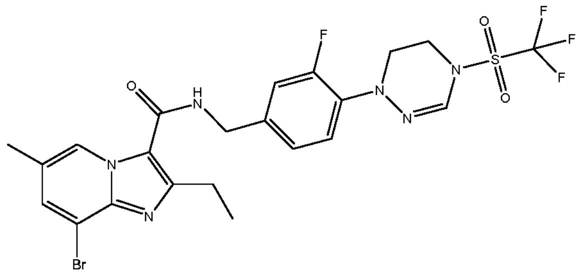

Compound 167

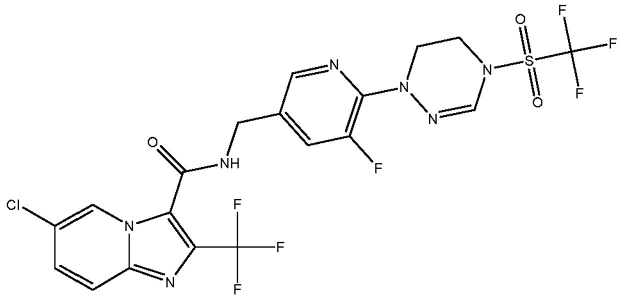

Compound 168

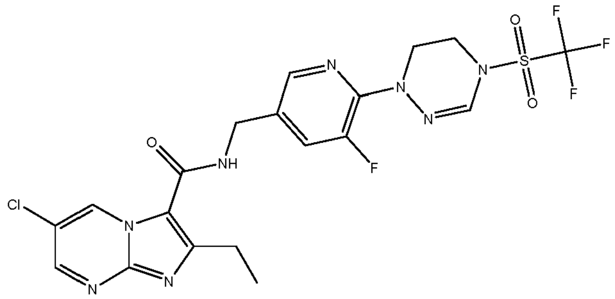

Compound 170

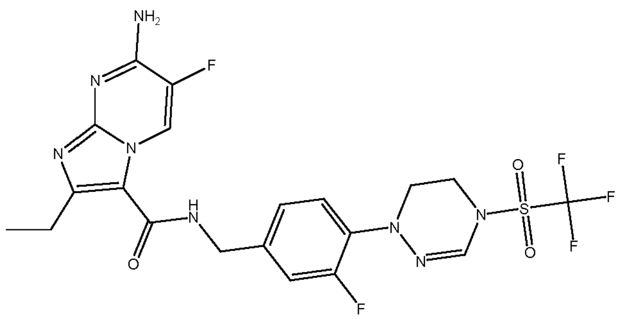

Compound 171

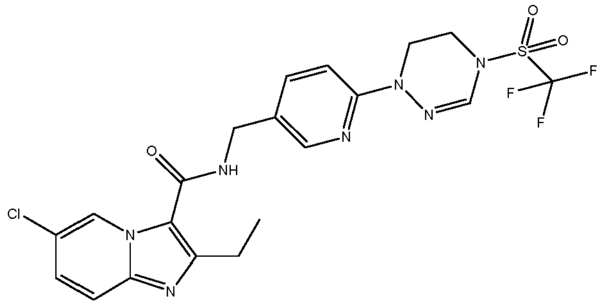

Compound 173

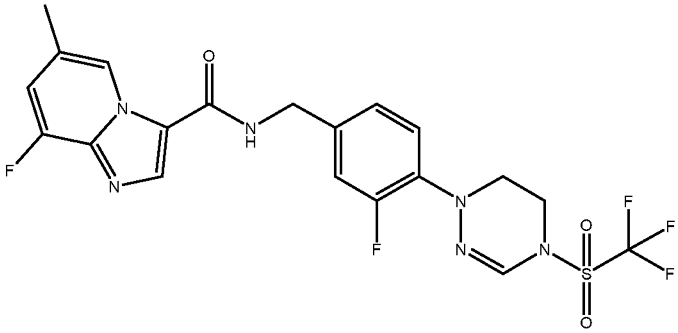

Compound 174

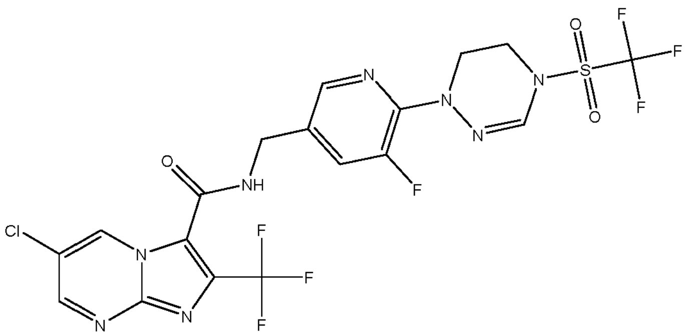

Compound 176

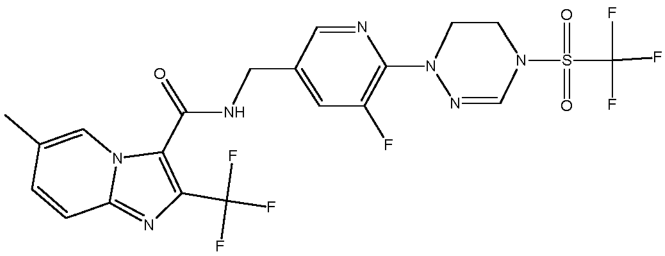

Compound 178

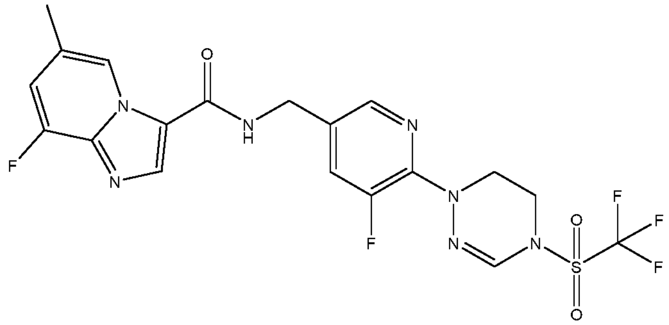

Compound 179

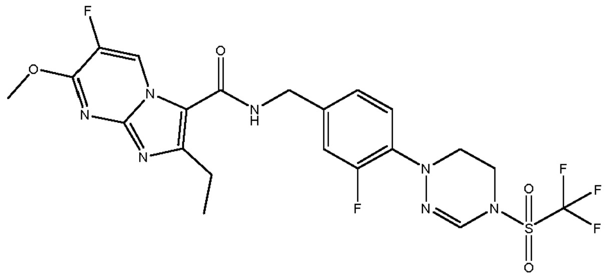

Compound 182

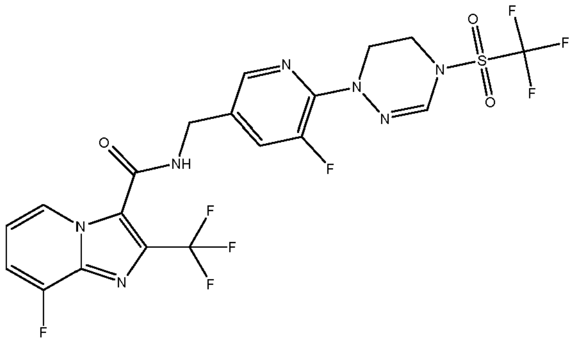

Compound 183

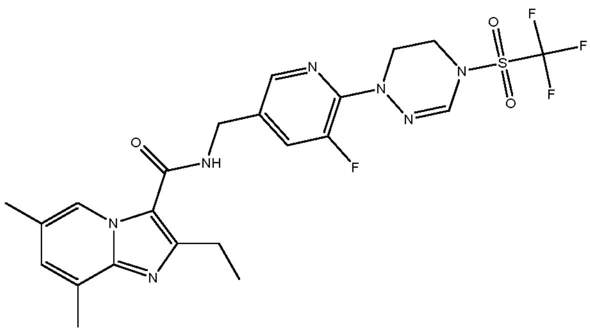

Compound 184

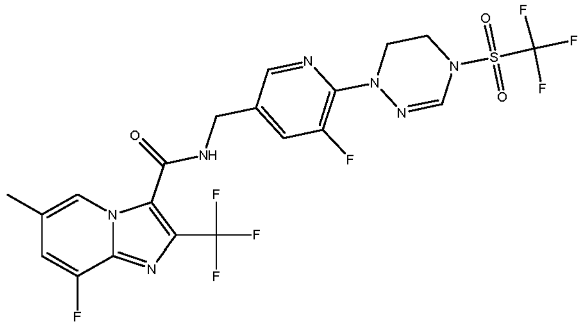

Compound 185

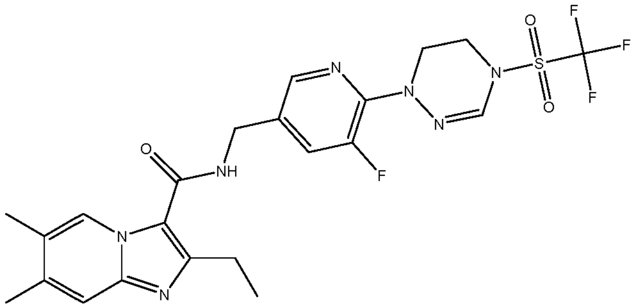

Compound 186

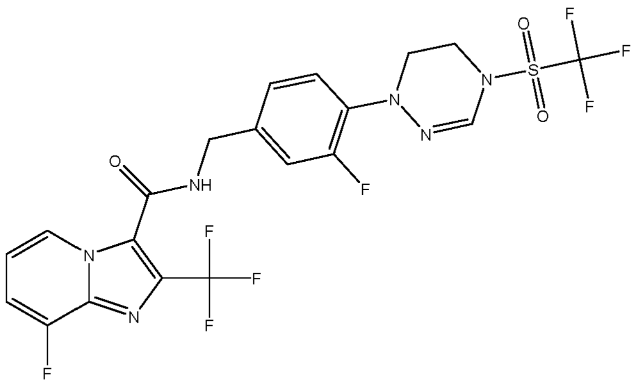

Compound 187

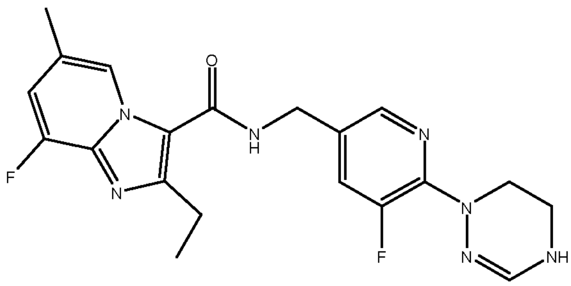

Compound 188 (depicted as a tautomer)

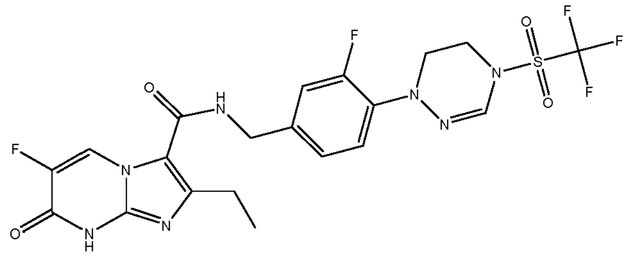

Compound 189

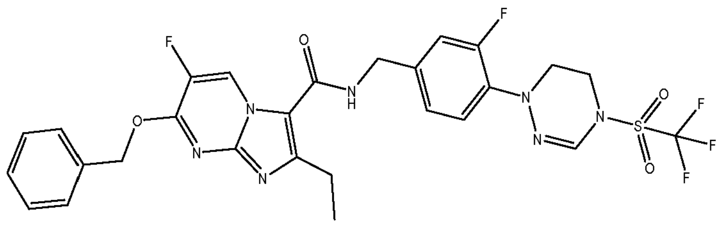

Compound 190

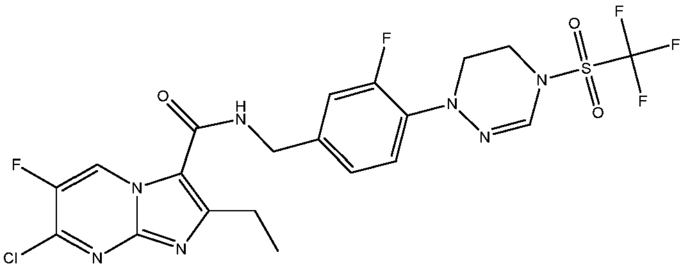

Compound 192

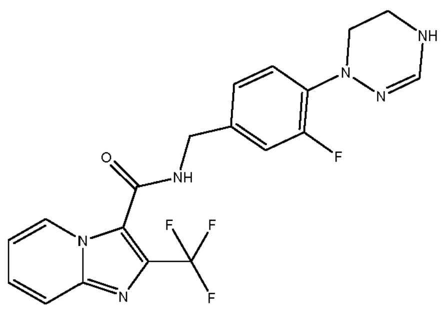

Compound 196

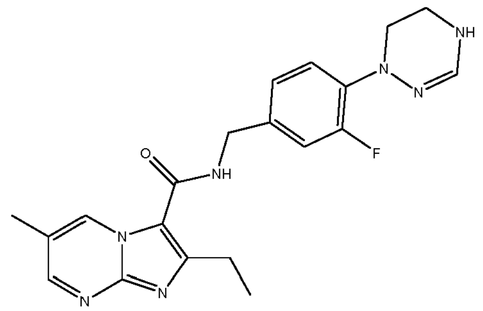

Compound 198

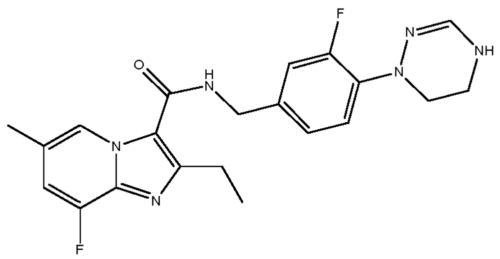

Compound 199

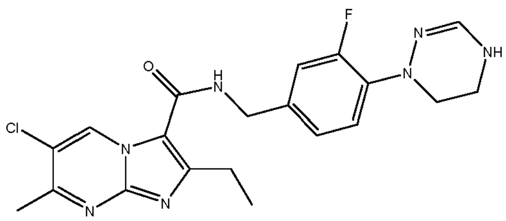

Compound 202

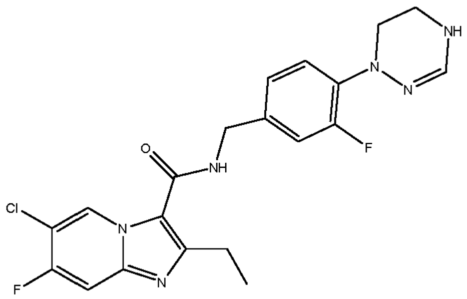

Compound 203

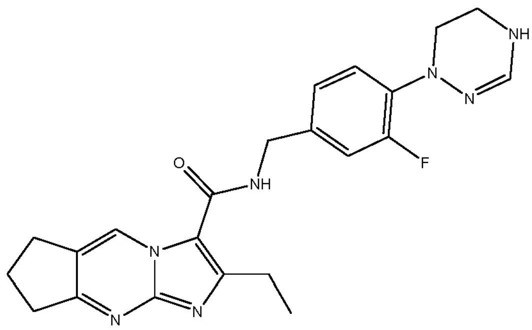

Compound 207

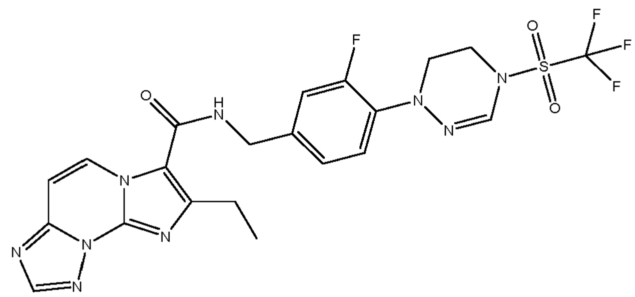

Compound 220

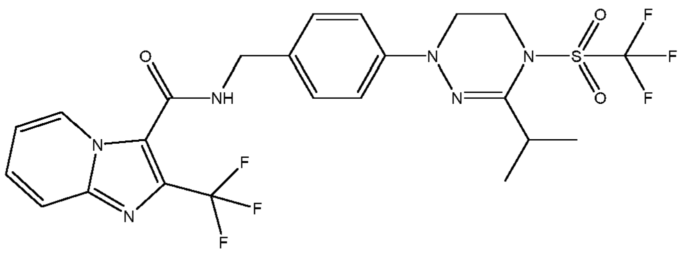

Compound 221

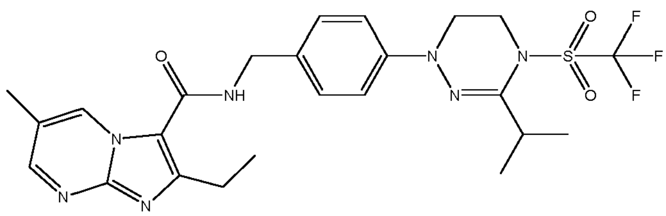

Compound 222

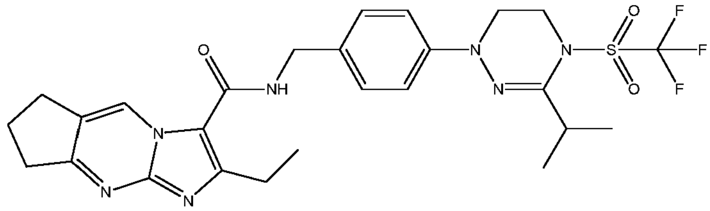

Compound 223

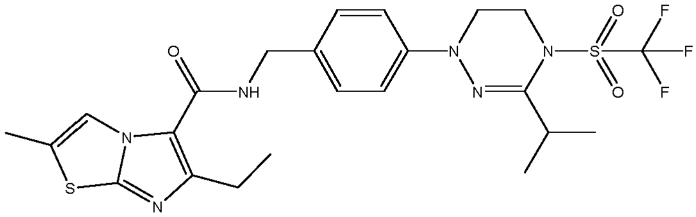

Compound 224

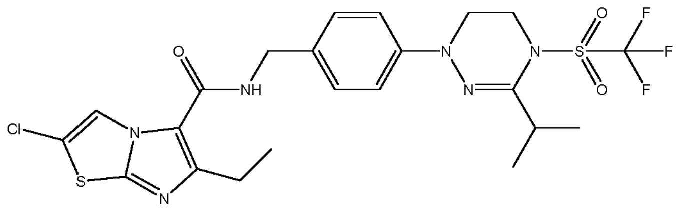

Compound 225

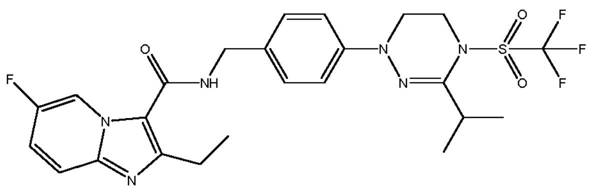

Compound 226

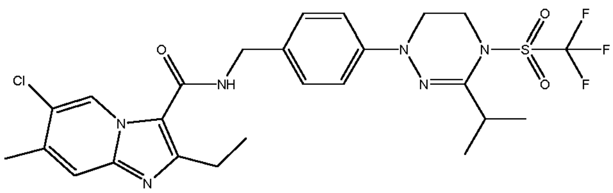

4. Characterizing data table

| Compound number | Melting point (°C) | LCMS | | | | | |
|---|---|---|---|---|---|---|---|
| | | Retention time | UV (%) | M exact | $[M+H]^+$ | $[M-H]^-$ | Method |
| 1 | 183.46 | 3.2 | 98.9 | 528.1 | 529.1 | 527.2 | A |
| 31 | | 3.26 | 97.1 | 528.1 | 529.3 | 527.4 | A |
| 32 | | 3.25 | 96.86 | 528.1 | 529.3 | 527.4 | A |
| 29 | 195.09 | 3.25 | 96.17 | 528.1 | 529.3 | 527.4 | A |
| 28 | 199.08 | 3.26 | 98.84 | 528.1 | 529.3 | 527.4 | A |
| 30 | 213.87 | 3.26 | 96.73 | 528.1 | 529.3 | 527.4 | A |
| 21 | 157.49 | 3.36 | 100 | 542.1 | 543.2 | 541.3 | A |
| 19 | 196.34 | 2.73 | 96.2 | 518.2 | 519.2 | 517.3 | A |
| 2 | 178.31 | 3.52 | 99.8 | 556.1 | 557.2 | 555.3 | A |
| 56 | 236.36 | 2.32 | 99.8 | 396.1 | 397.1 | 395.1 | A |
| 20 | 209.36 | 2.65 | 98 | 474.1 | 475.1 | 473.2 | A |
| 3 | 189.22 | 2.82 | 100 | 498.2 | 499.3 | 557.4 $[M+CH_3CO_2]^-$ | A |
| 57 | 211.47 | 1.85 | 95.7 | 366.2 | 367.2 | 425.4 $[M+CH_3CO_2]^-$ | A |
| 13 | 215.87 | 3.06 | 97.1 | 529.1 | 530.3 | 528.3 | A |
| 58 | | 2.32 | 97.7 | 488.2 | 489.4 | 547.5 $[M+CH_3CO_2]^-$ | A |
| 14 | 192.27 | 2.93 | 99.9 | 510.1 | 511.2 | 569.4 $[M+CH_3CO_2]^-$ | A |
| 59 | 202.94 | 2.52 | 99.7 | 424.2 | 425.2 | 423.3 | A |
| 15 | 206.66 | 2.53 | 99 | 410.2 | 411.2 | 409.2 | A |
| 6 | | 3.28 | 99.4 | 558.1 | 559.3 | 557.4 | A |
| 16 | 158.10 | 3.21 | 100 | 546.1 | 547.5 | 545.5 | B |
| 4 | | 2.23 | 99.1 | 410.2 | 411.6 | 409.5 | B |
| 18 | 245.36 | 2.56 | 99.7 | 439.2 | 439.3 | 437.3 | A |
| 5 | | 3.29 | 100 | 542.1 | 543.3 | 541.4 | A |
| 17 | 196.82 | 3.08 | 97.6 | 516.2 | 517.4 | 515.5 | A |
| 22 | 175.12 | 3.29 | 92.2 | 558.1 | 559.4 | 557.4 | A |
| 23 | 207.21 | 2.94 | 97.82 | 517.2 | 518.4 | 516.5 | A |
| 10 | 171.42 | 2.97 | 99.59 | 513.1 | 514.3 | 512.4 | A |
| 24 | 258.01 | 2.38 | 97.6 | 439.2 | 440.3 | 438.4 | A |
| 26 | | 2.93 | 98.42 | 525.1 | 526.4 | 524.5 | A |
| 11 | 179.76 | 3.17 | 98.6 | 514.1 | 515.3 | 513.4 | A |
| 12 | 109.51, 164.64, 179.98 | 2.81 | 99.8 | 495.1 | 496.3 | 494.4 | A |
| 9 | 153.26, 177.06 | 3.3 | 99.6 | 546.1 | 547.3 | 545.4 | A |
| 8 | 167.12 | 3.15 | 98.8 | 544.1 | 545.5 | 543.5 | B |

(continued)

| Compound number | Melting point (°C) | LCMS | | | | | |
|---|---|---|---|---|---|---|---|
| | | Retention time | UV (%) | M exact | $[M+H]^+$ | $[M-H]^-$ | Method |
| 25 | 125.56 | 3.24 | 97.97 | 558.1 | 559.3 | 557.5 | A |
| 7 | 208.41 | 3.19 | 98.34 | 541.1 | 542.3 | 540.5 | A |
| 27 | 174.88 | 3.16 | 100 | 559.1 | 560.3 | 558.4 | A |
| 60 | | 2.78 | 98.9 | 566.2 | 567.3 | 625.5 $[M+AcO]^-$ | A |
| 61 | 193.44 | 2.74 | 97.6 | 552.2 | 553.3 | 611.4 $[M+AcO]^-$ | A |
| 62 | | 2.94 | 97.2 | 537.1 | 538.2 | 596.5 $[M+CH_3CO_2]^-$ | A |
| 33 | 225.92 | 2.85 | 98.52 | 539.2 | 540.3 | 538.3 | A |
| 38 | 255.93 | 2.52 | 98.91 | 424.1 | 425.1 | 423.2 | A |
| 39 | 211.19 | 3.08 | 98.89 | 558.1 | 559.2 | 557.3 | A |
| 40 | | 2.98 | 100 | 572.1 | 573.3 | 571.4 | A |
| 63 | 159.95 | 3.12 | 100 | 566.2 | 567.4 | 565.4 | A |
| 41 | | 2.82 | 98.72 | 573.1 | 574.3 | 572.7 | A |
| 34 | 86.24, 147.08 | 3.04 | 100 | 540.1 | 541.2 | 599.4 $[M+AcO]^-$ | A |
| 35 | 152.93 | 3.25 | 99.56 | 564.1 | 565.3 | 563.4 | A |
| 64 | 193.79 | 3.26 | 98.82 | 558.1 | 559.3 | 557.3 | A |
| 65 | 228.15 | 3.3 | 98.84 | 563.2 | 564.4 | 562.5 | A |
| 42 | 147.57 | 3.06 | 98.5 | 524.1 | 525.3 | 523.4 | A |
| 43 | 169.05 | 3.02 | 100 | 538.2 | 539.3 | 537.4 | A |
| 66 | 211.30 | 3.08 | 98.23 | 492.1 | 493.2 | 491.2 | A |
| 67 | 206.99 | 3.22 | 99.6 | 558.1 | 559.3 | 557.3 | A |
| 36 | 142.03 | 3.11 | 98.33 | 554.2 | 555.3 | 553.3 | A |
| 37 | 193.36 | 3.37 | 99.74 | 576.1 | 577.2 | 575.3 | A |
| 44 | 173.29 | 3.1 | 99.83 | 558.1 | 559.3 | 617.5 $[M+AcO]^-$ | A |
| 45 | 155.29 | 3.29 | 99.85 | 588.1 | 589.3 | 587.3 | A |
| 68 | | 3.29 | 99.62 | 588.1 | 589.3 | 587.5 | A |
| 46 | 176.82 | 3.34 | 100 | 606.1 | 607.3 | 605.4 | A |
| 47 | 149.44 | 3.09 | 100 | 565.1 | 566.3 | 564.4 | A |
| 69 | 163.46 | 2.99 | 98.43 | 561.1 | 562.3 | 560.2 | A |
| 70 | 138.71 | 3.01 | 99.79 | 558.1 | 559.3 | 617.6 $[M+CH3COO]^-$ | A |
| 48 | 74.60 | 3.19 | 99.7 | 572.1 | 573.3 | 571.4 | A |
| 49 | | 3.37 | 99.7 | 606.1 | 607.3 | 605.3 | A |
| 50 | 101.66/ 150.99 | 3.06 | 100 | 573.1 | 574.3 | 572.3 | A |
| 51 | 185.08 | 3.04 | 97.89 | 524.1 | 525.3 | 523.3 | A |
| 52 | 164.28 | 3.11 | 99.62 | 554.2 | 555.5 | 553.3 | A |

(continued)

| Compound number | Melting point (°C) | LCMS | | | | | |
|---|---|---|---|---|---|---|---|
| | | Retention time | UV (%) | M exact | $[M+H]^+$ | $[M-H]^-$ | Method |
| 71 | | 3.14 | 98.26 | 549.1 | 550.3 | 548.4 | A |
| 72 | 216.25 | 3.07 | 98.33 | 519.1 | 520.2 | 518.2 | A |
| 53 | | 3.11 | 100 | 602.2 | 603.4 | 601.4 | A |
| 54 | 233.23 | 3.25 | 99.45 | 509.1 | 510.3 | 508.5 | A |
| 55 | 193.51 | 3.38 | 99.24 | 586.1 | 587.4 | 585.4 | A |
| 73 | 212.08 | 3.18 | 100 | 567.1 | 568.3 | 566.5 | A |
| 74 | | 3.42 | 99.2 | 585.2 | 586.4 | 584.5 | A |
| 75 | 135.16 | 3.16 | 97.19 | 572.1 | 573.3 | 571.4 | A |
| 76 | 232.40 | 2.95 | 99.82 | 479.1 | 480.3 | 478.4 | A |
| 77 | | 3.14 | 100 | 567.1 | 568.3 | 566.3 | A |

Further characterising data:

| Compound number | Melting point (°C) (DSC or MT) | LCMS | | | | | |
|---|---|---|---|---|---|---|---|
| | | Retention time | UV (%) | M exact | $[M+H]^+$ | $[M-H]^-$ | Method |
| 132 | 179.05 | 3.52 | 99.47 | 590.1 | 591.5 | 589.4 | A |
| 107 | 207.55 | 3.35 | 98.4 | 542.1 | 543.4 | 541.3 | A |
| 93 | 187.84 | 3.24 | 99.46 | 576.1 | 577.5 | 575.5 | A |
| 116 | 175.40 | 3.39 | 99.8 | 560.1 | 561.4 | 559.4 | A |
| 108 | | 3.41 | 100 | 572.1 | 573.5 | 571.4 | A |
| 146 | 173.85 | 3.12 | 99.58 | 547.1 | 548.4 | 546.3 | A |
| 120 | 183.66 | 3.36 | 98.02 | 508.1 | 509.4 | 507.3 | A |
| 92 | | 3.22 | 99.71 | 549.1 | 550.4 | 548.3 | A |
| 94 | 192.26 | 3.26 | 99.24 | 593 | 594.3 | 592.3 | A |
| 141 | 198.76 | 3.46 | 98.37 | 594.1 | 594.4 | 593.4 | A |
| 110 | 175.59 | 2.75 | 98.27 | 444.1 | 445.3 | 443.3 | A |
| 96 | 133.99 | 2.7 | 98.5 | 426.2 | 427.3 | 425.3 | A |
| 156 | 151.06 | 3.13 | 99.16 | 530.1 | 531.4 | 529.4 | A |
| 164 | | 3.12 | 100 | 526.1 | 527.4 | 525.4 | A |
| 91 | | 2.74 | 100 | 513.1 | 514.2 | 512.2 | C |
| 99 | | 2.79 | 100 | 552 | 555 | 553.1 | C |
| 123 | 157.82 | 3.06 | 100 | 556.1 | 557.4 | 555.4 | A |
| 147 | 147.39 | 2.82 | 97 | 588.1 | 539.3 | 537.3 | B |
| 157 | 183.19 | 3.35 | 99.4 | 564.1 | 565.3 | 563.3 | A |
| 152 | 169.75 | 3.15 | 100 | 552.1 | 553.3 | 551.3 | A |

(continued)

| Compound number | Melting point (°C) (DSC or MT) | LCMS | | | | | |
|---|---|---|---|---|---|---|---|
| | | Retention time | UV (%) | M exact | $[M+H]^+$ | $[M-H]^-$ | Method |
| 159 | 134.19 | 2.91 | 100 | 534.1 | 535.3 | 533.4 | B |
| 103 | 169.72 | 3.15 | 100 | 530.1 | 531.3 | 529.2 | A |
| 103 | 197.38 | 3.58 | 100 | 624 | 625.2 | 623.2 | A |
| 154 | 172.60 | 3.01 | 99.51 | 553.2 | 554.4 | 552.5 | A |
| 118 | 188.26 | 3.54 | 98.6 | 580 | 581.4 | 579.3 | A |
| 119 | 173.10 | 3.13 | 99.41 | 567.2 | 568.5 | 566.5 | A |
| 142 | 190.96 | 3.15 | 100 | 547.1 | 548.3 | 546.3 | A |
| 163 | 158.33 | 3.21 | 100 | 532.1 | 533.3 | 531.3 | A |
| 125 | 141.53 | 3.11 | 99.53 | 547.1 | 548.3 | 546.4 | A |
| 86 | 152.96 | 3.01 | 100 | 577.1 | 578.1 | 576.2 | A |
| 115 | 137.25 | 3.04 | 100 | 543.1 | 544.5 | 542.5 | A |
| 111 | 176.27 | 2.65 | 100 | 527.1 | 528.1 | 526.3 | C |
| 98 | 168.32 | 2.83 | 99.44 | 568.1 | 569.2 | 567.3 | C |
| 150 | 120.10 | 2.92 | 100 | 564.1 | 565.1 | 563.2 | C |
| 109 | 137.30 | 2.76 | 98.5 | 550.1 | 551.2 | 549.3 | C |
| 149 | 145.84 | 2.85 | 100 | 546.1 | 547.1 | 545.2 | C |
| 153 | 177.80 | 2.82 | 100 | 518.1 | 519 | 517.2 | C |
| 130 | | 2.75 | 100 | 51/1 | 532.1 | 530.2 | C |
| 133 | 165.25 | 3.27 | 99.55 | 538 | 539.3 | 537.3 | A |
| 126 | 239.37 | 2.88 | 100 | 562.1 | 563.4 | 561.3 | A |
| 129 | 135.91 | 3.19 | 99.77 | 546.1 | 547.4 | 545.4 | A |
| 101 | 171.00 | 3.26 | 100 | 552.2 | 553.5 | 551.5 | A |
| 161 | 167.34 | 2.93 | 100 | 527.1 | 528.4 | 526.4 | A |
| 88 | 194.58 | 3.02 | 100 | 538.1 | 539.3 | 537.4 | A |
| 127 | 179.93 | 3.12 | 99.4 | 534.1 | 535.3 | 533.3 | A |
| 104 | 154.40 | 2.93 | 100 | 520.1 | 521.4 | 519.4 | A |
| 128 | 170.22 | 3.04 | 100 | 516.1 | 517.3 | 515.3 | A |
| 158 | 163.01 | 3.09 | 100 | 583.2 | 584.5 | 582.5 | A |
| 87 | 171.19 | 3.51 | 99.75 | 580 | 581.3 | 579.4 | A |
| 155 | 181.61 | 3.2 | 100 | 561.1 | 562.4 | 560.4 | A |
| 151 | 183.62 | 3.39 | 100 | 564.1 | 565.3 | 563.4 | A |
| 112 | 146.66 | 3.12 | 100 | 586.1 | 587.4 | 585.4 | A |
| 137 | 136.03 | 3.04 | 100 | 568.1 | 569.4 | 567.3 | A |
| 160 | 127.38 | 3.14 | 99.38 | 564.1 | 565.4 | 563.4 | A |
| 113 | 194.44 | 3.27 | 99.1 | 552.2 | 553.5 | 551.5 | A |
| 85 | 158.44 | 3.48 | 100 | 560.1 | 561.4 | 559.3 | A |

(continued)

| Compound number | Melting point (°C) (DSC or MT) | LCMS | | | | | |
|---|---|---|---|---|---|---|---|
| | | Retention time | UV (%) | M exact | [M+H]+ | [M-H]- | Method |
| 145 | 149.39 | 3.29 | 100 | 568.1 | 569.3 | 567.3 | A |
| 95 | 154.17 & 147.66 | 3.29 | 100 | 540.2 | 541.4 | 539.4 | A |
| 124 | 161.38 | 3.35 | 100 | 586 | 587.3 | 585 | A |
| 144 | 146.89 | 3.35 | 100 | 586 | 587.3 | 585.3 | A |
| 114 | 169.31 | 3.4 | 97.63 | 604 | 605.3 | 603.3 | A |
| 117 | 164.15 | 3.42 | 100 | 616.1 | 617.3 | 615.3 | A |
| 102 | 174.33 | 3.32 | 100 | 560.1 | 561.4 | 559.4 | A |
| 148 | 157.00 | 3.24 | 99.21 | 548.1 | 549.3 | 547.3 | A |
| 89 | 158.88 | 3.29 | 98.1 | 544.1 | 545.4 | 543.4 | A |
| 162 | 160.65 | 3.22 | 99.3 | 544.1 | 545.4 | 543.4 | A |
| 105 | 172.82 | 3.21 | 98.93 | 556.2 | 557.4 | 555.4 | A |
| 143 | 226.52 | 301 | 100 | 561 | 562.3 | 560.3 | A |
| 136 | 147.95 | 3.37 | 100 | 572.1 | 573.3 | 571.3 | A |
| 135 | 167.06 | 3.14 | 99.4 | 552 | 558.1 | 551.1 | C |
| 131 | 199.18 | 3.04 | 98.71 | 554.1 | 555.4 | 558.4 | A |
| 134 | 185.60 | 3.02 | 100 | 556.1 | 557.3 | 555.3 | A |
| 106 | 198.37 | 3.2 | 99.02 | 556.2 | 557.4 | 555.3 | A |
| 100 | 174.31 | 3.02 | 100 | 570.1 | 571.3 | 569.3 | A |
| 139 | 176.81 | 3.21 | 100 | 540.2 | 541.4 | 539.4 | A |
| 140 | 183.14 | 3.41 | 99.01 | 560.1 | 561.3 | 559.3 | A |
| 90 | 233.3 (MT) | 3.2 | 97 | 569.1 | 570.1 | | D |
| 97 | | 3.45 | 1Â°Â° | 604.1 | 605.4 | 603.3 | A |
| 83 | | 3.02 | 99.46 | 576.1 | 577.3 | 575.3 | A |
| 121 | 149.7 (MT) | 3.712 | 98 | 561.1 | 562.1 | | D |
| 80 | | 3.13 | 97.74 | 575.1 | 576.4 | 574.3 | A |
| 138 | | 2.87 | 100 | 541.2 | 542.4 | 540.4 | A |
| 84 | 141.30 | 3.3 | 100 | 600.2 | 601.5 | 659.4 [M+CH3CO2]- | A |
| 81 | 173.01 | 3.46 | 100 | 626.2 | 627.5 | 685.5 [M+CH3COO]- | A |
| 82 | 187.66 | 3.45 | 100 | 616.1 | 617.4 | 615.4 | A |
| 165 | 189.8 (MT) | 3.553 | 99 | 553 | 554 | | D |
| 166 | | 3.12 | 100 | 604.1 | 605.1 | 603.2 | C |
| 167 | 153.0 (MT) | 3.89 | 98 | 587 | 588 | | D |
| 168 | 148.1 (MT) | 3.525 | 97 | 548.1 | 549.1 | | D |
| 169 | | 3.296 | 98 | 569.1 | 570.3 | | D |
| 170 | | 3.333 | 97 | 546.1 | 547.3 | | D |

(continued)

| Compound number | Melting point (°C) (DSC or MT) | LCMS | | | | | |
|---|---|---|---|---|---|---|---|
| | | Retention time | UV (%) | M exact | [M+H]+ | [M-H]- | Method |
| 171 | 203.43 | 3.22 | 99.48 | 529.1 | 530.3 | 538.2 | A |
| 173 | 183.2 (MT) | 3.82 | 99 | 516.1 | 516 | | D |
| 174 | 148.1 (MT) | 3.52 | 97 | 548 | 549 | | D |
| 175 | 146.4 (MT) | 4.048 | 98 | 566.1 | 567.1 | | D |
| 176 | 163.1 (MT) | 3.814 | 99 | 567.1 | 568.1 | | D |
| 177 | 166.4 (MT) | 3.693 | 95 | 545.1 | 546.1 | | D |
| 178 | 193.2 (MT) | 3.563 | 99 | 517.1 | 518.1 | | D |
| 179 | 169.8 (MT) | 3.721 | 99 | 561.1 | 562.1 | | D |
| 180 | | 3.457 | 99 | 599.1 | 600.1 | | D |
| 181 | 198.3 (MT) | 3.641 | 99 | 545.1 | 546.1 | | D |
| 182 | 184.7 | 3.762 | 97 | 571.1 | 572.1 | | D |
| 183 | 198.1 | 2.966 | 97 | 541.1 | 542.1 | | D |
| 184 | 193.2 (MT) | 3.931 | 98 | 585.1 | 586.1 | | D |
| 185 | 181.5 (MT) | 2.875 | 98 | 541.1 | 542.1 | | D |
| 186 | 158.1 (MT) | 3.987 | 99 | 570.1 | 571.2 | | D |
| 187 | 216.6 (MT) | 1.668 | 98 | 413.1 | 414.1 | | D |
| 188 | | 3.138 | 98 | 547.1 | 548.1 | | D |
| 189 | 169.8 (MT) | 4.286 | 99 | 637.1 | 638.2 | | D |
| 190 | | 4.015 | 98 | 567.1 | 566.1 | | D |
| 191 | 178.1 (MT) | 2.96 | 99 | 583.1 | 584.1 | | D |
| 192 | 189.9 (MT) | 1.816 | 98 | 420.1 | 421.1 | | D |
| 193 | 153.1 (MT) | 3.535 | 99 | 557.1 | 558.1 | | D |
| 194 | 137.9 (MT) | 4.136 | 98 | 596.1 | 597.1 | | D |
| 195 | 226.7 (MT) | 3.05 | 99 | 591.1 | 592.1 | | D |
| 196 | | 1.454 | 97 | 395.2 | 396.2 | | D |
| 198 | 173.1 (MT) | 1.807 | 99 | 412.2 | 413.2 | | D |
| 199 | 120.4 (MT) | 1.863 | 98 | 429.1 | 430.1 | | D |
| 200 | 166.4 (MT) | 3.431 | 99 | 539.1 | 540.1 | | D |
| 201 | 159.8 (MT) | 3.103 | 99 | 528.1 | 529.1 | | D |
| 202 | 214.9 (MT) | 2.03 | 97 | 432.1 | 433.1 | | D |
| 203 | 208.2 (MT) | 1.48 | 99 | 421.2 | 422.2 | | D |
| 204 | 186.4 (MT) | 3.655 | 99 | 560.1 | 561.1 | | D |
| 205 | 201.5 (MT) | 2.906 | 97 | 584.1 | 585.1 | | D |
| 206 | | 4.025 | 99 | 598.1 | 599.1 | | D |
| 207 | 157.42 | 2.88 | 100 | 553.1 | 554.4 | 552.4 | A |
| 208 | 178.11 | 3.29 | 100 | 543.1 | 544.3 | 542.3 | A |

(continued)

| Compound number | Melting point (°C) (DSC or MT) | LCMS | | | | | |
|---|---|---|---|---|---|---|---|
| | | Retention time | UV (%) | M exact | $[M+H]^+$ | $[M-H]^-$ | Method |
| 209 | 159.7 (MT) | 3.881 | 99 | 590.1 | 591.1 | | D |
| 210 | 178.5 (MT) | 4.33 | 99 | 594.1 | 595.1 | | D |
| 211 | 153.35 | 3.07 | 100 | 571.2 | 572.4 | 570.5 | A |
| 212 | | 3.04 | 98 | 541.2 | 542.4 | 540.4 | A |
| 213 | 159.38 | 3.46 | 100 | 560.1 | 561.4 | 559.3 | A |
| 214 | 106.9 | 3.28 | 97 | 548.1 | 549.4 | 547.4 | A |
| 215 | 152.81 | 3.26 | 97 | 522.2 | 523.3 | 521.4 | A |
| 216 | | 3.74 | 96.2 | 558.1 4 | 589.4 | 587.6 | A |
| 217 | | 3.03 | 100 | 565.2 | 566.4 | 564.4 | A |
| 218 | | 3.33 | 100 | 528.1 | 529.3 | 527.5 | A |
| 219 | | 3.36 | 99 | 550.2 | 551.2 | 549.3 | C |
| 220 | | 3.36 | 98 | 576.1 | 577.1 | 575.2 | C |
| 221 | | 3.18 | 96 | 551.2 | 552.2 | 550.3 | C |
| 222 | | 3.25 | 98 | 577.2 | 578.2 | 576.4 | C |
| 223 | | 3.44 | 99 | 556.1 | 557.2 | 555.2 | C |
| 224 | | 3.7 | 99 | 576.1 | 577.2 | 575.2 | C |
| 225 | | 3.35 | 96 | 554.2 | 555.2 | 553.3 | C |
| 226 | | 3.65 | 96 | 584.2 | 585.2 | 583.3 | C |
| 79 | 113.95 | 3.22 | 99.6 | 582.1 | 583.4 | 581.4 | A |

5. Biological Assays/ Pharmacological Examples

MIC determination for testing compounds against ***M. tuberculosis.***

**TEST 1**

Test compounds and reference compounds were dissolved in DMSO and 1 µl of solution was spotted per well in 96 well plates at 200x the final concentration. Column 1 and column 12 were left compound-free, and from column 2 to 11 compound concentration was diluted 3-fold. Frozen stocks *of Mycobacterium tuberculosis* strain EH4.0 expressing green-fluorescent protein (GFP) were previously prepared and titrated. To prepare the inoculum, 1 vial of frozen bacterial stock was thawed to room temperature and diluted to 5x10 exp5 colony forming units per ml in 7H9 broth. 200 µl of inoculum, which corresponds to 1x10 exp5 colony forming units, were transferred per well to the whole plate, except column 12. 200µl 7H9 broth were transferred to wells of column 12. Plates were incubated at 37°C in plastic bags to prevent evaporation. After 7 days, fluorescence was measured on a Gemini EM Microplate Reader with 485 excitation and 538 nm emission wavelengths and $IC_{50}$ and/or $pIC_{50}$ values (or the like, e.g. $IC_{50}$, $IC_{90}$, $pIC_{90}$, etc) were (or may be) calculated.

**TEST 2**

Appropriate solutions of experimental/test and reference compounds were made in 96 well plates with 7H9 medium. Samples *of Mycobacterium tuberculosis* strain H37Rv were taken from cultures in logarithmic growth phase. These were first diluted to obtain an optical density of 0.3 at 600 nm wavelength and then diluted 1/100, resulting in an inoculum of approximately 5x10 exp5 colony forming units per ml. 100µl of inoculum, which corresponds to 5x10 exp4

colony forming units, wer transferred per well to the whole plate, except column 12. Plates were incubated at 37°C in plastic bags to prevent evaporation. After 7 days, resazurin was added to all wells. Two days later, fluorescence was measured on a Gemini EM Microplate Reader with 543 excitation and 590 nm emission wavelengths and $MIC_{50}$ and/or $pIC_{50}$ values (or the like, e.g. $IC_{50}$, $IC_{90}$, $pIC_{90}$, etc) were (or may be) calculated.

**TEST 3: Time kill assays**

Bactericidal or bacteriostatic activity of the compounds can be determined in a time kill kinetic assay using the broth dilution method. In this assay, the starting inoculum of *M. tuberculosis* (strain H37Rv and H37Ra) is $10^6$ CFU / ml in Middlebrook (1x) 7H9 broth. The test compounds are tested alone or in combination with another compound (e.g. a compound with a different mode of action, such as with a cytochrome bd inhibitor) at a concentration ranging from 10-30μM to 0.9-0.3μM respectively. Tubes receiving no antibacterial agent constitute the culture growth control. The tubes containing the microorganism and the test compounds are incubated at 37 °C. After 0, 1, 4, 7, 14 and 21 days of incubation samples are removed for determination of viable counts by serial dilution ($10^0$ to $10^{-6}$) in Middlebrook 7H9 medium and plating (100 μl) on Middlebrook 7H11 agar. The plates are incubated at 37 °C for 21 days and the number of colonies are determined. Killing curves can be constructed by plotting the $\log_{10}$CFU per ml versus time. A bactericidal effect of a test compound (either alone or in combinaton) is commonly defined as 2-$\log_{10}$ decrease (decrease in CFU per ml) compared to Day 0. The potential carryover effect of the drugs is limited by using 0.4% charcoal in the agar plates, and by serial dilutions and counting the colonies at highest dilution possible used for plating.

**RESULTS**

Compounds of the invention/examples, for example when tested in Test 1 decribed above, may typically have a $pIC_{50}$ from 3 to 10 (e.g. from 4.0 to 9.0, such as from 5.0 to 8.0)

**6.** Biological Results

Compounds of the examples were tested in Test 1 described above (in section "Pharmacological Examples") and the following results were obtained:

Biological data table

| Compound number | $pIC_{50}$ | | Compound number | $pIC_{50}$ | | Compound number | $pIC_{50}$ |
|---|---|---|---|---|---|---|---|
| 1 | 8.15 | | 132 | 8.08 | | 97 | 7.47 |
| 31 | 8.04 | | 107 | 7.62 | | 83 | 6.43 |
| 32 | 7.94 | | 93 | 7.31 | | 121 | 7.89 |
| 29 | 8 | | 116 | 7.82 | | 80 | 6.30 |
| 28 | 8.6 | | 108 | 7.69 | | 138 | 8.21 |
| 30 | 7.9 | | 146 | 8.42 | | 84 | 6.70 |
| 21 | 8.6 | | 120 | 7.89 | | 81 | 6.30 |
| 19 | 7.4 | | 92 | 7.27 | | 82 | 6.30 |
| 2 | 7.8 | | 94 | 7.41 | | 165 | |
| 56 | 3.8 | | 141 | 8.31 | | 166 | 6.61 |
| 20 | 7.5 | | 110 | 7.73 | | 167 | 7.06 |
| 3 | 7.5 | | 96 | 7.44 | | 168 | 7.41 |
| 57 | 6.3 | | 156 | 8.86 | | 169 | 8.00 |
| 13 | 8.4 | | 164 | 9.61 | | 170 | 6.50 |
| 58 | 6.6 | | 91 | 7.19 | | 171 | 7.60 |

(continued)

| Compound number | $pIC_{50}$ | | Compound number | $pIC_{50}$ | | Compound number | $pIC_{50}$ |
|---|---|---|---|---|---|---|---|
| 14 | 8.2 | | 99 | 7.49 | | | |
| 59 | 6.7 | | 123 | 7.99 | | 173 | 7.30 |
| 15 | 7.6 | | 147 | 8.42 | | 174 | 6.30 |
| 6 | 8.2 | | 157 | 8.86 | | 175 | 8.80 |
| 16 | 8.2 | | 152 | 8.70 | | 176 | 7.80 |
| 4 | 7.4 | | 159 | 8.92 | | 177 | 8.10 |
| 18 | 7.8 | | 122 | 7.98 | | 178 | 6.70 |
| 5 | 8.1 | | 103 | 7.55 | | 179 | 6.90 |
| 17 | 7.7 | | 154 | 8.77 | | 180 | 8.40 |
| 22 | 6.4 | | 118 | 7.86 | | 181 | 8.20 |
| 23 | 7.9 | | 119 | 7.87 | | 182 | 7.50 |
| 10 | 7.2 | | 142 | 8.33 | | 183 | 6.70 |
| 24 | 7.8 | | 163 | 9.50 | | 184 | 7.50 |
| 26 | 8.6 | | 125 | 8.01 | | 185 | 7.33 |
| 11 | 7.1 | | 86 | 7.05 | | 186 | 8.44 |
| 12 | 8.5 | | 115 | 7.80 | | 187 | 6.30 |
| 9 | 9.2 | | 111 | 7.74 | | 188 | 6.30 |
| 8 | 6.6 | | 98 | 7.48 | | 189 | 6.30 |
| 25 | 8 | | 150 | 8.53 | | 190 | 6.76 |
| 7 | 7.2 | | 109 | 7.70 | | 191 | 7.42 |
| 27 | 8.7 | | 149 | 8.45 | | 192 | <6.301 |
| 60 | 6.7 | | 153 | 8.75 | | 193 | 9.05 |
| 61 | 6.3 | | 130 | 8.06 | | 194 | 8.62 |
| 62 | 6.3 | | 133 | 8.12 | | 195 | 7.98 |
| 33 | 7.6 | | 126 | 8.01 | | 196 | 6.46 |
| 38 | 7.2 | | 129 | 8.05 | | 198 | 7.03 |
| 39 | 7.1 | | 101 | 7.53 | | 199 | <6.301 |
| 40 | 7.2 | | 161 | 9.11 | | 200 | 8.26 |
| 63 | 6.3 | | 88 | 7.08 | | 201 | 7.92 |
| 41 | 7.3 | | 127 | 8.02 | | 202 | 6.41 |
| 34 | 8 | | 104 | 7.55 | | 203 | <6.301 |
| 35 | 8.3 | | 128 | 8.04 | | 204 | 8.35 |
| 64 | 6.5 | | 158 | 8.86 | | 205 | 7.18 |
| 65 | 6.3 | | 87 | 7.05 | | 206 | 8.61 |
| 42 | 8.2 | | 155 | 8.79 | | 207 | 6.73 |
| 43 | 8 | | 151 | 8.58 | | 208 | 7.16 |
| 66 | 6.6 | | 112 | 7.76 | | 209 | 7.95 |
| 67 | 6.7 | | 137 | 8.21 | | 210 | 8.52 |

(continued)

| Compound number | $pIC_{50}$ | | Compound number | $pIC_{50}$ | | Compound number | $pIC_{50}$ |
|---|---|---|---|---|---|---|---|
| 36 | 8.4 | | 160 | 8.92 | | 211 | 8.64 |
| 37 | 8.4 | | 113 | 7.79 | | 212 | 7.59 |
| 44 | 8.6 | | 85 | 7.02 | | 213 | 8.46 |
| 45 | 7.4 | | 145 | 8.39 | | 214 | 7.87 |
| 68 | 6.3 | | 95 | 7.42 | | 215 | 9.04 |
| 46 | 7 | | 124 | 8.00 | | 216 | 8.67 |
| 47 | 8.5 | | 144 | 8.38 | | 217 | 7.85 |
| 69 | 6.9 | | 114 | 7.79 | | 218 | 8.24 |
| 70 | 6.3 | | 117 | 7.83 | | 79 | 8.43 |
| 48 | 8.8 | | 102 | 7.53 | | | |
| 49 | 7.63 | | 148 | 8.42 | | | |
| 50 | 8.97 | | 89 | 7.08 | | | |
| 51 | 7.34 | | 162 | 9.41 | | | |
| 52 | 7.38 | | 105 | 7.56 | | | |
| 71 | 6.78 | | 143 | 8.35 | | | |
| 72 | 6.8 | | 136 | 8.20 | | | |
| 53 | 7.09 | | 135 | 8.14 | | | |
| 54 | 7.96 | | 131 | 8.06 | | | |
| 55 | 7.59 | | 134 | 8.12 | | | |
| 73 | 7.29 | | 106 | 7.59 | | | |
| 74 | 8.17 | | 100 | 7.50 | | | |
| 75 | 8.2 | | 139 | 8.24 | | | |
| 76 | 7.87 | | 140 | 8.26 | | | |
| 77 | 8.1 | | 90 | 7.13 | | | |

## 7. Further data on representative compounds of the invention/examples

The compounds of the invention/examples may have advantages associated with *in vitro* potency, kill kinetics (i.e. bactericidal effect) *in vitro,* PK properties, food effect, safety/toxicity (including liver toxicity, coagulation, 5-LO oxygenase), metabolic stability, Ames II negativity, MNT negativity, aqueous based solubility (and ability to formulate) and/or cardiovascular effect e.g. on animals (e.g. anesthetized guinea pig). The data below that was generated/calculated may be obtained using standard methods/assays, for instance that are available in the literature or which may be performed by a supplier (e.g. Microsomal Stability Assay - Cyprotex, Mitochondrial toxicity (Glu/Gal) assay - Cyprotex, as well as literature CYP cocktail inhibition assays). In some instances, GSH was measured (reactive metabolites, glucuronidation) to observe if a dihydrodiol is observed by LCMS (fragmentation ions), which would correspond to a dihydroxylation on the core heterocycle.

This following data was generated on Compound 1:

$$\text{cLogP} = 4.3 \ / \ \text{TPSA} = 107.7$$

CVS (Na Ch, Ca Ch, hERGdof), $IC_{50}$ = >10, >10, >10
Cocktail Cyp-450, $IC_{50}$ = >20 (except CYP3A4, which was not conclusive)

$$CLint\ (\mu l/min/mg\ prot) = (H)\ 29.6\ /\ (M)\ 21.5$$

The following data was generated on Compound 13:

$$cLogP = 3.3\ /\ TPSA = 120.7$$

CVS (Na Ch, Ca Ch, hERGdof), $IC_{50}$ = >10, >10, 7.4
Cocktail Cyp-450, $IC_{50}$ = >20 (except CYP3A4 and CY2D6, which were not conclusive)

$$CLint\ (\mu l/min/mg\ prot) = (H)\ 16.3\ /\ (M)\ 13.3$$

The following data was generated on Compound 20:

$$cLogP = 3.75\ /\ TPSA = 107.7$$

CVS (Na Ch, Ca Ch, hERGdof), $IC_{50}$ = >10, >10, >10
Cocktail Cyp-450, $IC_{50}$ = >20 (except CYP3A4, $IC_{50}$ = 13.2 μM)

$$CLint\ (\mu l/min/mg\ prot) = (H)\ 56.6\ /\ (M)\ 15.9$$

The following data was generated on Compound 73:

It was tested and showed no measure of GSH

$$cLog\ P= 3.2\ /\ TPSA\ 140.8$$

CVS (Ca, Na, Herg), $IC_{50}$ = >10
Cocktail Cyp-450, $IC_{50}$ = >20 (for all)

$$CLint\ (\mu l/min/mg\ prot) = (H)\ 18\ /\ (M)\ 93$$

The following data was generated on Compound 9

$$cLog\ P= 4.4\ /\ TPSA\ 107{,}8$$

CVS (Ca, Na, Herg), $IC_{50}$ = >10
Cocktail Cyp-450, $IC_{50}$ = >20 (for all)

$$CLint\ (\mu l/min/mg\ prot) = (H)\ 19\ /\ (M)\ 41$$

The following data was generated on Compound 26

$$cLog\ P= 3.1\ /\ TPSA\ 129.9$$

CVS (Ca, Na, Herg), $IC_{50}$ = >10
Cocktail Cyp-450, $IC_{50}$ = >20 (for all)

CLint (μl/min/mg prot) = (H) 37 / (M) 35

The following data was generated on Compound 16

cLog P= 4.4 / TPSA 107.8

CVS (Ca, Na, Herg), $IC_{50}$ = >10
Cocktail Cyp-450, $IC_{50}$ = >20 (for all)

CLint (μl/min/mg prot) = (H) 24 / (M) 18

The following data was generated on Compound 6

It was tested and showed no measure of GSH

cLog P= 4.3 / TPSA 117

CVS (Ca, Na, Herg), $IC_{50}$ = >10
Cocktail Cyp-450, $IC_{50}$ = >20 (for all)

CLint (μl/min/mg prot) = (H) 37.6 / (M) 49

<u>The following further data/results were generated</u>

Compound 1:

- Was found to have low mitotoxicity (<3 in the Glu/Gal assay) - hence no mitotoxicity alerts
- Had good bioavailaibility (as shown in rodents)

Compound 6:

- Was found to have low mitotoxicity (<3 in the Glu/Gal assay) - hence no mitotoxicity alerts
- Did not produce unwanted reactive metabolites (it showed no measure of GSH)

Compound 152:

- Found to have low mitotoxicity (<3 in the Glu/Gal assay) - hence no mitotoxicity alerts
- Had good bioavailaibility (as shown in rodents)
- The formation of reactive metabolites was blocked

Compound 161:

- Found to have low mitotoxicity (<3 in the Glu/Gal assay) - hence no mitotoxicity alerts
- Had good bioavailaibility (as shown in rodents)
- The formation of reactive metabolites was blocked

<u>Specific Data on Compound 161:</u>

TPSA = 120.6
HTEq Sol (μg/mL) - pH 2 : 33, pH 7: <0.02, FaSSIF : 5, FeSSIF : 16
Cocktail Cyp-450, $IC_{50}$ (μM) = >20

Cyp 3A4 induction (% control) - at 1 μM = 3.0
CLint Hep (ml/min/$10^6$cells) = (M) 0.012 / (R) 0.019 / (D) 0.0047 / (H) 0.0067
PPB (% unbound) (H) 1.5 / (M) 2.45
AMES II - negative (Score 1)
Glu/Gal - negative (ratio < 3)
GSH/CN - no reactive metabolites
Kinase panel - negative
CTCM (μM) - clean up to 5 μM
CVS (Na Ch, Ca Ch, hERGdof), $IC_{50}$ = >10, >10, 15.85

Oral bioavailability of Compound 161 in rat

Compound 161 was administed PO in rat (5 mg/kg, PEG4000 (sol.), 0.5 w/v Methocel (susp.) and the following results were obtained for the solution and suspension.

| | Solution (Compound 161) | Suspension (Compound 161) |
|---|---|---|
| **$C_{max}$ (ng/mL)** | **1228 ± 406** | **787 ± 226** |
| **$T_{max}$ (h)** | **4.0** | **2.0 (1.0 - 2.0)** |
| **$AUC_{0-inf}$ (ng.h/mL)** | **10880±1715** | **5610±2747** |
| **$t_{½}$ (h)** | **3.55±0.45** | **3.49±0.91** |
| **F (%)** | **106±17** | **55±27** |

Conclusions

Compounds of the invention/examples (e.g. as exemplified by Compound 161), may therefore have the advantage that:

- No *in vitro* cardiotoxicity is observed (for example either due to the CVS results or due to the Glu/Gal assay results);
- No reactive metabolite formation is observed (e.g. GSH); and/or
- There is a relatively higher unbound fraction,

for instance as compared to other compounds, for instance prior art compounds.

Certain compounds of the invention/examples may also have the additional advantage that they do not form degradants (e.g. that are undesired or may elicit unwanted side-effects).

Compounds of the invention/examples (for instance, as represented by Compound 161), may have the advantage that a faster oral absorption and improved bioavailability are displayed (as may be shown by the oral bioavailability data in rat).

**Claims**

1. A compound of formula (Ia)

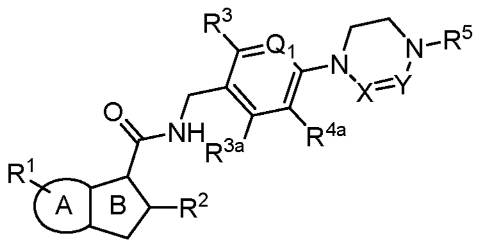

(Ia)

wherein

$Q_1$ represents Nor C($R^4$);
A is a 5- or 6-membered ring, which may be aromatic or non-aromatic, and optionally containing 1 or 2 heteroatoms selected from nitrogen and sulfur;
B is a 5-membered aromatic ring containing 1 or 2 nitrogen heteroatoms;
$R^1$ represents one or more optional substituents independently selected from halo, -$R^{6a}$, -O-$R^{6b}$, -C(=O)-$R^{6c}$, -C(=O)-N($R^7$)($R^8$), -CN and -N($R^{7a}$)$R^{7b}$; or any two $R^1$ groups may be taken together (when attached to adjacent atoms of the A ring) to form a 5- or 6-membered ring optionally containing one or two heteroatoms, and which ring is optionally substituted by one or two $C_{1-3}$ alkyl substituents;
$R^2$ is -$C_{1-4}$ alkyl optionally substituted by one or more substituents selected from halo and -$OC_{1-3}$ alkyl;
any two of $R^3$, $R^{3a}$, $R^4$ and $R^{4a}$ represent H, and the other two independently represent a substituent selected from H, F, -$C_{1-3}$ alkyl and -O-$C_{1-3}$ alkyl;
$R^5$ is H, -$R^{9a}$, -C(=O)-$R^{9b}$, -$SO_2$-$R^{10}$ or Het$^1$;
either one of X and Y represents -$CR^{11a}$ and the other represents N or -$CR^{11b}$;
$R^{6a}$ and $R^{6b}$ independently represent hydrogen or -$C_{1-4}$ alkyl optionally substituted by one or more substituents selected from halo, -O-$CH_3$ and phenyl;
$R^{6c}$ is -$C_{1-3}$ alkyl;
$R^7$ and $R^8$ are independently selected from Hand -$C_{1-3}$ alkyl;
$R^{7a}$ and $R^{7b}$ independently represent H, $C_{1-6}$ alkyl or $R^{7a}$ and $R^{7b}$ are linked together to form a 3- to 6-membered ring;
$R^{9a}$ represents -$C_{1-4}$ alkyl, optionally substituted by one or more substituents selected from halo, -$OC_{1-3}$ alkyl and Het$^2$;
$R^{9b}$ is hydrogen or -$C_{1-3}$ alkyl (optionally substituted by one or more fluoro atoms);
$R^{10}$ is -$C_{1-4}$ alkyl optionally substituted by one or more substituents selected from halo and -O-$CH_3$;
$R^{11a}$ and $R^{11b}$ independently represent H, $C_{1-4}$ alkyl (itself optionally substituted by one or more substituent(s) selected from fluoro, -CN, -$R^{12a}$, -$OR^{12b}$, -N($R^{12c}$)$R^{12d}$ and/or -C(O)N($R^{12e}$)$R^{12f}$) or -O-$C_{1-4}$ alkyl (itself optionally substituted by one or more substituent(s) selected from fluoro, -$R^{12g}$, -$OR^{12h}$ and/or -N($R^{12i}$)$R^{12j}$);
$R^{12a}$, $R^{12b}$, $R^{12c}$, $R^{12d}$, $R^{12e}$, $R^{12f}$, $R^{12g}$, $R^{12h}$, $R^{12i}$ and $R^{12j}$ independently represent hydrogen or $C_{1-3}$ alkyl (optionally substituted by one or more fluoro atoms);
Het$^1$ and Het$^2$ independently represent a 5- or 6-membered aromatic ring containing one or two heteroatoms, preferably selected from nitrogen and sulfur, optionally substituted by one or more substitutents selected from halo and $C_{1-3}$ alkyl (itself optionally substituted by one or more fluoro atoms),

or a pharmaceutically-acceptable salt thereof,

**2.** A compound according to claim 1 of formula (I)

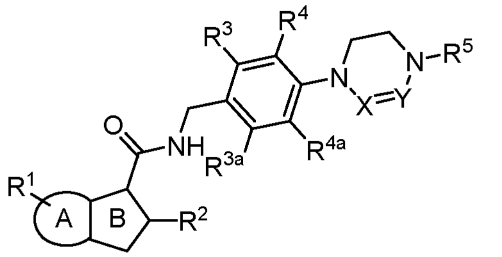

(I)

wherein

A is a 5- or 6-membered ring, which may be aromatic or non-aromatic, and optionally containing 1 or 2 heteroatoms selected from nitrogen and sulfur;
B is a 5-membered aromatic ring containing 1 or 2 nitrogen heteroatoms;
$R^1$ represents one or more optional substituents independently selected from halo, -$R^{6a}$, -O-$R^{6b}$, -C(=O)-$R^{6c}$, -C(=O)-N($R^7$)($R^8$), -CN and -N($R^{7a}$)$R^{7b}$;
$R^2$ is -$C_{1-4}$ alkyl optionally substituted by one or more substituents selected from halo and -$OC_{1-3}$ alkyl;
any two of $R^3$, $R^{3a}$, $R^4$ and $R^{4a}$ represent H, and the other two independently represent a substituent selected from H, F, -$C_{1-3}$ alkyl and -O-$C_{1-3}$ alkyl;
$R^5$ is H, -$R^{9a}$, -C(=O)-$R^{9b}$, -$SO_2$-$R^{10}$ or Het$^1$;
either one of X and Y represents -$CR^{11a}$ and the other represents N or -$CR^{11b}$;

$R^{6a}$ and $R^{6b}$ independently represent -$C_{1-4}$ alkyl optionally substituted by one or more substituents selected from halo and -O-$CH_3$;
$R^{6c}$ is -$C_{1-3}$ alkyl;
$R^7$ and $R^8$ are independently selected from Hand -$C_{1-3}$ alkyl;
$R^{7a}$ and $R^{7b}$ independently represent H, $C_{1-6}$ alkyl or $R^{7a}$ and $R^{7b}$ are linked together to form a 3- to 6-membered ring;
$R^{9a}$ represents -$C_{1-4}$ alkyl, optionally substituted by one or more substituents selected from halo, -O$C_{1-3}$ alkyl and $Het^2$;
$R^{9b}$ is hydrogen or -$C_{1-3}$ alkyl (optionally substituted by one or more fluoro atoms);
$R^{10}$ is -$C_{1-4}$ alkyl optionally substituted by one or more substituents selected from halo and -O-$CH_3$;
$R^{11a}$ and $R^{11b}$ independently represent H, $C_{1-4}$ alkyl (itself optionally substituted by one or more substituent(s) selected from fluoro, -CN, -$R^{12a}$, -O$R^{12b}$, -N($R^{12c}$)$R^{12d}$ and/or -C(O)N($R^{12e}$)$R^{12f}$) or -O-$C_{1-4}$ alkyl (itself optionally substituted by one or more substituent(s) selected from fluoro, -$R^{12g}$,
-O$R^{12h}$ and/or -N($R^{12i}$)$R^{12j}$);
$R^{12a}$, $R^{12b}$, $R^{12c}$, $R^{12d}$, $R^{12e}$, $R^{12f}$, $R^{12g}$, $R^{12h}$, $R^{12i}$ and $R^{12j}$ independently represent hydrogen or $C_{1-3}$ alkyl (optionally substituted by one or more fluoro atoms);
$Het^1$ and $Het^2$ independently represent a 5- or 6-membered aromatic ring containing one or two heteroatoms, preferably selected from nitrogen and sulfur, optionally substituted by one or more substitutents selected from halo and $C_{1-3}$ alkyl (itself optionally substituted by one or more fluoro atoms),
or a pharmaceutically-acceptable salt thereof.

**3.** A compound as claimed in Claim 1 or Claim 2, wherein:

there may be none, one or two $R^1$ substituents present on ring A;
$R^1$ (when present) represents one or two substituents independently selected from F, Cl, -$R^{6a}$, -O-$R^{6b}$, -C(=O)-$R^{6c}$, -C(=O)-N($R^7$)($R^8$), -CN and -N($R^{7a}$)$R^{7b}$;
$R^{6a}$ represents $C_{1-3}$ alkyl optionally substituted selected from -O-$C_{1-2}$ alkyl;
$R^{6b}$ and $R^{6c}$ represent $C_{1-3}$ alkyl, which is preferably unsubstituted;
$R^7$ and $R^8$ independently represent hydrogen or $C_{1-3}$ alkyl, which is preferably unsubstituted;
$R^{7a}$ and $R^{7b}$ are linked together to form a 4-6-membered ring.

**4.** A compound as claimed any one of the preceding claims, wherein:
Ring A is represented as follow:

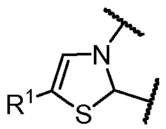
(II)

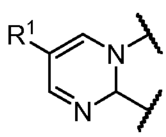
(III)

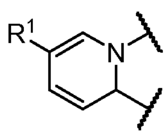
(IV)

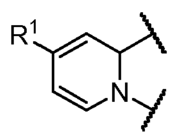
(V)

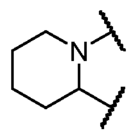
(VI)

**5.** A compound as claimed in any of the preceding claims, wherein:
Ring B is represented as follow:

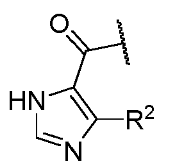
(VII)

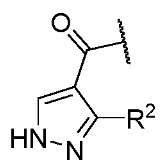
(VIII)

**6.** A compound as claimed in any of the preceding claims wherein:
the combined ring system, i.e. ring A and ring B may be represented as follow:

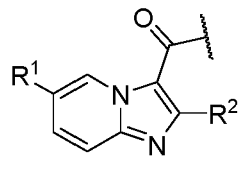

(IX)

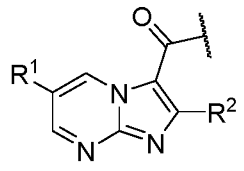

(X)

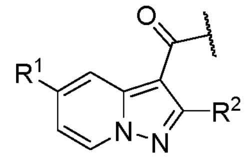

(XI)

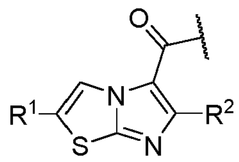

(XII)

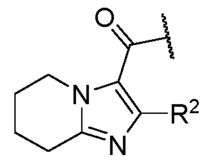

(XIII)

**7.** A compound as claimed in any one of the preceding claims, wherein:

$R^2$ is linear $-C_{1-4}$ alkyl optionally substituted by one or more substituents, for example selected from $-O-C_{1-2}$ alkyl;
any two of $R^3$, $R^{3a}$, $R^4$ and $R^{4a}$ represent H, and the other two independently represent a substituent selected from H, F, $-CH_3$ and $-OCH_3$;
$R^5$ is H, $-R^{9a}$, $-C(=O)-R^{9b}$, $-SO_2-R^{10}$ or $Het^1$;
$R^{9a}$ represents $C_{1-3}$ alkyl unsubstituted or substituted with one substituent;
$R^{9b}$ represents H or $C_{1-3}$ alkyl optionally substituted by one or more fluoro atoms (so forming a $-CF_3$ group);
$R^{10}$ represents $C_{1-4}$ alkyl optionally substituted by one or more substituents selected from fluoro and $-OC_{1-2}$ alkyl, and hence $R^{10}$ may represent $-CF_3$, $-CH_3$, i-propyl, - $CH_2C(H)(CH_3)_2$ (i-butyl), $-CH_2CH_2-OCH_3$; and/or
$Het^1$ and $Het^2$ independently represent a 5- or 6-membered heteroaryl ring containing one or two heteroatoms selected from nitrogen and sulfur, which ring is unsubstitued or substituted by one or two substituent selected from $C_{1-3}$ alkyl (itself optionally substituted by one or more fluoro atoms, so forming a $-CF_3$ group), and, hence, $Het^1$ and $Het^2$ may independently represent a thiazolyl group optionally substituted by a - $CF_3$ substituent.

**8.** A compound as claimed in any one of the preceding claims, wherein:

either one of X and Y represents $-CR^{11a}$ and the other represents N or $-CR^{11b}$ (and in an embodiment X represents N and Y represents $-CR^{11a}$);
when $R^{11a}$ or $R^{11b}$ represents $C_{1-4}$ alkyl, then it may be unsubstituted or substituted with -CN, $-OR^{12b}$ and/or $-N(R^{12c})R^{12d}$;
$R^{12b}$ represents H or $C_{1-2}$ alkyl;
$R^{12c}$ and $R^{12d}$ may independently represent $C_{1-2}$ alkyl;
hence, when $R^{11a}$ or $R^{11b}$ represents such a $C_{1-4}$ alkyl group, then it may be $-CH_3$, $-CH_2CH_3$, $-CH_2CH_2-OH$, $-CH_2CH_2-OCH_3$, $-C(H)(CH_3)_2$, $-CH_2-N(CH_3)_2$ or $-CH_2-CN$);
when $R^{11a}$ or $R^{11b}$ represents $-O-C_{1-4}$ alkyl, then it is preferably unsubstituted and may represent $-OC_{1-2}$ alkyl.

**9.** A compound as claimed in claim 1 that is:

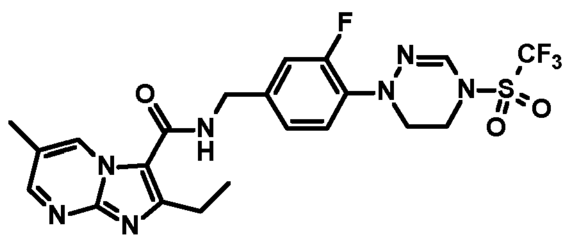

or a pharmaceutically acceptable salt thereof.

**10.** A compound as claimed in any one of claims 1 to 9, for use as a pharmaceutical.

**11.** A pharmaceutical composition comprising a pharmaceutically acceptable carrier and, as active ingredient, a therapeutically effective amount of a compound as claimed in any one of Claims 1 to 9.

**12.** A compound as claimed in any one of claims 1 to 9 for use in the treatment of a mycobacterial infection.

**13.** Use of a compound as claimed in any one of claims 1 to 9 for the manufacture of a medicament for the treatment of a mycobacterial infection.

**14.** A compound for use as claimed in Claim 12 or a use as claimed in Claim 13 wherein the mycobacterial infection is tuberculosis.

**15.** A combination of (a) a compound as claimed in any one of claims 1 to 9, and (b) one or more other anti-mycobacterial or anti-tuberculosis agent.

**16.** A product containing (a) a compound as claimed in any one of claims 1 to 9, and (b) one or more other anti-mycobacterial or anti-tuberculosis agent, as a combined preparation for simultaneous, separate or sequential use in the treatment of a bacterial infection.

**17.** A process for the preparation of a compound of formula (I) as claimed in Claim 2 or a compound of formula (Ia) as claimed in Claim 1, which process comprises:

(i) reaction of a compound of formula (XIV),

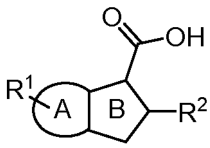

(XIV)

in which the integers are defined in Claim 1, with a compound of formula (XV) or (XVA), respectively,

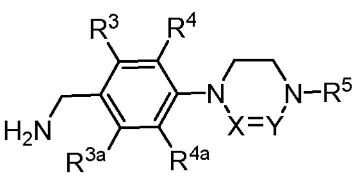

(XV)

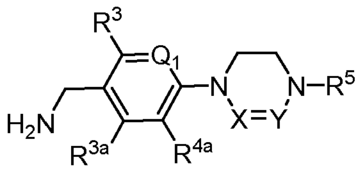

(XVA)

wherein the integers are as defined in Claim 1;
(ii) coupling of a compound of formula (XVII) or (XVIIA) respectively,

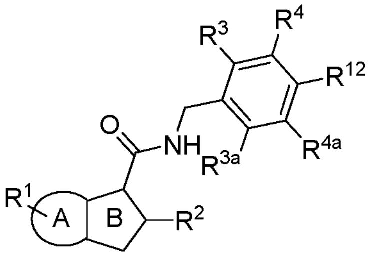

(XVII)

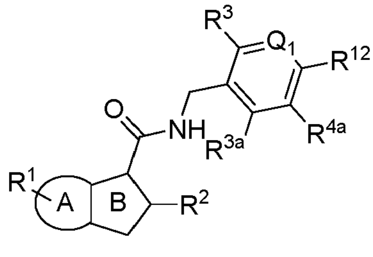

(XVIIA)

wherein the integers are as defined in Claim 1, and $R^{12}$ represents a suitable group, with a compound of formula

(XVI),

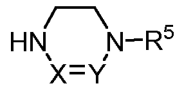

(XVI)

wherein $R^5$ is as defined in Claim 1;

(iii) for compounds of formula (I) or (Ia) in which X represents N (and $R^5$ preferably represents H), reaction of a compound of formula (XVIII) or (XVIIIA), respectively

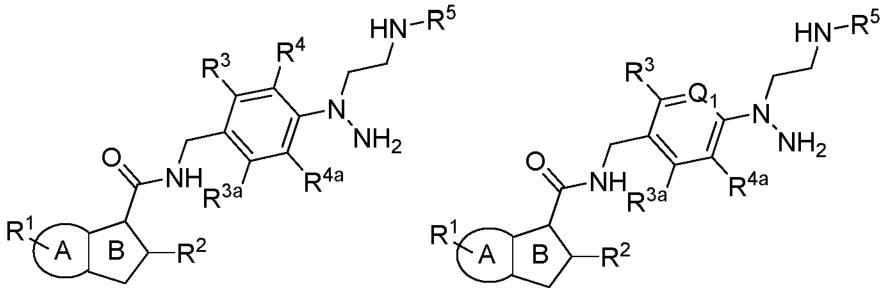

(XVIII) (XVIIIA)

wherein the integers are as defined in claim 1 (and $R^5$ preferably represents H), reaction with a compound of formula (XIX)

$R^{11x}C(OCH_3)_3$ (XIX)

or the like, wherein $R^{11x}$ represents $R^{11a}$ or $R^{11b}$ (as appropriate; and as defined in Claim 1);

(iv) for compounds of formula (I) or (Ia) in which X represents N (and preferably $R^5$ represents H), reaction of a comound of formula (XX) or (XXA), respectively

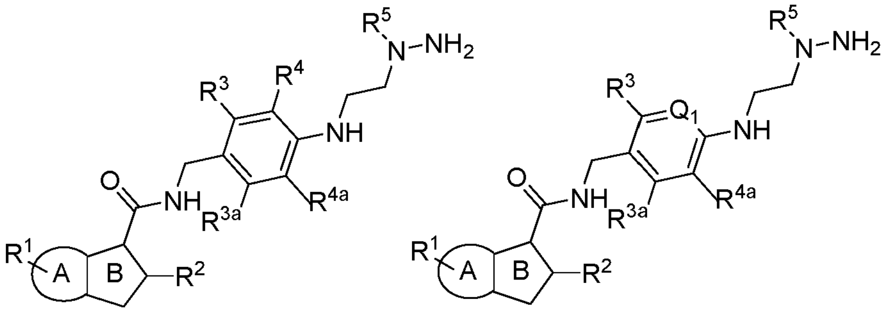

(XX) (XXA)

wherein the integers are as defined in Claim 1 (and $R^5$ preferably represents H), reaction with a compound of formula (XIX) as defined above; and/or

(v) for the preparation of a compound of formula (I) or (Ia) in which $R^5$ represents - $C(=O)-R^{9b}$, $-S(O)_2-R^{10}$ or $Het^1$, reaction of a corresponding compound of formula (I) in which $R^5$ represents H, with a compound of formula (XXI),

$LG^1-Z$ (XXI)

wherein Z represents $-C(=O)-R^{9b}$, $-S(O)_2-R^{10}$ or $Het^1$, and $LG^1$ represents a suitable leaving group, and wherein

the integers are as defined in Claim 1 and in the case of $Het^1$, the $LG^1$ is attached to an appropriate C atom of that heteroaromatic ring.

## Patentansprüche

**1.** Verbindung der Formel (Ia)

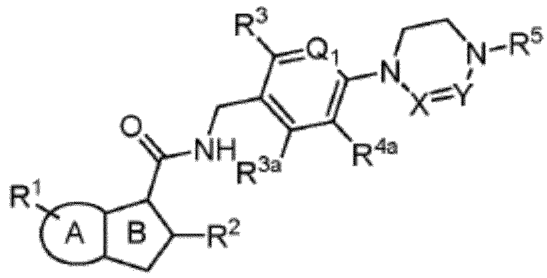

(Ia)

wobei

$Q_1$ für N oder $C(R^4)$ steht;
A ein 5- oder 6-gliedriger Ring ist, der aromatisch oder nichtaromatisch sein kann und optional 1 oder 2 Heteroatome enthält, die aus Stickstoff und Schwefel ausgewählt sind;
B ein 5-gliedriger aromatischer Ring ist, der 1 oder 2 Stickstoffheteroatome enthält;
$R^1$ für einen oder mehrere optionale Substituenten steht, die unabhängig voneinander ausgewählt sind aus Halogen, $-R^{6a}$, $-O-R^{6b}$, $-C(=O)-R^{6C}$,
$-C(=O)-N(R^7)(R^8)$, -CN und $-N(R^{7a})R^{7b}$; oder beliebige zwei $R^1$-Gruppen zusammengenommen werden können (wenn sie an benachbarte Atome des A-Rings gebunden sind), um einen 5- oder 6-gliedrigen Ring zu bilden, der optional ein oder zwei Heteroatome enthält und optional durch einen oder zwei $C_{1-3}$-Alkylsubstituenten substituiert ist;
$R^2$ $-C_{1-4}$-Alkyl ist, das optional durch einen oder mehrere Substituenten substituiert ist, die ausgewählt sind aus Halogen und $-OC_{1-3}$-Alkyl;
beliebige zwei von $R^3$, $R^{3a}$, $R^4$ und $R^{4a}$ für H stehen und die anderen beiden unabhängig voneinander für einen Substituenten stehen, der ausgewählt ist aus H, F, $-C_{1-3}$-Alkyl und $-O-C_{1-3}$-Alkyl;
$R^5$ H, $-R^{9a}$, $-C(=O)-R^{9b}$, $-SO_2-R^{10}$ oder $Het^1$ ist;
eines von X und Y für $-CR^{11a}$ steht und das andere für N oder $-CR^{11b}$ steht;
$R^{6a}$ und $R^{6b}$ unabhängig voneinander für Wasserstoff oder $-C_{1-4}$-Alkyl stehen, optional substituiert durch einen oder mehrere Substituenten, die ausgewählt sind aus Halogen, $-O-CH_3$ und Phenyl;
$R^{6C}$ $-C_{1-3}$-Alkyl ist;
$R^7$ und $R^8$ unabhängig voneinander ausgewählt sind aus H und $-C_{1-3}$-Alkyl-,
$R^{7a}$ und $R^{7b}$ unabhängig voneinander für H, $C_{1-6}$-Alkyl stehen oder $R^{7a}$ und $R^{7b}$ miteinander verbunden sind, um einen 3- bis 6-gliedrigen Ring zu bilden;
$R^{9a}$ für $-C_{1-4}$-Alkyl steht, das optional durch einen oder mehrere Substituenten substituiert ist, die ausgewählt sind aus Halogen, $-OC_{1-3}$-Alkyl und $Het^2$;
$R^{9b}$ Wasserstoff oder $-C_{1-3}$-Alkyl ist (das optional durch ein oder mehrere Fluoratome substituiert ist);
$R^{10}$ $-C_{1-4}$-Alkyl ist, das optional durch einen oder mehrere Substituenten substituiert ist, die ausgewählt sind aus Halogen und $-O-CH_3$;
$R^{11a}$ und $R^{11b}$ unabhängig voneinander für H, $C_{1-4}$-Alkyl (selbst optional substituiert durch einen oder mehrere Substituenten, die ausgewählt sind aus Fluor, -CN, $-R^{12a}$, $-OR^{12b}$, $-N(R^{12c})R^{12d}$ und/oder $-C(O)N(R^{12e})R^{12f}$) oder $-O-C_{1-4}$-Alkyl (selbst optional substituiert durch einen oder mehrere Substituenten, die ausgewählt sind aus Fluor, $-R^{12g}$, $-OR^{12h}$ und/oder $-N(R^{12i})R^{12j}$) stehen;
$R^{12a}$, $R^{12b}$, $R^{12C}$, $R^{12d}$, $R^{12e}$, $R^{12f}$, $R^{12g}$, $R^{12h}$, $R^{12i}$ und $R^{12j}$ unabhängig voneinander für Wasserstoff oder $C_{1-3}$-Alkyl (optional substituiert durch ein oder mehr Fluoratome) stehen;
$Het^1$ und $Het^2$ unabhängig voneinander für einen 5- oder 6-gliedrigen aromatischen Ring stehen, der ein oder zwei Heteroatome enthält, die vorzugsweise ausgewählt sind aus Stickstoff und Schwefel, optional substituiert durch einen oder mehrere Substituenten, die ausgewählt sind aus Halogen und $C_{1-3}$-Alkyl (selbst optional durch ein oder mehrere Fluoratome substituiert),

oder ein pharmazeutisch verträgliches Salz davon.

**2.** Verbindung nach Anspruch 1 der Formel (I)

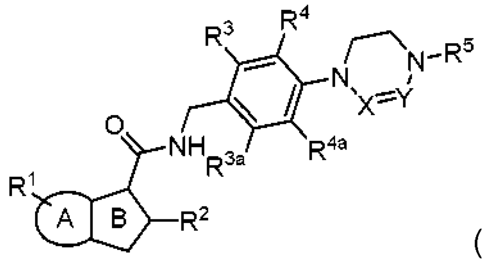

(I)

wobei

A ein 5- oder 6-gliedriger Ring ist, der aromatisch oder nichtaromatisch sein kann und optional 1 oder 2 Heteroatome enthält, die aus Stickstoff und Schwefel ausgewählt sind;
B ein 5-gliedriger aromatischer Ring ist, der 1 oder 2 Stickstoffheteroatome enthält;
$R^1$ für einen oder mehrere optionale Substituenten steht, die unabhängig voneinander ausgewählt sind aus Halogen, $-R^{6a}$, $-O-R^{6b}$,
$-C(=O)-R^{6C}$, $-C(=O)-N(R^7)(R^8)$, -CN und $-N(R^{7a})R^{7b}$;
$R^2$ $-C_{1-4}$-Alkyl ist, das optional durch einen oder mehrere Substituenten substituiert ist, die ausgewählt sind aus Halogen und $-OC_{1-3}$-Alkyl;
beliebige zwei von $R^3$, $R^{3a}$, $R^4$ und $R^{4a}$ für H stehen und die anderen beiden unabhängig voneinander für einen Substituenten stehen, der ausgewählt ist aus H, F, $-C_{1-3}$-Alkyl und $-O-C_{1-3}$-Alkyl;
$R^5$ H, $-R^{9a}$, $-C(=O)-R^{9b}$, $-SO_2-R^{10}$ oder $Het^1$ ist;
eines von X und Y für $-CR^{11a}$ steht und das andere für N oder $-CR^{11b}$ steht;
$R^{6a}$ und $R^{6b}$ unabhängig voneinander für $-C_{1-4}$-Alkyl stehen, optional substituiert durch einen oder mehrere Substituenten, die ausgewählt sind aus Halogen und $-O-CH_3$;
$R^{6C}$ $-C_{1-3}$-Alkyl ist;
$R^7$ und $R^8$ unabhängig voneinander ausgewählt sind aus H und $-C_{1-3}$-Alkyl-,
$R^{7a}$ und $R^{7b}$ unabhängig voneinander für H, $C_{1-6}$-Alkyl stehen oder $R^{7a}$ und $R^{7b}$ miteinander verbunden sind, um einen 3- bis 6-gliedrigen Ring zu bilden;
$R^{9a}$ für $-C_{1-4}$-Alkyl steht, das optional durch einen oder mehrere Substituenten substituiert ist, die ausgewählt sind aus Halogen, $-OC_{1-3}$-Alkyl und $Het^2$;
$R^{9b}$ Wasserstoff oder $-C_{1-3}$-Alkyl ist (das optional durch ein oder mehrere Fluoratome substituiert ist);
$R^{10}$ $-C_{1-4}$-Alkyl ist, das optional durch einen oder mehrere Substituenten substituiert ist, die ausgewählt sind aus Halogen und $-O-CH_3$;
$R^{11a}$ und $R^{11b}$ unabhängig voneinander für H, $C_{1-4}$-Alkyl (selbst optional substituiert durch einen oder mehrere Substituenten, die ausgewählt sind aus Fluor, -CN, $-R^{12a}$, $-OR^{12b}$, $-N(R^{12c})R^{12d}$ und/oder $-C(O)N(R^{12e})R^{12f}$) oder $-O-C_{1-4}$-Alkyl (selbst optional substituiert durch einen oder mehrere Substituenten, die ausgewählt sind aus Fluor, $-R^{12g}$,
$-OR^{12h}$ und/oder $-N(R^{12i})R^{12j}$) stehen;
$R^{12a}$ $R^{12b}$, $R^{12C}$, $R^{12d}$, $R^{12e}$, $R^{12f}$, $R^{12g,}$ $R^{12h}$, $R^{12i}$ und $R^{12j}$ unabhängig voneinander für Wasserstoff oder $C_{1-3}$-Alkyl (optional substituiert durch ein oder mehr Fluoratome) stehen;
$Het^1$ und $Het^2$ unabhängig voneinander für einen 5- oder 6-gliedrigen aromatischen Ring stehen, der ein oder zwei Heteroatome enthält, die vorzugsweise ausgewählt sind aus Stickstoff und Schwefel, optional substituiert durch einen oder mehrere Substituenten, die ausgewählt sind aus Halogen und $C_{1-3}$-Alkyl (selbst optional durch ein oder mehrere Fluoratome substituiert),
oder ein pharmazeutisch verträgliches Salz davon.

**3.** Verbindung nach Anspruch 1 oder 2, wobei:

an Ring A kein, ein oder zwei $R^1$-Substituenten vorhanden sein können;
$R^1$ (wenn vorhanden) für einen oder zwei Substituenten steht, die unabhängig voneinander ausgewählt sind aus F, Cl, $-R^{6a}$, $-O-R^{6b}$, $-C(=O)-R^{6C}$, $-C(=O)-N(R^7)(R^8)$, -CN und $-N(R^{7a})R^{7b}$;
$R^{6a}$ für $C_{1-3}$-Alkyl steht, das optional substituiert ist, ausgewählt aus $-O-C_{1-2}$-Alkyl;

$R^{6b}$ und $R^{6c}$ für $C_{1-3}$-Alkyl stehen, das vorzugsweise unsubstituiert ist;
$R^7$ und $R^8$ unabhängig voneinander für Wasserstoff oder $C_{1-3}$-Alkyl stehen, das vorzugsweise unsubstituiert ist;
$R^{7a}$ und $R^{7b}$ miteinander verknüpft sind, um einen 4- bis 6-gliedrigen Ring zu bilden.

**4.** Verbindung nach einem der vorstehenden Ansprüche, wobei:
Ring A wie folgt wiedergegeben wird:

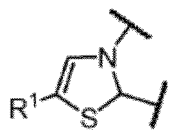
(II)

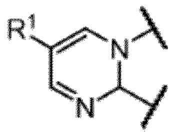
(III)

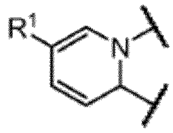
(IV)

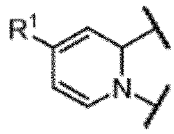
(V)

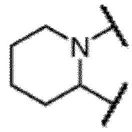
(VI)

**5.** Verbindung nach einem der vorstehenden Ansprüche, wobei:
Ring B wie folgt wiedergegeben wird:

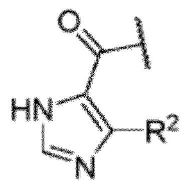
(VII)

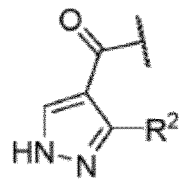
(VIII)

**6.** Verbindung nach einem der vorstehenden Ansprüche, wobei:
das kombinierte Ringsystem, d. h. Ring A und Ring B, wie folgt wiedergegeben werden kann:

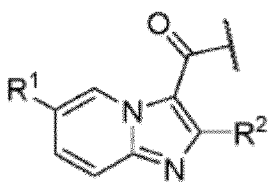
(IX)

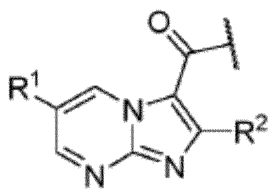
(X)

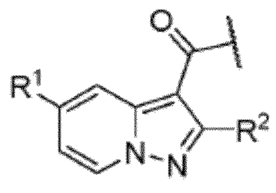
(XI)

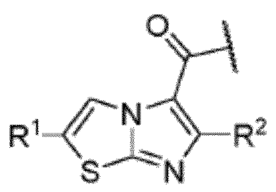
(XII)

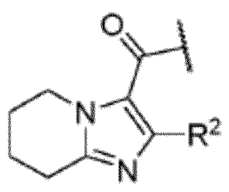
(XIII)

**7.** Verbindung nach einem der vorstehenden Ansprüche, wobei:

$R^2$ lineares -$C_{1-4}$-Alkyl ist, das optional durch einen oder mehrere Substituenten, beispielsweise ausgewählt aus -O-$C_{1-2}$-Alkyl, substituiert ist;
beliebige zwei von $R^3$, $R^{3a}$, $R^4$ und $R^{4a}$ für H stehen und die anderen beiden unabhängig voneinander für einen Substituenten stehen, der ausgewählt ist aus H, F, -$CH_3$ und -$OCH_3$;
$R^5$ H, -$R^{9a}$, -C(=O)-$R^{9b}$, -$SO_2$-$R^{10}$ oder $Het^1$ ist;
$R^{9a}$ für $C_{1-3}$-Alkyl steht, das unsubstituiert oder durch einen Substituenten substituiert ist;

$R^{9b}$ für H oder $C_{1-3}$-Alkyl steht, das optional durch ein oder mehrere Fluoratome substituiert ist (sodass eine $-CF_3$-Gruppe gebildet wird);
$R^{10}$ für $C_{1-4}$-Alkyl steht, das optional durch einen oder mehrere Substituenten substituiert ist, die ausgewählt sind aus Fluor und $-OC_{1-2}$-Alkyl, und somit $R^{10}$ für $-CF_3$, $-CH_3$, i-propyl, $-CH_2C(H)(CH_3)_2$ (i-butyl), $-CH_2CH_2-OCH_3$ stehen kann; und/oder
$Het^1$ und $Het^2$ unabhängig voneinander für einen 5- oder 6-gliedrigen Heteroarylring stehen, der ein oder zwei Heteroatome enthält, die ausgewählt sind aus Stickstoff und Schwefel, wobei der Ring unsubstituiert oder durch einen oder zwei Substituenten substituiert ist, die ausgewählt sind aus $C_{1-3}$-Alkyl (selbst optional substituiert durch ein oder mehrere Fluoratome, sodass eine $-CF_3$-Gruppe gebildet wird), und somit $Het^1$ und $Het^2$ unabhängig voneinander für eine Thiazolylgruppe stehen können, die optional durch einen $-CF_3$-Substituenten substituiert ist.

**8.** Verbindung nach einem der vorstehenden Ansprüche, wobei:

eines von X und Y für $-CR^{11a}$ steht und das andere für N oder $-CR^{11b}$ steht (und in einer Ausführungsform X für N steht und Y für $-CR^{11a}$ steht);
wenn $R^{11a}$ oder $R^{11b}$ für $C_{1-4}$-Alkyl steht, es dann unsubstituiert oder durch -CN, $-OR^{12b}$ und/oder $-N(R^{12c})R^{12d}$ substituiert sein kann;
$R^{12b}$ für H oder $C_{1-2}$-Alkyl steht;
$R^{12C}$ und $R^{12d}$ unabhängig voneinander für $C_{1-2}$-Alkyl stehen können;
somit, wenn $R^{11a}$ oder $R^{11b}$ für eine solche $C_{1-4}$-Alkylgruppe steht, es dann $-CH_3$, $-CH_2CH_3$, $-CH_2CH_2-OH$, $-CH_2CH_2-OCH_3$, $-C(H)(CH_3)_2$, $-CH_2-N(CH_3)_2$ oder $-CH_2-CN$) sein kann;
wenn $R^{11a}$ oder $R^{11b}$ für $-O-C_{1-4}$-Alkyl steht, es dann vorzugsweise unsubstituiert ist und für $-OC_{1-2}$-Alkyl stehen kann.

**9.** Verbindung nach Anspruch 1, die Folgendes ist:

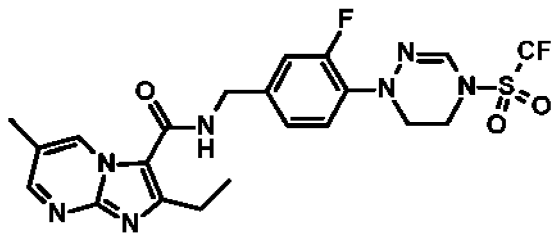

oder ein pharmazeutisch verträgliches Salz davon.

**10.** Verbindung nach einem der Ansprüche 1 bis 9, zur Verwendung als ein Pharmazeutikum.

**11.** Pharmazeutische Zusammensetzung, umfassend einen pharmazeutisch verträglichen Träger und, als Wirkstoff, eine therapeutisch wirksame Menge einer Verbindung nach einem der Ansprüche 1 bis 9.

**12.** Verbindung nach einem der Ansprüche 1 bis 9 zur Verwendung bei der Behandlung einer mykobakteriellen Infektion.

**13.** Verwendung einer Verbindung nach einem der Ansprüche 1 bis 9 für die Herstellung eines Medikaments für die Behandlung einer mykobakteriellen Infektion.

**14.** Verbindung zur Verwendung nach Anspruch 12 oder Verwendung nach Anspruch 13, wobei die mykobakterielle Infektion Tuberkulose ist.

**15.** Kombination aus (a) einer Verbindung nach einem der Ansprüche 1 bis 9 und (b) einem oder mehreren anderen antimykobakteriellen oder Anti-Tuberkulose-Mitteln.

**16.** Produkt, das (a) eine Verbindung nach einem der Ansprüche 1 bis 9 und (b) ein oder mehrere andere antimykobakterielle oder Anti-Tuberkulose-Mittel enthält, als eine kombinierte Zubereitung für die gleichzeitige, separate oder sequentielle Verwendung bei der Behandlung einer bakteriellen Infektion.

**17.** Verfahren für die Herstellung einer Verbindung der Formel (I) nach Anspruch 2 oder einer Verbindung der Formel (Ia) nach Anspruch 1, wobei das Verfahren umfasst:

(i) Umsetzen einer Verbindung der Formel (XIV),

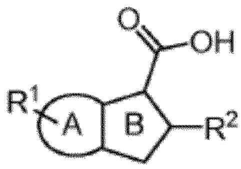

(XIV)

bei der die Ganzzahlen in Anspruch 1 definiert sind, mit einer Verbindung der Formel (XV) beziehungsweise (XVA),

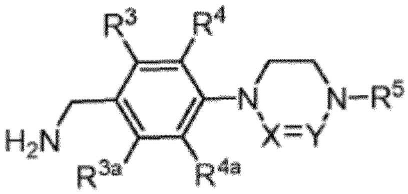

(XV)

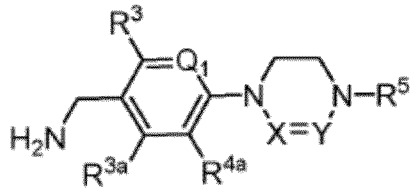

(XVA)

bei der die Ganzzahlen wie in Anspruch 1 definiert sind;
(ii) Kuppeln einer Verbindung der Formel (XVII) beziehungsweise (XVIIA),

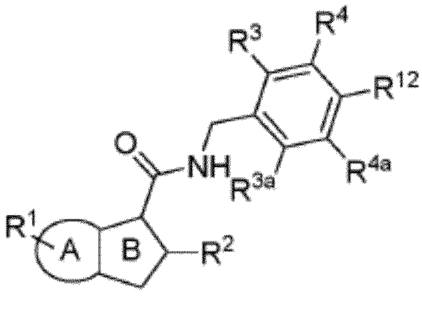

(XVII)

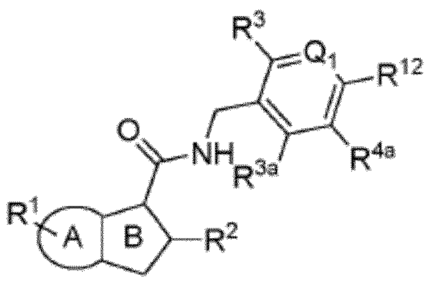

(XVIIA)

bei der die Ganzzahlen wie in Anspruch 1 definiert sind und $R^{12}$ für eine geeignete Gruppe steht, mit einer Verbindung der Formel (XVI),

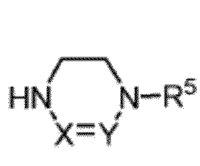

(XVI)

bei der $R^5$ wie in Anspruch 1 definiert ist;
(iii) für Verbindungen der Formel (I) oder (Ia), in denen X für N steht (und $R^5$ vorzugsweise für H steht), Umsetzen einer Verbindung der Formel (XVIII) beziehungsweise (XVIIIA),

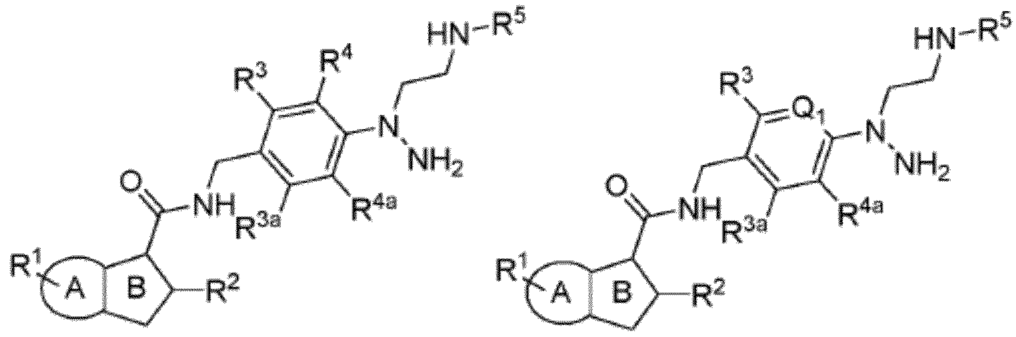

(XVIII) (XVIIIA)

bei der die Ganzzahlen wie in Anspruch 1 definiert sind (und $R^5$ vorzugsweise für H steht), Umsetzen mit einer Verbindung der Formel (XIX)

$R^{11x}C(OCH_3)_3$ (XIX)

oder dergleichen, bei der $R^{11x}$ für $R^{11a}$ oder $R^{11b}$ steht (je nachdem; und wie in Anspruch 1 definiert);

(iv) für Verbindungen der Formel (I) oder (Ia), in der X für N steht (und vorzugsweise $R^5$ für H steht), Umsetzen einer Verbindung der Formel (XX) beziehungsweise (XXA),

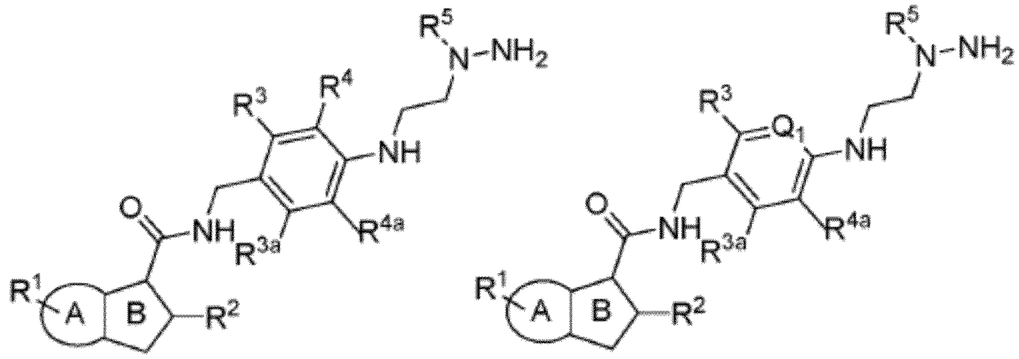

(XX) (XXA)

bei der die Ganzzahlen wie in Anspruch 1 definiert sind (und $R^5$ vorzugsweise für H steht), Umsetzen mit einer Verbindung der Formel (XIX) wie oben definiert; und/oder

(v) für die Herstellung einer Verbindung der Formel (I) oder (Ia), bei der $R^5$ für $-C(=O)-R^{9b}$, $-S(O)_2-R^{10}$ oder $Het^1$ steht, Umsetzen einer entsprechenden Verbindung der Formel (I), bei der $R^5$ für H steht, mit einer Verbindung der Formel (XXI),

$LG^1-Z$ (XXI)

bei der Z für $-C(=O)-R^{9b}$, $-S(O)_2-R^{10}$ oder $Het^1$ steht und $LG^1$ für eine geeignete Abgangsgruppe steht, und bei der die Ganzzahlen wie in Anspruch 1 definiert sind und im Falle von $Het^1$ das $LG^1$ an ein geeignetes C-Atom dieses heteroaromatischen Rings gebunden ist.

## Revendications

**1.** Composé de formule (Ia)

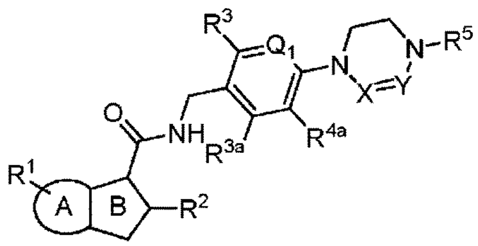

(Ia)

dans lequel

$Q_1$ représente N ou $C(R^4)$ ;
A est un cycle à 5 ou 6 chaînons, qui peut être aromatique ou non aromatique, et contenant facultativement 1 ou 2 hétéroatomes choisis parmi azote et soufre ;
B est un cycle aromatique à 5 chaînons contenant 1 ou 2 hétéroatomes d'azote ;
$R^1$ représente un ou plusieurs substituants facultatifs choisis indépendamment parmi halo, $-R^{6a}$, $-O-R^{6b}$, $-C(=O)-R^{6c}$,
$-C(=O)-N(R^7)(R^8)$, -CN et $-N(R^{7a})R^{7b}$ ; ou deux groupes $R^1$ quelconques peuvent être pris ensemble (lorsqu'ils sont liés à des atomes adjacents du cycle A) pour former un cycle à 5 ou 6 chaînons contenant facultativement un ou deux hétéroatomes, et lequel cycle est facultativement substitué par un ou deux substituants $C_{1-3}$ alkyle ;
$R^2$ est $-C_{1-4}$ alkyle facultativement substitué par un ou plusieurs substituants choisis parmi halo et $-OC_{1-3}$ alkyle ;
deux quelconques de $R^3$, $R^{3a}$, $R^4$ et $R^{4a}$ représentent H, et les deux autres représentent indépendamment un substituant choisi parmi H, F, $-C_{1-3}$ alkyle et $-O-C_{1-3}$ alkyle ;
$R^5$ est H, $-R^{9a}$, $-C(=O)-R^{9b}$, $-SO_2-R^{10}$ ou $Het^1$ ;
l'un ou l'autre de X et Y représente $-CR^{11a}$ et l'autre représente N ou $-CR^{11b}$ ;
$R^{6a}$ et $R^{6b}$ représentent indépendamment hydrogène ou $-C_{1-4}$ alkyle facultativement substitué par un ou plusieurs substituants choisis parmi halo, $-O-CH_3$ et phényle ;
$R^{6c}$ est $-C_{1-3}$ alkyle ;
$R^7$ et $R^8$ sont choisis indépendamment parmi H et $-C_{1-3}$ alkyle ;
$R^{7a}$ et $R^{7b}$ représentent indépendamment H, $C_{1-6}$ alkyle ou $R^{7a}$ et $R^{7b}$ sont liés ensemble pour former un cycle à 3 à 6 chaînons ;
$R^{9a}$ représente $-C_{1-4}$ alkyle, facultativement substitué par un ou plusieurs substituants choisis parmi halo, $-OC_{1-3}$ alkyle et $Het^2$ ;
$R^{9b}$ est hydrogène ou $-C_{1-3}$ alkyle (facultativement substitué par un ou plusieurs atomes fluoro) ;
$R^{10}$ est $-C_{1-4}$ alkyle facultativement substitué par un ou plusieurs substituants choisis parmi halo et $-O-CH_3$ ;
$R^{11a}$ et $R^{11b}$ représentent indépendamment H, $C_{1-4}$ alkyle (lui-même facultativement substitué par un ou plusieurs substituants choisis parmi fluoro, -CN, $-R^{12a}$, $-OR^{12b}$,
$-N(R^{12c})R^{12d}$ et/ou $-C(O)N(R^{12e})R^{12f}$) ou $-O-C_{1-4}$ alkyle (lui-même facultativement substitué par un ou plusieurs substituants choisis parmi fluoro, $-R^{12g}$, $-OR^{12h}$ et/ou $-N(R^{12i})R^{12j}$) ;
$R^{12a}$, $R^{12b}$, $R^{12c}$, $R^{12d}$, $R^{12e}$, $R^{12f}$, $R^{12g}$, $R^{12h}$, $R^{12i}$ et $R^{12j}$ représentent indépendamment hydrogène ou $C_{1-3}$ alkyle (facultativement substitué par un ou plusieurs atomes fluoro) ;
$Het^1$ et $Het^2$ représentent indépendamment un cycle aromatique à 5 ou 6 chaînons contenant un ou deux hétéroatomes, de préférence choisis parmi azote et soufre, facultativement substitués par un ou plusieurs substituants choisis parmi halo et $C_{1-3}$ alkyle (lui-même facultativement substitué par un ou plusieurs atomes fluoro),
ou un sel pharmaceutiquement acceptable de celui-ci.

**2.** Composé selon la revendication 1 de formule (I)

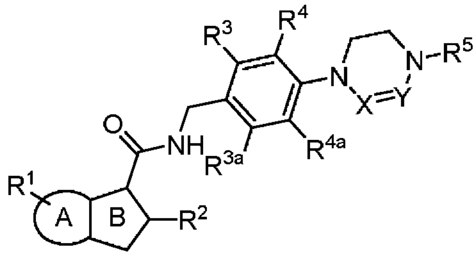

(I)

dans lequel

A est un cycle à 5 ou 6 chaînons, qui peut être aromatique ou non aromatique, et contenant facultativement 1 ou 2 hétéroatomes choisis parmi azote et soufre ;
B est un cycle aromatique à 5 chaînons contenant 1 ou 2 hétéroatomes d'azote ;
$R^1$ représente un ou plusieurs substituants facultatifs choisis indépendamment parmi halo, $-R^{6a}$, $-O-R^{6b}$, $-C(=O)-R^{6c}$, $-C(=O)-N(R^7)(R^8)$, -CN et $-N(R^{7a})R^{7b}$ ;
$R^2$ est $-C_{1-4}$ alkyle facultativement substitué par un ou plusieurs substituants choisis parmi halo et $-OC_{1-3}$ alkyle ;
deux quelconques de $R^3$, $R^{3a}$, $R^4$ et $R^{4a}$ représentent H, et les deux autres représentent indépendamment un substituant choisi parmi H, F, $-C_{1-3}$ alkyle et $-O-C_{1-3}$ alkyle ;
$R^5$ est H, $-R^{9a}$, $-C(=O)-R^{9b}$, $-SO_2-R^{10}$ ou $Het^1$ ;
l'un ou l'autre de X et Y représente $-CR^{11a}$ et l'autre représente N ou $-CR^{11b}$ ;
$R^{6a}$ et $R^{6b}$ représentent indépendamment $-C_{1-4}$ alkyle facultativement substitué par un ou plusieurs substituants choisis parmi halo et $-O-CH_3$ ;
$R^{6c}$ est $-C_{1-3}$ alkyle ;
$R^7$ et $R^8$ sont choisis indépendamment parmi H et $-C_{1-3}$ alkyle ;
$R^{7a}$ et $R^{7b}$ représentent indépendamment H, $C_{1-6}$ alkyle ou $R^{7a}$ et $R^{7b}$ sont liés ensemble pour former un cycle à 3 à 6 chaînons ;
$R^{9a}$ représente $-C_{1-4}$ alkyle, facultativement substitué par un ou plusieurs substituants choisis parmi halo, $-OC_{1-3}$ alkyle et $Het^2$ ;
$R^{9b}$ est hydrogène ou $-C_{1-3}$ alkyle (facultativement substitué par un ou plusieurs atomes fluoro) ;
$R^{10}$ est $-C_{1-4}$ alkyle facultativement substitué par un ou plusieurs substituants choisis parmi halo et $-O-CH_3$ ;
$R^{11a}$ et $R^{11b}$ représentent indépendamment H, $C_{1-4}$ alkyle (lui-même facultativement substitué par un ou plusieurs substituants choisis parmi fluoro, -CN, $-R^{12a}$, $-OR^{12b}$,
$-N(R^{12c})R^{12d}$ et/ou $-C(O)N(R^{12e})R^{12f}$) ou $-O-C_{1-4}$ alkyle (lui-même facultativement substitué par un ou plusieurs substituants choisis parmi fluoro, $-R^{12g,}$
$-OR^{12\,h}$ et/ou $-N(R^{12i})R^{12j}$) ;
$R^{12a}$, $R^{12b}$, $R^{12c}$, $R^{12d}$, $R^{12e}$, $R^{12f}$, $R^{12g,}$ $R^{12h}$, $R^{12i}$ et $R^{12j}$ représentent indépendamment hydrogène ou $C_{1-3}$ alkyle (facultativement substitué par un ou plusieurs atomes fluoro) ;
$Het^1$ et $Het^2$ représentent indépendamment un cycle aromatique à 5 ou 6 chaînons contenant un ou deux hétéroatomes, de préférence choisis parmi azote et soufre, facultativement substitués par un ou plusieurs substituants choisis parmi halo et $C_{1-3}$ alkyle (lui-même facultativement substitué par un ou plusieurs atomes fluoro),
ou un sel pharmaceutiquement acceptable de celui-ci.

**3.** Composé selon la revendication 1 ou la revendication 2, dans lequel :

il peut n'y avoir aucun, ou y avoir un ou deux substituants $R^1$ présents sur le cycle A ;
$R^1$ (lorsqu'il est présent) représente un ou deux substituants choisis indépendamment parmi F, Cl, $-R^{6a}$, $-O-R^{6b}$, $-C(=O)-R^{6c}$, $-(=O)-N(R^7)(R^8)$, -CN et $-N(R^{7a})R^{7b}$ ;
$R^{6a}$ représente $C_{1-3}$ alkyle facultativement substitué choisi parmi $-O-C_{1-2}$ alkyle ;
$R^{6b}$ et $R^{6c}$ représentent $C_{1-3}$ alkyle, qui est de préférence non substitué ;
$R^7$ et $R^8$ représentent indépendamment hydrogène ou $C_{1-3}$ alkyle, qui est de préférence non substitué ;
$R^{7a}$ et $R^{7b}$ sont liés ensemble pour former un cycle à 4 à 6 chaînons.

**4.** Composé selon l'une quelconque des revendications précédentes, dans lequel :
le cycle A est représenté comme suit :

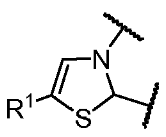
(II)

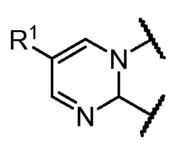
(III)

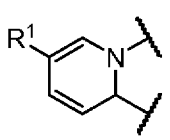
(IV)

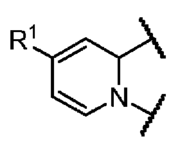
(V)

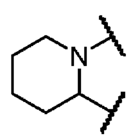
(VI)

.

**5.** Composé selon l'une quelconque des revendications précédentes, dans lequel :
le cycle B est représenté comme suit :

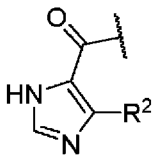 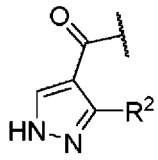

(VII) (VIII)

6. Composé selon l'une quelconque des revendications précédentes, dans lequel : le système cyclique combiné, c'est-à-dire le cycle A et le cycle B, peut être représenté comme suit :

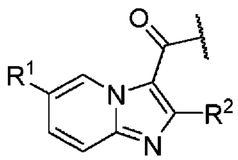

(IX)

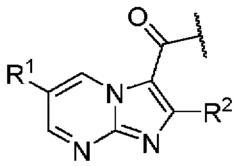

(X)

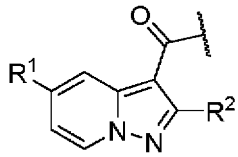

(XI)

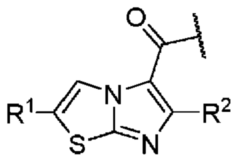

(XII)

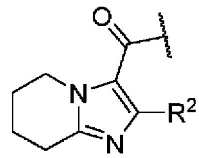

(XIII)

7. Composé selon l'une quelconque des revendications précédentes, dans lequel :

$R^2$ est $-C_{1-4}$ alkyle linéaire facultativement substitué par un ou plusieurs substituants, par exemple choisis parmi $-O-C_{1-2}$ alkyle ;
deux quelconques de $R^3$, $R^{3a}$, $R^4$ et $R^{4a}$ représentent H, et les autres deux représentent indépendamment un substituant choisi parmi H, F, $-CH_3$ et $-OCH_3$ ;
$R^5$ est H, $-R^{9a}$, $-C(=O)-R^{9b}$, $-SO_2-R^{10}$ ou $Het^1$ ;
$R^{9a}$ représente $C_{1-3}$ alkyle non substitué ou substitué par un substituant ;
$R^{9b}$ représente H ou $C_{1-3}$ alkyle facultativement substitué par un ou plusieurs atomes fluoro (formant ainsi un groupe $-CF_3$) ;
$R^{10}$ représente $C_{1-4}$ alkyle facultativement substitué par un ou plusieurs substituants choisis parmi fluoro et $-OC_{1-2}$ alkyle, et donc $R^{10}$ peut représenter $-CF_3$, $-CH_3$, i-propyle, $-CH_2C(H)(CH_3)_2$ (i-butyle), $-CH_2CH_2OCH_3$ ; et/ou
$Het^1$ et $Het^2$ représentent indépendamment un cycle hétéroaryle à 5 ou 6 chaînons contenant un ou deux hétéroatomes choisis parmi azote et soufre, lequel cycle est non substitué ou substitué par un ou deux substituants choisis parmi $C_{1-3}$ alkyle (lui-même facultativement substitué par un ou plusieurs atomes fluoro, formant ainsi un groupe $-CF_3$), et, donc, $Het^1$ et $Het^2$ peuvent représenter indépendamment un groupe thiazolyle facultativement substitué par un substituant $-CF_3$.

8. Composé selon l'une quelconque des revendications précédentes, dans lequel :

l'un ou l'autre de X et Y représente $-CR^{11a}$ et l'autre représente N ou $-CR^{11b}$ (et dans un mode de réalisation X représente N et Y représente $-CR^{11a}$) ;
lorsque $R^{11a}$ ou $R^{11b}$ représente $C_{1-4}$ alkyle, alors il peut être non substitué ou substitué par -CN, $-OR^{12b}$ et/ou $-N(R^{12c})R^{12d}$ ;
$R^{12b}$ représente H ou $C_{1-2}$ alkyle ;
$R^{12c}$ et $R^{12d}$ peuvent représenter indépendamment $C_{1-2}$ alkyle ;
donc, lorsque $R^{11a}$ ou $R^{11b}$ représente un tel groupe $C_{1-4}$ alkyle, alors il peut être $-CH_3$, $-CH_2CH_3$, $-CH_2CH_2-OH$, $-CH_2CH_2OCH_3$, $-C(H)(CH_3)_2$, $-CH_2-N(CH_3)_2$ ou $-CH_2-CN$) ;

lorsque $R^{11a}$ ou $R^{11b}$ représente -O-$C_{1-4}$ alkyle, alors il est de préférence non substitué et peut représenter -O$C_{1-2}$ alkyle.

**9.** Composé selon la revendication 1, qui est :

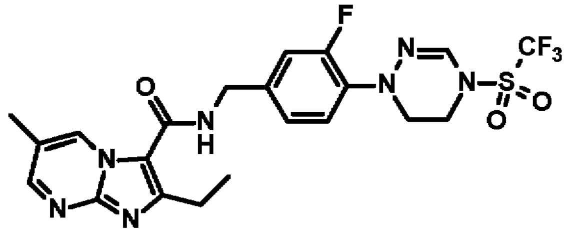

ou un sel pharmaceutiquement acceptable de celui-ci.

**10.** Composé selon l'une quelconque des revendications 1 à 9, pour une utilisation en tant que produit pharmaceutique.

**11.** Composition pharmaceutique comprenant un support pharmaceutiquement acceptable et, en tant que principe actif, une quantité thérapeutiquement efficace d'un composé selon l'une quelconque des revendications 1 à 9.

**12.** Composé selon l'une quelconque des revendications 1 à 9 pour une utilisation dans le traitement d'une infection mycobactérienne.

**13.** Utilisation d'un composé selon l'une quelconque des revendications 1 à 9 pour la fabrication d'un médicament pour le traitement de l'infection mycobactérienne.

**14.** Composé pour une utilisation selon la revendication 12 ou une utilisation selon la revendication 13, dans lequel l'infection mycobactérienne est la tuberculose.

**15.** Combinaison de (a) un composé selon l'une quelconque des revendications 1 à 9, et (b) un ou plusieurs autres agents antimycobactériens ou antituberculose.

**16.** Produit contenant (a) un composé selon l'une quelconque des revendications 1 à 9, et (b) un ou plusieurs autres agents antimycobactériens ou antituberculose, en tant que préparation combinée pour une utilisation simultanée, séparée ou séquentielle dans le traitement d'une infection bactérienne.

**17.** Procédé pour la préparation d'un composé de formule (I) selon la revendication 2 ou d'un composé de formule (Ia) selon la revendication 1, lequel procédé comprend :

(i) la réaction d'un composé de formule (XIV),

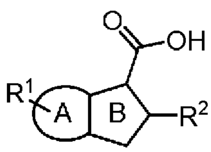

(XIV)

dans lequel les nombres entiers sont définis dans la revendication 1, avec un composé de formule (XV) ou (XVA), respectivement,

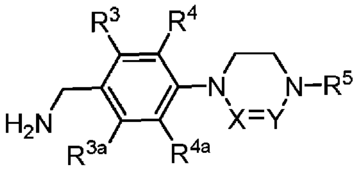

(XV)

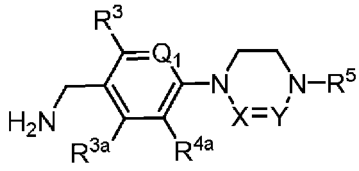

(XVA)

dans lequel les nombres entiers sont tels que définis dans la revendication 1 ;
(ii) le couplage d'un composé de formule (XVII) ou (XVIIA) respectivement,

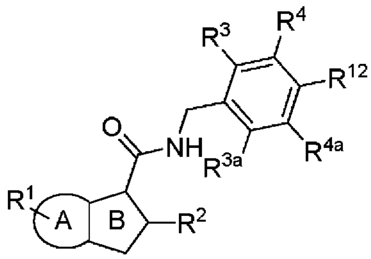

(XVII)

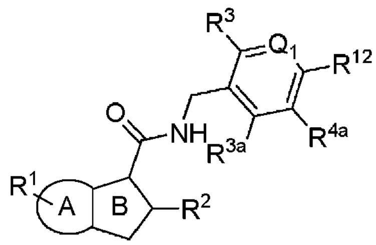

(XVIIA)

dans lequel les nombres entiers sont tels que définis dans la revendication 1, et $R^{12}$ représente un groupe approprié, avec un composé de formule (XVI),

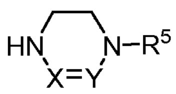

(XVI)

dans lequel $R^5$ est tel que défini dans la revendication 1 ;
(iii) pour des composés de formule (I) ou (Ia) dans lesquels X représente N (et $R^5$ représente de préférence H), la réaction d'un composé de formule (XVIII) ou (XVIIIA), respectivement

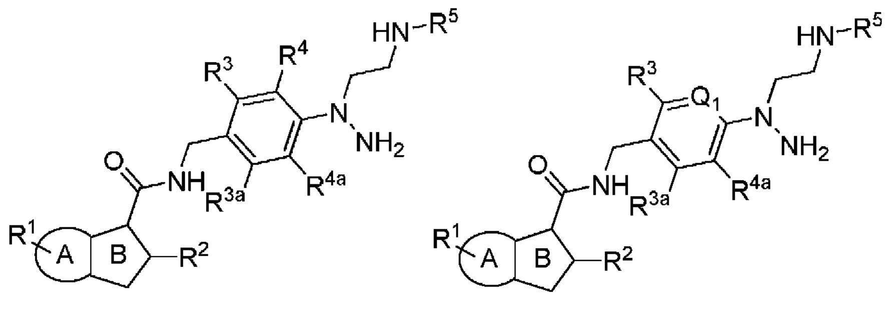

(XVIII) (XVIIIA)

dans lequel les nombres entiers sont tels que définis dans la revendication 1 (et $R^5$ représente de préférence H), la réaction avec un composé de formule (XIX)

$R^{11x}C(OCH_3)_3$ (XIX)

ou similaires, dans lequel $R^{11x}$ représente $R^{11a}$ ou $R^{11b}$ (selon le cas ; et tel que défini dans la revendication 1) ;
(iv) pour des composés de formule (I) ou (Ia) dans lesquels X représente N (et $R^5$ représente de préférence

H), la réaction d'un composé de formule (XX) ou (XXA), respectivement

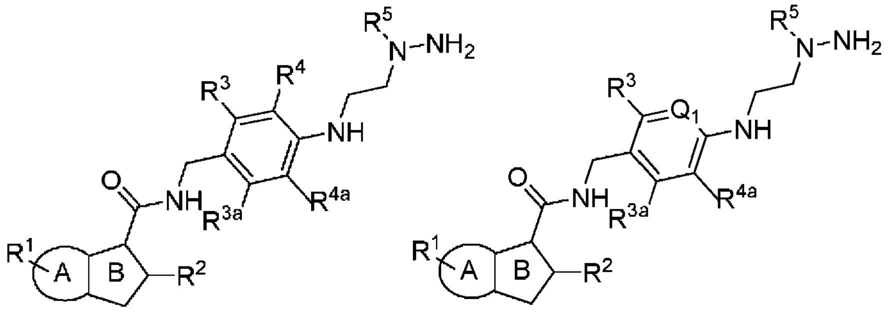

(XX) (XXA)

dans lequel les nombres entiers sont tels que définis dans la revendication 1 (et $R^5$ représente de préférence H), la réaction avec un composé de formule (XIX) tel que défini ci-dessus ; et/ou

(v) pour la préparation d'un composé de formule (I) ou (Ia) dans lequel $R^5$ représente -C(=O)-$R^{9b}$, -$S(O)_2$-$R^{10}$ ou $Het^1$, la réaction d'un composé correspondant de formule (I) dans lequel $R^5$ représente H, avec un composé de formule (XXI),

$LG^1$-Z (XXI)

dans lequel Z représente -C(=O)-$R^{9b}$, -$S(O)_2$-$R^{10}$ ou $Het^1$, et $LG^1$ représente un groupe partant approprié, et dans lequel les nombres entiers sont tels que définis dans la revendication 1 et dans le cas de $Het^1$, le $LG^1$ est lié à un atome C approprié de ce cycle hétéroaromatique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011113606 A
- WO 2014015167 A
- WO 2015014993 A
- WO 20144015167 A
- WO 2017001660 A
- WO 2017001661 A
- WO 2017216281 A
- WO 2017216283 A
- WO 2013033070 A
- WO 2013033167 A
- WO 2004011436 A

### Non-patent literature cited in the description

- **PETHE et al.** Discovery of Q203, a potent clinical candidate for the treatment of tuberculosis. *Journal article Nature Medicine,* 2013, vol. 19, 1157-1160
- *J. Medicinal Chemistry,* 2014, vol. 57 (12), 5293-5305
- **BUNDEGAARD, H.** Design of Prodrugs. Elesevier, 1985, I-92
- *CHEMICAL ABSTRACTS,* 1216142-18-5
- *CHEMICAL ABSTRACTS,* 1232431-05-8
- *CHEMICAL ABSTRACTS,* 4949-44-4
- *CHEMICAL ABSTRACTS,* 2318-25-4
- *CHEMICAL ABSTRACTS,* 2059140-68-8
- *CHEMICAL ABSTRACTS,* 1908481-13-9
- *CHEMICAL ABSTRACTS,* 1352395-28-8
- *CHEMICAL ABSTRACTS,* 867131-26-8
- *CHEMICAL ABSTRACTS,* 662117-63-7
- *CHEMICAL ABSTRACTS,* 13418-77-4
- *CHEMICAL ABSTRACTS,* 1536994-62-3
- *CHEMICAL ABSTRACTS,* 1000843-61-7
- *CHEMICAL ABSTRACTS,* 4214-73-7
- *CHEMICAL ABSTRACTS,* 10201-73-7
- *CHEMICAL ABSTRACTS,* 73221-19-9
- *CHEMICAL ABSTRACTS,* 2059954-47-9
- *CHEMICAL ABSTRACTS,* 36936-27-3
- *CHEMICAL ABSTRACTS,* 1393574-54-3
- *CHEMICAL ABSTRACTS,* 108990-72-3
- *CHEMICAL ABSTRACTS,* 874830-60-1
- *CHEMICAL ABSTRACTS,* 1368682-64-7
- *CHEMICAL ABSTRACTS,* 1131613-58-5
- *CHEMICAL ABSTRACTS,* 2060043-79-8
- *CHEMICAL ABSTRACTS,* 2089471-58-7
- *CHEMICAL ABSTRACTS,* 1517795-25-3
- *CHEMICAL ABSTRACTS,* 2089471-57-6
- *CHEMICAL ABSTRACTS,* 1369332-25-1
- *CHEMICAL ABSTRACTS,* 1216036-36-0
- *CHEMICAL ABSTRACTS,* 1007875-19-5
- *CHEMICAL ABSTRACTS,* 874830-67-8
- *CHEMICAL ABSTRACTS,* 3993-78-0
- *CHEMICAL ABSTRACTS,* 1683-85-8
- *CHEMICAL ABSTRACTS,* 1785357-12-1
- *CHEMICAL ABSTRACTS,* 2230730-23-9
- *CHEMICAL ABSTRACTS,* 732306-31-9
- *CHEMICAL ABSTRACTS,* 20712-16-7
- *CHEMICAL ABSTRACTS,* 1072-98-6
- *CHEMICAL ABSTRACTS,* 352-24-9
- *CHEMICAL ABSTRACTS,* 1033203-32-5
- *CHEMICAL ABSTRACTS,* 57963-11-8
- *CHEMICAL ABSTRACTS,* 1033203-31-4
- *CHEMICAL ABSTRACTS,* 1187449-01-9
- *CHEMICAL ABSTRACTS,* 403854-21-7
- *CHEMICAL ABSTRACTS,* 2393-23-9
- *CHEMICAL ABSTRACTS,* 40439-76-7
- *CHEMICAL ABSTRACTS,* 1781072-41-0
- *CHEMICAL ABSTRACTS,* 20781-20-8
- *CHEMICAL ABSTRACTS,* 2927-71-1
- *CHEMICAL ABSTRACTS,* 110-46-3
- *CHEMICAL ABSTRACTS,* 1020253-14-8
- *CHEMICAL ABSTRACTS,* 57260-73-8
- *CHEMICAL ABSTRACTS,* 73221-19-9],0.34